(19) Europäisches Patentamt
European Patent Office
Office européen des brevets

(11) **EP 2 402 464 A1**

(12) **EUROPEAN PATENT APPLICATION**

(43) Date of publication:
**04.01.2012 Bulletin 2012/01**

(51) Int Cl.:
**C12Q 1/68** (2006.01)

(21) Application number: **11182820.8**

(22) Date of filing: **06.11.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.11.2006 US 858965 P**

(62) Document number(s) of the earlier application(s) in accordance with Art. 76 EPC:
**07861767.7 / 2 092 082**

(71) Applicant: **Source Precision Medicine, Inc. d/b/a Source MDX.**
**Boulder, CO 80301 (US)**

(72) Inventors:
• **Bankaitis-Davis, Danute**
**Longmont, CO 80503 (US)**

• **Siconolfi, Lisa**
**Westminster, CO 80234 (US)**
• **Storm, Kathleen**
**Longmont, CO 80503 (US)**
• **Wassmann, Karl**
**Dover, MA 02030 (US)**

(74) Representative: **Finnie, Isobel Lara et al**
**Mintz, Levin, Cohn, Ferris, Glovsky and Popeo IP, LLP**
**Alder Castle, 10 Nobel Street**
**London EC2V 7JX (GB)**

Remarks:
This application was filed on 26-09-2011 as a divisional application to the application mentioned under INID code 62.

(54) **Gene expression profiling for identification, monitoring, and treatment of colorectal cancer**

(57)    A method is provided in various embodiments for determining a profile data set for a subject with colorectal cancer or conditions related to colorectal cancer based on a sample from the subject, wherein the sample provides a source of RNAs. The method includes using amplification for measuring the amount of RNA corresponding to at least 1 constituent from Tables 1-5. The profile data set comprises the measure of each constituent and amplification is performed under measurement conditions that are substantially repeatable.

Fig. 1

**EP 2 402 464 A1**

**Description**

**REFERENCE TO RELATED APPLICATIONS**

This application claims the benefit of U.S. Provisional Application No. 60/858965 filed November 13, 2006 the contents of which are incorporated by reference in its entirety.

**FIELD OF THE INVENTION**

The present invention relates generally to the identification of biological markers associated with the identification of colorectal cancer. More specifically, the present invention relates to the use of gene expression data in the identification, monitoring and treatment of colorectal cancer and in the characterization and evaluation of conditions induced by or related to colorectal cancer.

**BACKGROUND OF THE INVENTION**

Colorectal cancer is a type of cancer that develops in the gastrointestinal system (GI system), specifically in the colon, or the rectum. The GI system consists of the small intestine, the large intestine (also known as the colon), the rectum, and the anus. The colon is a muscular tube, about five feet long on average, and has four sections: the ascending colon which begins where the small bowel attaches to the colon and extends upward on the rights side of the abdomen; the transverse colon, which runs across the body from the right to left side in the upper abdomen; the descending colon, which continues downward on the left side; and the sigmoid colon, which joins the rectum, which in turn joins the anus. The wall of each of the sections of the colon and rectum has several layers of tissue. Colorectal cancer starts in the innermost layer of tissue of the colon or rectum and can grow through some or all of the other layers. The stage (*i.e.,* the extent of spread) of colorectal cancer depends on how deeply it invades into these layers.

Colorectal cancer develops slowly over a period of several years, usually beginning as a non-cancerous or pre-cancerous polyp which develops on the lining of the colon or rectum. Certain kinds of polyps, called adenomatous polyps (or adenomas), are highly likely to become cancerous. Other kinds of polyps, called hyperplastic polyps and inflammatory polyps, indicate an increased chance of developing adenomatous polyps and cancer, particularly if growing in the ascending colon. A pre-cancerous condition known as dysplasia is common in people suffering from diseases which clause chronic inflammation in the colon, such as ulcerative colitis or Chrohn's Disease.

Over 95% of colorectal cancers are adenocarcinomas, a cancer of the glandular cells that line the inside layer of the wall of the colon and rectum. Other types of colorectal tumors include carcinoid tumors, which develop from hormone producing cells of the colon; gastrointestinal stromal tumors, which develop in the interstitial cells of Cajal within the wall of the colon; and lymphomas of the digestive system.

Once cancer forms within a colorectal polyp, it eventually grows into the wall of the colon or rectum. Once cancer cells are in the wall, they can grow into blood vessels or lymph vessels, at which point the cancer metastizes.

Colorectal cancer is the third most common cancer diagnosed in men and women, and is the second leading cause of cancer-related deaths in the United States. Risk factors for colorectal cancer include age (increased chance after age 50); personal history of colorectal cancer, polyps, or chronic inflammatory bowel disease; ethnic background (Jews of Eastern European descent have higher rates of colorectal cancer); a diet mostly from animal sources (high in fat); physical inactivity; obesity; smoking (30-40% increased risk for colorectal cancer); and high alcohol intake. Additionally, individuals with a family history of colorectal cancer have an increased risk for developing the disease. About 30% of people who develop colorectal cancer have disease that is familial. About another 10% of people who develop colorectal cancer have an inherited genetic susceptibility to the disease; approximately 3-5% of colorectal cancers are associated with a syndrome called hereditary non-polyposis colorectal cancer (HNPCC), approximately 1% of colorectal cancers are associated with an inherited syndrome called familial adenomatous polyposis (FAP).

FAP is a disease where people develop hundreds of polyps in their colon and rectum, typically between the ages of 5 and 40 years. Cancer develops in one or more of these polyps as early as age 20. By age 40, almost all people with FAP will have developed cancer if preventative surgery is not done. HNPCC also develops at a relatively young age. However, individuals with HNPCC develop only a few polyps. Women with HNPCC have a high risk of developing endometrial cancer. Other cancers associated with HNPCC include cancer of the ovary, stomach, small intestine, pancreas, kidney, ureter, and bile duct. The lifetime risk of developing colorectal cancer for people with HNPCC is about 80%, compared to near 100% for those with FAP.

From the time the first abnormal cells in polyps start to grow, it takes about 10-15 years for them to develop into colorectal cancer. An individual can live asymptomatic for several years with precancerous polyps that develop into colorectal cancer without knowing it. Once symptoms do start presenting, they include changes in bowel habits (*e.g.*, constipation, diarrhea, narrowing of the stool), stomach cramping or bloating, bright red blood in stool, unexplained weight loss, constant fatigue, constant sensation of needing a bowel movement, naseau and vomiting, gaseousness, and anemia.

Treatment of colorectal cancer varies according to type, location, extent, and aggressiveness of the cancer, and can include any one or combination of the following procedures: surgery, radiation therapy, and chemotherapy, and targeted therapy (*e.g.*, monoclonal antibodies). Surgery is the main treatment for colorectal cancer. At early stages it may be possible to remove cancerous polyps through a colonoscope, by passing a wire loop through the colonoscope to cut the polyp from the wall of the colon with an electrical current. The most common operation for colon cancer is a segmental resection, in which the cancer a length of the normal colon on either side of the cancer, and nearby lymph nodes are removed, and the remaining sections of the colon are reattached.

Radiation therpy uses high energy rays to destroy cancer cells, and is used after colorectal surgery to destroy small deposits of cancer that may not be detected during surgery, or when the cancer has attached to an internal organ or lining of the abdomen. Radiation therpy is also used to treat local recurrences of rectal cancer. Several types of radiation therapy are available, including external-beam radiation therapy, endocavitry radiation therapy, and brachytherapy. Radiation therapy is also often used after surgery in combination with chemotherapy.

Chemotherapy can also be used to shrink primary tumors, relieve symptoms of advanced colorectal cancer, or as an adjuvant therapy. Fluorouracil (5-FU) is the drug most often used to treat colon cancer. In adjuvant therapy, it is often administered with leucovorin via an IV injection regimen to increase its effectiveness. Capecitabine (Xeloda™) is an orally administered chemotherapeutic that is converted to 5-FU once it reaches the tumor site. Other chemotherapeutics which have been found to increase the effectiveness 5-FU and leucovorin when given in combination include Irinotecan (Camptosar™), and Oxaliplatin.

Targeted therapies such as monoclonal antibodies are being used more frequently to specifically attack cancer cells with fewer side effects than radiation therapy or chemotherapy. Monoclonal antibodies that have been approved for the treatment of colon cancer include Cetuximab (Erbitux™), and Bevacizumab (Avastin™).

Since individuals with colon cancer can live for several years asymptomatic while the disease progresses, regular screenings are essential to detect colorectal cancer at an early stage, or to prevent abnormal polyps from developing into colorectal cancer. Diagnosis for colorectal cancer is typically done through a combination of a medical history, physical exam, blood tests for anemia or tumor markers (*e.g.*, carcinoembryonic antigen, or CA19-9); and one or more screening methods for polyps or abnormalities in the lining of the colorectal wall.

A number of different screening methods for colorectal cancer are available. However, most procedures are highly invasive and painful. Take home test kits such as the fecal occult blood test (FOBT), or fecal immunochemical test (FIT), use a chemical reaction to detect occult (hidden blood) in the feces due to ruptured blood vessels at the surface of colorectal polyps of adenomas or cancers, damaged by the passage of feces. However, since occult in the stool could be indicative of a variety of gastrointestinal disorders, a colonoscopy or sigmoidoscopy is necessary to verify that positive FOBT or FIT results are due to colorectal cancer.

A colonoscopy involves a colonoscope which is a longer version of a sigmoidoscope, connected to a camera or monitor, and is inserted through the rectum to enable a doctor to visualize the lining of the entire colon. Polyps detected by such screening methods can be removed through a colonoscope or biopsied to determine whether the polyp is cancerous, benign, or a result of inflammation.

Additional screening techniques include invasive imaging techniques such as a barium enema with air contrast, or virtual colonoscopy. A barium enema with air contrast involves pumping barium sulfate and air through the anus to partially fill and open up the colon, then x-ray to image the lining of the colon. Virtual colonoscopy uses only air pumped through the anus to distend the colon, then a helical or spiral CT scan to image the lining of the colon. Ultrasound, CT scan, PET scan, and MRI can also be used to image the lining of the colorectal wall. However, if abnormalities such as polyps are found by any such imaging technique, a procedure such as a colonoscopy or CT guided needle biopsy is still necessary to remove or biopsy the polyp. It is nearly impossible to detect or verify a diagnosis of colorectal cancer in a non-invasive manner, and without causing the patient pain and discomfort. Thus a need exists for better ways to diagnose and monitor the progression and treatment of colorectal cancer.

Additionally, information on any condition of a particular patient and a patient's response to types and dosages of therapeutic or nutritional agents has become an important issue in clinical medicine today not only from the aspect of efficiency of medical practice for the health care industry but for improved outcomes and benefits for the patients. Thus, there is the need for tests which can aid in the diagnosis and monitor the progression and treatment of colorectal cancer.

## SUMMARY OF THE INVENTION

The invention is in based in part upon the identification of gene expression profiles (Precision Profiles™) associated with colon cancer. These genes are referred to herein as colon cancer associated genes or colon cancer associated constituents. More specifically, the invention is based upon the surprising discovery that detection of as few as one colon cancer associated gene in a subject derived sample is capable of identifying individuals with or without colon cancer with at least 75% accuracy. More particularly, the invention is based upon the surprising discovery that the methods provided by the invention are capable of detecting colon cancer by assaying blood samples.

In various aspects the invention provides methods of evaluating the presence or absence (*e.g.*, diagnosing or prognosing) of colon cancer, based on a sample from the subject, the sample providing a source of RNAs, and determining a quantitative measure of the amount of at least one constituent of any constituent (*e.g.*, colon cancer associated gene) of any of Tables 1, 2, 3, 4, and 5 and arriving at a measure of each constituent.

Also provided are methods of assessing or monitoring the response to therapy in a subject having colon cancer, based on a sample from the subject, the sample providing a source of RNAs, determining a quantitative measure of the amount of at least one constituent of any constituent of Tables 1, 2, 3, 4, 5 or 6 and arriving at a measure of each constituent. The therapy, for example, is immunotherapy. Preferably, one or more of the constituents listed in Table 6 is measured. For example, the response of a subject to immunotherapy is monitored by measuring the expression of TNFRSF10A, TMPRSS2, SPARC, ALOX5, PTPRC, PDGFA, PDGFB, BCL2, BAD, BAK1, BAG2, KIT, MUC1, ADAM17, CD19, CD4, CD40LG, CD86, CCR5, CTLA4, HSPA1A, IFNG, IL23A, PTGS2, TLR2, TGFB1, TNF, TNFRSF13B, TNFRSF10B, VEGF, MYC, AURKA, BAX, CDH1, CASP2, CD22, IGF1R, ITGA5, ITGAV, ITGB1,

ITGB3, IL6R, JAK1, JAK2, JAK3, MAP3K1, PDGFRA, COX2, PSCA, THBS1, THBS2, TYMS, TLR1, TLR3, TLR6, TLR7, TLR9, TNFSF10, TNFSF13B, TNFRSF17, TP53, ABL1, ABL2, ANT1, KRAS, BRAF, RAF1, ERBB4, ERBB2, ERBB3, AKT2, EGFR, IL12 or IL15. The subject has received an immunotherapeutic drug such as anti CD19 Mab, rituximab, epratuzumab, lumiliximab, visilizumab (Nuvion), HuMax-CD38, zanolimumab, anti CD40 Mab, anti-CD40L, Mab, galiximab anti-CTLA-4 MAb, ipilimumab, ticilimumab, anti-SDF-1 MAb, panitumumab, nimotuzumab, pertuzumab, trastuzumab, catumaxomab, ertumaxomab, MDX-070, anti ICOS, anti IFNAR, AMG-479, anti- IGF-1R Ab, R1507, IMC-A12, antiangiogenesis MAb, CNTO-95, natalizumab (Tysabri), SM3, IPB-01, hPAM-4, PAM4, Imuteran, huBrE-3 tiuxetan, BrevaRex MAb, PDGFR MAb, IMC-3G3, GC-1008, CNTO-148 (Golimumab), CS-1008, belimumab, anti-BAFF MAb, or bevacizumab. Alternatively, the subject has received a placebo.

In a further aspect the invention provides methods of monitoring the progression of colon cancer in a subject, based on a sample from the subject, the sample providing a source of RNAs, by determining a quantitative measure of the amount of at least one constituent of any constituent of Tables 1, 2, 3, 4, and 5 as a distinct RNA constituent in a sample obtained at a first period of time to produce a first subject data set and determining a quantitative measure of the amount of at least one constituent of any constituent of Tables 1, 2, 3, 4, and 5 as a distinct RNA constituent in a sample obtained at a second period of time to produce a second subject data set. Optionally, the constituents measured in the first sample are the same constituents measured in the second sample. The first subject data set and the second subject data set are compared allowing the progression of colon cancer in a subject to be determined. The second subject is taken *e.g.*, one day, one week, one month, two months, three months, 1 year, 2 years, or more after the first subject sample. Optionally the first subject sample is taken prior to the subject receiving treatment, *e.g.* chemotherapy, radiation therapy, or surgery and the second subject sample is taken after treatment. In various aspects the invention provides a method for determining a profile data set, *i.e.*, a colon cancer profile, for characterizing a subject with colon cancer or conditions related to colon cancer based on a sample from the subject, the sample providing a source of RNAs, by using amplification for measuring the amount of RNA in a panel of constituents including at least 1 constituent from any of Tables 1-5, and arriving at a measure of each constituent. The profile data set contains the measure of each constituent of the panel.

The methods of the invention further include comparing the quantitative measure of the constituent in the subject derived sample to a reference value or a baseline value, *e.g.* baseline data set. The reference value is for example an index value. Comparison of the subject measurements to a reference value allows for the present or absence of colon cancer to be determined, response to therapy to be monitored or the progression of colon cancer to be determined. For example, a similarity in the subject data set compares to a baseline data set derived form a subject having colon cancer indicates that presence of colon cancer or response to therapy that is not efficacious. Whereas a similarity in the subject data set compares to a baseline data set derived from a subject not having colon cancer indicates the absence of colon cancer or response to therapy that is efficacious. In various embodiments, the baseline data set is derived from one or more other samples from the same subject, taken when the subject is in a biological condition different from that in which the subject was at the time the first sample was taken, with respect to at least one of age, nutritional history, medical condition, clinical indicator, medication, physical activity, body mass, and environmental exposure, and the baseline profile data set may be derived from one or more other samples from one or more different subjects.

The baseline data set or reference values may be derived from one or more other samples from the same subject taken under circumstances different from those of the first sample, and the circumstances may be selected from the group consisting of (i) the time at which the first sample is taken (*e.g.*, before, after, or during treatment cancer treatment), (ii) the site from which the first sample is taken, (iii) the biological condition of the subject when the first sample is taken.

The measure of the constituent is increased or decreased in the subject compared to the expression of the constituent in the reference, *e.g.*, normal reference sample or baseline value. The measure is increased or decreased 10%, 25%, 50% compared to the reference level. Alternately, the measure is increased or decreased 1, 2, 5 or more fold compared to the reference level.

In various aspects of the invention the methods are carried out wherein the measurement conditions are substantially repeatable, particularly within a degree of repeatability of better than ten percent, five percent or more particularly within a degree of repeatability of better than three percent, and/or wherein efficiencies of amplification for all constituents are substantially similar, more particularly wherein the efficiency of amplification is within ten percent, more particularly wherein the efficiency of amplification for all constituents is within five percent, and still more particularly wherein the efficiency of amplification for all constituents is within three percent or less.

In addition, the one or more different subjects may have in common with the subject at least one of age group, gender, ethnicity, geographic location, nutritional history, medical condition, clinical indicator, medication, physical activity, body mass, and environmental exposure. A clinical indicator may be used to assess colon cancer or a condition related to colon cancer of the one or more different subjects, and may also include interpreting the calibrated profile data set in the context of at least one other clinical indicator, wherein the at least one other clinical indicator includes blood chemistry, X-ray or other radiological or metabolic imaging technique, molecular markers in the blood, other chemical assays, and physical findings.

At least 1, 2, 3, 4, 5, 6, 7, 8, 9, 10, 15, 20, 30, 40, 50 or more constituents are measured. Preferably, XIN2, C1QA, CDKN2A, CCR7, CNKSR2, C1QB , EGR1, MSH2, MSH6 or RHOC is measured.

In one aspect, two constituents from Table 1 are measured. The first constituent is ACSL5, ALDH1A1, APC, AXIN2, BAX, CA4, CCND3, CD44, CD63, CFLAR, GADD45A, IGFBP4, ITGA3, MGMT, MSH2, or MSH6 and the second constituent is any other constituent from Table 1.

In another aspect two constituents from Table 2 are measured. The first constituent is ADAM17, ALOX5, APAF1, C1QA, CASP1, CASP3, CCL3, CCL5, CCR5, CD19, CD4, CD8A, CTLA4, CXCL1, CXCR3, DPP4, EGR1, GZMB, HLADRA, HMOX1, HSPA1A, ICAM1, IFI16, IFNG, IL10, IL18, IL18BP, IL1B, IL1R1, IL1RN, IL23A, IL32, IL8, IRF1, LTA, MAPK14, MHC2TA, MIF, MMP9, MNDA, MYC, NFKB1, PLA2G7, PLAUR, PTGS2, PTPRC, SERPINA1, SSI3, TGFB1, TIMP1, TLR2, TNF, or TNPRSF1A, and the second constituent is any other constituent from Table 2.

In a further aspect two constituents from Table 3 are measured. The first constituent is ABL1, ABL2, AKT1, APAF1, ATM, BAD, BAX, BCL2, BRAF, BRCA1, CASP8, CDK2, CDK4, CDK5, CDKN1A, CDKN2A, CFLAR, COL18A1, E2F1, EGR1, ERBB2, FOS, GZMA, HRAS, IFITM1, IL1B, IL8, ITGA1, ITGA3, ITGAE, ITGB1, MMP9, MSH2, MYC, MYCL1, NFKB1, NME4, NOTCH2, NRAS, PCNA, PLAUR, PTCH1, RB1, RHOA, RHOC, S100A4, SEMA4D, SERPINE1, SKI, SKIL, SMAD4, TGFB1, or TNF and the second constituent is any other constituent from Table 3.

In yet another aspect two constituents from Table 4 are measured. The first constituent is, CEBPB, CREBBP, EGR1, EGR2, FOS, ICAM1, MAP2K1, NAB1, NFKB1, NR4A2, SRC, TGFB1, and TOPBP1 and the second constituent is from the group consisting of NAB 1, NR4A2, PDGFA, PTEN, TGFB1, TNFRSF6, or TOPBP1, and the second constituent is any other constituent from Table 4.

In a further aspect two constituents from Table 5 are measured. The first constituent is ADAM17, APC, AXIN2, BAX, BCAM, C1QA, C1QB, CA4, CASP9, CAV1, CCL3, CCL5, CCR7, CD59, CD97, CNKSR2, CTNNA1, CTSD, DAD1, DIABLO, E2F1, EGR1, ESR1, ETS2, FOS, G6PD, GNB1, GSK3B, HMGA1, HMOX1, HOXA10, IFI16, IGF2BP2, IKBKE, IL8, ING2, IQGAP1, IRF1, ITGAL, LARGE, LGALS8, LTA, MAPK14, MLH1, MME, MMP9, MNDA, MSH2, MSH6, MTA1, MTF1, MYD88, NBEA, NCOA1, NRAS, PLEK2, PLXDC2, PTEN, PTPRK, RBM5, S100A4, SERPINE1, SERPING1, SIAH2, SPARC, SRF, ST14, TGFB1, TIMP1, TLR2, TNF, TNFRSF1A, TNFSF5, or UBE2C and the second constituent is any other constituent from Table 5.

The panel of constituents are selected so as to distinguish from a normal and a colorectal cancer-diagnosed subject. The colorectal cancer-diagnosed subject is diagnosed with different stages of cancer. Alternatively, the panel of constituents is selected as to permit characterizing the severity of colon cancer in relation to a normal subject over time so as to track movement toward normal as a result of successful therapy and away from normal in response to cancer recurrence. Thus in some embodiments, the methods of the invention are used to determine efficacy of treatment of a particular subject.

Preferably, the constituents are selected so as to distinguish, *e.g.*, classify between a normal and a colon cancer-diagnosed subject with at least 75%, 80%, 85%, 90%, 95%, 97%, 98%, 99% or greater accuracy. By "accuracy" is meant that the method has the ability to distinguish, *e.g.*, classify, between subjects having colon cancer or conditions associated with colon cancer, and those that do not. Accuracy is determined for example by comparing the results of the Gene Precision Profiling™ to standard accepted clinical methods of diagnosing colorectal cancer, *e.g.*, one or more symptoms of colorectal cancer such changes in bowel habits (*e.g.*, constipation, diarrhea, narrowing of the stool), stomach cramping or bloating, bright red blood in stool, unexplained weight loss, constant fatigue, constant sensation of needing a bowel movement, naseau and vomiting, gaseousness, and anemia.

For example the combination of constituents are selected according to any of the models enumerated in Tables 1A, 2A, 3A, 4A, or 5A.

In some embodiments, the methods of the present invention are used in conjunction with standard accepted clinical methods to diagnose colon cancer. By colorectal cancer or conditions related to colorectal cancer is meant the growth of abnormal cells in the colon or the rectum, capable of invading and destroying other colorectal cells, and includes adenocarcinomas, carcinoid tumors, gastrointestinal stromal tumors, and lymphomas of the digestive system. The term colorectal cancer encompasses both colon cancer and rectal cancer.

The sample is any sample derived from a subject which contains RNA. For example, the sample is blood, a blood fraction, body fluid, a population of cells or tissue from the subject, a colon cell, or a rare circulating tumor cell or circulating endothelial cell found in the blood.

Optionally one or more other samples can be taken over an interval of time that is at least one month between the first sample and the one or more other samples, or taken over an interval of time that is at least twelve months between the first sample and the one or more samples, or they may be taken pre-therapy intervention or post-therapy intervention. In such embodiments, the first sample may be derived from blood and the baseline profile data set may be derived from tissue or body fluid of the subject other than blood. Alternatively, the first sample is derived from tissue or bodily fluid of the subject and the baseline profile data set is derived from blood.

Also included in the invention are kits for the detection of colon cancer in a subject, containing at least one reagent for the detection or quantification of any constituent measured according to the methods of the invention and instructions for using the kit.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description and claims.


**BRIEF DESCRIPTION OF THE DRAWINGS**


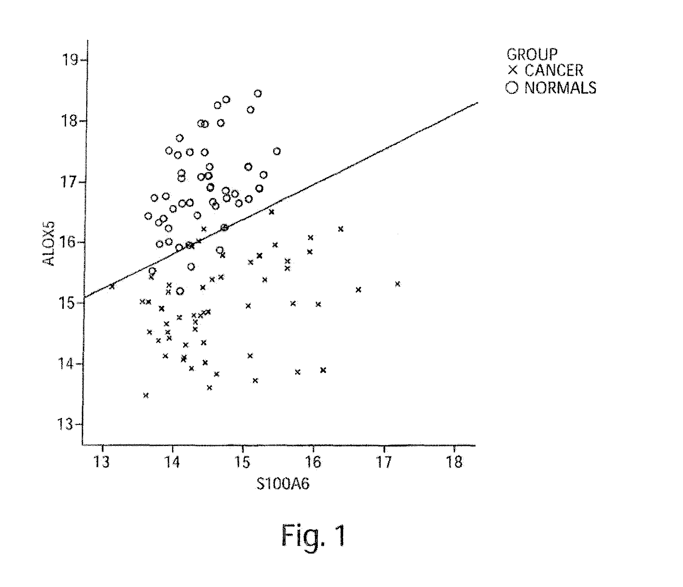

Figure 1 is a graphical representation of a 2-gene model for cancer based on disease-specific genes, capable of distinguishing between subjects afflicted with cancer and normal subjects with a discrimination line overlaid onto the graph as an example of the Index Function evaluated at a particular logit value. Values above and to the left of the line represent subjects predicted to be in the normal population. Values below and to the right of the line represent subjects predicted to be in the cancer population. ALOX5 values are plotted along the Y-axis, S100A6 values are plotted along the X-axis.

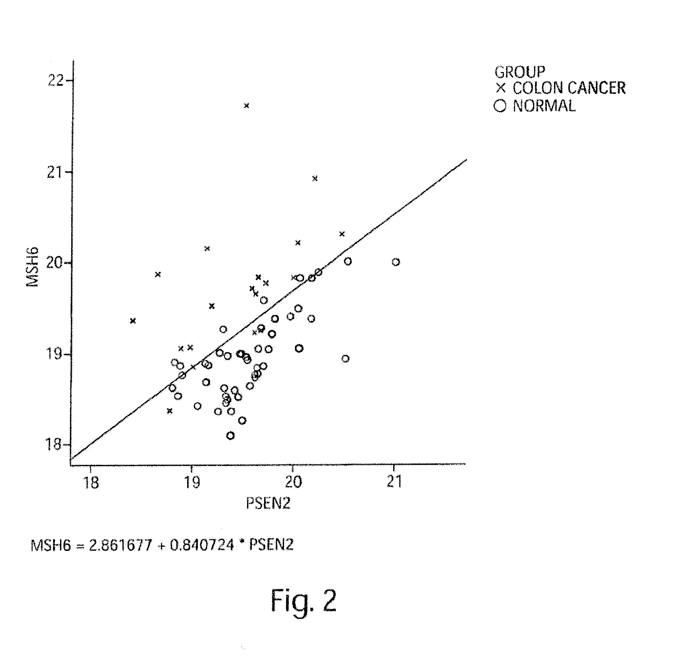

Figure 2 is a graphical representation of a 2-gene model, MSH6 and PSEN2, based on the Precision Profile™ for Colorectal Cancer (Table 1), capable of distinguishing between subjects afflicted with colon cancer and normal subjects, with a discrimination line overlaid onto the graph as an example of the Index Function evaluated at a particular logit value. Values below and to the right of the line represent subjects predicted to be in the normal population. Values above and to the left of the line represent subjects predicted to be in the colon cancer population. MSH6 values are plotted along the Y-axis, PSEN2 values are plotted along the X-axis.

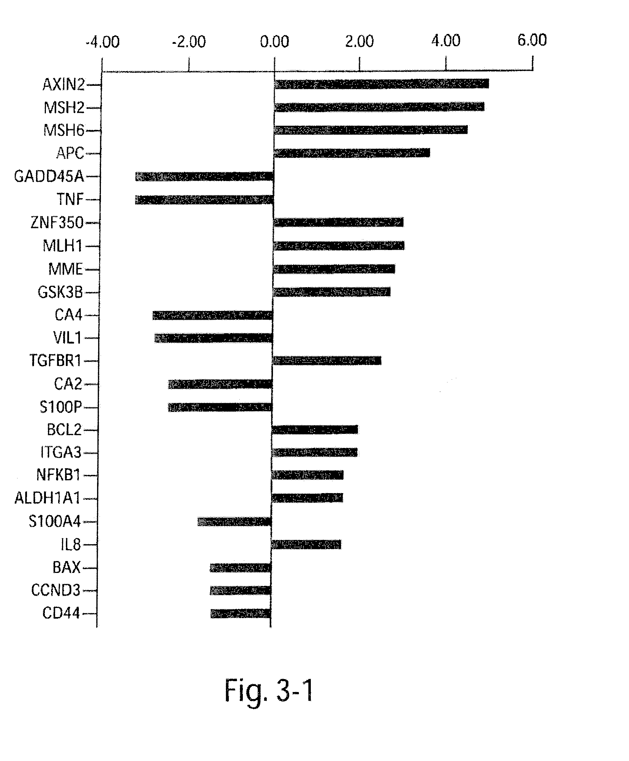

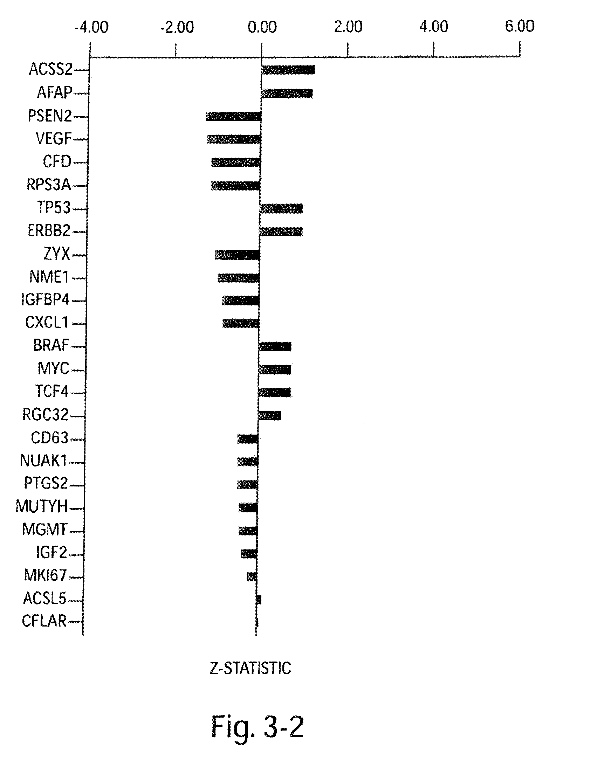

Figure 3 is a graphical representation of the Z-statistic values for each gene shown in Table 1B. A negative Z statistic means up-regulation of gene expression in colon cancer vs. normal patients; a positive Z statistic means down-regulation of gene expression in colon cancer vs. normal patients.

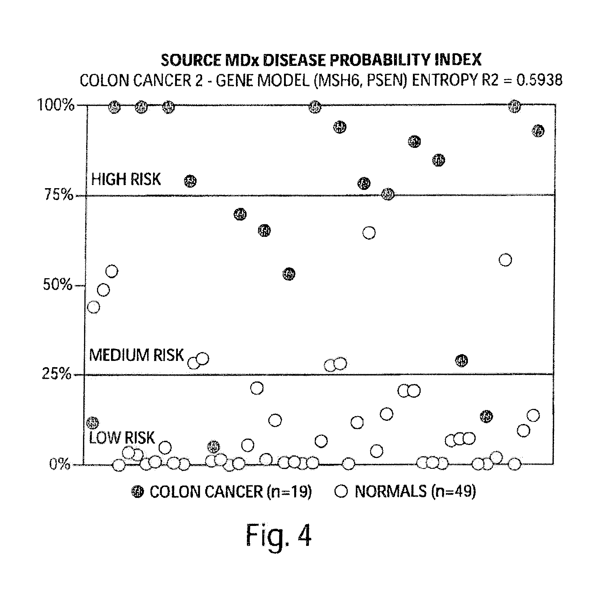

Figure 4 is a graphical representation of a colon cancer index based on the 2-gene logistic regression model, MSH6 and PSEN2, capable of distinguishing between normal, healthy subjects and subjects suffering from colon cancer.

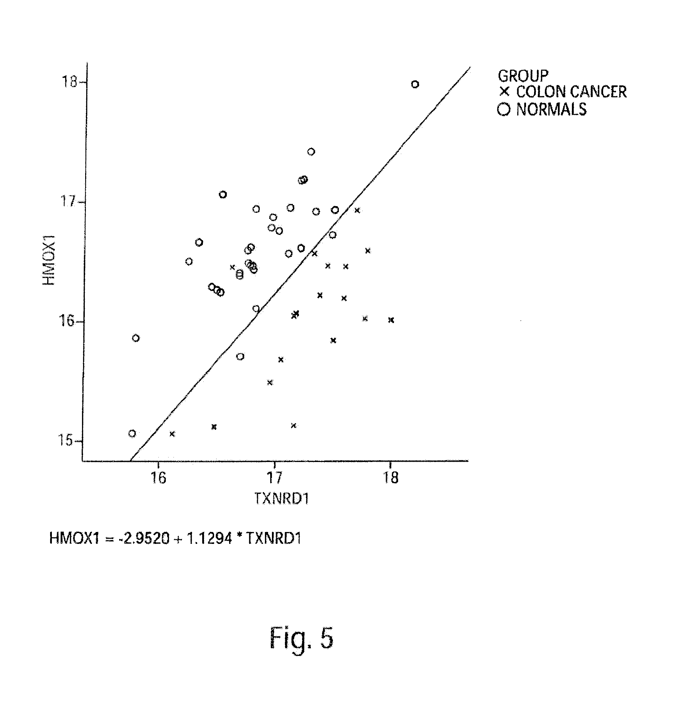

Figure 5 is a graphical representation of a 2-gene model, HMOX1 and TXNRD1, based on the Precision Profile™ for Inflammatory Response (Table 2), capable of distinguishing between subjects afflicted with colon cancer and normal subjects, with a discrimination line overlaid onto the graph as an example of the Index Function evaluated at a particular logit value. Values above and to the left of the line represent subjects predicted to be in the normal population. Values below and to the right of the line represent subjects predicted to be in the colon cancer population. HMOX1 values are plotted along the Y-axis, TXNRD1 values are plotted along the X-axis.

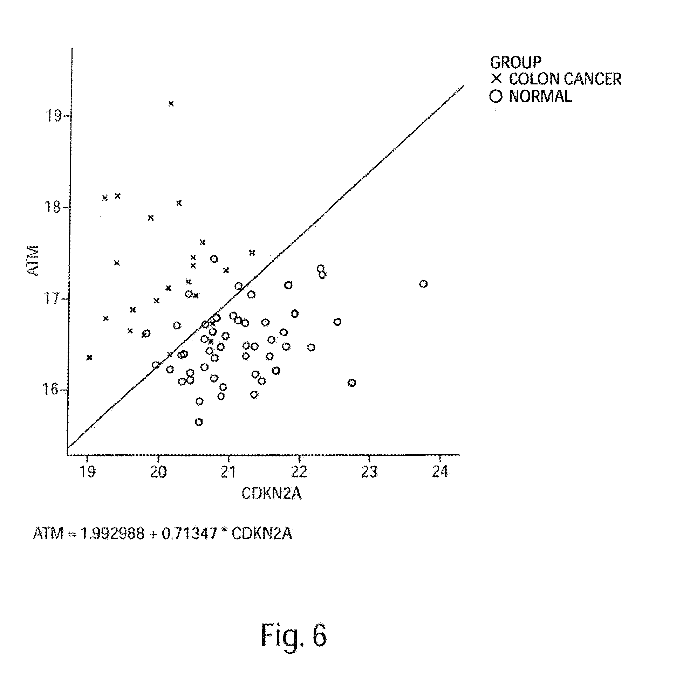

Figure 6 is a graphical representation of a 2-gene model, ATM and CDKN2A, based on the Human Cancer General Precision Profile™ (Table 3), capable of distinguishing between subjects afflicted with colon cancer and normal subjects, with a discrimination line overlaid onto the graph as an example of the Index Function evaluated at a particular logit value. Values below and to the right of the line represent subjects predicted to be in the normal population. Values above and to the left of the line represent subjects predicted to be in the colon cancer population.

ATM values are plotted along the Y-axis, CDKN2A values are plotted along the X-axis.

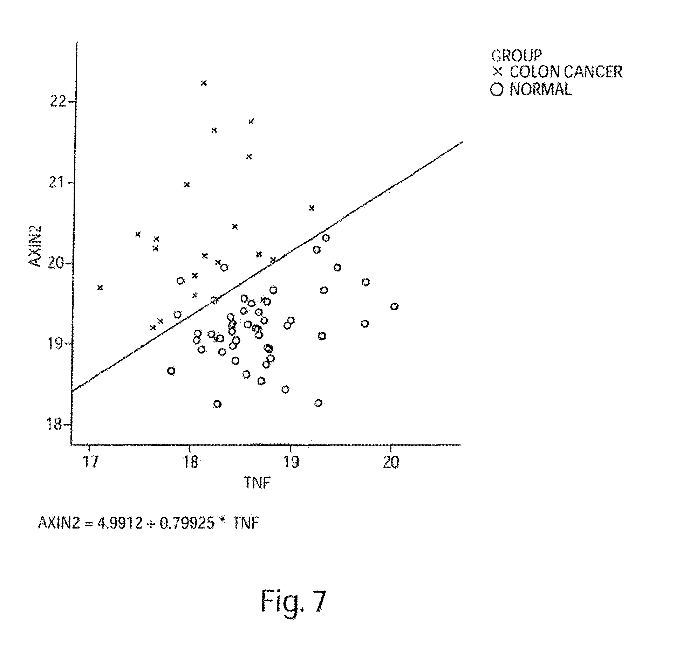

Figure 7 is a graphical representation of a 2-gene model, AXIN2 and TNF, based on the Cross-Cancer Precision Profile™ (Table 5), capable of distinguishing between subjects afflicted with colon cancer and normal subjects, with a discrimination line overlaid onto the graph as an example of the Index Function evaluated at a particular logit value. Values below and to the right of the line represent subjects predicted to be in the normal population. Values above and to the left of the line represent subjects predicted to be in the colon cancer population. AXIN2 values are plotted along the Y-axis, TNF values are plotted along the X-axis.

## DETAILED DESCRIPTION

### *Definitions*

The following terms shall have the meanings indicated unless the context otherwise requires:

*"Accuracy"* refers to the degree of conformity of a measured or calculated quantity (a test reported value) to its actual (or true) value. Clinical accuracy relates to the proportion of true outcomes (true positives (TP) or true negatives (TN)) versus misclassified outcomes (false positives (FP) or false negatives (FN)), and may be stated as a sensitivity, specificity, positive predictive values (PPV) or negative predictive values (NPV), or as a likelihood, odds ratio, among other measures.

*"Algorithm"* is a set of rules for describing a biological condition. The rule set may be defined exclusively algebraically but may also include alternative or multiple decision points requiring domain-specific knowledge, expert interpretation or other clinical indicators.

An *"agent"* is a *"composition"* or a *"stimulus",* as those terms are defined herein, or a combination of a *composition* and a *stimulus.*

*"Amplification"* in the context of a quantitative RT-PCR assay is a function of the number of DNA replications that are required to provide a quantitative determination of its concentration. *"Amplification"* here refers to a degree of sensitivity and specificity of a quantitative assay technique. Accordingly, amplification provides a measurement of concentrations of constituents that is evaluated under conditions wherein the efficiency of amplification and therefore the degree of sensitivity and reproducibility for measuring all constituents is substantially similar.

A *"baseline profile data set"* is a set of values associated with constituents of a *Gene Expression Panel* (Precision Profile™) resulting from evaluation of a biological sample (or population or set of samples) under a desired *biological condition* that is used for mathematically normative purposes. The desired biological condition may be, for example, the condition of a subject (or population or set of subjects) before exposure to an agent or in the presence of an untreated disease or in the absence of a disease. Alternatively, or in addition, the desired biological condition may be health of a subject or a population or set of subjects. Alternatively, or in addition, the desired biological condition may be that associated with a population or set of subjects selected on the basis of at least one of age group, gender, ethnicity, geographic location, nutritional history, medical condition, clinical indicator, medication, physical activity, body mass, and environmental exposure.

A *"biological condition"* of a subject is the condition of the subject in a pertinent realm that is under observation, and such realm may include any aspect of the subject capable of being monitored for change in condition, such as health; disease including cancer; trauma; aging; infection; tissue degeneration; developmental steps; physical fitness; obesity, and mood. As can be seen, a condition in this context may be chronic or acute or simply transient. Moreover, a targeted biological condition may be manifest throughout the organism or population of cells or may be restricted to a specific organ (such as skin, heart, eye or blood), but in either case, the condition may be monitored directly by a sample of the affected population of cells or indirectly by a sample derived elsewhere from the subject. The term *"biological condition"* includes a *"physiological condition".*

*"Body fluid"* of a subject includes blood, urine, spinal fluid, lymph, mucosal secretions, prostatic fluid, semen, haemolymph or any other body fluid known in the art for a subject.

*"Calibrated profile data set"* is a function of a member of a first *profile data set* and a corresponding member of a *baseline profile data set* for a given constituent in a panel.

A *"circulating endothelial cell"* ("CEC") is an endothelial cell from the inner wall of blood vessels which sheds into the bloodstream under certain circumstances, including inflammation, and contributes to the formation of new vasculature associated with cancer pathogenesis. CECs may be useful as a marker of tumor progression and/or response to antiangiogenic therapy.

A *"circulating tumor cell"* ("CTC") is a tumor cell of epithelial origin which is shed from the primary tumor upon metastasis, and enters the circulation. The number of circulating tumor cells in peripheral blood is associated with prognosis in patients with metastatic cancer. These cells can be separated and quantified using immunologic methods that detect epithelial cells.

A *"clinical indicator"* is any physiological datum used alone or in conjunction with other data in evaluating the *physiological condition* of a collection of cells or of an organism. This term includes pre-clinical indicators.

*"Clinical parameters"* encompasses all non-sample or non-Precision Profiles™ of a subject's health status or other characteristics, such as, without limitation, age (AGE), ethnicity (RACE), gender (SEX), and family history of cancer.

*"Colorectal cancer"* is a type of cancer that develops in the colon, or the rectum and includes adenocarcinomas, carcinoid tumors, gastrointestinal stromal tumors, and lymphomas of the digestive system. The term colorectal cancer encompasses both colon cancer and rectal cancer. The terms colorectal cancer and colon cancer are used interchangeably herein.

A *"composition"* includes a chemical compound, a nutraceutical, a pharmaceutical, a homeopathic formulation, an allopathic formulation, a naturopathic formulation, a combination of compounds, a toxin, a food, a food supplement, a mineral, and a complex mixture of substances, in any physical state or in a combination of physical states.

To *"derive"* a profile data set from a *sample* includes determining a set of values associated with constituents of a *Gene Expression Panel* (Precision Profile™) either (i) by direct measurement of such constituents in a biological *sample.*

*"Distinct RNA or protein constituent"* in a panel of constituents is a distinct expressed product of a gene, whether RNA or protein. An *"expression"* product of a gene includes the gene product whether RNA or protein resulting from translation of the messenger RNA.

"FN" is false negative, which for a disease state test means classifying a disease subject incorrectly as non-disease or normal.

"FP" is false positive, which for a disease state test means classifying a normal subject incorrectly as having disease.

A *"formula,"* *"algorithms,"* or *"model"* is any mathematical equation, algorithmic, analytical or programmed process, statistical technique, or comparison, that takes one or more continuous or categorical inputs (herein called *"parameters")* and calculates an output value, sometimes referred to as an *"index"* or *"index value."* Non-limiting examples of *"formulas"* include comparisons to reference values or profiles, sums, ratios, and regression operators, such as coefficients or exponents, value transformations and normalizations (including, without limitation, those normalization schemes based on clinical parameters, such as gender, age, or ethnicity), rules and guidelines, statistical classification models, and neural networks trained on historical populations. Of particular use in combining constituents of a Gene Expression Panel (Precision Profile™) are linear and non-linear equations and statistical significance and classification analyses to determine the relationship between levels of constituents of a Gene Expression Panel (Precision Profile™) detected in a subject sample and the subject's risk of colorectal cancer. In panel and combination construction, of particular interest are structural and synactic statistical classification algorithms, and methods of risk index construction, utilizing pattern recognition features, including, without limitation, such established techniques such as cross-correlation, Principal Components Analysis (PCA), factor rotation, Logistic Regression Analysis (LogReg), Kolmogorov Smirnoff tests (KS), Linear Discriminant Analysis (LDA), Eigengene Linear Discriminant Analysis (ELDA), Support Vector Machines (SVM), Random Forest (RF), Recursive Partitioning Tree (RPART), as well as other related decision tree classification techniques (CART, LART, LARTree, FlexTree, amongst others), Shrunken Centroids (SC), StepAIC, K-means, Kth-Nearest Neighbor, Boosting, Decision Trees, Neural Networks, Bayesian Networks, Support Vector Machines, and Hidden Markov Models, among others. Other techniques may be used in survival and time to event hazard analysis, including Cox, Weibull, Kaplan-Meier and Greenwood models well known to those of skill in the art. Many of these techniques are useful either combined with a consituentes of a Gene Expression Panel (Precision Profile™) selection technique, such as forward selection, backwards selection, or stepwise selection, complete enumeration of all potential panels of a given size, genetic algorithms, voting and committee methods, or they may themselves include biomarker selection methodologies in their own technique. These may be coupled with information criteria, such as Akaike's Information Criterion (AIC) or Bayes Information Criterion (BIC), in order to quantify the tradeoff between additional biomarkers and model improvement, and to aid in minimizing overfit. The resulting predictive models may be validated in other clinical studies, or cross-validated within the study they were originally trained in, using such techniques as Bootstrap, Leave-One-Out (LOO) and 10-Fold cross-validation (10-Fold CV). At various-steps, false discovery rates (FDR) may be estimated by value permutation according to techniques known in the art.

A *"Gene Expression Panel"* (Precision Profile™) is an experimentally verified set of constituents, each constituent being a distinct expressed product of a gene, whether RNA or protein, wherein constituents of the set are selected so that their measurement provides a measurement of a targeted *biological condition.*

A *"Gene Expression Profile"* is a set of values associated with constituents of a *Gene Expression Panel* (Precision Profile™) resulting from evaluation of a biological sample (or population or set of samples).

A "*Gene Expression Profile InflammationIndex"* is the value of an index function that provides a mapping from an instance of a *Gene Expression Profile* into a single-valued measure of inflammatory condition.

A *Gene Expression Profile Cancer Index"* is the value of an index function that provides a mapping from an instance of a *Gene Expression Profile* into a single-valued measure of a cancerous condition.

The "*health*" of a subject includes mental, emotional, physical, spiritual, allopathic, naturopathic and homeopathic condition of the subject.

"*Index*" is an arithmetically or mathematically derived numerical characteristic developed for aid in simplifying or disclosing or informing the analysis of more complex quantitative information. A disease or population index may be determined by the application of a specific algorithm to a plurality of subjects or samples with a common biological condition.

"*Inflammation*" is used herein in the general medical sense of the word and may be an acute or chronic; simple or suppurative; localized or disseminated; cellular and tissue response initiated or sustained by any number of chemical, physical or biological agents or combination of agents.

"*Inflammatory state*" is used to indicate the relative biological condition of a subject resulting from inflammation, or characterizing the degree of inflammation.

A "*large number*" of data sets based on a common panel of genes is a number of data sets sufficiently large to permit a statistically significant conclusion to be drawn with respect to an instance of a data set based on the same panel.

"*Negative predictive value*" or "*NPV*" is calculated by TN/(TN + FN) or the true negative fraction of all negative test results. It also is inherently impacted by the prevalence of the disease and pre-test probability of the population intended to be tested. See, *e.g.*, O'Marcaigh AS, Jacobson RM, "Estimating the Predictive Value of a Diagnostic Test, How to Prevent Misleading or Confusing Results," Clin. Ped. 1993, 32(8): 485-491, which discusses specificity, sensitivity, and positive and negative predictive values of a test, *e.g.*, a clinical diagnostic test. Often, for binary disease state classification approaches using a continuous diagnostic test measurement, the sensitivity and specificity is summarized by Receiver Operating Characteristics (ROC) curves according to Pepe et al., "Limitations of the Odds Ratio in Gauging the Performance of a Diagnostic, Prognostic, or Screening Marker," Am. J. Epidemiol 2004, 159 (9): 882-890, and summarized by the Area Under the Curve (AUC) or c-statistic, an indicator that allows representation of the sensitivity and specificity of a test, assay, or method over the entire range of test (or assay) cut points with just a single value. See also, *e.g.*, Shultz, "Clinical Interpretation of Laboratory Procedures," chapter 14 in Teitz, Fundamentals of Clinical Chemistry, Burtis and Ashwood (eds.), 4th edition 1996, W.B. Saunders Company, pages 192-199; and Zweig et al., "ROC Curve Analysis: An Example Showing the Relationships Among Serum Lipid and Apolipoprotein Concentrations in Identifying Subjects with Coronory Artery Disease," Clin. Chem., 1992, 38(8): 1425-1428. An alternative approach using likelihood functions, BIC, odds ratios, information theory, predictive values, calibration (including goodness-of-fit), and reclassification measurements is summarized according to Cook, "Use and Misuse of the Receiver Operating Characteristic Curve in Risk Prediction," Circulation 2007, 115: 928-935.

A "*normal*" subject is a subject who is generally in good health, has not been diagnosed with colorectal cancer, is asymptomatic for colorectal cancer, and lacks the traditional laboratory risk factors for colorectal cancer.

A "*normative*" condition of a subject to whom a composition is to be administered means the condition of a subject before administration, even if the subject happens to be suffering from a disease.

A "*panel*" of genes is a set of genes including at least two constituents.

A "*population of cells*" refers to any group of cells wherein there is an underlying commonality or relationship between the members in the population of cells, including a group of cells taken from an organism or from a culture of cells or from a biopsy, for example.

"*Positive predictive value*" or "*PPV*" is calculated by TP/(TP+FP) or the true positive fraction of all positive test results. It is inherently impacted by the prevalence of the disease and pre-test probability of the population intended to be tested.

"*Risk*" in the context of the present invention, relates to the probability that an event will occur over a specific time period, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of lower risk cohorts, across population divisions (such as tertiles, quartiles, quintiles, or deciles, etc.) or an average population risk, which can vary by how clinical risk factors are assessed. Odds ratios, the proportion of positive events to negative events for a given test result, are also commonly used (odds are according to the formula p/(1-p) where p is the probability of event and (1- p) is the probability of no event) to no-conversion.

"*Risk evaluation,*" or "*evaluation of risk*" in the context of the present invention encompasses making a prediction of the probability, odds, or likelihood that an event or disease state may occur, and/or the rate of occurrence of the event or conversion from one disease state to another, *i.e.*, from a normal condition to cancer or from cancer remission to cancer, or from primary cancer occurrence to occurrence of a cancer metastasis. Risk evaluation can also comprise prediction of future clinical parameters, traditional laboratory risk factor values, or other indices of cancer results, either in absolute or relative terms in reference to a previously measured population. Such differing use may require different consituentes of a Gene Expression Panel (Precision Profile™) combinations and indivualized panels, mathematical algorithms, and/or cut-off points, but be subject to the same aforementioned measurements of accuracy and performance for the respective intended use.

A *"sample"* from a subject may include a single cell or multiple cells or fragments of cells or an aliquot of body fluid, taken from the subject, by means including venipuncture, excretion, ejaculation, massage, biopsy, needle aspirate, lavage sample, scraping, surgical incision or intervention or other means known in the art. The sample is blood, urine, spinal fluid, lymph, mucosal secretions, prostatic fluid, semen, haemolymph or any other body fluid known in the art for a subject. The sample is also a tissue sample. The sample is or contains a circulating endothelial cell or a circulating tumor cell.

*"Sensitivity"* is calculated by TP/(TP+FN) or the true positive fraction of disease subjects.

*"Specificity"* is calculated by TN/(TN+FP) or the true negative fraction of non-disease or normal subjects.

By "*statistically significant",* it is meant that the alteration is greater than what might be expected to happen by chance alone (which could be a "false positive"). Statistical significance can be determined by any method known in the art. Commonly used measures of significance include the *p*-value, which presents the probability of obtaining a result at least as extreme as a given data point, assuming the data point was the result of chance alone. A result is often considered highly significant at a p-value of 0.05 or less and statistically significant at a p-value of 0.10 or less. Such p-values depend significantly on the power of the study performed.

A *"set"* or *"population"* of samples or subjects refers to a defined or selected group of samples or subjects wherein there is an underlying commonality or relationship between the members included in the set or population of samples or subjects.

A *"Signature Profile"* is an experimentally verified subset of a *Gene Expression Profile* selected to discriminate a biological condition, agent or physiological mechanism of action.

A *"Signature Panel"* is a subset of a *Gene Expression Panel (Precision Profile™),* the constituents of which are selected to permit discrimination of a *biological condition, agent* or physiological mechanism of action.

A *"subject"* is a cell, tissue, or organism, human or non-human, whether *in vivo,* ex vivo or in *vitro,* under observation. As used herein, reference to evaluating the biological condition of a subject based on a sample from the subject, includes using blood or other tissue sample from a human subject to evaluate the human subject's condition; it also includes, for example, using a blood sample itself as the subject to evaluate, for example, the effect of therapy or an agent upon the sample.

A *"stimulus"* includes (i) a monitored physical interaction with a subject, for example ultraviolet A or B, or light therapy for seasonal affective disorder, or treatment of psoriasis with psoralen or treatment of cancer with embedded radioactive seeds, other radiation exposure, and (ii) any monitored physical, mental, emotional, or spiritual activity or inactivity of a subject.

*"Therapy"* includes all interventions whether biological, chemical, physical, metaphysical, or combination of the foregoing, intended to sustain or alter the monitored biological condition of a subject.

*"TN"* is *true negative,* which for a disease state test means classifying a non-disease or normal subject correctly.

*"TP"* is *true positive,* which for a disease state test means correctly classifying a disease subject.

The PCT patent application publication number WO 01/25473, published April 12, 2001, entitled "Systems and Methods for Characterizing a Biological Condition or Agent Using Calibrated Gene Expression Profiles," filed for an invention by inventors herein, and which is herein incorporated by reference, discloses the use of Gene Expression Panels (Precision Profiles™) for the evaluation of (i) biological condition (including with respect to health and disease) and (ii) the effect of one or more agents on biological condition (including with respect to health, toxicity, therapeutic treatment and drug interaction).

In particular, the Gene Expression Panels (Precision Profile™) described herein may be used, without limitation, for measurement of the following: therapeutic efficacy of natural or synthetic compositions or stimuli that may be formulated individually or in combinations or mixtures for a range of targeted biological conditions; prediction of toxicological effects and dose effectiveness of a composition or mixture of compositions for an individual or for a population or set of individuals or for a population of cells; determination of how two or more different agents administered in a single treatment might interact so as to detect any of synergistic, additive, negative, neutral or toxic activity; performing pre-clinical and clinical trials by providing new criteria for pre-selecting subjects according to informative profile data sets for revealing disease status; and conducting preliminary dosage studies for these patients prior to conducting phase 1 or 2 trials. These Gene Expression Panels (Precision Profiles™) may be employed with respect to samples derived from subjects in order to evaluate their biological condition.

The present invention provides Gene Expression Panels (Precision Profiles™) for the evaluation or characterization of colorectal cancer and conditions related to colorectal cancer in a subject. In addition, the Gene Expression Panels described herein also provide for the evaluation of the effect of one or more agents for the treatment of colorectal cancer and conditions related to colorectal cancer.

The Gene Expression Panels (Precision Profiles™) are referred to herein as the Precision Profile™ for Colorectal Cancer, the Precision Profile™ for Inflammatory Response, the Human Cancer General Precision Profile™, the Precision Profile™ for EGR1, and the Cross-Cancer Precision Profile™. The Precision Profile™ for Colorectal Cancer includes one or more genes, *e.g.*, constituents, listed in Table 1, whose expression is associated with colorectal cancer or conditions related to colorectal cancer. The Precision Profile™ for Inflammatory Response includes one or more genes, *e.g.*; constituents, listed in Table 2, whose expression is associated with inflammatory response and cancer. The Human Cancer General Precision Profile™ includes one or more genes, *e.g.*, constituents, listed in Table 3, whose expression is associated generally with human cancer (including without limitation prostate, breast, ovarian, cervical, lung, colon, and skin cancer).

The Precision Profile™ for EGR1 includes one or more genes, *e.g.*, constituents listed in Table 4, whose expression is associated with the role early growth response (EGR) gene family plays in human cancer. The Precision Profile™ for EGR1 is composed of members of the early growth response (EGR) family of zinc finger transcriptional regulators; EGR1, 2, 3 & 4 and their binding proteins; NAB1 & NAB2 which function to repress transcription induced by some members of the EGR family of transactivators. In addition to the early growth response genes, The Precision Profile™ for EGR1 includes genes involved in the regulation of immediate early gene expression, genes that are themselves regulated by members of the immediate early gene family (and EGR1 in particular) and genes whose products interact with EGR1, serving as co-activators of transcriptional regulation.

The Cross-Cancer Precision Profile™ includes one or more genes, *e.g.*, constituents listed in Table 5, whose expression has been shown, by latent class modeling, to play a significant role across various types of cancer, including without limitation, prostate, breast, ovarian, cervical, lung, colon, and skin cancer. Each gene of the Precision Profile™ for Colorectal Cancer, the Precision Profile™ for Inflammatory Response, the Human Cancer General Precision Profile™ the Precision Profile™ for EGR1, and the Cross-Cancer Precision Profile™ is referred to herein as a colorectal cancer associated gene or a colorectal cancer associated constituent. In addition to the genes listed in the Precision Profiles™ herein, colorectal cancer associated genes or colorectal cancer associated constituents include oncogenes, tumor suppression genes, tumor progression genes, angiogenesis genes, and lymphogenesis genes.

The present invention also provides a method for monitoring and determining the efficacy of immunotherapy, using the Gene Expression Panels (Precision Profiles™) described herein. Immunotherapy target genes include, without limitation, TNFRSF10A, TMPRSS2, SPARC, ALOX5, PTPRC, PDGFA, PDGFB, BCL2, BAD, BAK1, BAG2, KIT, MUC1, ADAM17, CD19, CD4, CD40LG, CD86, CCR5, CTLA4, HSPA1A, IFNG, IL23A, PTGS2, TLR2, TGFB1, TNF, TNFRSF13B, TNFRSF10B, VEGF, MYC, AURKA, BAX, CDH1, CASP2, CD22, IGF1R, ITGA5, ITGAV, ITGB1, ITGB3, IL6R, JAK1, JAK2, JAK3, MAP3K1, PDGFRA, COX2, PSCA, THBS1, THBS2, TYMS, TLR1, TLR3, TLR6, TLR7, TLR9, TNFSF10, TNFSF13B, TNFRSF17, TP53, ABL1, ABL2, AKT1, KRAS , BRAF, RAF1, ERBB4, ERBB2, ERBB3, AKT2, EGFR, IL12, and IL15. For example, the present invention provides a method for monitoring and determining the efficacy of immunotherapy by monitoring the immunotherapy associated genes, *i.e.*, constituents, listed in Table 6.

It has been discovered that valuable and unexpected results may be achieved when the quantitative measurement of constituents is performed under repeatable conditions (within a degree of repeatability of measurement of better than twenty percent, preferably ten percent or better, more preferably five percent or better, and more preferably three percent or better). For the purposes of this description and the following claims, a degree of repeatability of measurement of better than twenty percent may be used as providing measurement conditions that are "substantially repeatable". In particular, it is desirable that each time a measurement is obtained corresponding to the level of expression of a constituent in a particular sample, substantially the same measurement should result for substantially the same level of expression. In this manner, expression levels for a constituent in a Gene Expression Panel (Precision Profile™) may be meaningfully compared from sample to sample. Even if the expression level measurements for a particular constituent are inaccurate (for example, say, 30% too low), the criterion of repeatability means that all measurements for this constituent, if skewed, will nevertheless be skewed systematically, and therefore measurements of expression level of the constituent may be compared meaningfully. In this fashion valuable information may be obtained and compared concerning expression of the constituent under varied circumstances.

In addition to the criterion of repeatability, it is desirable that a second criterion also be satisfied, namely that quantitative measurement of constituents is performed under conditions wherein efficiencies of amplification for all constituents are substantially similar as defined herein. When both of these criteria are satisfied, then measurement of the expression level of one constituent may be meaningfully compared with measurement of the expression level of another constituent in a given sample and from sample to sample.

The evaluation or characterization of colorectal cancer is defined to be diagnosing colorectal cancer, assessing the presence or absence of colorectal cancer, assessing the risk of developing colorectal cancer or assessing the prognosis of a subject with colorectal cancer, assessing the recurrence of colorectal cancer or assessing the presence or absence of a metastasis. Similarly, the evaluation or characterization of an agent for treatment of colorectal cancer includes identifying agents suitable for the treatment of colorectal cancer. The agents can be compounds known to treat colorectal cancer or compounds that have not been shown to treat colorectal cancer.

The agent to be evaluated or characterized for the treatment of colorectal cancer may be an alkylating agent (*e.g.*, Cisplatin, Carboplatin, Oxaliplatin, BBR3464, Chlorambucil, Chlormethine, Cyclophosphamides; Ifosmade, Melphalan, Carmustine, Fotemustine, Lomustine, Streptozocin, Busulfan, Dacarbazine, Mechlorethamine, Procarbazine, Temozolomide, ThioTPA, and Uramustine); an anti-metabolite (*e.g.*, purine (azathioprine, mercaptopurine), pyrimidine (Capecitabine, Cytarabine, Fluorouracil, Gemcitabine), and folic acid (Methotrexate, Pemetrexed, Raltitrexed)); a vinca alkaloid (*e.g.*, Vincristine, Vinblastine, Vinorelbine, Vindesine); a taxane (*e.g.*, paclitaxel, docetaxel, BMS-247550); an anthracycline (*e.g.*, Daunorubicin, Doxorubicin, Epirubicin, Idarubicin, Mitoxantrone, Valrubicin, Bleomycin, Hydroxyurea, and Mitomycin); a topoisomerase inhibitor (*e.g.*, Topotecan, Irinotecan Etoposide, and Teniposide); a monoclonal antibody (*e.g.*, Alemtuzumab, Bevacizumab, Cetuximab, Gemtuzumab, Panitumumab, Rituximab, and Trastuzumab); a photosensitizer (*e.g.*, Aminolevulinic acid, Methyl aminolevulinate, Porfimer sodium, and Verteporfin); a tyrosine kinase inhibitor (*e.g.*, Gleevec™); an epidermal growth factor receptor inhibitor (*e.g.*, Iressa™, erlotinib (Tarceva™), gefitinib); an FPTase inhibitor (*e.g.*, FTIs (R115777, SCH66336, L-778,123)); a KDR inhibitor (*e.g.*, SU6668, PTK787); a proteosome inhibitor (*e.g.*, PS341); a TS/DNA synthesis inhibitor (*e.g.*, ZD9331, Raltirexed (ZD1694, Tomudex), ZD9331, 5-FU)); an S-adenosyl-methionine decarboxylase inhibitor (*e.g.*, SAM468A); a DNA methylating agent (*e.g.*, TMZ); a DNA binding agent (*e.g.*, PZA); an agent which binds and inactivates $O^6$-alkylguanine AGT (*eg.,* BG); a *c-raf*-1 antisense oligo-deoxynucleotide (*e.g.,* ISIS-5132 (CGP-69846A)); tumor immunotherapy (see Table 6); a steroidal and/or non-steroidal antiinflammatory agent (*e.g.*, corticosteroids, COX-2 inhibitors); or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin.

Colorectal cancer and conditions related to colorectal cancer is evaluated by determining the level of expression (*e.g.*, a quantitative measure) of an effective number (*e.g.*, one or more) of constituents of a Gene Expression Panel (Precision Profile™) disclosed herein (*i.e.*, Tables 1-5). By an effective number is meant the number of constituents that need to be measured in order to discriminate between a normal subject and a subject having colorectal cancer. Preferably the constituents are selected as to discriminate between a normal subject and a subject having colorectal cancer with at least 75% accuracy, more preferably 80%, 85%, 90%, 95%, 97%, 98%, 99% or greater accuracy.

The level of expression is determined by any means known in the art, such as for example quantitative PCR. The measurement is obtained under conditions that are substantially repeatable. Optionally, the qualitative measure of the constituent is compared to a reference or baseline level or value (*e.g.* a baseline profile set). In one embodiment, the reference or baseline level is a level of expression of one or more constituents in one or more subjects known not to be suffering from colorectal cancer (*e.g.*, normal, healthy individual(s)). Alternatively, the reference or baseline level is derived from the level of expression of one or more constituents in one or more subjects known to be suffering from colorectal cancer. Optionally, the baseline level is derived from the same subject from which the first measure is derived. For example, the baseline is taken from a subject prior to receiving treatment or surgery for colorectal cancer, or at different time periods during a course of treatment. Such methods allow for the evaluation of a particular treatment for a selected individual. Comparison can be performed on test (*e.g.*, patient) and reference samples (*e.g.*, baseline) measured concurrently or at temporally distinct times. An example of the latter is the use of compiled expression information, *e.g.*, a gene expression database, which assembles information about expression levels of cancer associated genes.

A reference or baseline level or value as used herein can be used interchangeably and is meant to be relative to a number or value derived from population studies, including without limitation, such subjects having similar age range, subjects in the same or similar ethnic group, sex, or, in female subjects, pre-menopausal or post-menopausal subjects, or relative to the starting sample of a subject undergoing treatment for colorectal cancer. Such reference values can be derived from statistical analyses and/or risk prediction data of populations obtained from mathematical algorithms and computed indices of colorectal cancer. Reference indices can also be constructed and used using algorithms and other methods of statistical and structural classification.

In one embodiment of the present invention, the reference or baseline value is the amount of expression of a cancer associated gene in a control sample derived from one or more subjects who are both asymptomatic and lack traditional laboratory risk factors for colorectal cancer.

In another embodiment of the present invention, the reference or baseline value is the level of cancer associated genes in a control sample derived from one or more subjects who are not at risk or at low risk for developing colorectal cancer.

In a further embodiment, such subjects are monitored and/or periodically retested for a diagnostically relevant period of time ("longitudinal studies") following such test to verify continued absence from colorectal cancer (disease or event free survival). Such period of time may be one year, two years, two to five years, five years, five to ten years, ten years, or ten or more years from the initial testing date for determination of the reference or baseline value. Furthermore, retrospective measurement of cancer associated genes in properly banked historical subject samples may be used in establishing these reference or baseline values, thus shortening the study time required, presuming the subjects have been appropriately followed during the intervening period through the intended horizon of the product claim.

A reference or baseline value can also comprise the amounts of cancer associated genes derived from subjects who show an improvement in cancer status as a result of treatments and/or therapies for the cancer being treated and/or evaluated.

In another embodiment, the reference or baseline value is an index value or a baseline value. An index value or baseline value is a composite sample of an effective amount of cancer associated genes from one or more subjects who do not have cancer.

For example, where the reference or baseline level is comprised of the amounts of cancer associated genes derived from one or more subjects who have not been diagnosed with colorectal cancer, or are not known to be suffering from colorectal cancer, a change (*e.g.*, increase or decrease) in the expression level of a cancer associated gene in the patient-derived sample as compared to the expression level of such gene in the reference or baseline level indicates that the subject is suffering from or is at risk of developing colorectal cancer. In contrast, when the methods are applied prophylacticly, a similar level of expression in the patient-derived sample of a colorectal cancer associated gene compared to such gene in the baseline level indicates that the subject is not suffering from or is at risk of developing colorectal cancer.

Where the reference or baseline level is comprised of the amounts of cancer associated genes derived from one or more subjects who have been diagnosed with colorectal cancer, or are known to be suffering from colorectal cancer, a similarity in the expression pattern in the patient-derived sample of a colorectal cancer gene compared to the colorectal cancer baseline level indicates that the subject is suffering from or is at risk of developing colorectal cancer.

Expression of a colorectal cancer gene also allows for the course of treatment of colorectal cancer to be monitored. In this method, a biological sample is provided from a subject undergoing treatment, *e.g.*, if desired, biological samples are obtained from the subject at various time points before, during, or after treatment. Expression of a colorectal cancer gene is then determined and compared to a reference or baseline profile. The baseline profile may be taken or derived from one or more individuals who have been exposed to the treatment. Alternatively, the baseline level may be taken or derived from one or more individuals who have not been exposed to the treatment. For example, samples may be collected from subjects who have received initial treatment for colorectal cancer and subsequent treatment for colorectal cancer to monitor the progress of the treatment.

Differences in the genetic makeup of individuals can result in differences in their relative abilities to metabolize various drugs. Accordingly, the Precision Profile™ for Colorectal Cancer (Table 1), the Precision Profile™ for Inflammatory Response (Table 2), the Human Cancer General Precision Profile™ (Table 3), the Precision Profile™ for EGR1 (Table 4), and the Cross-Cancer Precision Profile™ (Table 5) disclosed herein, allow for a putative therapeutic or prophylactic to be tested from a selected subject in order to determine if the agent is suitable for treating or preventing colorectal cancer in the subject Additionally, other genes known to be associated with toxicity may be used. By suitable for treatment is meant determining whether the agent will be efficacious, not efficacious, or toxic for a particular individual. By toxic it is meant that the manifestations of one or more adverse effects of a drug when administered therapeutically. For example, a drug is toxic when it disrupts one or more normal physiological pathways.

To identify a therapeutic that is appropriate for a specific subject, a test sample from the subject is exposed to a candidate therapeutic agent, and the expression of one or more of colorectal cancer genes is determined. A subject sample is incubated in the presence of a candidate agent and the pattern of colorectal cancer gene expression in the test sample is measured and compared to a baseline profile, *e.g.*, a colorectal cancer baseline profile or a non-colorectal cancer baseline profile or an index value. The test agent can be any compound or composition. For example, the test agent is a compound known to be useful in the treatment of colorectal cancer. Alternatively, the test agent is a compound that has not previously been used to treat colorectal cancer.

If the reference sample, *e.g.*, baseline is from a subject that does not have colorectal cancer a similarity in the pattern of expression of colorectal cancer genes in the test sample compared to the reference sample indicates that the treatment is efficacious. Whereas a change in the pattern of expression of colorectal cancer genes in the test sample compared to the reference sample indicates a less favorable clinical outcome or prognosis. By *"efficacious"* is meant that the treatment leads to a decrease of a sign or symptom of colorectal cancer in the subject or a change in the pattern of expression of a colorectal cancer gene such that the gene expression pattern has an increase in similarity to that of a reference or baseline pattern. Assessment of colorectal cancer is made using standard clinical protocols. Efficacy is determined in association with any known method for diagnosing or treating colorectal cancer.

A Gene Expression Panel (Precision Profile™) is selected in a manner so that quantitative measurement of RNA or protein constituents in the Panel constitutes a measurement of a biological condition of a subject. In one kind of arrangement, a calibrated profile data set is employed. Each member of the calibrated profile data set is a function of (i) a measure of a distinct constituent of a Gene Expression Panel (Precision Profile™) and (ii) a baseline quantity.

Additional embodiments relate to the use of an index or algorithm resulting from quantitative measurement of constituents, and optionally in addition, derived from either expert analysis or computational biology (a) in the analysis of complex data sets; (b) to control or normalize the influence of uninformative or otherwise minor variances in gene expression values between samples or subjects; (c) to simplify the characterization of a complex data set for comparison to other complex data sets, databases or indices or algorithms derived from complex data sets; (d) to monitor a biological condition of a subject; (e) for measurement of therapeutic efficacy of natural or synthetic compositions or stimuli that may be formulated individually or in combinations or mixtures for a range of targeted biological conditions; (f) for predictions of toxicological effects and dose effectiveness of a composition or mixture of compositions for an individual or for a population or set of individuals or for a population of cells; (g) for determination of how two or more different agents administered in a single treatment might interact so as to detect any of synergistic, additive, negative, neutral of toxic activity (h) for performing pre-clinical and clinical trials by providing new criteria for pre-selecting subjects according to informative profile data sets for revealing disease status and conducting preliminary dosage studies for these patients prior to conducting Phase I or 2 trials.

Gene expression profiling and the use of index characterization for a particular condition or agent or both may be used to reduce the cost of Phase 3 clinical trials and may be used beyond Phase 3 trials; labeling for approved drugs; selection of suitable medication in a class of medications for a particular patient that is directed to their unique physiology; diagnosing or determining a prognosis of a medical condition or an infection which may precede onset of symptoms or alternatively diagnosing adverse side effects associated with administration of a therapeutic agent; managing the health care of a patient; and quality control for different batches of an agent or a mixture of agents.

The subject

The methods disclosed herein may be applied to cells of humans, mammals or other organisms without the need for undue experimentation by one of ordinary skill in the art because all cells transcribe RNA and it is known in the art how to extract RNA from all types of cells.

A subject can include those who have not been previously diagnosed as having colorectal cancer or a condition related to colorectal cancer. Alternatively, a subject can also include those who have already been diagnosed as having colorectal cancer or a condition related to colorectal cancer. Diagnosis of colorectal cancer is made, for example, from any one or combination of the following procedures: a medical history; physical exam; blood tests for anemia or tumor markers (*e.g.*, carcinoembryonic antigen, or CA19-9); and one or more screening methods for polyps or abnormalities in the lining of the colorectal wall. Screening methods for polyps or abnormalities include but are not limited to: digital rectal examination (DRE); fecal occult blood test (FOBT); fecal immunochemical test (FIT); colonoscopy or sigmoidoscopy; barium enema with air contrast; virtual colonoscopy; biopsy (*e.g.*, CT guided needle biopsy); and imaging techniques (*e.g.*, ultrasound, CT scan, PET scan, and MRI).

Optionally, the subject has been previously treated with a surgical procedure for removing colorectal cancer or a condition related to colorectal cancer, including but not limited to any one or combination of the following treatments: laparoscopic surgery, colonic segmental resection, polypectomy and local excision to remove superificial cancer and polyps, local transanal resection, lower anterior or abdominoperineal resection, colo-anal anastomosis, coloplasty, abdominoperineal resection, pelvic exteneration, and urostomy. Optionally, the subject has previously been treated with a therapeutic agent such as radiation therapy (*e.g.*, external beam radiation therapy, endocavitary radiation therapy, and brachytherapy), chemotherapy (*e.g.*, 5-FU, Leucovorin, Capecitabine (Xeloda™), Irinotecan (Camptosar™), and/or Oxaliplatin (Eloxitan™)), and targeted therapies (*e.g.*, Cetuximab (Erbitux ™), or Bevacizumab (Avastin™)), alone, in combination, or in succession with a surgical procedure for removing colorectal cancer. Optionally, the subject may be treated with any of the agents previously described; alone, or in combination with a surgical procedure for removing colorectal cancer and/or radiation therapy as previously described.

A subject can also include those who are suffering from, or at risk of developing colorectal cancer or a condition related to colorectal cancer, such as those who exhibit known risk factors for colorectal cancer or conditions related to colorectal cancer. Known risk factors for colorectal cancer include, but are not limited to: age (increased chance after age 50); personal history of colorectal cancer, polyps, or chronic inflammatory bowel disease; ethnic background (Jews of Eastern European descent have higher rates of colorectal cancer); a diet mostly from animal sources (high in fat); physical inactivity; obesity; smoking (30-40% increased risk for colorectal cancer); high alcohol intake; and family history of colorectal cancer, hereditary polyposis colorectal cancer, or familial adenomatous polyposis.

Selecting Constituents of a Gene Expression Panel (Precision Profile")

The general approach to selecting constituents of a Gene Expression Panel (Precision Profile™) has been described in PCT application publication number WO 01/25473, incorporated herein in its entirety. A wide range of Gene Expression Panels (Precision Profiles™) have been designed and experimentally validated, each panel providing a quantitative measure of biological condition that is derived from a sample of blood or other tissue. For each panel, experiments have verified that a Gene Expression Profile using the panel's constituents is informative of a biological condition. (It has also been demonstrated that in being informative of biological condition, the Gene Expression Profile is used, among other things, to measure the effectiveness of therapy, as well as to provide a target for therapeutic intervention).

In addition to the the Precision Profile™ for Colorectal Cancer (Table 1), the Precision Profile™ for Inflammatory Response (Table 2), the Human Cancer General Precision Profile™ (Table 3), the Precision Profile™ for EGR1 (Table 4), and the Cross-Cancer Precision Profile™ (Table 5), include relevant genes which may be selected for a given Precision Profiles™, such as the Precision Profiles™ demonstrated herein to be useful in the evaluation of colorectal cancer and conditions related to colorectal cancer.

Inflammation and Cancer

Evidence has shown that cancer in adults arises frequently in the setting of chronic inflammation. Epidemiological and experimental studies provide stong support for the concept that inflammation facilitates malignant growth. Inflammatory components have been shown to 1) induce DNA damage, which contributes to genetic instability (*e.g.*, cell mutation) and transformed cell proliferation (Balkwill and Mantovani, Lancet 357:539-545 (2001)); 2) promote angiogenesis, thereby enhancing tumor growth and invasiveness (Coussens L.M. and Z. Werb, Nature 429:860-867 (2002)); and 3) impair myelopoiesis and hemopoiesis, which cause immune dysfunction and inhibit immune surveillance (Kusmartsev and Gabrilovic, Cancer Immunol. Immunother. 51:293-298 (2002); Serafini et al., Cancer Immunol. Immunther. 53:64-72 (2004)).

Studies suggest that inflammation promotes malignancy via proinflammatory cytokines, including but not limited to IL-1β, which enhance immune suppression through the induction of myeloid suppressor cells, and that these cells down regulate immune surveillance and allow the outgrowth and proliferation of malignant cells by inhibiting the activation and/or function of tumor-specific lymphocytes. (Bunt et al., J. Immunol. 176: 284-290 (2006). Such studies are consistent with findings that myeloid suppressor cells are found in many cancer patients, including lung and breast cancer, and that chronic inflammation in some of these malignancies may enhance malignant growth (Coussens L.M. and Z. Werb, 2002).

Additionally, many cancers express an extensive repertoire of chemokines and chemokine receptors, and may be characterized by dis-regulated production of chemokines and abnormal chemokine receptor signaling and expression. Tumor-associated chemokines are thought to play several roles in the biology of primary and metastatic cancer such as: control of leukocyte infiltration into the tumor, manipulation of the tumor immune response, regulation of angiogenesis, autocrine or paracrine growth and survival factors, and control of the movement of the cancer cells. Thus, these activities likely contribute to growth within/outside the tumor microenvironment and to stimulate anti-tumor host responses.

As tumors progress, it is common to observe immune deficits not only within cells in the tumor microenvironment but also frequently in the systemic circulation. Whole blood contains representative populations of all the mature cells of the immune system as well as secretory proteins associated with cellular communications. The earliest observable changes of cellular, immune activity are altered levels of gene expression within the various immune cell types. Immune responses are now understood to be a rich, highly complex tapestry of cell-cell signaling events driven by associated pathways and cascades-all involving modified activities of gene transcription. This highly interrelated system of cell response is immediately activated upon any immune challenge, including the events surrounding host response to colorectal cancer and treatment. Modified gene expression precedes the release of cytokines and other immunologically important signaling elements.

As such, inflammation genes, such as the genes listed in the Precision Profiles™ for Inflammatory Response (Table 2) are useful for distinguishing between subjects suffering from colorectal cancer and normal subjects, in addition to the other gene panels, *i.e.*, Precision Profiles™, described herein.

Early Growth Response Gene Family and Cancer

The early growth response (EGR) genes are rapidly induced following mitogenic stimulation in diverse cell types, including fibroblasts, epithelial cells and B lymghocytes. The EGR genes are members of the broader "Immediate Early Gene" (IEG) family, whose genes are activated in the first round of response to extracellular signals such as growth factors and neurotransmitters, prior to new protein synthesis. The IEG's are well known as early regulators of cell growth and differentiation signals, in addition to playing a role in other cellular processes. Some other well characterized members of the IEG family include the c-myc, c-fos and c-jun oncogenes. Many of the immediate early gene products function as transcription factors and DNA-binding proteins, though other IEG's also include secreted proteins, cytoskeletal proteins and receptor subunits. EGR1 expression is induced by a wide variety of stimuli. It is rapidly induced by mitogens such as platelet derived growth factor (PDGF), fibroblast growth factor (FGF), and epidermal growth factor (EGF), as well as by modified lipoproteins, shear/mechanical stresses, and free radicals. Interestingly, expression of the EGR1 gene is also regulated by the oncogenes v-raf, v-fps and v-src as demonstrated in transfection analysis of cells using promoter-reporter constructs. This regulation is mediated by the serum response elements (SREs) present within the EGR1 promoter region. It has also been demonstrated that hypoxia, which occurs during development of cancers, induces EGR1 expression. EGR1 subsequently enhances the expression of endogenous EGFR, which plays an important role in cell growth (over-expression of EGFR can lead to transformation). Finally, EGR1 has also been shown to be induced by Smad3, a signaling component of the TGFB pathway.

In its role as a transcriptional regulator, the EGR1 protein binds specifically to the G+C rich EGR consensus sequence present within the promoter region of genes activated by EGR1. EGR1 also interacts with additional proteins (CREBBP/EP300) which co-regulate transcription of EGR1 activated genes. Many of the genes activated by EGR1 also stimulate the expression of EGR1, creating a positive feedback loop. Genes regulated by EGR1 include the mitogens: platelet derived growth factor (PDGFA), fibroblast growth factor (FGF), and epidermal growth factor (EGF) in addition to TNF, IL2, PLAU, ICAM1, TP53, ALOX5, PTEN, FN1 and TGFB1.

As such, early growth response genes, or genes associated therewith, such as the genes listed in the Precision Profile™ for EGR1 (Table 4) are useful for distinguishing between subjects suffering from colorectal cancer and normal subjects, in addition to the other gene panels, *i.e.*, Precision Profiles™, described herein.

In general, panels may be constructed and experimentally validated by one of ordinary skill in the art in accordance with the principles articulated in the present application.

Gene Epression Profiles Based on Gene Expression Panels of the Present Invention

Tables 1A-1C were derived from a study of the gene expression patterns described in Example 3 below. Table 1A describes all 1 and 2-gene logistic regression models based on genes from the Precision Profile™ for Colorectal Cancer (Table 1) which are capable of distinguishing between subjects suffering from colorectal cancer and normal subjects with at least 75% accuracy. For example, the first row of Table 1A, describes a 2-gene model, MSH6 and PSEN2, capable of correctly classifying colorectal cancer-afflicted subjects with 84.2% accuracy, and normal subjects with 87.5% accuracy.

Tables 2A-2C were derived from a study of the gene expression patterns described in Example 4 below. Table 2A describes all 1 and 2-gene logistic regression models based on genes from the Precision Profile™ for Inflammatory Response (Table 2), which are capable of distinguishing between subjects suffering from colorectal cancer and normal subjects with at least 75% accuracy. For example, the first row of Table 2A, describes a 2-gene model, HMOX1 and TXNRD1, capable of correctly classifying colorectal cancer-afflicted subjects with 94,4% accuracy, and normal subjects with 93.8% accuracy.

Tables 3A-3C were derived from a study of the gene expression patterns described in Example 5 below. Table 3A describes all I and 2-gene logistic regression models based on genes from the Human Cancer General Precision Profile™ (Table 3), which are capable of distinguishing between subjects suffering from colorectal cancer and normal subjects with at least 75% accuracy. For example, the first row of Table 3A, describes a 2-gene model, ATM and CDKN2A, capable of correctly classifying colorectal cancer-afflicted subjects with 91.3% accuracy, and normal subjects with 88% accuracy.

Tables 4A-4B were derived from a study of the gene expression patterns described in Example 6 below. Table 4A describes all 2-gene logistic regression models based on genes from the Precision Profile™ for EGR1 (Table 4), which are capable of distinguishing between subjects suffering from colorectal cancer and normal subjects with at least 75% accuracy. For example, the first row of Table 4A, describes a 2-gene model, NAB2 and TGFB1, capable of correctly classifying colorectal cancer-afflicted subjects with 81.8% accuracy, and normal subjects with 82% accuracy.

Tables 5A-5C were derived from a study of the gene expression patterns described in Example 7 below. Table 5A describes all 1 and 2-gene logistic regression models based on genes from the Cross-Cancer Precision Profile™ (Table 5), which are capable of distinguishing between subjects suffering from colorectal cancer and normal subjects with at least 75% accuracy. For example, the first row of Table 5A, describes a 2-gene model, AXIN2 and TNF, capable of correctly classifying colorectal cancer-afflicted subjects with 90.5% accuracy, and normal subjects with 93.9% accuracy.

Design of assays

Typically, a sample is run through a panel in replicates of three for each target gene (assay); that is, a sample is divided into aliquots and for each aliquot the concentrations of each constituent in a Gene Expression Panel (Precision Profile™) is measured. From over thousands of constituent assays, with each assay conducted in triplicate, an average coefficient of variation was found (standard deviation/average)* 100, of less than 2 percent among the normalized ΔCt measurements for each assay (where normalized quantitation of the target mRNA is determined by the difference in threshold cycles between the internal control (*e.g.*, an endogenous marker such as 18S rRNA, or an exogenous marker) and the gene of interest. This is a measure called "intra-assay variability". Assays have also been conducted on different occasions using the same sample material. This is a measure of "inter-assay variability": Preferably, the average coefficient of variation of intra- assay variability or inter-assay variability is less than 20%, more preferably less than 10%, more preferably less than 5%, more preferably less than 4%, more preferably less than 3%, more preferably less than 2%, and even more preferably less than 1%.

It has been determined that it is valuable to use the quadruplicate or triplicate test results to identify and eliminate data points that are statistical "outliers"; such data points are those that differ by a percentage greater, for example, than 3% of the average of all three or four values. Moreover, if more than one data point in a set of three or four is excluded by this procedure, then all data for the relevant constituent is discarded.

Measurement of Gene Expression for a Constituent in the Panel

For measuring the amount of a particular RNA in a sample, methods known to one of ordinary skill in the art were used to extract and quantify transcribed RNA from a sample with respect to a constituent of a Gene Expression Panel (Precision Profile™). (See detailed protocols below. Also see PCT application publication number WO 98/24935 herein incorporated by reference for RNA analysis protocols). Briefly, RNA is extracted from a sample such as any tissue, body fluid, cell (*e.g.*, circulating tumor cell) or culture medium in which a population of cells of a subject might be growing. For example, cells may be lysed and RNA eluted in a suitable solution in which to conduct a DNAse reaction. Subsequent to RNA extraction, first strand synthesis may be performed using a reverse transcriptase. Gene Amplification, more specifically quantitative PCR assays, can then be conducted and the gene of interest calibrated against an internal marker such as 18S rRNA (Hirayama et al., Blood 92, 1998: 46-52). Any other endogenous marker can be used, such as 28S-25S rRNA and 5S RNA. Samples are measured in multiple replicates, for example, 3 replicates. In an embodiment of the invention, quantitative PCR is performed using amplification, reporting agents and instruments such as those supplied commercially by Applied Biosystems (Foster City, CA). Given a defined efficiency of amplification of target transcripts, the point (*e.g.*, cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (e,g., a reference standard curve) or normalized to a standard with limited variability can be used to quantify the number of target templates in an unknown sample.

Although not limited to amplification methods, quantitative gene expression techniques may utilize amplification of the target transcript. Alternatively or in combination with amplification of the target transcript, quantitation of the reporter signal for an internal marker generated by the exponential increase of amplified product may also be used. Amplification of the target template may be accomplished by isothermic gene amplification strategies or by gene amplification by thermal cycling such as PCR.

It is desirable to obtain a definable and reproducible correlation between the amplified target or reporter signal, *i.e.*, internal marker, and the concentration of starting templates. It has been discovered that this objective can be achieved by careful attention to, for example, consistent primer-template ratios and a strict adherence to a narrow permissible level of experimental amplification efficiencies (for example 80.0 to 100% +/- 5% relative efficiency, typically 90.0 to 100% +/- 5% relative efficiency, more typically 95.0 to 100% +/- 2 %, and most typically 98 to 100% +/- 1 % relative efficiency). In determining gene expression levels with regard to a single Gene Expression Profile, it is necessary that all constituents of the panels, including endogenous controls, maintain similar amplification efficiencies, as defined herein, to permit accurate and precise relative measurements for each constituent. Amplification efficiencies are regarded as being "substantially similar", for the purposes of this description and the following claims, if they differ by no more than approximately 10%, preferably by less than approximately 5%, more preferably by less than approximately 3%, and more preferably by less than approximately 1%. Measurement conditions are regarded as being "substantially repeatable, for the purposes of this description and the following claims, if they differ by no more than approximately +/- 10% coefficient of variation (CV), preferably by less than approximately +/- 5% CV, more preferably +/- 2% CV. These constraints should be observed over the entire range of concentration levels to be measured associated with the relevant biological condition. While it is thus necessary for various embodiments herein to satisfy criteria that measurements are achieved under measurement conditions that are substantially repeatable and wherein specificity and efficiencies of amplification for all constituents are substantially similar, nevertheless, it is within the scope of the present invention as claimed herein to achieve such measurement conditions by adjusting assay results that do not satisfy these criteria directly, in such a manner as to compensate for errors, so that the criteria are satisfied after suitable adjustment of assay results.

In practice, tests are run to assure that these conditions are satisfied. For example, the design of all primer-probe sets are done in house, experimentation is performed to determine which set gives the best performance. Even though primer-probe design can be enhanced using computer techniques known in the art, and notwithstanding common practice, it has been found that experimental validation is still useful. Moreover, in the course of experimental validation, the selected primer-probe combination is associated with a set of features:

The reverse primer should be complementary to the coding DNA strand. In one embodiment, the primer should be located across an intron-exon junction, with not more than four bases of the three-prime end of the reverse primer complementary to the proximal exon. (If more than four bases are complementary, then it would tend to competitively amplify genomic DNA.)

In an embodiment of the invention, the primer probe set should amplify cDNA of less than 110 bases in length and should not amplify, or generate fluorescent signal from, genomic DNA or transcripts or cDNA from related but biologically irrelevant loci.

A suitable target of the selected primer probe is first strand cDNA, which in one embodiment may be prepared from whole blood as follows:

(a) <u>Use of whole blood for *ex vivo* assessment of a biological condition</u>

Human blood is obtained by venipuncture and prepared for assay. The aliquots of heparinized, whole blood are mixed with additional test therapeutic compounds and held at 37˚C in an atmosphere of 5% $CO_2$ for 30 minutes. Cells are lysed and nucleic acids, *e.g.*, RNA, are extracted by various standard means.

Nucleic acids, RNA and or DNA, are purified from cells, tissues or fluids of the test population of cells. RNA is preferentially obtained from the nucleic acid mix using a variety of standard procedures (or RNA Isolation Strategies, pp. 55-104, in RNA Methodologies, A laboratory guide for isolation and characterization, 2nd edition, 1998, Robert E. Farrell, Jr., Ed., Academic Press), in the present using a filter-based RNA isolation system from Ambion (RNAqueous ™, Phenol-free Total RNA Isolation Kit, Catalog #1912, version 9908; Austin, Texas).

(b) <u>Amplification strategies.</u>

Specific RNA are amplified using message specific primers or random primers. The specific primers are synthesized from data obtained from public databases (*e.g.*, Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples (see, for example, RT PCR, Chapter 15 in RNA Methodologies. A Laboratory Guide for Isolation and Characterization, 2nd edition, 1998, Robert E. Farrell, Jr., Ed., Academic Press; or Chapter 22 pap.143-151, RNA Isolation and Characterization Protocols, Methods in Molecular Biology, Volume 86, 1998, R. Rapley and D. L. Manning Eds., Human Press, or Chapter 14 Statistical refinement of primer design parameters; or Chapter 5, pp.55-72, PCR Applications: protocols for functional genomics, M.A.Innis, D.H. Gelfand and J,J. Sninsky, Eds., 1999, Academic Press). Amplifications are carried out in either isothermic conditions or using a thermal cycler (for example, a ABI 9600 or 9700 or 7900 obtained from Applied Biosystems, Foster City, CA; see Nucleic acid detection methods, pp. 1-24, in Molecular Methods for Virus Detection, D.L.Wiedbrauk and D.H., Farkas, Eds., 1995, Academic Press). Amplified nucleic acids are detected using fluorescent-tagged detection oligonucleotide probes (see, for example, TaqmanTMPCR Reagent Kit, Protocol, part number 402823, Revision A, 1996, Applied Biosystems, Foster City CA) that are identified and synthesized from publicly known databases as described for the amplification primers.

For example, without limitations, amplified cDNA is detected and quantified using detection systems such as the ABI Prism®7900 Sequence Detection System (Applied Biosystems (Foster City, CA)), the Cepheid SmartCycler® and Cepheid GeneXpert® Systems, the Fluidigm BioMark™ System, and the Roche LightCycler® 480 Real-Time PCR System. Amounts of "specific RNAs contained in the test sample can be related to the relative quantity of fluorescence observed (see for example, Advances in Quantitative PCR Technology: 5' Nuclease Assays, Y.S. Lie and C.J. Petropolus, Current Opinion in Biotechnology, 1998, 9:43-48, or Rapid Thermal Cycling and PCR Kinetics, pp. 211-229, chapter 14 in PCR applications: protocols for functional genomics, M.A. Innis, D.H. Gelfand and J.J. Sninsky, Eds., 1999, Academic Press). Examples of the procedure used with several of the above-mentioned detection systems are described below. In some embodiments, these procedures can be used for both whole blood RNA and RNA extracted from cultured cells (*e.g.*, without limitation, CTCs, and CECs). In some embodiments, any tissue, body fluid, or cell(s) (*e.g.*, circulating tumor cells (CTCs) or circulating endothelial cells (CECs)) may be used for *ex vivo* assessment of a biological condition affected by an agent. Methods herein may also be applied using proteins where sensitive quantitative techniques, such as an Enzyme Linked ImmunoSorbent Assay (ELISA) or mass spectroscopy, are available and well-known in the art for measuring the amount of a protein constituent (see WO 98/24935 herein incorporated by reference).

An example of a procedure for the synthesis of first strand cDNA for use in PCR amplification is as follows:

<u>Materials</u>

1. Applied Biosystems TAQMAN Reverse Transcription Reagents Kit (P/N 808-0234). Kit Components: 10X TaqMan RT Buffer, 25 mM Magnesium chloride, deoxyNTPs mixture, Random Hexamers, RNase Inhibitor, MultiScribe Reverse Transcriptase (50 U/mL) (2) RNase / DNase free water (DEPC Treated Water from Ambion (P/N 9915G), or equivalent).

<u>Methods</u>

1. Place RNase Inhibitor and MuttiScribe Reverse Transcriptase on ice immediately. All other reagents can be thawed at room temperature and then placed on ice.
2. Remove RNA samples from -80oC freezer and thaw at room temperature and then place immediately on ice.
3. Prepare the following cocktail of Reverse Transcriptase Reagents for each 100 mL RT reaction (for multiple samples, prepare extra cocktail to allow for pipetting error):

| 1 reaction (mL) | | 11X, *e.g.* 10 samples (μL) |
|---|---|---|
| 10X RT Buffer | 10.0 | 110.0 |
| 25 mM MgCl$_2$ | 22.0 | 242.0 |
| dNTPs | 20.0 | 220.0 |
| Random Hexamers | 5.0 | 55.0 |
| RNAse Inhibitor | 2.0 | 22.0 |
| Reverse Transcriptase | 2.5 | 27.5 |
| Water | 18.5 | 203.5 |
| Total: | 80.0 | 880.0 (80 μL per sample) |

4. Bring each RNA sample to a total volume of 20 μL, in a 1.5 mL microcentrifuge tube (for example, remove 10 μL RNA and dilute to 20 μL with RNase / DNase free water, for whole blood RNA use 20 μL total RNA) and add 80 μL RT reaction mix from step 5,2,3. Mix by pipetting up and down.
5. Incubate sample at room temperature for 10 minutes.
6. Incubate sample at 37˚C for 1 hour.
7. Incubate sample at 90˚C for 10 minutes.
8. Quick spin samples in microcentrifuge.
9. Place sample on ice if doing PCR immediately, otherwise store sample at -20˚C for future use.
10. PCR QC should be run on all RT samples using 18S and β-actin.

Following the synthesis of first strand DNA, one particular embodiment of the approach for amplification of first strand cDNA by PCR, followed by detection and quantification of constituents of a Gene Expression Panel (Precision Profile™) is performed using the ABI Prism® 7900 Sequence Detection System as follows:

Materials

1. 20X Primer/Probe Mix for each gene of interest.
2. 20X Primer/Probe Mix for 18S endogenous control.
3. 2X Taqman Universal PCR Master Mix.
4. cDNA transcribed from RNA extracted from cells.
5. Applied Biosystems 96-Well Optical Reaction Plates.
6. Applied Biosystems Optical Caps, or optical-clear film.
7. Applied Biosystem Prism® 7700 or 7900 Sequence Detector.

Methods

1. Make stocks of each Primer/Probe mix containing the Primer/Probe for the gene of interest, Primer/Probe for 18S endogenous control, and 2X PCR Master Mix as follows. Make sufficient excess to allow for pipetting error *e.g.*, approximately 10% excess. The following example illustrates a typical set up for one gene with quadruplicate samples testing two conditions (2 plates).

| | 1X (1 well) (μL) |
|---|---|
| 2X Master Mix | 7.5 |
| 20X 18S Primer/Probe Mix | 0.75 |
| 20X Gene of interest Primer/Probe Mix | 0.75 |
| Total | 9.0 |

2. Make stocks of cDNA targets by diluting 95μL of DNA into 2000μL of water. The amount of cDNA is adjusted to give Ct values between 10 and 18, typically between 12 and 16.
3. Pipette 9 μL of Primer/Probe mix into the appropriate wells of an Applied Biosystems 384-Well Optical Reaction Plate.
4. Pipette 10μL of cDNA stock solution into each well of the Applied Biosystems 384-Well Optical Reaction Plate.

5. Seal the plate with Applied Biosystems Optical Caps, or optical-clear film.

6. Analyze the plate on the ABI Prism®7900 Sequence Detector.

In another embodiment of the invention, the use of the primer probe with the first strand cDNA as described above to permit measurement of constituents of a Gene Expression Panel (Precision Profile™) is performed using a QPCR assay on Cepheid SmartCycler® and GeneXpert® Instruments as follows:

I. To run a QPCR assay in duplicate on the Cepheid SmartCycler® instrument containing three target genes and one reference gene, the following procedure should be followed.

A. With 20X Primer/Probe Stocks.

<u>Materials</u>

1. SrnartMix™-HM lyophilized Master Mix.
2. Molecular grade water.
3. 20X Primer/Probe Mix for the 18S endogenous control gene. The endogenous control gene will be dual labeled with VIC-MGB or equivalent.
4. 20X Primer/Probe Mix for each for target gene one, dual labeled with FAM-BHQ1 or equivalent.
5. 20X Primer/Probe Mix for each for target gene two, dual labeled with Texas Red-BHQ2 or equivalent.
6. 20X Primer/Probe Mix for each for target gene three, dual labeled with Alexa 647-BHQ3 or equivalent.
7. Tris buffer, pH 9.0
8. cDNA transcribed from RNA extracted from sample.
9. SmartCycler® 25 $\mu$L tube.
10. Cepheid SmartCycler® instrument.

<u>Methods</u>

1. For each cDNA sample to be investigated, add the following to a sterile 650 $\mu$L tube.

| | |
|---|---|
| SmartMix™-HM lyophilized Master Mix | 1 bead |
| 20X 18S Primer/Probe Mix | 2.5 $\mu$L |
| 20X Target Gene 1 Primer/Probe Mix | 2.5 $\mu$L |
| 20X Target Gene 2 Primer/Probe Mix | 2.5 $\mu$L |
| 20X Target Gene 3 Primer/Probe Mix | 2.5 $\mu$L |
| Tris Buffer, pH 9.0 | 2.5 $\mu$L |
| Sterile Water | 34.5 $\mu$L |
| Total | 47 $\mu$L |

Vortex the mixture for 1 second three times to completely mix the reagents. Briefly centrifuge the tube after vortexing.

2. Dilute the DNA sample so that a 3 $\mu$L addition to the reagent mixture above will give an 18S reference gene CT value between 12 and 16.

3. Add 3 $\mu$L of the prepared cDNA sample to the reagent mixture bringing the total volume to 50 $\mu$L. Vortex the mixture for 1 second three times to completely mix the reagents. Briefly centrifuge the tube after vortexing.

4. Add 25 $\mu$L of the mixture to each of two SmartCycer® tubes, cap the tube and spin for 5 seconds in a microcentrifuge having an adapter for SmartCycler® tubes.

5. Remove the two SmartCycler® tubes from the microcentrifuge and inspect for air bubbles. If bubbles are present, re-spin, otherwise, load the tubes into the SmartCycler® instrument.

6. Run the appropriate QPCR protocol on the SmartCycler®, export the data and analyze the results.

B. With Lyophilized SmartBeads™.

<u>Materials</u>

1. SmartMix™-HM lyophilized Master Mix.
2. Molecular grade water.
3. SmartBeads™ containing the 18S endogenous control gene dual labeled with VIC-MGB or equivalent, and the three target genes, one dual labeled with FAM-BHQ1 or equivalent, one dual labeled with Texas Red-BHQ2 or equivalent and one dual labeled with Alexa 647-BHQ3 or equivalent.
4. Tris buffer, pH 9.0
5. cDNA transcribed from RNA extracted from sample.
6. SmartCycler 25 μL tube.
7. Cepheid SmartCycler® instrument.

Methods

1. For each cDNA sample to be investigated, add the following to a sterile 650 μL tube.

| | |
|---|---|
| SmartMix™-HM lyophilized Master Mix | 1 bead |
| SmartBead™ containing four primer/probe sets | 1 bead |
| Tris Buffer, pH 9.0 | 2.5 μL |
| Sterile Water | 44.5 μL |
| Total | 47 μL |

Vortex the mixture for 1 second three times to completely mix the reagents, Briefly centrifuge the tube after vortexing.
2. Dilute the cDNA sample so that a 3 μL addition to the reagent mixture above will give an 18S reference gene CT value between 12 and 16.
3. Add 3 μL of the prepared cDNA sample to the reagent mixture bringing the total volume to 50 μL. Vortex the mixture for I second three times to completely mix the reagents. Briefly centrifuge the tube after vortexing.
4. Add 25 μL of the mixture to each of two SmartCycler® tubes, cap the tube and spin for 5 seconds in a microcentrifuge having an adapter for SmartCycler® tubes.
5. Remove the two SmartCycler®tubes from the microcentrifuge and inspect for air bubbles. If bubbles are present, re-spin, otherwise, load the tubes into the SmartCycler® instrument.
6. Run the appropriate QPCR protocol on the SmartCycler®, export the data and analyze the results.

II. To run a QPCR assay on the Cepheid GeneXpert® instrument containing three target genes and one reference gene, the following procedure should be followed. Note that to do duplicates, two self contained cartridges need to be loaded and run on the GeneXpert® instrument.

Materials

1. Cepheid GeneXpert® self contained cartridge preloaded with a lyophilized SmartMix™-HM master mix bead and a lyophilized SmartBead™ containing four primer-probe sets.
2. Molecular grade water, containing Tris buffer, pH 9.0.
3. Extraction and purification reagents.
4. Clinical sample (whole blood, RNA, etc.)
5. Cepheid GeneXpert® instrument.

Methods

1. Remove appropriate GeneXpert® self contained cartridge from packaging.
2. Fill appropriate chamber of self contained cartridge with molecular grade water with Tris buffer, pH 9.0.
3. Fill appropriate chambers of self contained cartridge with extraction and purification reagents.
4. Load aliquot of clinical sample into appropriate chamber of self contained cartridge.
5. Seal cartridge and load into GeneXpert® instrument.
6. Run the appropriate extraction and amplification protocol on the GeneXpert® and analyze the resultant data.

In yet another embodiment of the invention, the use of the primer probe with the first strand cDNA as described above to permit measurement of constituents of a Gene Expression Panel (Precision Profile™) is performed using a QPCR assay on the Roche LightCycler® 480 Real-Time PCR System as follows:

Materials

1. 20X Primer/Probe stock for the 18S endogenous control gene. The endogenous control gene may be dual labeled with either VIC-MGB or VIC-TAMRA.
2. 20X Primer/Probe stock for each target gene, dual labeled with either FAM-TAMRA or FAM-BHQ1.
3. 2X LightCycler® 490 Probes Master (master mix).
4. 1X cDNA sample stocks transcribed from RNA extracted from samples.
5. 1X TE buffer, pH 8.0.
6. LightCycler® 480 384-well plates.
7. Source MDx 24 gene Precision Profile™ 96-well intermediate plates.
8. RNase/DNase free 96-well plate.
9. 1.5 mL microcentrifuge tubes.
10. Beckman/Coulter Biomek® 3000 Laboratory Automation Workstation.
11. Velocityl Bravo™ Liquid Handling Platform.
12. LightCycler® 480 Real-Time PCR System.

Methods

1. Remove a Source MDx 24 gene Precision Profile™ 96-well intermediate plate from the freezer, thaw and spin in a plate centrifuge.
2. Dilute four (4) 1X cDNA sample stocks in separate 1.5 mL microcentrifuge tubes with the total final volume for each of 540 $\mu$L.
3. Transfer the 4 diluted cDNA samples to an empty RNase/DNase free 96-well plate using the Biomek® 3000 Laboratory Automation Workstation.
4. Transfer the cDNA samples from the cDNA plate created in step 3 to the thawed and centrifuged Source MDx 24 gene Precision Profile™ 96-well intermediate plate using Biomek® 3000 Laboratory Automation.Workstation. Seal the plate with a foil seal and spin in a plate centrifuge.
5. Transfer the contents of the cDNA-loaded Source MDx 24 gene Precision Profile™ 96-well intermediate plate to a new LightCycter® 480 384-well plate using the Bravo™ Liquid Handling Platform. Seal the 384-well plate with a LightCycler® 480 optical sealing foil and spin in a plate centrifuge for 1 minute at 2000 rpm.
6. Place the sealed in a dark 4°C refrigerator for a minimum of 4 minutes.
7. Load the plate into the LightCycler® 480 Real-Time PCR System and start the LightCycler® 480 software. Chose the appropriate run parameters and start the run.
8. At the conclusion of the run, analyze the data and export the resulting CP values to the database.

In some instances, target gene FAM measurements may be beyond the detection limit of the particular platform instrument used to detect and quantify constituents of a Gene Expression Panel (Precision Profile™). To address the issue of "undetermined" gene expression measures as lack of expression for a particular gene, the detection limit may be reset and the "undetermined" constituents may be "flagged". For example without limitation, the ABI Prism® 7900HT Sequence Detection System reports target gene FAM measurements that are beyond the detection limit of the instrument (>40 cycles) as "undetermined". Detection Limit Reset is performed when at least 1 of 3 target gene FAM $C_T$ replicates are not detected after 40 cycles and are designated as "undetermined". "Undetermined" target gene FAM $C_T$ replicates are re-set to 40 and flagged. $C_T$ normalization ($\Delta C_T$) and relative expression calculations that have used re-set FAM $C_T$ values are also flagged.

Baseline profile data sets

The analyses of samples from single individuals and from large groups of individuals provide a library of profile data sets relating to a particular panel or series of panels. These profile data sets may be stored as records in a library for use as baseline profile data sets. As the term "baseline" suggests, the stored baseline profile data sets serve as comparators for providing a calibrated profile data set that is informative about a biological condition or agent. Baseline profile data sets may be stored in libraries and classified in a number of cross-referential ways. One form of classification may rely on the characteristics of the panels from which the data sets are derived. Another form of classification may be by particular biological condition, *e.g.*, colorectal cancer. The concept of a biological condition encompasses any state in which a cell or population of cells may be found at any one time. This state may reflect geography of samples, sex of subjects or any other discriminator. Some of the discriminators may overlap. The libraries may also be accessed for records associated with a single subject or particular clinical trial. The classification of baseline profile data sets may further be annotated with medical information about a particular subject, a medical condition, and/or a particular agent.

The choice of a baseline profile data set for creating a calibrated profile data set is related to the biological condition to be evaluated, monitored, or predicted, as well as, the intended use of the calibrated panel, *e.g.*, as to monitor drug development, quality control or other uses. It may be desirable to access baseline profile data sets from the same subject for whom a first profile data set is obtained or from different subject at varying times, exposures to stimuli, drugs or complex compounds; or may be derived from like or dissimilar populations or sets of subjects. The baseline profile data set may be normal, healthy baseline.

The profile data set may arise from the same subject for which the first data set is obtained, where the sample is taken at a separate or similar time, a different or similar site or in a different or similar biological condition. For example, a sample may be taken before stimulation or after stimulation with an exogenous compound or substance, such as before or after therapeutic treatment. Alternatively the sample is taken before or include before or after a surgical procedure for colorectal cancer. The profile data set obtained from the unstimulated sample may serve as a baseline profile data set for the sample taken after stimulation. The baseline data set may also be derived from a library containing profile data sets of a population or set of subjects having some defining characteristic or biological condition. The baseline profile data set may also correspond to some *ex vivo* or *in vitro* properties associated with an *in vitro* cell culture. The resultant calibrated profile data sets may then be stored as a record in a database or library along with or separate from the baseline profile data base and optionally the first profile data s*et al*.though the first profile data set would normally become incorporated into a baseline profile data set under suitable classification criteria. The remarkable consistency of Gene Expression Profiles associated with a given biological condition makes it valuable to store profile data, which can be used, among other things for normative reference purposes. The normative reference can serve to indicate the degree to which a subject conforms to a given biological condition (healthy or diseased) and, alternatively or in addition, to provide a target for. clinical intervention.

Calibrated data

Given the repeatability achieved in measurement of gene expression, described above in connection with "Gene Expression Panels" (Precision Profiles™) and "gene amplification", it was concluded that where differences occur in measurement under such conditions, the differences are attributable to differences in biological condition. Thus, it has been found that calibrated profile data sets are highly reproducible in samples taken from the same individual under the same conditions. Similarly, it has been found that calibrated profile data sets are reproducible in samples that are repeatedly tested. Also found have been repeated instances wherein calibrated profile data sets obtained when samples from a subject are exposed *ex vivo* to a compound are comparable to calibrated profile data from a sample that has been exposed to a sample *in vivo.*

Calculation of calibrated profile data sets and computational aids

The calibrated profile data set may be expressed in a spreadsheet or represented graphically for example, in a bar chart or tabular form but may also be expressed in a three dimensional representation. The function relating the baseline and profile data may be a ratio expressed as a logarithm. The constituent may be itemized on the x-axis and the logarithmic scale may be on the y-axis. Members of a calibrated data set may be expressed as a positive value representing a relative enhancement of gene expression or as a negative value representing a relative reduction in gene expression with respect to the baseline.

Each member of the calibrated profile data set should be reproducible within a range with respect to similar samples taken from the subject under similar conditions. For example, the calibrated profile data sets may be reproducible within 20%, and typically within 10%. In accordance with embodiments of the invention, a pattern of increasing, decreasing and no change in relative gene expression from each of a plurality of gene loci examined in the Gene Expression Panel (Precision Profile™) may be used to prepare a calibrated profile set that is informative with regards to a biological condition, biological efficacy of an agent treatment conditions or for comparison to populations or sets of subjects or samples, or for comparison to populations of cells. Patterns of this nature may be used to identify likely candidates for a drug trial, used alone or in,combination with other clinical indicators to be diagnostic or prognostic with respect to a biological condition or may be used to guide the development of a pharmaceutical or nutraceutical through manufacture, testing and marketing.

The numerical data obtained from quantitative gene expression and numerical data from calibrated gene expression relative to a baseline profile data set may be stored in databases or digital storage mediums and may be retrieved for purposes including managing patient health care or for conducting clinical trials or for characterizing a drug. The data may be transferred in physical or wireless networks via the World Wide Web, email, or internet access site for example or by hard copy so as to be collected and pooled from distant geographic sites.

The method also includes producing a calibrated profile data set for the panel, wherein each member of the calibrated profile data set is a function of a corresponding member of the first profile data set and a corresponding member of a baseline profile data set for the panel, and wherein the baseline profile data set is related to the colorectal cancer or conditions related to colorectal cancer to be evaluated, with the calibrated profile data set being a comparison between the first profile data set and the baseline profile data set, thereby providing evaluation of colorectal cancer or conditions related to colorectal cancer of the subject.

In yet other embodiments, the function is a mathematical function and is other than a simple difference, including a second function of the ratio of the corresponding member of first profile data set to the corresponding member of the baseline profile data set, or a logarithmic function. In such embodiments, the first sample is obtained and the first profile data set quantified at a first location, and the calibrated profile data set is produced using a network to access a database stored on a digital storage medium in a second location, wherein the database may be updated to reflect the first profile data set quantified from the sample. Additionally, using a network may include accessing a global computer network.

In an embodiment of the present invention, a descriptive record is stored in a single database or multiple databases where the stored data includes the raw gene expression data (first profile data set) prior to transformation by use of a baseline profile data set, as well as a record of the baseline profile data set used to generate the calibrated profile data set including for example, annotations regarding whether the baseline profile data set is derived from a particular Signature Panel and any other annotation that facilitates interpretation and use of the data.

Because the data is in a universal format, data handling may readily be done with a computer. The data is organized so as to provide an output optionally corresponding to a graphical representation of a calibrated data set.

The above described data storage on a computer may provide the information in a form that can be accessed by a user. Accordingly, the user may load the information onto a second access site including downloading the information. However, access may be restricted to users having a password or other security device so as to protect the medical records contained within. A feature of this embodiment of the invention is the ability of a user to add new or annotated records to the data set so the records become part of the biological information.

The graphical representation of calibrated profile data sets pertaining to a product such as a drug provides an opportunity for standardizing a product by means of the calibrated profile, more particularly a signature profile. The profile may be used as a feature with which to demonstrate relative efficacy, differences in mechanisms of actions, etc, compared to other drugs approved for similar or different uses.

The various embodiments of the invention may be also implemented as a computer program product for use with a computer system. The product may include program code for deriving a first profile data set and for producing calibrated profiles. Such implementation may include a series of computer instructions fixed either on a tangible medium, such as a computer readable medium (for example, a diskette, CD-ROM, ROM, or fixed disk), or transmittable to a computer system via a modem or other interface device, such as a communications adapter coupled to a network. The network coupling may be for example, over optical or wired communications lines or via wireless techniques (for example, microwave, infrared or other transmission techniques) or some combination of these. The series of computer instructions preferably embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (for example, shrink wrapped software), preloaded with a computer system (for example, on system ROM or fixed disk), or distributed from a server or electronic bulletin board over a network (for example, the Internet or World Wide Web). In addition, a computer system is further provided including derivative modules for deriving a first data set and a calibration profile data set.

The calibration profile data sets in graphical or tabular form, the associated databases, and the calculated index or derived algorithm, together with information extracted from the panels, the databases, the data sets or the indices or algorithms are commodities that can be sold together or separately for a variety of purposes as described in WO 01/25473.

In other embodiments, a clinical indicator may be used to assess the colorectal cancer or conditions related to colorectal cancer of the relevant set of subjects by interpreting the calibrated profile data set in the context of at least one other clinical indicator, wherein the at least one other clinical indicator is selected from the group consisting of blood chemistry, X-ray or other radiological or metabolic imaging technique, molecular markers in the blood (*e.g.*, carcinoembryonic antigen, CA19-9), other chemical assays, and physical findings.

Index construction

In combination, (i) the remarkable consistency of Gene Expression Profiles with respect to a biological condition across a population or set of subject or samples, or across a population of cells and (ii) the use of procedures that provide substantially reproducible measurement of constituents in a Gene Expression Panel (Precision Profile™) giving rise to a Gene Expression Profile, under measurement conditions wherein specificity and efficiencies of amplification for all constituents of the panel are substantially similar, make possible the use of an index that characterizes a Gene Expression Profile, and which therefore provides a measurement of a biological condition.

An index may be constructed using an index function that maps values in a Gene Expression Profile into a single value that is pertinent to the biological condition at hand. The values in a Gene Expression Profile are the amounts of each constituent of the Gene Expression Panel (Precision Profile™). These constituent amounts form a profile data set, and the index function generates a single value-the index- from the members of the profile data set.

The index function may conveniently be constructed as a linear sum of terms, each term being what is referred to herein as a "contribution function" of a member of the profile data set. For example, the contribution function may be a constant times a power of a member of the profile data set. So the index function would have the form

$$I = \sum CiMi^{P(i)},$$

where I is the index, Mi is the value of the member i of the profile data set, $Ci$ is a constant, and P(i) is a power to which Mi is raised, the sum being formed for all integral values of i up to the number of members in the data set. We thus have a linear polynomial expression. The role of the coefficient Ci for a particular gene expression specifies whether a higher $\Delta$Ct value for this gene either increases (a positive Ci) or decreases (a lower value) the likelihood of colorectal cancer, the $\Delta$Ct values of all other genes in the expression being held constant.

The values $Ci$ and P(i) may be determined in a number of ways, so that the index I is informative of the pertinent biological condition. One way is to apply statistical techniques, such as latent class modeling, to the profile data sets to correlate clinical data or experimentally derived data, or other data pertinent to the biological condition. In this connection, for example, may be employed the software from Statistical Innovations, Belmont, Massachusetts, called Latent Gold®. Alternatively, other simpler modeling techniques may be employed in a manner known in the art. The index function for colorectal cancer may be constructed, for example, in a manner that a greater degree of colorectal cancer (as determined by the profile data set for the any of the Precision Profiles™ (listed in Tables 1-5) described herein) correlates with a large value of the index function.

Just as a baseline profile data set, discussed above, can be used to provide an appropriate normative reference, and can even be used to create a Calibrated profile data set, as discussed above, based on the normative reference, an index that characterizes a Gene Expression Profile can also be provided with a normative value of the index function used to create the index. This normative value can be determined with respect to a relevant population or set of subjects or samples or to a relevant population of cells, so that the index may be interpreted in relation to the normative value. The relevant population or set of subjects or samples, or relevant population of cells may have in common a property that is at least one of age range, gender, ethnicity, geographic location, nutritional history, medical condition, clinical indicator, medication, physical activity, body mass, and environmental exposure.

As an example, the index can be constructed, in relation to a normative Gene Expression Profile for a population or set of healthy subjects, in such a way that a reading of approximately 1 characterizes normative Gene Expression Profiles of healthy subjects. Let us further assume that the biological condition that is the subject of the index is colorectal cancer; a reading of 1 in this example thus corresponds to a Gene Expression Profile that matches the norm for healthy subjects. A substantially higher reading then may identify a subject experiencing colorectal cancer, or a condition related to colorectal cancer. The use of 1 as identifying a normative value, however, is only one possible choice; another logical choice is to use 0 as identifying the normative value. With this choice, deviations in the index from zero can be indicated in standard deviation units (so that values lying between -1 and +1 encompass 90% of a normally distributed reference population or set of subjects. Since it was determined that Gene Expression Profile values (and accordingly constructed indices based on them) tend to be normally distributed, the 0-centered index constructed in this manner is highly informative, It therefore facilitates use of the index in diagnosis of disease and setting objectives for treatment.

Still another embodiment is a method of providing an index pertinent to colorectal cancer or conditions related to colorectal cancer of a subject based on a first sample from the subject, the first sample providing a source of RNAs, the method comprising deriving from the first sample a profile data set, the profile data set including a plurality of members, each member being a quantitative measure of the amount of a distinct RNA constituent in a panel of constituents selected so that measurement of the constituents is indicative of the presumptive signs of colorectal cancer, the panel including at least one of any of the genes listed in the Precision Profiles (listed in Tables 1-5). In deriving the profile data set, such measure for each constituent is achieved under measurement conditions that are substantially repeatable, at least one measure from the profile data set is applied to an index function that provides a mapping from at least one measure of the profile data set into one measure of the presumptive signs of colorectal cancer, so as to produce an index pertinent to the colorectal cancer or conditions related to colorectal cancer of the subject.

As another embodiment of the invention, an index function I of the form

$$I = C_0 + \Sigma\ C_i M_{1i}{}^{P1(i)}\ M_{2i}{}^{P2(i)},$$

can be employed, where $M_1$ and $M_2$ are values of the member i of the profile data set, $C_i$ is a constant determined without reference to the profile data set, and P1 and P2 are powers to which $M_1$ and $M_2$ are raised. The role of P1(i) and P2(i) is to specify the specific functional form of the quadratic expression, whether in fact the equation is linear, quadratic, contains cross-product terms, or is constant. For example, when P1 = P2 = 0, the index function is simply the sum of constants; when P1 = 1 and P2 = 0, the index function is a linear expression; when P1 = P2 =1, the index function is a quadratic expression.

The constant $C_0$ serves to calibrate this expression to the biological population of interest that is characterized by having colorectal cancer. In this embodiment, when the index value equals 0, the odds are 50:50 of the subject having colorectal cancer vs a normal subject. More generally, the predicted odds of the subject having colorectal cancer is [exp $(I_i)$], and therefore the predicted probability of having colorectal cancer is [exp$(I_i)$]/[1+exp$((I_i)$)]. Thus; when the index exceeds 0, the predicted probability that a subject has colorectal cancer is higher than 0.5, and when it falls below 0, the predicted probability is less than 0.5.

The value of $C_0$ may be adjusted to reflect the prior probability of being in this population based on known exogenous risk factors for the subject. In an embodiment where $C_0$ is adjusted as a function of the subject's risk factors, where the subject has prior probability $p_i$ of having colorectal cancer based on such risk factors, the adjustment is made by increasing (decreasing) the unadjusted $C_0$ value by adding to $C_0$ the natural logarithm of the following ratio: the prior odds of having colorectal cancer taking into account the risk factors/the overall prior odds of having colorectal cancer *without* taking into account the risk factors.

Performance and Accuracy Measures of the Invention

The performance and thus absolute and relative clinical usefulness of the invention may be assessed in multiple ways as noted above. Amongst the various assessments of performance, the invention is intended to provide accuracy in clinical diagnosis and prognosis. The accuracy of a diagnostic or prognostic test, assay, or method concerns the ability of the test, assay, or method to distinguish between subjects having colorectal cancer is based on whether the subjects have an "effective amount" or a "significant, alteration" in the levels of a cancer associated gene. By "effective amounts" or "significant alteration", it is meant that the measurement of an appropriate number of cancer associated gene (which may be one or more) is different than the predetermined cut-off point (or threshold value) for that cancer associated gene and therefore indicates that the subject has colorectal cancer for which the cancer associated gene(s) is a determinant.

The difference in the level of cancer associated gene(s) between normal and abnormal is preferably statistically significant. As noted below, and without any limitation of the invention, achieving statistical significance, and thus the preferred analytical and clinical accuracy, generally but not always requires that combinations of several cancer associated gene(s) be used together in panels and combined with mathematical algorithms in order to achieve a statistically significant cancer associated gene index.

In the categorical diagnosis of a disease state, changing the cut point or threshold value of a test (or assay) usually changes the sensitivity and specificity, but in a qualitatively inverse relationship. Therefore, in assessing the accuracy and usefulness of a proposed medical test, assay, or method for assessing a subject's condition, one should always take both sensitivity and specificity into account and be mindful of what the cut point is at which the sensitivity and specificity are being reported because sensitivity and specificity may vary significantly over the range of cut points. Use of statistics such as AUC, encompassing all potential cut point values, is preferred for most categorical risk measures using the invention, while for continuous risk measures, statistics of goodness-of-fit and calibration to observed results or other gold standards, are preferred.

Using such statistics, an "acceptable degree of diagnostic accuracy", is herein defined as a test or assay (such as the test of the invention for determining an effective amount or a significant alteration of cancer associated gene(s), which thereby indicates the presence of a colorectal cancer in which the AUC (area under the ROC curve for the test or assay) is at least 0.60, desirably at least 0.65, more desirably at least 0.70, preferably at least 0.75, more preferably at least 0.80, and most preferably at least 0.85.

By a "very high degree of diagnostic accuracy", it is meant a test or assay in which the AUC (area under the ROC curve for the test or assay) is at least 0.75, desirably at least 0.775, more desirably at least 0.800, preferably at least 0.825, more preferably at least 0.850, and most preferably at least 0.875.

The predictive value of any test depends on the sensitivity and specificity of the test, and on the prevalence of the condition in the population being tested. This notion, based on Bayes' theorem, provides that the greater the likelihood that the condition being screened for is present in an individual or in the population (pre-test probability), the greater the validity of a positive test and the greater the likelihood that the result is a true positive. Thus, the problem with using a test in any population where there is a low likelihood of the condition being present is that a positive result has limited value (*i.e.*, more likely to be a false positive). Similarly, in populations at very high risk, a negative test result is more likely to be a false negative.

As a result, ROC and AUC can be misleading as to the clinical utility of a test in low disease prevalence tested populations (defined as those with less than 1% rate of occurrences (incidence) per annum, or less than 10% cumulative prevalence over a specified time horizon). Alternatively, absolute risk and relative risk ratios as defined elsewhere in this disclosure can be employed to determine the degree of clinical utility. Populations of subjects to be tested can also be categorized into quartiles by the test's measurement values, where the top quartile (25% of the population) comprises the group of subjects with the highest relative risk for developing colorectal cancer, and the bottom quartile comprising the group of subjects having the lowest relative risk for developing colorectal cancer. Generally, values derived from tests or assays having over 2.5 times the relative risk from top to bottom quartile in a low prevalence population are considered to have a "high degree of diagnostic accuracy," and those with five to seven times the relative risk for each quartile are considered to have a "very high degree of diagnostic accuracy." Nonetheless, values derived from tests or assays having only 1.2 to 2.5 times the relative risk for each quartile remain clinically useful are widely used as risk factors for a disease. Often such lower diagnostic accuracy tests must be combined with additional parameters in order to derive meaningful clinical thresholds for therapeutic intervention, as is done with the aforementioned global risk assessment indices.

A health economic utility function is yet another means of measuring the performance and clinical value of a given test, consisting of weighting the potential categorical test outcomes based on actual measures of clinical and economic value for each. Health economic performance is closely related to accuracy, as a health economic utility function specifically assigns an economic value for the benefits of correct classification and the costs of misclassification of tested subjects. As a performance measure, it is not unusual to require a test to achieve a level of performance which results in an increase in health economic value per test (prior to testing costs) in excess of the target price of the test.

In general, alternative methods of determining diagnostic accuracy are commonly used for continuous measures, when a disease category or risk category (such as those at risk for having a bone fracture) has not yet been clearly defined by the relevant medical societies and practice of medicine, where thresholds for therapeutic use are not yet established, or where there is no existing gold standard for diagnosis of the pre-disease. For continuous measures of risk, measures of diagnostic accuracy for a calculated index are typically based on curve fit, and calibration between the predicted continuous value and the actual observed values (or a historical index calculated value) and utilize measures such as R squared, Hosmer-Lemeshow *P*-value statistics and confidence intervals. It is not unusual for predicted values using such algorithms to be reported including a confidence interval (usually 90% or 95% CI) based on a historical observed cohort's predictions, as in the test for risk of future breast cancer recurrence commercialized by Genomic Health, Inc. (Redwood City, California).

In general, by defining the degree of diagnostic accuracy, *i.e.*, cut points on a ROC curve, defining an acceptable AUC value, and determining the acceptable ranges in relative concentration of what constitutes an effective amount of the cancer associated gene(s) of the invention allows for one of skill in the art to use the cancer associated gene(s) to identify, diagnose, or prognose subjects with a pre-determined level of predictability and performance.

Results from the cancer associated gene(s) indices thus derived can then be validated through their calibration with actual results, that is, by comparing the predicted versus observed rate of disease in a given population, and the best predictive cancer associated gene(s) selected for and optimized through mathematical models of increased complexity. Many such formula may be used; beyond the simple non-linear transformations, such as logistic regression, of particular interest in this use of the present invention are structural and synactic classification algorithms, and methods of risk index construction, utilizing pattern recognition features, including established techniques such as the Kth-Nearest Neighbor, Boosting, Decision Trees, Neural Networks, Bayesian Networks, Support Vector Machines, and Hidden Markov Models, as well as other formula described herein.

Furthermore, the application of such techniques to panels of multiple cancer associated gene(s) is provided, as is the use of such combination to create single numerical "risk indices" or "risk scores" encompassing information from multiple cancer associated gene(s) inputs. Individual B cancer associated gene(s) may also be included or excluded in the panel of cancer associated gene(s) used in the calculation of the cancer associated gene(s) indices so derived above, based on various measures of relative performance and calibration in validation, and employing through repetitive training methods such as forward, reverse, and stepwise selection, as well as with genetic algorithm approaches, with or without the use of constraints on the complexity of the resulting cancer associated gene(s) indices.

The above measurements of diagnostic accuracy for cancer associated gene(s) are only a few of the possible measurements of the clinical performance of the invention. It should be noted that the appropriateness of one measurement of clinical accuracy or another will vary based upon the clinical application, the population tested, and the clinical consequences of any potential misclassification of subjects. Other important aspects of the clinical and overall performance of the invention include the selection of cancer associated gene(s) so as to reduce overall cancer associated gene (s) variability (whether due to method (analytical) or biological (pre-analytical variability, for example, as in diurnal variation), or to the integration and analysis of results (post-analytical variability) into indices and cut-off ranges), to assess analyte stability or sample integrity, or to allow the use of differing sample matrices amongst blood, cells, serum, plasma, urine, etc.

Kits

The invention also includes a colorectal cancer detection reagent, *i.e.*, nucleic acids that specifically identify one or more colorectal cancer or condition related to colorectal cancer nucleic acids (*e.g.*, any gene listed in Tables 1-5, oncogenes, tumor suppression genes, tumor progression genes, angiogenesis genes and lymphogenesis genes; sometimes referred to herein as colorectal cancer associated genes or colorectal cancer associated constituents) by having homologous nucleic acid sequences, such as oligonucleotide sequences, complementary to a portion of the colorectal cancer genes nucleic acids or antibodies to proteins encoded by the colorectal cancer gene nucleic acids packaged together in the form of a kit. The oligonucleotides can be fragments of the colorectal cancer genes. For example the oligonucleotides can be 200, 150, 100, 50, 25, 10 or less nucleotides in length. The kit may contain in separate containers a nucleic acid or antibody (either already bound to a solid matrix or packaged separately with reagents for binding them to the matrix), control formulations (positive and/or negative), and/or a detectable label. Instructions (*i.e.*, written, tape, VCR, CD-ROM, etc.) for carrying out the assay may be included in the kit. The assay may for example be in the form of PCR, a Northern hybridization or a sandwich ELISA, as known in the art.

For example, colorectal cancer gene detection reagents can be immobilized on a solid matrix such as a porous strip to form at least one colorectal cancer gene detection site. The measurement or detection region of the porous strip may include a plurality of sites containing a nucleic acid. A test strip may also contain sites for negative and/or positive controls. Alternatively, control sites can be located on a separate strip from the test strip. Optionally, the different detection sites may contain different amounts of immobilized nucleic acids, *i.e.*, a higher amount in the first detection site and lesser amounts in subsequent sites. Upon the addition of test sample, the number of sites displaying a detectable signal provides a quantitative indication of the amount of colorectal cancer genes present in the sample. The detection sites may be configured in any suitably detectable shape and are typically in the shape of a bar or dot spanning the width of a test strip.

Alternatively, colorectal cancer detection genes can be labeled (*e.g.*, with one or more fluorescent dyes) and immobilized on lyophilized beads to form at least one colorectal cancer gene detection site. The beads may also contain sites for negative and/or positive controls. Upon addition of the test sample, the number of sites displaying a detectable signal provides a quantitative indication of the amount of colorectal cancer genes present in the sample.

Alternatively, the kit contains a nucleic acid substrate array comprising one or more nucleic acid sequences. The nucleic acids on the array specifically identify one or more nucleic acid sequences represented by colorectal cancer genes (see Tables 1-5). In various embodiments, the expression of 1, 2, 3, 4, 5, 6, 7, 8, 9, 10, 15, 20, 25, 40 or 50 or more of the sequences represented by colorectal cancer genes (see Tables 1-5) can be identified by virtue of binding to the array. The substrate array can be on, *i.e.*, a solid substrate, *i.e.*, a "chip" as described in U.S. Patent No. 5,744,305. Alternatively, the substrate array can be a solution array, *i.e.*, Luminex, Cyvera, Vitra and Quantum Dots' Mosaic.

The skilled artisan can routinely make antibodies, nucleic acid probes, *i.e.*, oligonucleotides, aptamers, siRNAs, antisense oligonucleotides, against any of the colorectal cancer genes listed in Tables 1-5.

**OTHER EMBODIMENTS**

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Examples 1:** Patient Population

RNA was isolated using the PAXgene System from blood samples obtained from a total of 23 subjects suffering from colon cancer and 50 healthy, normal (*i.e.*, not suffering from or diagnosed with colon cancer) subjects. These RNA samples were used for the gene expression analysis studies described in Examples 3-7 below.

The inclusion criteria for the colon cancer subjects that participated in the study were as follows: each of the subjects had defined, newly diagnosed disease, the blood samples were obtained prior to initiation of any treatment for colon cancer, and each subject in the study was 18 years or older, and able to provide consent.

The following criteria were used to exclude subjects from the study: any treatment with immunosuppressive drugs, corticosteroids or investigational drugs; diagnosis of acute and chronic infectious diseases (renal or chest infections, previous TB, HIV infection or AIDS, or active cytomegalovirus); symptoms of severe progression or uncontrolled renal, hepatic, hematological, gastrointestinal, endocrine, pulmonary, neurological, or cerebral disease; and pregnancy.

**Example 2:** Numeration and Classification Methodology based on Logistic Regression Models **Introduction**

The following methods were used to generate I, 2, and 3-gene models capable of distinguishing between subjects diagnosed with colon cancer and normal subjects, with at least 75% classification accurary, as described in Examples 3-7 below.

Given measurements on G genes from samples of $N_1$ subjects belonging to group 1 and $N_2$ members of group 2, the purpose was to identify models containing g < G genes which discriminate between the 2 groups. The groups might be such that one consists of reference subjects (*e.g.*, healthy, normal subjects) while the other group might have a specific disease, or subjects in group 1 may have disease A while those in group 2 may have disease B.

Specifically, parameters from a linear logistic regression model were estimated to predict a subject's probability of belonging to group 1 given his (her) measurements on the g genes in the model. After all the models were estimated (all G 1-gene models were estimated, as well as all $\binom{G}{2} = G*(G-1)/2$ 2-gene models, and all (G 3) =G*(G-1)*(G-2)/6 3-gene models based on G

genes (number of combinations taken 3 at a time from G)), they were evaluated using a 2-dimensional screening process. The first dimension employed a *statistical* screen (significance of incremental p-values) that eliminated models that were likely to overfit the data and thus may not validate when applied to new subjects. The second dimension employed a clinical screen to eliminate models for which the expected misclassification rate was higher than an acceptable level. As a threshold analysis, the gene models showing less than 75% discrimination between $N_1$ subjects belonging to group 1 and $N_2$ members of group 2 (*i.e.*, misclassification of 25% or more of subjects in either of the 2 sample groups), and genes with incremental p-values that were not statistically significant, were eliminated.

**Methodological, Statistical and Computing Tools Used**

The Latent GOLD program (Vermunt and Magidson, 2005) was used to estimate the logistic regression models. For efficiency in processing the models, the LG-Syntax™ Module available with version 4.5 of the program (Vermunt and Magidson, 2007) was used in batch mode, and all g-gene models associated with a particular dataset were submitted in a single run to be estimated. That is, all 1-gene models were submitted in a single run, all 2-gene models were submitted in a second run, etc.

**The Data**

The data consists of ΔCT values for each sample subject in each of the 2 groups (*e.g.*, cancer subject vs. reference (*e.g.*, healthy, normal subjects) on each of G(k) genes obtained from a particular class k of genes. For a given disease, separate analyses were performed based on disease specific genes, including without limitation genes specific for prostate, breast, ovarian, cervical, lung, colon, and skin cancer, (k=1), inflammatory genes (k=2), human cancer general genes (k=3), genes from a cross cancer gene panel (k=4), and genes in the EGR family (k=5).

**Analysis Steps**

The steps in a given analysis of the G(k) genes measured on $N_1$ subjects in group 1 and $N_2$ subjects in group 2 are as follows:

1) Eliminate low expressing genes: In some instances, target gene FAM measurements were beyond the detection limit (*i.e.*, very high ΔCT values which indicate low expression) of the particular platform instrument used to detect and quantify constituents of a Gene Expression Panel (Precision Profile™). To address the issue of "undetermined" gene expression measures as lack of expression for a particular gene, the detection limit was reset and the "undetermined" constituents were "flagged", as previously described. $C_T$ normalization ($\Delta C_T$) and relative expression calculations that have used re-set FAM $C_T$ values were also flagged. In some instances, these low expressing genes (*i.e.*, re-set FAM $C_T$ values) were eliminated from the analysis in step 1 if 50% or more $\Delta C_T$ values from either of the 2 groups were flagged. Although such genes were eliminated from the statistical analyses described herein, one skilled in the art would recognize that such genes may be relevant in a disease state.

2) Estimate logistic regression (logit) models predicting P(i) = the probability of being in group 1 for each subject i = 1,2,..., $N_1+N_2$. Since there are only 2 groups, the probability of being in group 2 equals 1-P(i). The maximum likelihood (ML) algorithm implemented in Latent GOLD 4.0 (Vermunt and Magidson, 2005) was used to estimate the model parameters. All 1-gene models were estimated first, followed by all 2-gene models and in cases where the sample sizes $N_1$ and $N_2$ were sufficiently large, all 3-gene models were estimated.

3) Screen out models that fail to meet the statistical or clinical criteria: Regarding the *Statistical* criteria, models were retained if the incremental p-values for the parameter estimates for each gene (*i.e.*, for each predictor in the model) fell below the cutoff point alpha = 0.05. Regarding the clinical criteria, models were retained if the percentage of cases within each group (*e.g.*, disease group, and reference group (*e.g.*, healthy, normal subjects) that was correctly predicted to be in that group was at least 75%. For technical details, see the section "Application of the Statistical and Clinical Criteria to Screen Models".

4) Each model yielded an index that could be used to rank the sample subjects. Such an index value could also be computed for new cases not included in the sample. See the section "Computing Model-based Indices for each Subject" for details on how this index was calculated.

5) A cutoff value somewhere between the lowest and highest index value was selected and based on this cutoff, subjects with indices above the cutoff were classified (predicted to be) in the disease group, those below the cutoff were classified into the reference group (*i.e.*, normal, healthy subjects). Based on such classifications, the percent of each group that is correctly classified was determined. See the section labeled "Classifying Subjects into Groups" for details on how the cutoff was chosen.

6) Among all models that survived the screening criteria (Step 3), an entropy-based $R^2$ statistic was used to rank the models from high to low, *i.e.*, the models with the highest percent classification rate to the lowest percent classification rate. The top 5 such models are then evaluated with respect to the percent correctly classified and the one having the highest percentages was selected as the single "*best*" model. A discrimination plot was provided for the best model having an 85% or greater percent classification rate. For details on how this plot was developed, see the section "Discrimination Plots" below.

While there are several possible $R^2$ statistics that might be used for this purpose, it was determined that the one based on entropy was most sensitive to the extent to which a model yields clear separation between the 2 groups. Such sensitivity provides a model which can be used as a tool by a practitioner (*e.g.*, primary care physician, oncologist, etc.) to ascertain the necessity of future screening or treatment options. For more detail on this issue, see the section labeled "Using $R^2$ Statistics to Rank Models" below.

## Computing Model-based Indices for each Subject

The model parameter estimates were used to compute a numeric value (logit, odds or probability) for each diseased and reference subject (*e.g.*, healthy, normal subject) in the sample. For illustrative purposes only, in an example of a 2-gene logit model for cancer containing the genes ALOX5 and S100A6, the following parameter estimates listed in Table A were obtained:

**Table A:**

| | | |
|---|---|---|
| Cancer | alpha(1) | 18.37 |
| Normals | alpha(2) | -18.37 |
| | | |
| Predictors | | |
| ALOX5 | beta(1) | -4.81 |
| | | |
| S100A6 | beta(2) | 2.79 |

For a given subject with particular $\Delta C_T$ values observed for these genes, the predicted logit associated with cancer vs. reference (*i.e.*, normals) was computed as:

$$\text{LOGIT (ALOX5, S100A6)} = [\text{alpha}(1) - \text{alpha}(2)] + \text{beta}(1)^* \text{ ALOX5} + \text{beta}(2)^* \text{ S100A6}.$$

The predicted odds of having cancer would be:

$$\text{ODDS (ALOX5, S100A6)} = \exp[\text{LOGIT (ALOX5, S100A6)}]$$

and the predicted probability of belonging to the cancer group is:
P (ALOX5, S100A6) = ODDS (ALOX5, S100A6) / [1 + ODDS (ALOX5, S100A6)]

Note that the ML estimates for the alpha parameters were based on the relative proportion of the group sample sizes. Prior to computing the predicted probabilities, the alpha estimates may be adjusted to take into account the relative proportion in the population to which the model will be applied (for example, without limitation, the incidence of prostate cancer in the population of adult men in the U.S., the incidence of breast cancer in the population of adult women in the U.S., etc.)

## Classifying Subjects into Groups

The "modal classification rule" was used to predict into which group a given case belongs. This rule classifies a case into the group for which the model yields the highest predicted probability. Using the same cancer example previously described (for illustrative purposes only), use of the modal classification rule would classify any subject having $P > 0.5$ into the cancer group, the others into the reference group (*e.g.*, healthy, normal subjects). The percentage of all $N_1$ cancer subjects that were correctly classified were computed as the number of such subjects having $P > 0.5$ divided by $N_1$. Similarly, the percentage of all $N_2$ reference (*e.g.*, normal healthy) subjects that were correctly classified were computed as the number of such subjects having $P \leq 0.5$ divided by $N_2$. Alternatively, a cutoff point $P_0$ could be used instead of the modal classification rule so that any subject i having $P(i) > P_0$ is assigned to the cancer group, and otherwise to the Reference group (*e.g.*, normal, healthy group).

## Application of the Statistical and Clinical Criteria to Screen Models,

### Clinical screening criteria

In order to determine whether a model met the clinical 75% correct classification criteria, the following approach was used:

A. All sample subjects were ranked from high to low by their predicted probability P (*e.g.*, see Table B).
B. Taking $P_0(i) = P(i)$ for each subject, one at a time, the percentage of group 1 and group 2 that would be correctly classified, $P_1(i)$ and $P_2(i)$ was computed.
C. The information in the resulting table was scanned and any models for which none of the potential cutoff probabilities met the clinical criteria (*i.e.*, no cutoffs $P_0(i)$ exist such that both $P_1(i) > 0.75$ and $P_2(i) > 0.75$) were eliminated. Hence, models that did *not* meet the clinical criteria were eliminated.

The example shown in Table B has many cut-offs that meet this criteria. For example, the cutoff $P_0 = 0.4$ yields correct classification rates of 92% for the reference group (*i.e.*, normal, healthy subjects), and 93% for Cancer subjects. A plot based on this cutoff is shown in Figure 1 and described in the section "Discrimination Plots".

### Statistical screening criteria

In order to determine whether a model met the *statistical* criteria, the following approach was used to compute the incremental p-value for each gene g =1,2,..., G as follows:

i. Let LSQ(0) denote the overall model L-squared output by Latent GOLD for an unrestricted model.
ii. Let LSQ(g) denote the overall model L-squared output by Latent GOLD for the restricted version of the model where the effect of gene g is restricted to 0.

iii. With 1 degree of freedom, use a 'components of chi-square' table to determine the p-value associated with the LR difference statistic LSQ(g) - LSQ(0).

Note that this approach required estimating g restricted models as well as 1 unrestricted model.

## Discrimination Plots

For a 2-gene model, a discrimination plot consisted of plotting the $\Delta C_T$ values for each subject in a scatterplot where the values associated with one of the genes served as the vertical axis, the other serving as the horizontal axis. Two different symbols were used for the points to denote whether the subject belongs to group 1 or 2.

A line was appended to a discrimination graph to illustrate how well the 2-gene model discriminated between the 2 groups. The slope of the line was determined by computing the ratio of the ML parameter estimate associated with the gene plotted along the horizontal axis divided by the corresponding estimate associated with the gene plotted along the vertical axis. The intercept of the line was determined as a function of the cutoff point. For the cancer example model based on the 2 genes ALOX5 and S100A6 shown in Figure 1, the equation for the line associated with the cutoff of 0.4 is LOX5 = 7.7 +0.58* S100A6. This line provides correct classification rates of 93% and 92% (4 of 57 cancer subjects misclassified and only 4 of 50 reference (*i.e.*, normal) subjects misclassified).

For a 3-gene model, a 2-dimensional slice defined as a linear combination of 2 of the genes was plotted along one of the axes, the remaining gene being plotted along the other axis. The particular linear combination was determined based on the parameter estimates. For example, if a 3rd gene were added to the 2-gene model consisting of ALOX5 and S100A6 and the parameter estimates for ALOX5 and S100A6 were beta(1) and beta(2) respectively, the linear combination beta(1)* ALOX5+ beta(2)* S100A6 could be used. This approach can be readily extended to the situation with 4 or more genes in the model by taking additional linear combinations. For example, with 4 genes one might use beta(1)* ALOX5+ beta(2)* S100A6-along one axis and beta(3)*gene3 + beta(4)*gene4 along the other, or beta(1)* ALOX5+ beta(2)* S 100A6+ beta(3)*gene3 along one axis and gene4 along the other axis. When producing such plots with 3 or more genes, genes with parameter estimates having the same sign were chosen for combination.

## Using $R^2$ Statistics to Rank Models

The $R^2$ in traditional OLS (ordinary least squares) linear regression of a continuous dependent variable can be interpreted in several different ways, such as 1) proportion of variance accounted for, 2) the squared correlation between the observed and predicted values, and 3) a transformation of the F-statistic. When the dependent variable is not continuous but categorical (in our models the dependent variable is dichotomous membership in the diseased group or reference group), this standard $R^2$ defined in terms of *variance* (see definition 1 above) is only one of several possible measures. The term 'pseudo $R^2$' has been coined for the generalization of the standard variance-based $R^2$ for use with categorical dependent variables, as well as other settings where the usual assumptions that justify OLS do not apply.

The general definition of the (pseudo) $R^2$ for an estimated model is the reduction of errors compared to the errors of a baseline model. For the purpose of the present invention, the *estimated* model is a logistic regression model for predicting group membership based on 1 or more continuous predictors ($\Delta C_T$ measurements of different genes). The *baseline* model is the regression model that contains no predictors; that is, a model where the regression coefficients are restricted to 0. More precisely, the pseudo $R^2$ is defined as:

$$R^2 = [\text{Error(baseline)}- \text{Error(model)}]/\text{Error(baseline)}$$

Regardless how error is defined, if prediction is perfect, Error(model) = 0 which yields

$R^2$ = 1. Similarly, if all of the regression coefficients do in fact turn out to equal 0, the model is equivalent to the baseline, and thus $R^2$=0. In general, this pseudo $R^2$ falls somewhere between 0 and 1.

When Error is defined in terms of variance, the pseudo $R^2$ becomes the *standard* $R^2$. When the dependent variable is dichotomous group membership, scores of 1 and 0, -1 and +1, or any other 2 numbers for the 2 categories yields the same value for $R^2$. For example, if the dichotomous dependent variable takes on the scores of 1 and 0, the variance is defined as P*(1-P) where P is the probability of being in 1 group and 1-P the probability of being in the other.

A common alternative in the case of a dichotomous dependent variable, is to define error in terms of entropy. In this situation, entropy can be defined as P*ln(P)*(1-P)*ln(1-P) (for further discussion of the variance and the entropy based $R^2$, see Magidson, Jay, "Qualitative Variance, Entropy and Correlation Ratios for Nominal Dependent Variables," Social Science Research 10 (June), pp. 177-194).

The $R^2$ statistic was used in the enumeration methods described herein to identify the "best" gene-model. $R^2$

can be calculated in different ways depending upon how the error variation and total observed variation are defined. For example, four different $R^2$ measures output by Latent GOLD are based on:

a) Standard variance and mean squared error (MSE)
b) Entropy and minus mean log-likelihood (-MLL)
c) Absolute variation and mean absolute error (MAE)
d) Prediction errors and the proportion of errors under modal assignment (PPE)

Each of these 4 measures equal 0 when the predictors provide zero discrimination between the groups, and equal 1 if the model is able to classify each subject into their actual group with 0 error. For each measure, Latent GOLD defines the total variation as the error of the baseline (intercept-only) model which restricts the effects of all predictors to 0. Then for each, $R^2$ is defined as the proportional reduction of errors in the estimated model compared to the baseline model. For the 2-gene cancer example used to illustrate the enumeration methodology described herein, the baseline model classifies all cases as being in the diseased group since this group has a larger sample size, resulting in 50 misclassifications (all 50 normal subjects are misclassified) for a prediction error of 50/107 = 0.467. In contrast, there are only 10 prediction errors ( = 10/107 = 0.093) based on the 2-gene model using the modal assignment rule, thus yielding a prediction error $R^2$ of 1 - 0.093/.467 = 0.8. As shown in Exhibit 1, 4 normal and 6 cancer subjects would be misclassified using the modal assignment rule. Note that the modal rule utilizes $P_0 = 0.5$ as the cutoff. If $P_0 = 0.4$ were used instead, there would be only 8 misclassified subjects.

The sample discrimination plot shown in Figure 1 its for a 2-gene model for cancer based on disease-specific genes. The 2 genes in the model are ALOX5 and S100A6 and only 8 subjects are misclassified (4 blue circles corresponding to normal subjects fall to the right and below the line, while 4 red Xs corresponding to misclassified cancer subjects lie above the line).

To reduce the likelihood of obtaining models that capitalize on chance variations in the observed samples the models may be limited to contain only M genes as predictors in the model. (Although a model may meet the significance criteria, it may overfit data and thus would not be expected to validate when applied to a new sample of subjects.) For example, for M = 2, all models would be estimated which contain:

A. I-gene-G such models

B. 2-gene models -- $\binom{G}{2} = G*(G\text{-}1)/2$ such models

C. 3-gene models -- (G3) =G*(G-1)*(G-2)/6 such models

## Computation of the Z-statistic

The Z-Statistic associated with the test of significance between the mean $\Delta C_T$ values for the cancer and normal groups for any gene g was calculated as follows: i. Let LL[g] denote the log of the likelihood function that is maximized under the logistic regression model that predicts group membership (Cancer vs. Normal) as a function of the $\Delta C_T$ value associated with gene g. There are 2 parameters in this modet-an intercept and a slope.
ii. Let LL(0) denote the overall model L-squared output by Latent GOLD for the restricted version of the model where the slope parameter reflecting the effect of gene g is restricted to 0. This model has only 1 unrestricted parameter - the intercept.
iii. With 2-1 = 1 degree of freedom (the difference in the number of unrestricted parameters in the models), one can use a 'components of chi-square' table to determine the p-value associated with the Log Likelihood difference statistic LLDiff = -2*(mL[0] - LL[g]) = 2*(LL[g] - LL[0]).
iv. Since the chi-squared statistic with 1 df is the square of a Z-statistic, the magnitude of the Z-statistic can be computed as the square root of the LLDiff. The sign of Z is negative if the mean $\Delta C_T$ value for the cancer group on gene g is less than the corresponding mean for the normal group, and positive if it is greater.
v. These Z-statistics can be plotted as a bar graph. The length of the bar has a monotonic relationship with the p-value.

**Table B:** $\Delta C_T$ Values and Model Predicted Probability of Cancer for Each Subject

| ALOX5 | S100A6 | P | Group | | | | ALOX5 | S100A6 | P | Group | |
|---|---|---|---|---|---|---|---|---|---|---|---|
| 13.92 | 16.13 | 1.0000 | Cancer | | | | 16.52 | 15.38 | 0.5343 | Cancer | |
| 13.90 | 15.77 | 1.0000 | Cancer | | | | 15-54 | 13.67 | 0.5255 | Normal | |

(continued)

| ALOX5 | S100A6 | P | Group | | | ALOX5 | S100A6 | P | Group |
|---|---|---|---|---|---|---|---|---|---|
| 13.75 | 15.17 | 1.0000 | Cancer | | | 15.29 | 13.11 | 0.4537 | Cancer |
| 13.62 | 14.51 | 1.0000 | Cancer | | | 15.96 | 14.23 | 0.4201 | Cancer |
| 15.33 | 17.16 | 1.0000 | Cancer | | | 15.96 | 14.20 | 0.3928 | Normal |
| 13.86 | 14.61 | 1.0000 | Cancer | | | 16.25 | 14.69 | 0.3887 | Cancer |
| 14.14 | 15.09 | 1.0000 | Cancer | | | 16.04 | 14.32 | 0.3874 | Cancer |
| 13.49 | 13.60 | 0.9999 | Cancer | | | 16.116 | 14.71 | 0.3863 | Normal |
| 15.24 | 16.61 | 0.9999 | Cancer | | | 15.97 | 14.18 | 0.3710 | Cancer |
| 14.03 | 14.45 | 0.9999 | Cancer | | | 15.93 | 14.06 | 0.3407 | Normal |
| 14.98 | 16.05 | 0.9999 | Cancer | | | 16.23 | 14.41 | 0.2378 | Cancer |
| 13.95 | 14.25 | 0.9999 | Cancer | | | 18.02 | 13.91 | 0.1743 | Normal |
| 14.09 | 14.13 | 0.9998 | Cancer | | | 15.99 | 13.78 | 0.1501 | Normal |
| 15.01 | 15.69 | 0.9997 | Cancer | | | 16.74 | 15.05 | 0.1359 | Normals |
| 14.13 | 14.15 | 0.9997 | cancer | | | 16.66 | 14.90 | 0.1349 | Normal |
| 14.37 | 14.43 | 0.9996 | Cancer | | | 16.91 | 15.20 | 0.0994 | Normal |
| 14.14 | 13.88 | 0.9994 | Cancer | | | 16.47 | 14.31 | 0.0721 | Normal |
| 14.33 | 14.17 | 0.9993 | Cancer | | | 16.63 | 14.57 | 0.0672 | Normal |
| 14.97 | 15.06 | 0.9988 | Cancer | | | 16.82 | 14.84 | 0.0596 | Normal |
| 14.59 | 14.30 | 0.9984 | Cancer | | | 16.75 | 14.73 | 0.0587 | Normal |
| 14.45 | 13.93 | 0.9978 | Cancer | | | 16.69 | 14.54 | 0.0474 | Normal |
| 14.40 | 13.77 | 0.9972 | Cancer | | | 17.13 | 15.25 | 0.0416 | Normal |
| 14.72 | 14.31 | 0.9971 | Cancer | | | 16.97 | 14.72 | 00329 | Normal |
| 14.81 | 14.38 | 0.9963 | Cancer | | | 16.35 | 13.76 | 0.0285 | Normal |
| 14.54 | 13.91 | 09963 | Cancer | | | 18.41 | 13.93 | 0.0255 | Normal |
| 14.88 | 14.48 | 0.9962 | Cancer | | | 16.68 | 14.20 | 0.0205 | Normal |
| 14.85 | 14.42 | 0.9959 | Cancer | | | 18.58 | 13.97 | 0.0169 | Normal |
| 15.40 | 15.30 | 0.9951 | Cancer | | | 18.66 | 14.09 | 0.0167 | Normal |
| 15.58 | 15.60 | 0.9951 | Cancer | | | 16.92 | 14.49 | 0.0140 | Normal |
| 14.82 | 14.28 | 0.9950 | Cancer | | | 16.93 | 14.51 | 0.0139 | Normal |
| 14.78 | 14.06 | 0.9924 | Cancer | | | 17.27 | 15.04 | 0.0123 | Normal |
| 14.68 | 13.88 | 0.9922 | Cancer | | | 16.45 | 13.60 | 0.0116 | Normal |
| 14.54 | 13.64 | 0.9922 | Cancer | | | 17.52 | 15.44 | 0,0110 | Normal |
| 15.86 | 15.91 | 0.9920 | Cancer | | | 17.12 | 14.46 | 0.0051 | Normal |
| 15.71 | 15.60 | 0.9909 | Cancer | | | 17.13 | 14.48 | 0.0048 | Normal |
| 16.24 | 16.36 | 0.9858 | Cancer | | | 16.78 | 13.86 | 0.0047 | Normal |
| 16.09 | 15.94 | 0.9774 | Cancer | | | 17.10 | 14.36 | 0.0041 | Normal |
| 15.26 | 14.41 | 0.9705 | Cancer | | | 16.75 | 13.69 | 0.0034 | Normal |
| 14.93 | 13.81 | 0.9693 | Cancer | | | 17.27 | 14.49 | 0.0027 | Normals |
| 15.44 | 14.67 | 0.9670 | Cancer | | | 17.07 | 14.08 | 0.0022 | Normal |

(continued)

| ALOX5 | S100A6 | P | Group | | | ALOX5 | S100A6 | P | Group |
|---|---|---|---|---|---|---|---|---|---|
| 15.69 | 15.08 | 0.9663 | Cancer | | | 17.16 | 14.08 | 0.0014 | Normal |
| 15.40 | 14.54 | 0.9615 | Cancer | | | 17.50 | 14.41 | 0.0007 | Normal |
| 15.80 | 15.21 | 0.9586 | Cancer | | | 17.50 | 14.18 | 0.0004 | Normal |
| 15.98 | 15.43 | 0.9485 | Cancer | | | 17.45 | 1d.02 | 0.0003 | Normal |
| 15.20 | 14.08 | 0.9461 | Normal | | | 17.53 | 13.90 | 0.0001 | Normal |
| 15.03 | 13.62 | 0.9196 | Cancer | | | 18.21 | 15.06 | 0.0001 | Normal |
| 15.20 | 13.91 | 0.9184 | Cancer | | | 17.99 | 14.63 | 0.0001 | Normal |
| 15.04 | 13.54 | 0.8972 | Cancer | | | 17.73 | 14.06 | 0.0001 | Normal |
| 15.30 | 13.92 | 0.0774 | Cancer | | | 17.97 | 14.40 | 0.0001 | Normal |
| 15.80 | 14.69 | 0.8404 | Cancer | | | 17.98 | 14.35 | 0.0001 | Normal |
| 15.61 | 14.23 | 0.7939 | Normal | | 69 | 18.47 | 15.16 | 0.0001 | Normal |
| 15.89 | 14.64 | 0,7577 | Normal | | | 18.28 | 14.59 | 0.0000 | Normal |
| 15.44 | 13.66 | 0.6445 | Cancer | | | 18.37 | 14.71 | 0.0000 | Normal |

**Examples 3**: Precision Profile™ for Colorectal Cancer

Custom primers and probes were prepared for the targeted 70 genes shown in the Precision Profile™ for Colorectal Cancer (shown in Table 1), selected to be informative relative to biological state of colon cancer patients. Gene expression profiles for the 70 colon cancer specific genes were analyzed using the 19 of the RNA samples obtained from colon cancer subjects, and the 50 RNA samples obtained from healthy, normal subjects, as described in Example 1.

Logistic regression models yielding the best discrimination between subjects diagnosed with colon cancer and normal subjects were generated using the enumeration and classification methodology described in Example 2. A listing of all 1 and 2-gene logistic regression models capable of distinguishing between subjects diagnosed with colon cancer and normal subjects with at least 75% accuracy is shown in Table 1 A, (read from left to right).

As shown in Table 1A, the 1 and 2-gene models are identified in the first two columns on the left side of Table 1A, ranked by their entropy $R^2$ value (shown in column 3, ranked from high to low). The number of subjects correctly classified or misclassified by each 1 or 2-gene model for each patient group (*i.e.*, normal vs. colon cancer) is shown in columns 4-7. The percent normal subjects and percent colon cancer subjects correctly classified by the corresponding gene model is shown in columns 8 and 9. The incremental p-value for each first and second gene in the 1 or 2-gene model is shown in columns 10 and 11 (note p-values smaller than $1 \times 10^{-17}$ are reported as '0'). The total number of RNA samples analyzed in each patient group (i.e., normals vs. colon cancer), after exclusion of missing values, is shown in columns 12 and 13. The values missing from the total sample number for normal and/or colon cancer subjects shown in columns 12 and 13 correspond to instances in which values were excluded from the logistic regression analysis due to reagent limitations and/or instances where replicates did not meet quality metrics.

For example, the "best" logistic regression model (defined as the model with the highest entropy $R^2$ value, as described in Example 2) based on the 70 genes included in the Precision Profile™ for Colorectal Cancer is shown in the first row of Table 1A, read left to right. The first row of Table 1A lists a 2-gene model, MASH6 and PSEN2, capable of classifying normal subjects with 87.5% accuracy, and colon cancer subjects with 84.2% accuracy. A total number of 48 normal and 19 colon cancer RNA samples were analyzed for this 2-gene model, after exclusion of missing values. As shown in Table 1A, this 2-gene model correctly classifies 42 of the normal subjects as being in the normal patient population, and misclassifies 6 of the normal subjects as being in the colon cancer patient population. This 2-gene model correctly classifies 16 of the colon cancer subjects as being in the colon cancer patient population, and misclassifies 3 of the colon cancer subjects as being in the normal patient population. The p-value for the 1st gene, MSH6 is 6.6E-11, the incremental p-value for the second gene, PSEN2, is 1.2E-06,

A discrimination plot of the 2-gene model, MSH6 and PSEN2, is shown in Figure 2. As shown in Figure 2, the normal subjects are represented by circles, whereas the colon cancer subjects are represented by X's. The line appended to the discrimination graph in Figure 2 illustrates how well the 2-gene model discriminates between the 2 groups. Values below and to the right of the line represent subjects predicted by the 2-gene model to be in the normal population. Values above and to the left of the line represent subjects predicted to be in the colon cancer population. As shown in Figure 2, 5 normal subjects (circles) and 3 colon cancer subjects (X's) are classified in the wrong patient population.

The following equation describes the discrimination line shown in Figure 2:

$$MSH6 = 2.861677 + 0.840724 * PSEN2$$

The intercept (alpha) and slope (beta) of the discrimination line was computed as follows. A cutoff of 0.286 was used to compute alpha (equals -0.91489 in logit units).

Subjects above and to the left of this discrimination line have a predicted probability of being in the diseased group higher than the cutoff probability of 0.286.

The intercept $C_0$ = 2.81677 was computed by taking the difference between the-intercepts for the 2 groups [-10.544 -(10.544)= -21.088] and subtracting the log-odds of the cutoff probability (-0.91489). This quantity was then multiplied by -1/X where X is the coefficient for MSH6 (7.0494).

A ranking of the top 49 colon cancer specific genes for which gene expression profiles were obtained, from most to least significant, is shown in Table 1B. Table 1B summarizes the results of significance tests (Z-statistic and p-values) for the difference in the mean expression levels for normal subjects and subjects suffering from colon cancer. A negative Z-statistic means that the $\Delta C_T$ for the colon cancer subjects is less than that of the normals, i.e., genes having a negative Z-statistic are up-regulated in colon cancer subjects as compared to normal subjects. A positive Z-statistic means that the $\Delta C_T$ for the colon cancer subjects is higher than that of of the normals, *i.e.*, genes with a positive Z-statistic are down-regulated in colon cancer subjects as compared to normal subjects. Figure 3 shows a graphical representation of the Z-statistic for each of the 49 genes shown in Table 1B, indicating which genes are up-regulated and down-regulated in colon cancer subjects as compared to normal subjects.

The expression values ($\Delta C_T$) for the 2-gene model, MSH6 and PSEN2, for each of the 19 colon cancer samples and 48 normal subject samples used in the analysis, and their predicted probability of having colon cancer, is shown in Table 1C. As shown in Table 1C, the predicted probability of a subject having colon cancer, based on the 2-gene model, MSH6 and PSEN2, is based on a scale of 0 to 1, "0" indicating no colon cancer (*i.e.*, normal healthy subject), "1" indicating the subject has colon cancer. A graphical representation of the predicted probabilities of a subject having colon cancer (*i.e.,* a colon cancer index), based on this 2-gene model, is shown in Figure 4. Such an index can be used as a tool by a practitioner (*e.g.*, primary care physician, oncologist, etc.) for diagnosis of colon cancer and to ascertain the necessity of future screening or treatment options.

**Example 4:** (Precision Profile™ for Inflammatory Response,

Custom primers and probes were prepared for the targeted 72 genes shown in the Precision Profile™ for Inflammatory Response (shown in Table 2), selected to be informative relative to biological state of inflammation and cancer. Gene expression profiles for the 72 inflammatory response genes were analyzed using 18 of the RNA samples obtained from colon cancer subjects, and 32 of the RNA samples obtained from, healthy, normal subjects, as described in Example 1.

Logistic regression models yielding the best discrimination between subjects diagnosed with colon cancer and normal subjects were generated using the enumeration and classification methodology described in Example 2. A listing of all 1 and 2-gene logistic regression models capable of distinguishing between subjects diagnosed with colon cancer and normal subjects with at least 75% accuracy is shown in Table 2A, (read from left to right).

As shown in Table 2A, the 1 and 2-gene models are identified in the first two columns on the left side of Table 2A, ranked by their entropy $R^2$ value (shown in column 3, ranked from high to low). The number of subjects correctly classified or misclassified by each 1 or 2-gene model for each patient group (*i.e.*, normal vs. colon cancer) is shown in columns 4-7. The percent normal subjects and percent colon cancer subjects correctly classified by the corresponding gene model is shown in columns 8 and 9. The incremental p-value for each first and second gene in the 1 or 2-gene model is shown in columns 10 and 11 (note p-values smaller than $1 \times 10^{-17}$ are reported as '0'). The total number of RNA samples analyzed in each patient group (*i.e.*, normals vs. colon cancer) after exclusion of missing values, is shown in columns 12-13. The values missing from the total sample number for normal and/or colon cancer subjects shown in columns 12-13 correspond to instances in which values were excluded from the logistic regression analysis due to reagent limitations and/or instances where replicates did not meet quality metrics.

For example, the "best" logistic regression model (defined as the model with the highest entropy $R^2$ value, as described in Example 2) based on the 72 genes included in the Precision Profile™ for Inflammatory Response is shown in the first row of Table 2A, read left to right. The first row of Table 2A lists a 2-gene model, HMOX1 and TXNRD1. capable of classifying normal subjects with 93.8% accuracy, and colon cancer subjects with 94.4% accuracy. All 32 normal and 18 colon cancer RNA samples were analyzed for this 2-gene model, no values were excluded. As shown in Table 2A, this 2-gene model correctly classifies 30 of the normal subjects as being in the normal patient population, and misclassifies 2 of the normal subjects as being in the colon cancer patient population. This 2-gene model correctly classifies 17 of the colon cancer subjects as being in the colon cancer patient population, and misclassifies 1 of the colon cancer subjects as being in the normal patient population. The p-value for the 1st gene, HMOX1, is 2.3E-09, the incremental p-value for the second gene, TXNRD1 is 2.1E-08.

A discrimination plot of the 2-gene model, HMOX1 and TXNRD1, is shown in Figure 5. As shown in Figure 5, the normal subjects are represented by circles, whereas the colon cancer subjects are represented by X's. The line appended to the discrimination graph in Figure 5 illustrates how well the 2-gene model discriminates between the 2 groups. Values to the left of the line represent subjects predicted by the 2-gene model to be in the normal population. Values to the right of the line represent subjects predicted to be in the colon cancer population. As shown in Figure 5, 2 normal subjects (circles) and 1 colon cancer subject (X's) are classified in the-wrong patient population.

The following equation describes the discrimination line shown in Figure 5:

$$HMOX1 = -2.9520 + 1.1294 * TXNRD1$$

The intercept (alpha) and slope (beta) of the discrimination line was computed as follows. A cutoff of 0.41465 was used to compute alpha (equals -0.34478 in logit units).

Subjects to the right of this discrimination line have a predicted probability of being in the diseased group higher than the cutoff probability of 0.41465.

The intercept $C_0$ = -2.9520 was computed by taking the difference between the intercepts for the 2 groups [-9.5916 -(9.5916)= -19.1832] and subtracting the log-odds of the cutoff probability (-0.34478). This quantity was then multiplied by -1/X where X is the coefficient for HMOX1 (-6.3815).

A ranking of the top 68 inflammatory response genes for which gene expression profiles were obtained, from most to least significant, is shown in Table 2B. Table 2B summarizes the results of significance tests (p-values) for the difference in the mean expression levels for normal subjects and subjects suffering from colon cancer.

The expression values ($\Delta C_T$) for the 2-gene model, HMOX1 and TXNRD1. for each of the 18 colon cancer subjects and 32 normal subject samples used in the analysis, and their predicted probability of having colon cancer is shown in Table 2C. In Table 2C, the predicted probability of a subject having colon cancer, based on the 2-gene model HMOX1 and TXNRD1, is based on a scale of 0 to 1, "0" indicating no colon cancer (*i.e.*, normal healthy subject), "1" indicating the subject has colon cancer. This predicted probability can be used to create a colon cancer index based on the 2-gene model HMOX1 and TXNRD1, that can be used as a tool by a practitioner (*e.g.*, primary care physician, oncologist, etc.) for diagnosis of colon cancer and to ascertain the necessity of future screening or treatment options.

**Example 5:** Human Cancer General Precision Profile™

Custom primers and probes were prepared for the targeted 91 genes shown in the Human Cancer Precision Profile™ (shown in Table 3), selected to be informative relative to the biological condition of human cancer, including but not limited to ovarian, breast, cervical, prostate, lung, colon, and skin cancer. Gene expression profiles for these 91 genes were analyzed using 23 of the RNA samples obtained from colon cancer subjects, and the 50 RNA samples obtained from the healthy, normal subjects, as described in Example 1.

Logistic regression models yielding the best discrimination between subjects diagnosed with colon cancer and normal subjects were generated using the enumeration and classification methodology described in Example 2. A listing of all 1 and 2-gene logistic regression models capable of distinguishing between subjects diagnosed with colon cancer and normal subjects with at least 75% accuracy is shown in Table 3A, (read from left to right).

As shown in Table 3A, the 1 and 2-gene models are identified in the first two columns on the left side of Table 3A, ranked by their entropy $R^2$ value (shown in column 3, ranked from high to low). The number of subjects correctly classified or misclassified by each 1 or 2-gene model for each patient group (*i.e.*, normal vs. colon cancer) is shown in columns 4-7. The percent normal subjects and percent colon cancer subjects correctly classified by the corresponding gene model is shown in columns 8 and 9. The incremental p-value for each first and second gene in the 1 or 2-gene model is shown in columns 10-11 (note p-values smaller than $1 \times 10^{-17}$ are reported as '0'). The total number of RNA samples analyzed in each patient group (*i.e.*, normals vs. colon cancer) after exclusion of missing values, is shown in columns 12 and 13. The values missing from the total sample number for normal and/or colon cancer subjects shown in columns 12-13 correspond to instances in which values were excluded from the logistic regression analysis due to reagent limitations and/or instances where replicates did not meet quality metrics.

For example, the "best" logistic regression model (defined as the model with the highest entropy $R^2$ value, as described in Example 2) based on the 91 genes included in the Human Cancer General Precision Profile™ is shown in the first row of Table 3A, read left to right. The first row of Table 3A lists a 2-gene model, ATM and CDKN2A, capable of classifying normal subjects with 88% accuracy, and colon cancer subjects with 91.3% accuracy. All 50 normal and 23 colon cancer RNA samples were analyzed for this 2-gene model, no values were excluded. As shown in Table 3A, this 2-gene model correctly classifies 44 of the normal subjects as being in the normal patient population, and misclassifies 6 of the normal subjects as being in the colon cancer patient population. This 2-gene model correctly classifies 21 of the colon cancer subjects as being in the colon cancer patient population, and misclassifies 2 of the colon cancer subjects as being in the normal patient population. The p-value for the 1st gene, ATM, is 4.2E-07, the incremental p-value for the second gene, CDKN2A is 2.8E-08.

A discrimination plot of the 2-gene model, ATM and CDKN2A, is shown in Figure 6. As shown in Figure 6, the normal subjects are represented by circles, whereas the colon cancer subjects are represented by X's. The line appended to the discrimination graph in Figure 6 illustrates how well the 2-gene model discriminates between the 2 groups. Values below and to the right of the line represent subjects predicted by the 2-gene model to be in the normal population. Values above and to the left of the line represent subjects predicted to be in the colon cancer population. As shown in Figure 6, 6 normal subjects (circles) and 2 colon cancer subjects (X's) are classified in the wrong patient population.

The following equation describes the discrimination line shown in Figure 6:

$$ATM = 1.992988 + 0.71347 * CDKN2A$$

The intercept (alpha) and slope (beta) of the discrimination line was computed as follows. A cutoff of 0.2123 was used to compute alpha (equals -1.31112 in logit units).

Subjects above and to the left of this discrimination line have a predicted probability of being in the diseased group higher than the cutoff probability of 0.2123.

The intercept $C_0$ = 1.992988 was computed by taking the difference between the intercepts for the 2 groups [-5.3332 -(5.3332)= -10.6664] and subtracting the log-odds of the cutoff probability (-I.31112). This quantity was then multiplied by -1/X where X is the coefficient for ATM (4.6941).

A ranking of the top 79 genes for which gene expression profiles were obtained, from most to least significant is shown in Table 3B. Table 3B summarizes the results of significance tests (p-values) for the difference in the mean expression levels for normal subjects and subjects suffering from colon cancer.

The expression values ($\Delta C_T$) for the 2-gene model, ATM and CDKN2A, for each of the 23 colon cancer subjects and 50 normal subject samples used in the analysis, and their predicted probability of having colon cancer is shown in Table 3C. In Table 3C, the predicted probability of a subject having colon cancer, based on the 2-gene model ATM and CDKN2A is based on a scale of 0 to 1, "0" indicating no colon cancer (i.e., normal healthy subject), "1" indicating the subject has colon cancer. This predicted probability can be used to create a colon cancer index based on the 2-gene model ATM and CDKN2A, that can be used as a tool by a practitioner (e.g., primary care physician, oncologist, etc.) for diagnosis of colon cancer and to ascertain the necessity of future screening or treatment options.

**Example 6**: EGR1 Precision Profile™

Custom primers and probes were prepared for the targeted 39 genes shown in the Precision Profile™ for EGR1 (shown in Table 4), selected to be informative of the biological role early growth response genes play in human cancer (including but not limited to ovarian, breast, cervical, prostate, lung, colon, and skin cancer). Gene expression profiles for these 39 genes were analyzed using 22 of the RNA samples obtained from colon cancer subjects, and the 50 RNA samples obtained from normal subjects, as described in Example 1.

Logistic regression models yielding the best discrimination between subjects diagnosed with colon cancer and normal subjects were generated using the enumeration and classification methodology described in Example 2. A listing of all 2-gene logistic regression models capable of distinguishing between subjects diagnosed with colon cancer and normal subjects with at least 75% accuracy is shown in Table 4A, (read from left to right).

As shown in Table 4A, the 2-gene models are identified in the first two columns on the left side of Table 4A, ranked by their entropy $R^2$ value (shown in column 3, ranked from high to low). The number of subjects correctly classified or misclassified by each 2-gene model for each patient group (i.e., normal vs. colon cancer) is shown in columns 4-7. The percent normal subjects and percent colon cancer subjects correctly classified by the corresponding gene model is shown in columns 8 and 9. The incremental p-value for each first and second gene in the 2-gene model is shown in columns 10-11 (note p-values smaller than $1 \times 10^{-17}$ are reported as '0'). The total number of RNA samples analyzed in each patient group (i.e., normals vs. colon cancer) after exclusion of missing values, is shown in columns 12 and 13. The values missing from the total sample number for normal and/or colon cancer subjects shown in columns 12-13 correspond to instances in which values were excluded from the logistic regression analysis due to reagent limitations and/or instances where replicates did not meet quality metrics.

For example, the "best" logistic regression model (defined as the model with the highest entropy $R^2$ value, as described in Example 2) based on the 39 genes included in the Precision Profile™ for EGR1 is shown in the first row of Table 4A, read left to right. The first row of Table 4A lists a 2-gene model, NAB2 and TGFB1, capable of classifying normal subjects with 82% accuracy, and colon cancer subjects with 81.8% accuracy. All 50 normal and 22 colon cancer RNA samples were analyzed for this 2-gene model, no values were excluded. As shown in Table 4A, this 2-gene model correctly classifies 41 of the normal subjects as being in the normal patient population, and misclassifies 9 of the normal subjects as being in the colon cancer patient population. This 2-gene model correctly classifies 18 of the colon cancer subjects as being in the colon cancer patient population, and misclassifies 4 of the colon cancer subjects as being in the normal patient population. The p-value for the 1st gene, NAB2, is 6.4E-09, the incremental p-value for the second gene, TGFB1 is 4.6E-07.

A ranking of the top 33 genes for which gene expression profiles were obtained, from most to least significant is shown in Table 4B, Table 4B summarizes the results of significance tests (p-values) for the difference in the mean expression levels for normal subjects and subjects suffering from colon cancer.

**Example 7:** Cross-Cancer (Precision Profile™

Custom primers and probes were prepared for the targeted 110 genes shown in the Cross Cancer Precision Profile™ (shown in Table 5), selected to be informative relative to the biological condition of human cancer, including but not limited to ovarian, breast, cervical, prostate, lung, colon, and skin cancer. Gene expression profiles for these 110 genes were analyzed using 23 of the RNA samples obtained from colon cancer subjects, and the 50 RNA samples obtained from healthy, normal subjects, as described in Example 1.

Logistic regression models yielding the best discrimination between subjects diagnosed with colon cancer and normal subjects were generated using the enumeration and classification methodology described in Example 2. A listing of all 1 and 2-gene logistic regression models capable of distinguishing between subjects diagnosed with colon cancer and normal subjects with at least 75% accuracy is shown in Table 5A, (read from left to right).

As shown in Table 5A, the 1 and 2-gene models are identified in the first two columns on the left side of Table 5A, ranked by their entropy $R^2$ value (shown in column 3, ranked from high to low). The number of subjects correctly classified or misclassified by each 1 or 2-gene model for each patient group (i.e., normal vs. colon cancer) is shown in columns 4-7. The percent normal subjects and percent colon cancer subjects correctly classified by the corresponding gene model is shown in columns 8 and 9. The incremental p-value for each first and second gene in the 1 or 2-gene model is shown in columns 10-11 (note p-values smaller than $1\times10^{-17}$ are reported as '0'). The total number of RNA samples analyzed in each patient group (i.e., normals vs. colon cancer) after exclusion of missing values, is shown in columns 12 and 13. The values missing from the total sample number for normal and/or colon cancer subjects shown in columns 12-13 correspond to instances in which values were excluded from the logistic regression analysis due to reagent limitations and/or instances where replicates did not meet quality metrics.

For example, the "best" logistic regression model (defined as the model with the highest entropy $R^2$ value, as described in Example 2) based on the 110 genes in the Human Cancer General Precision Profile™ is shown in the first row of Table 5A, read left to right. The first row of Table 5A lists a 2-gene model, AXIN2 and TNF, capable of classifying normal subjects with 93.9% accuracy, and colon cancer subjects with 90.5% accuracy. Forty-nine of the normal RNA samples and 21 of the colon cancer RNA samples were used to analyze this 2-gene model after exclusion of missing values. As shown in Table 5A, this 2-gene model correctly classifies 46 of the normal subjects as being in the normal patient population and misclassifies 3 of the normal subjects as being in the colon cancer population. This 2-gene model correctly classifies 19 of the colon cancer subjects as being in the colon cancer patient population, and misclassifies only 2 of the colon cancer subjects as being in the normal patient population. The p-value for the 1st gene, AXIN2, is 9.0E-10, the incremental p-value for the second gene, TNF is 2.4E-05.

A discrimination plot of the 2-gene model, AXIN2 and TNF, is shown in Figure 7. As shown in Figure 7, the normal subjects are represented by circles, whereas the colon cancer subjects are represented by X's. The line appended to the discrimination graph in Figure 7 illustrates how well the 2-gene model discriminates between the 2 groups. Values below and to the right of the line represent subjects predicted by the 2-gene model to be in the normal population. Values above and to the left of the line represent subjects predicted to be in the colon cancer population. As shown in Figure 7, 3 normal subjects (circles) and only 2 colon cancer subjects (X's) are classified in the wrong patient population.

The following equation describes the discrimination line shown in Figure 7:

$$AXIN2 = 4.9912 + 0.79925 * TNF$$

The intercept (alpha) and slope (beta) of the discrimination line was computed as follows. A cutoff of 0.3966 was used to compute alpha (equals -0.41965 in logit units).

Subjects above and to the left of this discrimination line have a predicted probability of being in the diseased group higher than the cutoff probability of 0.3966.

The intercept $C_0$ = 4.9912 was computed by taking the difference between the intercepts for the 2 groups [-11.6595 -(11.6595)= -23.319] and subtracting the log-odds of the cutoff probability (-0.41965). This quantity was then multiplied by -1/X where X is the coefficient for AXIN2 (4.5879).

A ranking of the top 107 genes for which gene expression profiles were obtained, from most to least significant is shown in Table 5B. Table 5B summarizes the results of significance tests (p-values) for the difference in the mean expression levels for normal subjects and subjects suffering from colon cancer.

The expression values ($\Delta C_T$) for the 2-gene model, AXIN2 and TNF, for each of the 21 colon cancer subjects and 49 normal subject samples used in the analysis, and their predicted probability of having colon cancer is shown in Table 5C. In Table 5C, the predicted probability of a subject having colon cancer, based on the 2-gene model AXIN2 and TNF is based on a scale of 0 to 1, "0" indicating no colon cancer (*i.e.*, normal healthy subject), "1" indicating the subject has colon cancer. This predicted probability can be used to create a colon cancer index based on the 2-gene model AXIN2 and TNF, that can be used as a tool by a practitioner (*e.g.*, primary care physician, oncologist, etc.) for diagnosis of colon cancer and to ascertain the necessity of future screening or treatment options.

These data support that Gene Expression Profiles with sufficient precision and calibration as described herein (1) can determine subsets of individuals with a known biological condition, particularly individuals with colorectal cancer or individuals with conditions related to colorectal cancer; (2) may be used to monitor the response of patients to therapy; (3) may be used to assess the efficacy and safety of therapy; and (4) may be used to guide the medical management of a patient by adjusting therapy to bring one or more relevant Gene Expression Profiles closer to a target set of values, which may be normative values or other desired or achievable values.

Gene Expression Profiles are used for characterization and monitoring of treatment efficacy of individuals with colorectal cancer, or individuals with conditions related to colorectal cancer. Use of the algorithmic and statistical approaches discussed above to achieve such identification and to discriminate in such fashion is within the scope of various embodiments herein.

The references listed below are hereby incorporated herein by reference.

## References

Magidson, J. GOLDMineR User's Guide (1998). Belmont, MA: Statistical Innovations Inc. Vermunt and Magidson (2cm5). Latent GOLD 4.0 Technical Guide, Belmont MA: Statistical Innovations.

Vermunt and Magidson, (2007). LG-Syntax™ User's Guide: Manual for Latent GOLD® 4.5 Syntax Module, Belmont MA: Statistical Innovations.

Vermunt J.K. and J. Magidson. Latent Class Cluster Analysis in (2002) J. A. Hagenaars and A. L. McCutcheon (eds.), Applied Latent Class Analysis, 89-106. Cambridge: Cambridge University Press.

Magidson, J. "maximum Likelihood Assessment of Clinical Trials Based on an Ordered Categorical Response." (1996) Drug Information Journal, Maple Glen, PA: Drug Information Association, Vol. 30, No. 1, pp 143-170.

**TABLE 1:** Precision Profile" for Colorectal Cancer

| Gene Symbol | GeneName | Gene Acession Number |
|---|---|---|
| ACSL5 | acyl-CoA synthetase long-chain family member 5 | NM_16234 |
| ACSS2 | acyl-CoA synthetase short-chain family member 2 | NM_018677 NM_139274 |
| AFAP | actin filament associated protein | NM_021 638 |
| ALDH1A1 | aldehyde dehydrogenase 1 family, member A1 | NM_000689 |
| ALX4 | aristaless-like homeobox 4 | NM-021926 |

(continued)

| Gene Symbol | GeneName | Gene Acession Number |
|---|---|---|
| APC | adenomatosis polyposis coli | NM_000038 |
| AXIN2 | axin 2 (conductin, axil) | N_004655 |
| BAX | BCL2-associated X protein | NM_138761 |
| BCL2 | B-cell CLII/lymphoma 2 | NM_000633 |
| BRAF | v-raf murine sarcoma viral oncogene homolog B1 | NM_004333 |
| CA2 | carbonic anhydrase II | NM_000067 |
| CA4 | carbonic anhydrase IV | NM_000717 |
| CA7 | carbonic anhydrase VII | NM_005182 |
| CCND3 | cyclin D3 | NM_001760 |
| CD44 | CD44 antigen (homing function and Indian blood group system) | NM_000610 |
| CD63 | CD63 antigen (melanoma 1 antigen) | NM_0001780 |
| CDC2 | cell division cycle 2, G 1 to S and G2 to M | NM_001786 |
| CDX2 | caudal type homeo box transcription factor 2 | NM_001265 |
| CFD | D component of complement (adipsin) | NM_001928 |
| CFLAR | CASP8 and FADD-like apoptosis regulator | NM_003879 |
| CLDN1 | claudin 1 | NM_021101 |
| CXCL1 | chemokine (C-X-C motif) ligand 1 (melanoma growth stimulating activity, alpha) | NM_001511 |
| DEFA6 | defensin, alpha 6, Paneth cell-specific | NM_001926 |
| ERBB2 | V-erb-b2 erythroblastic leukemia viral oncogene homolog 2, neuro/ glioblastoma derived oncogene homolog (avian) | NM_004448 |
| ERBB3 | V-erb-b2 Erythroblastic Leukemia Viral Oncogene Homolog 3 | NM_001982 |
| GADD45A | growth arrest and DNA-damage-inducible, alpha | NM_001924 |
| GPX2 | glutathione peroxidase 2 (gastrointestinal) | NM-002083 |
| GSK3B | glycogen synthase kinase 3 beta | NM_002093 |
| GSTA2 | glutathione S-transferase A2 | NM_000846 |
| GSTT2 | glutathione S-transferase theta 2 | NM_000854 |
| IGF2 | Putative insulin-like growth factor II associated protein. | NM_000612 |
| IGFBP4 | insulin-like growth factor binding protein 4 | NM_001552 |
| IL8 | interleukin 8 | NM_000584 |
| ITGA3 | integrin, alpha 3 (antigen CD49C, alpha 3 subunit of VLA-3 receptor) | NM_005501 |
| KRT19 | keratin 19 | NM_002276 |
| KRT20 | keratin 20 | NM_019010 |
| MGMT | O-6-methylguanine-DNA methyltransferase | NM_002412 |
| MKI67 | antigen identified by monoclonal antibody Ki-67 | NM_002417 |
| MLH1 | mutL homolog 1, colon cancer, nonpolyposis type 2 (E. coli) | NM_000249 |
| MME | membrane metallo-endopeptidase (neutral endopeptidase, enkephalinase, CALLA, CD10) | NM_000902 |
| MSH2 | mutS homolog 2, colon cancer, nonpolyposis type 1 (E. coli) | NM_000251 |

(continued)

| Gene Symbol | GeneName | Gene Acession Number |
|---|---|---|
| MSH6 | mutS homolog 6 (E. coli) | NM_000179 |
| MUTYH | mutY homolog (E. coli) | NM_012222 |
| MYC | v-myc myelocytomatosis viral oncogene homolog (avian) | NM_002467 |
| NFKB1 | nuclear factor of kappa light polypeptide gene enhancer in B-cells 1 (p105) | NM_003998 |
| NME1 | non-metastatic cells 1, protein (NM23A) expressed in | NM_198175 |
| NR2E1 | nuclear receptor subfamily 2, group E, member I | NM_003269 |
| NUAK1 | NUAK family, SNF1-like kinase, 1 | NM_014840 |
| PKLR | pyruvate kinase, liver and RBC | NM_000298 |
| PPARG | peroxisome proliferative activated receptor, gamma | NM_138712 |
| PSEN2 | presenilin 2 (Alzheimer disease 4) | NM_000447 |
| PTGS2 | prostaglandin-endoperoxide synthase 2 (prostaglandin G/H synthase and cyclooxygenase) | NM_000963 |
| RGC32 | response gene to complement 32 | NM_014059 |
| RPS3A | ribosomal protein S3A | NM_001006 |
| S100A4 | S100 calcium binding protein A4 | NM_002961 |
| S100P | S 100 calcium binding protein P | NM_005980 |
| SAA1 | serum amyloid A1 | NM_199161 |
| SERPINB5 | serpin peptidase inhibitor, clade B (ovalbumin), member 5 | NM_002639 |
| SLC25A21 | solute carrier family 25 (mitochondrial oxodicarboxylate carrier), member 21 | NM_002539 |
| SLURP1 | secreted LY6/PLAUR domain containing 1 | NM_020427 |
| SMARCA1 | SWT/SNF related, matrix associated, actin dependent regulator of chromatin, subfamily a, member 1 | NM_139035 |
| TCF4 | transcription factor 4 | NM_003199 |
| TGFBR1 | transforming growth factor, beta receptor I (activin A receptor type II-like kinase. 53kDa) | NM_004612 |
| THY1 | Thy-1 cell surface antigen | NM_006288 |
| TNF | tumor necrosis factor (TNF superfamily, member 2) | NM_000594 |
| TP53 | tumor protein p53 (Li-Fraumeni syndrome) | NM_000546 |
| VEGF | vascular endothelial growth factor | NM_003376 |
| VIL1 | villin 1 | NM_007127 |
| ZNF350 | zinc finger protein 350 | NM_021632 |
| ZYX | Zyxin | NM_003461 |

**TABLE 2:** Precision Profile™ for Inflammatory Response,

| Gene Symbol | Gene Name | Gene Accession Number |
|---|---|---|
| ADAM17 | a disintegrin and metalloproteinase domain 17 (tumor necrosis factor, alpha, converting enzyme) | NM_003183 |

(continued)

| Gene Symbol | Gene Name | Gene Accession Number |
|---|---|---|
| ALOX5 | arachidonate 5-lipoxygenase | NM_000698 |
| APAF1 | apoptotic Protease Activating Factor 1 | NM_013229 |
| C1QA | complement component 1, q subcomponent, alpha polypeptide | NM_015991 |
| CASP1 | caspase 1, apoptosis-related cysteine peptidase (interleukin 1, beta, convertase) | NM_033292 |
| CASP3 | caspase 3, apoptosis-related cysteine peptidase | NM_004346 |
| CCL3 | chemokine (C-C motif) ligand 3 | NK_002983 |
| CCL5 | chemokine (C-C motif) ligand 5 | NM_002985 |
| CCR3 | chemokine (C-C motif) receptor 3 | NM_001837 |
| PCR5 | chemokine (C-C motif) receptor 5 | NM_000579 |
| CD19 | CD19 Antigen | NM_001770 |
| CD4 | CD4 antigen (p55) | NM_000616 |
| CD86 | CD86 antigen (CD28 antigen ligand 2, B7-2 antigen) | NM_006889 |
| CD8A | CD8 antigen, alpha polypeptide | NM_001768 |
| CSF2 | colony stimulating factor 2 (granulocyte-macrophage) | NM_000758 |
| CTLA4 | cytotoxic T-lymphocyte-associated protein 4 | NM_005214 |
| CXCL1 | chemokine (C-X-C motif) ligand 1 (melanoma growth stimulating activity, alpha) | NM_001511 |
| CXCL10 | chemokine (C-X-C motif) ligand 10 | NM_001565 |
| CXCR3 | chemokine (C-X-C motif) receptor 3 | NM_001504 |
| DPP4 | Dipeptidylpeptidase 4 | MM_001935 |
| EGR1 | early growth response-1 | NM_001964 |
| ELA2 | elastase 2, neutrophil | NM_001972 |
| GZMB | granzyme B (granzyme 2, cytotoxic T-lymphocyte-associated serine esterase 1) | NM_004131 |
| HLA-DRA | major histocomputibility complex, class II, DR alpha | NM_019111 |
| HMGB1 | high-mobility group box 1 | NM_002128 |
| HMOX1 | heme oxygenase (decycling) 1 | NM_002133 |
| HSPA1A | heat shock protein 70 | NM_005345 |
| ICAM1 | intercellular adhesion molecule 1 | NM_000201 |
| IFI16 | interferon inducible protein 16, gamma | NM_005531 |
| IFNG | interferon gamma | NM_000619 |
| IL10 | interleukin 10 | NM_000572 |
| IL12B | interleukin 12 p40 | NN_002187 |
| IL15 | Interleukin 15 | NM_000585 |
| IL18 | interleukin 18 | NM_001562 |
| IL18BP | IL-18 Binding Protein | NM_005699 |
| IL1B | interleukin 1, beta | NM_000576 |
| IL1R1 | interleukin 1 receptor, type I | NM_000877 |

(continued)

| Gene Symbol | Gene Name | Gene Accession Number |
|---|---|---|
| IL1BN | interleukin 1 receptor antagonist | NM_173843 |
| IL23A | interleukin 23, alpha subunit p19 | NM_016584 |
| IL32 | interleukin 32 | NM_001012631 |
| IL5 | interleukin 5 (colony-stimulating factor, eosinophil) | NM_000879 |
| IL6 | interleukin 6 (interferon, beta 2) | NM_000600 |
| IL8 | interleukin 8 | NM_000584 |
| IRF1 | interferon regulatory factor I | NM_002198 |
| LTA | lymphotoxin alpha (TNF superfamily, member 1) | NM_000595 |
| MAPK14 | mitogen-activated protein kinase 14 | NM_001315 |
| MHC2TA | class II, major histocompatibility complex, transactivator | NM_000246 |
| MIF | macrophage migration inhibitory factor (glycosylation-inhibiting factor) | NM_002415 |
| NMP12 | matrix metallopeptidase 12 (macrophage elastase) | NM_002426 |
| MMP9 | matrix metallopeptidase 9 (gelatinase B, 92kDa gelatinase, 92kDa type IV collagenase) | NM_004994 |
| MNDA | myeloid cell nuclear differentiation antigen | NM_002432 |
| MYC | v-myc myelocytomatosis viral oncogene homolog (avian) | NM_002467 |
| NFKB1 | nuclear factor of kappa light polypeptide gene enhancer in B-cells 1 (p105) | NM_003998 |
| PLA2G7 | phospholipase A2, group VII (platelet-activating factor acetylhydrolase, plasma) | NM_005084 |
| PLAUR | plasminogen activator, urokinase receptor | NM_002659 |
| PTGS2 | prostaglandin-endoperoxide synthase 2 (prostaglandin G/H synthase and cyclooxygenase) | NM_000963 |
| PTPRC | protein tyrasine phosphatase, receptor type, C | NM_002838 |
| SERPINA1 | serine (or cysteine) proteinase inhibitor, clade A (alpha-1 antiproteinase, antitrypsin), member 1 | NM_000295 |
| SERPINE1 | serpin peptidase inhibitor, clade E (nexin, plasminogen Activator inhibitor type 1), member 1 | NM_000602 |
| SS1-3 | suppressor of cytokine signaling 3 | NM_003955 |
| TGFB1 | transforming growth factor, beta 1 (Camurati-Engelmann disease) | NM_000660 |
| TIMP1 | tissue inhibitor of metalloproteinase I | NM_003254 |
| TLR2 | toll-like receptor 2 | NM_003264 |
| TLR4 | toll-like receptor 4 | NM_003266 |
| TNF | tumor necrosis factor (TNF superfamily, member 2) | NM_000594 |
| TNFRSF13B | tumor necrosis factor receptor superfamily, member 13B | NM_012452 |
| TNFRSF1A | tumor necrosis factor receptor superfamily, member 1A | NM_001065 |
| TNFSF5 | CD40 ligand (TNF superfamily, member 5, hyper-IgM syndrome) | NM_000074 |
| TNFSF6 | Fas ligand (TNF superfamily, member 6) | NM_000639 |
| TOSO | Fas apoptotic inhibitory molecule 3 | NM_005449 |

(continued)

| Gene Symbol | Gene Name | Gene Accession Number |
|---|---|---|
| TXNRD1 | thioredoxin reductase | NM_003330 |
| VEGF | vascular endothelial growth factor | NM_003376 |

TABLE 3: Human Cancer General Precision Profile™

| Gene Symbol | Gene Name | Gene Accession Number |
|---|---|---|
| ABL1 | v-abl Abelson murine leukemia viral oncogene homolog 1 | NM_007313 |
| ABL2 | v-abl Abelson murine leukemia viral oncogene homolog 2 (arg, Abelson-related gene) | NM_007314 |
| AKT1 | v-akt murine thymoma viral oncogene homolog I | NM_005163 |
| ANGPT1 | angiopoietin 1 | NM_001145 |
| ANGPT2 | angiopoietin 2 | NM_001147 |
| APAF1 | Apoptotic Protease Activating Factor 1 | NM_013229 |
| ATM | ataxia telangiectasia mutated (includes complementation groups A, C and D) | NM_138293 |
| BAD | BCL2-antagonist of cell death | NM_004322 |
| BAX | BCL2-associated X protein | NM_138761 |
| BCL2 | BCL2-antagonist of cell death | NM_004322 |
| BRAF | γ-raf murine sarcoma viral oncogene homolog B1 | NM_004333 |
| BRCA1 | breast cancer 1, early onset | NM_007294 |
| CASP8 | caspase 8, apoptosis-related cysteine peptidase | NM_001228 |
| CCNE1 | Cyclin E1 | NM_001238 |
| CDC25A | cell division cycle 25A | NM_001789 |
| CDK2 | cyclin-dependent kinase 2 | NM_001798 |
| CDK4 | cyclin-dependent kinase 4 | NM_000075 |
| CDK5 | Cyclin-dependent kinase 5 | NM_004935 |
| CDKN1A | cyclin-dependent kinase inhibitor 1A (p21, Cip1) | NM_000389 |
| CDKN2A | cyclin-dependent kinase inhibitor 2A (melanoma, p16, inhibits CDK4) | NM_000077 |
| CFLAR | CASP8 and FADD-like apoptosis regulator | NM_003879 |
| COL18A1 | collagen, type XVIII, alpha1 | NM_030582 |
| E2F1 | E2F transcription factor 1 | NM_005225 |
| EGFR | epidermal growth factor receptor (crythroblastic leukemia viral (v-erb-b) oncogene homolog, avian) | NM_005228 |
| EGR1 | Early growth response-1 | NM-001964 |
| ERBB2 | V-erb-b2 erythroblastic leukemia viral oncogene homolog 2, neuro/glioblastoma derived oncogene homolog (avian) | NM_004448 |
| FAS | Fas (TNF receptor superfamily, member 6) | NM_000043 |
| FGFR2 | fibroblast growth factor receptor 2 (bacteria-expressed kinase, keratinocyte growth factor receptor, craniofacial dysostosis 1) | NM_000141 |
| FOS | v-fos FBJ murine osteosarcoma viral oncogene homolog | NM_005252 |

(continued)

| Gene Symbol | Gene Name | Gene Accession Number |
|---|---|---|
| GZMA | Granyme A (granzyme 1, cytotoxic T-lymphocyte-associated serine esterase 3) | NM_006144 |
| HRAS | v-Ha-ras Harvey rat sarcoma viral oncogene homolog | NM_005343 |
| ICAM1 | Intercellular adhesion molecule 1 | NM_000201 |
| IF16 | interferon, alpha-inducible protein 6 | NM_002038 |
| IFITM1 | interferon induced transmembrane protein 1 (9-27) | NM_00341 |
| IFNG | interferon gamma | NM_000619 |
| IGF1 | insulin-like growth factor 1 (somatomedin C) | NM_000618 |
| IGFBP3 | insulin-like growth factor binding protein 3 | NN_001013398 |
| IL18 | Interleukin 18 | NM_001562 |
| IL1B | Interleukin 1, beta | NM_000576 |
| IL8 | interleukin 8 | NK_000584 |
| ITGA1 | integrin, alpha 1 | NM_181501 |
| ITGA3 | integrin, alpha 3 (antigen CD49C, alpha 3 subunit of VLA-3 receptor) | NM_005501 |
| ITGAE | integrin, alpha E (antigen CD103, human mucosal lymphocyte antigen 1; alpha polypeptide) | N_002208 |
| ITGB1 | integrin, beta I (fibronectin receptor, beta polypeptide, antigen CD29 includes MDF2, MSK12) | NM_002211 |
| JUN | v-jun sarcoma virus 17 oncogene homolog (avian) | NM_002228 |
| KDR | kinase insert domain receptor (a type III receptor tyrosine kinase) | NM_002253 |
| MCAM | melanoma cell adhesion molecule | NM_006500 |
| MMP2 | matrix metallopeptidase 2 (gelatinase A, 72kDa gelatinase, 72kDa type IV collagenase) | NM_04530 |
| NMP9 | matrix metallopeptidase 9 (gelatinase B, 92kDa gelatinase, 92kDa type IV collagenase) | NM_004994 |
| MSH2 | mutS homolog 2, colon cancer, nonpolyposis type I (E. coli) | NM_000251 |
| MYC | v-myc myelocytomatosis viral oncogene homolog (avian) | NM_002467 |
| MYCL1 | v-myc myelocytomatosis viral oncogene homolog 1, lung carcinoma derived (avian) | NM-001033081 |
| NFKB1 | nuclear factor of kappa light polypeptide gene enhancer in B-cells 1 (p105) | NM_003998 |
| NME1 | non-metastatic cells 1, protein (NM23A) expressed in | NM_198175 |
| NME4 | non-metastatic cells 4, protein expressed in | NM_005009 |
| NOTCH2 | Notch homolog 2 | NM_024408 |
| NOTCH4 | Notch homolog 4 (Drosophila) | NM_004557 |
| NRAS | neuroblastoma RAS viral (v-ras) oncogene homolog | NM_002524 |
| PCNA | proliferating cell nuclear antigen | NM_002592 |
| PDGFRA | platelet-derived growth factor receptor, alpha polypeptide | NM_006206 |
| PLAU | plasminogen activator, urokinase | NM_002658 |
| PLAUR | plasminogen activator, urokinase receptor | NM_002659 |

(continued)

| Gene Symbol | Gene Name | Gene Accession Number |
|---|---|---|
| PTCH1 | patched homolog 1 (Drosophila) | NK_000264 |
| PTEN | phosphatase and tensin homolog (mutated in multiple advanced cancers 1) | NM_000314 |
| RAF1 | v-raf- murine leukemia viral oncogene homolog 1 | NM_002880 |
| RB1 | retinoblastoma I (including osteosarcoma) | NM_000321 |
| RHOA | ras homolog gene family, member A | NM_001664 |
| RHOC | ras homolog gene family, member C | NM_175744 |
| S100A4 | S100 calcium binding protein A4 | NM_002961 |
| SEMA4D | sema domain, immunoglobulin domain (Ig), transmembrane domain (TM) and short cytoplasmic domain, (semaphorin) 4D | NM_006378 |
| SERPINB5 | serpin Peptidase inhibitor, clade B (ovalbumin), member 5 | NM_002639 |
| SERPINE1 | serpin peptidase inhibitor, clade E (nexin, plasminogen activator inhibitor type I), member 1 | NM_000602 |
| SKI | v-ski sarcoma viral oncogene homolog (avian) | NM_003036 |
| SKIL | SKI-like oncogene | NM_005414 |
| SMAD4 | SMAD family member 4 | NM_005359 |
| SOCS1 | suppressor of cytokine signaling 1 | NM_003745 |
| SRC | v-src sarcoma (Schmidt-Ruppin A-2) viral oncogene homolog (avian) | NM_198291 |
| TERT | telomerase-reverse transcriptase | NM_003219 |
| TGFB1 | transforming growth factor, beta 1 (Camurati-Engelmann disease) | NM_000660 |
| THBS1 | thrombospondin 1 | NM_003246 |
| TEMP1 | tissue inhibitor of metalloproteinase 1 | NM_003254 |
| TIMP3 | Tissue inhibitor of metalloproteinase 3 (Sorsby fundus dystrophy, pseudoinflammatory) | NM_000362 |
| TNF | tumor necrosis factor (TNF superfamily, member 2) | NM_000594 |
| TNFRSF10A | tumor necrosis factor receptor superfamily, member 10a | NM_003844 |
| TNFRSF10B | tumor necrosis factor receptor superfamily, member 10b | NM_003842 |
| TNFRSF1A | tumor necrosis factor receptor superfamily, member 1A | NM_001065 |
| TP53 | tumor protein p53 (Li-Fraumeni syndrome) | NM_000546 |
| VEGF | vascular endothelial growth factor | NM_003376 |
| VHL | von Hippel-Lindau tumor suppressor | NM_000551 |
| WNT1 | wingless-type MMTV integration site family, member 1 | NM_005430 |
| WT1 | Wilms tumor 1 | NM_000378 |

**TABLE 4:** Precision Profile™ for EGR1

| Gene Symbol | Gene Name | Gene Accession Number |
|---|---|---|
| ALOX5 | arachidonate 5-lípoxygenase | NM_000698 |
| APOA1 | apolipoprotein A-I | NM_000039 |
| CCND32 | cyclin D2 | NM_001759 |

(continued)

| Gene Symbol | Gene Name | Gene Accession Number |
|---|---|---|
| CDKN2D | cyclin-dependent kinase inhibitor 2D (p19, inhibits CDK4) | NM_001800 |
| CEBPB | CCAAT/enhancer binding protein(C/EBP), beta | NM_005194 |
| CREBBP | CREB binding protein (Rubinstein-Taybi syndrome) | NM_004380 |
| EGFR | epidermal growth factor receptor (erythroblastic leukemia viral (v-erb-b) oncogene homolog, avian) | NM_005228 |
| EGR1 | early growth response 1 | NM_001964 |
| EGR2 | early growth response 2 (Krox-20 homolog, Drosophila) | NK_000399 |
| EGR3 | early growth response 3 | NM_004430 |
| EGR4 | early growth response 4 | NM_001965 |
| FP300 | E1A binding protein p300 | NM_001429 |
| F3 | coagulation factor III (thromboplastin, tissue factor) | NM_001993 |
| FGF2 | fibroblast growth factor 2 (basic) | NM_002006 |
| FN1 | fibronectin 1 | NM_00212482 |
| FOS | v-fos FBJ murine osteosarcoma viral oncogene homolog | NM_005252 |
| ICAM1 | Intercellular adhesion molecule 1 | NM_000201 |
| JUN | jun oncogene | NM_002228 |
| MAP2K1 | mitogen-activated protein kinase kinase 1 | NM_002755 |
| MAPK1 | mitogen-activated protein kinase 1 | NM_002745 |
| NAB1 | NGFT-A binding protein 1 (EGR1 binding protein 1) | NM_005966 |
| NAB2 | NGF1-A binding protein 2 (EGR1 binding protein 2) | NM_005967 |
| NFATC2 | nuclear factor of activated T-cells, cytoplasmic, calcineurin-dependent 2 | NM_173091 |
| NFkB1 | nuclear factor of kappa light polypeptide gene enhancer in B-cell 1 (p105) | NM_003998 |
| NR4A2 | nuclear receptor subfamily 4, group A, member 2 | NM_006186 |
| PDGFA, | platelet-derived growth factor alpha polypeptide | NM_002607 |
| PLAU | plasminogen activator, urokinase | NM_00658 |
| PTEN | phosphatase and tensin homolog (mutated in multiple advanced cancers 1) | NM_000314 |
| RAF1 | γ-raf-1 murine leukemia viral oncogene homolog 1 | NM_002880 |
| S100A6 | S100 calcium binding protein A6 | NM_014624 |
| SERPINE1 | serpin peptidase inhibitor, clade E (nexin, plasminogen activator inhibitor type 1), member 1 | NM_000302 |
| SMAD3 | SMAD, mothers against DPP homolog 3 (Drosophila | NM_005902 |
| SRF | v-src sarcoma (Schmide-Ruppin A-2) viral oncogene homolog (avian) | NM_198291 |
| TGFB1 | transforming growth factor, beta 1 | NM_000660 |
| THBS1 | thombospondin 1 | NM_003246 |
| TOPBP1 | topoisomerase (DNA) II binding protein 1 | NM_007027 |
| TNFRSF6 | Fas (TNF receptor superfamily, member 6) | NK_000043 |

(continued)

| Gene Symbol | Gene Name | Gene Accession Number |
|---|---|---|
| TP53 | tumor protein p53 (Li-Fraumeni syndrome) | NM_000546 |
| WT1 | Wilms tumor 1 | NM_000378 |

**Table 5:** Cross-Cancer Precision Profile™

| Gene Symbol | Gene Name Number | Gene Accession |
|---|---|---|
| ACPP | acid phosphatase, prostate | NM_001099 |
| ADAM17 | a disintegrin and metalloproteinase domain 17 (tumor necrosis factor, alpha, converting enzyme) | NM_003183 |
| ANLN | anillin, actin binding protein (scraps homolog, Drosophila) | NM_018685 |
| APC | adenomatosis polyposis coli | NM_000038 |
| AXIN2 | axin 2 (conductin, axil) | NM_004655 |
| BAX | BCL2-associated X protein | MM_138761 |
| BCAM | basal cell adhesion molecule (Lutheran blood group) | NM_05581 |
| C1QA | complement component 1, q subcomponent, alpha polypeptide | NM_015991 |
| C1QB | complement component 1, q subcomponent, B chain | NM_000491 |
| CA4 | carbonic anhydrase IV | NM_000717 |
| CASP3 | caspase 3, apoptosis-related cysteine peptidase | NM_004346 |
| CASP9 | caspase 9, apoptosis-related cysteine peptidase | NM-001229 |
| CAV1 | caveolin 1, caveolae protein, 22kDa | NM_001753 |
| CCL3 | chemokine (C-C motif) ligand 3 | NM_002983 |
| CCL5 | chemokine (C-C motif) ligand 5 | NM_002985 |
| CCR7 | chemokine (C-C motif) receptor 7 | NM_001838 |
| CD40LG | CD40 ligand (TNF superfamily, member 5, hyper-IgM syndrome) | NM_000074 |
| CD59 | CD59 antigen p18-20 | NM_000611 |
| CD97 | CD97 molecule | NM_075481 |
| CDH1 | cadherin 1, type 1, E-cadherin (epithelial) | NM_004360 |
| CEACAM1 | carcinoembryonic antigen-related cell adhesion molecule I (biliary glycoprotein) | NM_001712 |
| CNKSR2 | connector enhancer of kinase suppressor of Ras 2 | NM_014927 |
| CTNNA1 | catenin (cadherin-associated protein), alpha 1, 102kDa | NM_001903 |
| CTSD | cathopsin D (lysosomal aspartyl peptidase) | NM_001909 |
| CXCL1 | chemokine (C-X-C motif) ligand I (melanoma growth stimulating activity, alpha) | NM_001511 |
| DAD1 | defender against cell death 1 | NM_001344 |
| DIABLO | diablo homolog (Drosophila) | NM_019887 |
| DLC1 | deleted in liver cancer 1 | NM_182643 |
| E2F1 | E2F transcription factor 1 | NM_005225 |
| EGR1 | early growth response-1 | NM_001964 |

(continued)

| Gene Symbol | Gene Name Number | Gene Accession |
|---|---|---|
| ELBA2 | elastase 2, neutrophil | NM_001972 |
| ESR1 | estrogen receptor 1 | NM_000125 |
| ESR2 | estrogen receptor 2 (ER beta) | NM_001437 |
| ETS2 | v-ets erythroblastosis virus E26 oncogene homolog 2 (avian) | NM_005239 |
| FOS | γ-fos FBJ murine osteosarcoma viral oncogene homolog | NM_005252 |
| G6PD | glucose-6-phosphate dehydrogenase | NM_000402 |
| GADD45A | growth arrest and DNA-damage-inducible, alpha | NM_001924 |
| GNB1 | guanine nucleotide binding protein (G protein), beta polypeptide 1 I | NM_002074 |
| GSK3B | glycogen synthase kinase 3 beta | NM_002093 |
| HMGA1 | high nobility group AT-hook 1 | NM_145899 |
| HMOX1 | heme oxygenase (decycling) 1 | NM_002133 |
| HOXA10 | homeobox A10 | NM_018951 |
| HSPA1A | heat shock protein 70 | NM_005345 |
| IFI16 | interferon inducible protein 16, gamma | NM_005531 |
| IGF2BP2 | insulin-like growth factor 2 mRNA binding protein 2 | NM_006548 |
| IGFBP3 | insulin-like growth factor binding protein 3 | NM_001013398 |
| IKBKE | inhibitor of kappa light polypeptide gene enhancer in B-cells, kinase epsilon | NM_014002 |
| IL8 | interleukin 8 | NM_000584 |
| ING2 | inhibitor of growth family, member 2 | NM_001564 |
| IQGAP1 | IQ motif containing GTPase activating protein 1 | NM_003870 |
| IRF1 | interferon regulatory factor 1 | NM_002198 |
| ITGAL | integrin, alpha L (antigen CD11A (p180), lymphocyte function-associated antigen 1; alpha polypeptide) | NM_002209 |
| LARGE | like-glycosyltransferase | NM_004737 |
| LGALS8 | lectin, galactoside-binding, soluble, 8 (galectin 8) | NM_006499 |
| LTA | lymphotoxin alpha (TNF superfamily, member 1) | NM_000595 |
| MAPK14 | mitogen-activated protein kinase 14 | NM_001315 |
| MCAM | melanoma cell adhesion molecule | NM_006500 |
| MEIS1 | Meis1, myeloid ecotropic viral integration site I homolog (mouse) | NM_002398 |
| MLH1 | mutL homolog 1, colon cancer, nonpolyposis type 2 (E. coli) | NM_000249 |
| MME | membrane metallo-endopeptidase (neutral endopeptidase, enkephalinase, CALLA, CD10) | NM_000902 |
| MMP9 | matrix metallopeptidase 9 (gelatinase B, 92kDa gelatinase, 92kDa type IV collagenase) | NM_004994 |
| MNDA | myeloid cell nuclear differentiation antigen | NM_002432 |
| MSH2 | mutS homolog 2, colon cancer, nonpolyposis type 1 (E. coli) | NM_000251 |
| MSH6 | mutS homolog 6 (E. coli) | NM_000179 |
| MTA1 | metastasis associated I | NM_004689 |
| MTF1 | metal-regulatory transcription factor 1 | NM_005955 |

(continued)

| Gene Symbol | Gene Name Number | Gene Accession |
|---|---|---|
| MYC | v-myc myelocytomatosis viral oncogene homolog (avian) | NM_002467 |
| MYD88 | myeloid differentiation primary response gene (88) | NM_002468 |
| NBEA | neurobeachin | NM_015678 |
| NCOA1 | nuclear receptor coactivator 1 | NM_003743 |
| NEDD4L | neural precursor cell expressed, developmentally down-regulated 4-like | NM-015277 |
| NRAS | neuroblastoma RAS viral (v-ras) oncogene homolog, | NM_002524 |
| NUDT4 | nudix (nucleoside diphosphate linked moiety X)-type motif 4 | NM_019094 |
| PLAU | plasminogen activator, urokinase | NM_002658 |
| PLEK2 | pleckstrin 2 | NM_016445 |
| PLXDC2 | plexin domain containing 2 | NM_032812 |
| PPARG | peroxisome proliferative activated receptor, gamma | NM_138712 |
| PTEN | phosphatase and tensin homolog (mutated in multiple advanced cancers 1) | NM_000314 |
| PTGS2 | prostaglandin-endoperoxide synthase 2 (prostaglandin G/H synthase and cyclooxygenase) | NM_000963 |
| PTPRC | protein tyrosine phosphatase, receptor type, C | NM_002838 |
| PTPRK | protein tyrosine phosphatase, receptor type, K | NM_002844 |
| RBM5 | RNA binding motif protein 5 | NM_005778 |
| RP5-1077B9.4 | invasion inhibitory protein 45 | NM_001025374 |
| S100A11 | S100 calcium binding protein A11 | NM_005620 |
| S100A4 | S100 calcium binding protein A4 | NM_002961 |
| SCGB2A1 | secretoglobin, family 2A, member 1 | NM_002407 |
| SERPINA1 | serine (or cysteine) proteinase inhibitor, clade A (alpha-1 antiproteinase, antitrypsin), member 1 | NM_000295 |
| SERPINE1 | serpin peptidase inhibitor, clade E (nexin, plasminogen activator inhibitor type 1), member 1 | NM_000602 |
| SERPING1 | serpin peptidase inhibitor, clade G (C1 inhibitor), member 1, (angioedema, hereditary) | NM_000062 |
| SIAH2 | seven in absentia homolog 2 (Drosophila) | NM_005067 |
| SLC43A1 | solute carrier family 43, member | NM_003627 |
| SP1 | Sp1 transcription factor | NM_138473 |
| SPARC | secreted protein, acidic, cysteine-rich (osteonectin) | NM_003118 |
| SRF | serum response factor (c-fos serum response element-binding transcription factor) | NM_003131 |
| ST14 | suppression of tumorigenicity 14 (colon carcinoma) | N_021978 |
| TEGT | testis enhanced gene transcript (BAX inhibitor 1) | NM_003217 |
| TGFB1 | transforming growth factor, beta I (Camurati-Engelmann disease) | NM_000660 |
| TIMP1 | tissue inhibitor of metalloproteinase 1 | NM_003254 |
| TLR2 | toll-like receptor 2 | NM_003264 |
| TNF | tumor necrosis factor (TNF superfamily, member 2) | NM_000594 |

(continued)

| Gene Symbol | Gene Name Number | Gene Accession |
|---|---|---|
| **TNFRSF1A** | tumor necrosis factor receptor superfamily, member 1A | NM_001065 |
| **TXNRD1** | thioredoxin reductase | NM_003330 |
| **UBE2C** | ubiquitin-conjugating enzyme E2C | NM_007019 |
| **USP7** | ubiquitin specific peptidase 7 (herpes virus-associated) | NM_003470 |
| **VEGFA** | vascular endothelial growth factor | NM_003376 |
| **VIM** | vimentin | NM_003380 |
| **XK** | X-linked Kx blood group (McLeod syndrome) | NM_021083 |
| **XRCC1** | X-ray repair complementing defective repair in Chinese hamster cells 1 | NM_006297 |
| **ZNF185** | zinc finger protein 185 (LIM domain) | NM_007150 |
| **ZNF350** | zinc finger protein 350 | NM_021632 |

**TABLE 6**: Precision Profile™ for Immunotherapy

| Gene Symbol |
|---|
| ABL1 |
| ABL2 |
| MADAM17 |
| ALOX5 |
| CD19 |
| CD4 |
| CD40LG |
| CD86 |
| CCR5 |
| CTLA4 |
| EGFR |
| ERBB2 |
| HSPA1A |
| IFNG |
| IL12 |
| IL15 |
| IL23A |
| KIT |
| MUC1 |
| MYC |
| PDGFRA |
| PTGS2 |
| PTPRC |
| RAF1 |
| TGFB1 |

(continued)

| Gene Symbol |
| --- |
| TLR2 |
| TNF |
| TNFRSF10B |
| TNFRSF13B |
| VEGF |

Table 1A

| | | | | | | Normal | Colon | | | | |
|---|---|---|---|---|---|---|---|---|---|---|---|
| | | | | | N= | 50 | 19 | | | | |
| 2-gene models and | | Entropy | #normal | #normal | #cc | #cc | Correct | Correct | | | total used (excludes missing) | |
| 1-gene models | | R-sq | correct | FALSE | Correct | FALSE | Classification | Classification | p-val 1 | p-val 2 | # normals | # disease |
| MSH6 | PSEN2 | 0.55 | 42 | 6 | 16 | 3 | 87.5% | 84.2% | 6.6E-11 | 1.2E-06 | 48 | 19 |
| CA4 | MME | 0.49 | 44 | 6 | 17 | 2 | 88.0% | 89.5% | 2.2E-08 | 1.3E-08 | 50 | 19 |
| APC | CFLAR | 0.45 | 43 | 7 | 16 | 3 | 86.0% | 84.2% | 1.8E-09 | 2.2E-06 | 50 | 19 |
| AXIN2 | MUTYH | 0.44 | 39 | 10 | 16 | 3 | 79.6% | 84.2% | 2.4E-09 | 0.0012 | 49 | 19 |
| MSH6 | MUTYH | 0.44 | 43 | 6 | 16 | 3 | 87.8% | 84.290 | 3.0E-09 | 0.0001 | 49 | 19 |
| MSH2 | PSEN2 | 0.42 | 41 | 8 | 16 | 3 | 83.7% | 84.2% | 1.1E-08 | 0.0017 | 49 | 19 |
| AXIN2 | TNF | 0.41 | 41 | 9 | 15 | 4 | 82.0% | 79.0% | 1.6E-06 | 0.0054 | 50 | 19 |
| AXIN2 | IGFBP4 | 0.39 | 42 | 8 | 16 | 3 | 84.0% | 84.2% | 2.2E-08 | 0.0095 | 50 | 19 |
| MSH2 | MUTYH | 0.39 | 39 | 10 | 15 | 4 | 79.6% | 79.0% | 2.3E-08 | 0.0093 | 49 | 19 |
| BAX | MSH6 | 0.39 | 42 | 7 | 16 | 3 | 85.7% | 84.2% | 0.0011 | 5.6E-08 | 49 | 19 |
| AC5L5 | AXIN2 | 0.39 | 39 | 11 | 16 | 3 | 78.0% | 84.2% | 0.0143 | 2.2E-08 | 50 | 19 |
| AXIN2 | MSH2 | 0.38 | 44 | 6 | 15 | 4 | 88.0% | 79.0% | 0.0097 | 0.0149 | 50 | 19 |
| MSH6 | TNF | 0.38 | 39 | 10 | 15 | 4 | 79.6% | 79.0% | 4.7E-06 | 0.0015 | 49 | 19 |
| MSH2 | S100P | 0.38 | 39 | 11 | 15 | 4 | 78.0% | 79.0% | 4.9E-07 | 0.0123 | 50 | 19 |
| MSH6 | NME1 | 0.38 | 40 | 9 | 14 | 4 | 81.6% | 77.8% | 8.0E-08 | 0.0029 | 49 | 18 |
| MSH2 | NME1 | 0.38 | 39 | 11 | 15 | 3 | 78.0% | 83.3% | 7.9E-08 | 0.0178 | 50 | 18 |
| AXIN2 | PSEN2 | 0.37 | 38 | 11 | 16 | 3 | 77.6% | 84.2% | 8.7E-08 | 0.0199 | 49 | 19 |
| ACSL5 | MSH6 | 0.37 | 39 | 10 | 15 | 4 | 79.6% | 79.0% | 0.0021 | 4.5E-08 | 49 | 19 |
| MSH6 | VEGF | 0.37 | 43 | 6 | 15 | 4 | 87.8% | 79.0% | 8.8E-08 | 0.0023 | 49 | 19 |
| CD63 | MSH6 | 0.37 | 40 | 9 | 15 | 4 | 81.6% | 79.0% | 0.0024 | 5.0E-08 | 49 | 19 |
| APC | AXIN2 | 0.37 | 40 | 10 | 15 | 4 | 80.0% | 79.0% | 0.0350 | 6.3E-05 | 50 | 19 |
| MSH6 | TP53 | 0.37 | 37 | 12 | 14 | 4 | 75.5% | 77.8% | 1.8E-07 | 0.0051 | 49 | 18 |

54

| | | | | | | Normal | Colon | | | | |
|---|---|---|---|---|---|---|---|---|---|---|---|
| | | | | | N= | 50 | 19 | | | | |
| 2-gene models and | | Entropy | #normal | #normal | #cc | #cc | Correct | Correct | | | total used (excludes missing) |
| 1-gene models | | R-sq | correct | FALSE | Correct | FALSE | Classification | Classification | p-val 1 | p-val 2 | # normals | # disease |
| CFLAR | MSH2 | 0.37 | 41 | 9 | 16 | 3 | 82.0% | 84.2% | 0.0229 | 5.0E-08 | 50 | 19 |
| AXIN2 | MSH6 | 0.36 | 43 | 6 | 15 | 4 | 87.8% | 79.0% | 0.0030 | 0.0425 | 49 | 19 |
| MSH26 | S100A4 | 0.36 | 38 | 10 | 15 | 4 | 79.2% | 79.0% | 2.5E-07 | 0.0027 | 48 | 19 |
| AXIN2 | GSK3B | 0.36 | 43 | 7 | 15 | 4 | 86.0% | 79.0% | 3.4E-06 | 0.0415 | 50 | 19 |
| AXIN2 | MME | 0.36 | 40 | 10 | 16 | 3 | 80.0% | 84.2% | 4.8E-06 | 0.0439 | 50 | 19 |
| CFLAR | MSH6 | 0.36 | 40 | 9 | 15 | 4 | 81.6% | 79.0% | 0.0035 | 7.0E-08 | 49 | 19 |
| MSH2 | TNF | 0.36 | 41 | 9 | 16 | 3 | 82.0% | 84.2% | 1.1E-05 | 0.0294 | 50 | 19 |
| MSH2 | VEGF | 0.36 | 41 | 9 | 16 | 3 | 82.0% | 84.2% | 1.3E-07 | 0.0295 | 50 | 19 |
| MSH2 | RPS3A | 0.36 | 38 | 12 | 15 | 4 | 76.0% | 79.0% | 1.2E-07 | 0.0305 | 50 | 19 |
| AXIN2 | MYC | 0.36 | 44 | 6 | 15 | 4 | 88.0% | 79.0% | 8.9E-08 | 0.0475 | 50 | 19 |
| AXIN2 | ZNF350 | 0.36 | 38 | 12 | 15 | 4 | 76.0% | 79.0% | 1.0E-05 | 0.0479 | 50 | 19 |
| MSH2 | S100A4 | 0.35 | 41 | 8 | 15 | 4 | 83.7% | 79.0% | 4.1E-07 | 0.0341 | 49 | 19 |
| MSH6 | S100P | 0.34 | 37 | 12 | 15 | 4 | 75.5% | 79.0% | 2.6E-06 | 0.0098 | 49 | 19 |
| GADD45A | GSK3B | 0.33 | 42 | 8 | 15 | 4 | 84.0% | 79.0% | 1.4E-05 | 4.9E-05 | 50 | 19 |
| MGMT | MSH6 | 0.33 | 39 | 9 | 15 | 4 | 81.3% | 79.0% | 0.0142 | 3.0E-07 | 48 | 19 |
| IGFBP4 | MSH6 | 0.33 | 42 | 7 | 15 | 4 | 85.7% | 79.0% | 0.0164 | 3.7E-07 | 49 | 19 |
| CCND3 | MSH6 | 0.32 | 38 | 10 | 15 | 4 | 79.2% | 79.0% | 0.0231 | 1.1E-06 | 48 | 19 |
| AXIN2 | | 0.31 | 40 | 10 | 15 | 4 | 80.0% | 79.0% | 4.9E-07 | | 50 | 19 |
| MSH6 | VIL1 | 0.31 | 37 | 12 | 15 | 4 | 75.5% | 79.0% | 2.5E-05 | 0.0357 | 49 | 19 |
| CD44 | MSH6 | 0.31 | 37 | 12 | 15 | 4 | 75.5% | 79.0% | 0.0384 | 1.4E-06 | 49 | 19 |
| MSH6 | RPS3A | 0.31 | 38 | 11 | 15 | 4 | 77.6% | 79.0% | 1.2E-06 | 0.0442 | 49 | 19 |
| MSH2 | | 0.30 | 38 | 12 | 15 | 4 | 76.0% | 79.0% | 7.2E-07 | | 50 | 19 |

| 2-gene models and | | Entropy | #normal | #normal | #cc | #cc | Normal<br>50<br>Correct<br>Classification | Colon<br>19<br>Correct<br>Classification | | | total used (excludes missing) | |
|---|---|---|---|---|---|---|---|---|---|---|---|---|
| 1-gene models | | R-sq | correct | FALSE | Correct | FALSE | | | p-val 1 | p-val 2 | # normals | # disease |
| CA4 | GSK3B | 0.29 | 40 | 10 | 15 | 4 | 80.0% | 79.0% | 7.2E-05 | 5.7E-05 | 50 | 19 |
| APC | S100P | 0.28 | 38 | 12 | 15 | 4 | 76.0% | 79.0% | 3.0E-05 | 0.0024 | 50 | 19 |
| ITGA3 | TNF | 0.28 | 40 | 10 | 15 | 4 | 80.0% | 79.0% | 0.0004 | 1.9E-05 | 50 | 19 |
| CD44 | NFKB1 | 0.26 | 39 | 11 | 15 | 4 | 78.0% | 79.0% | 1.7E-05 | 9.6E-06 | 50 | 19 |
| APC | VEGF | 0.26 | 40 | 10 | 15 | 4 | 80.0% | 79.0% | 9.4E-06 | 0.0070 | 50 | 19 |
| APC | NME1 | 0.26 | 39 | 11 | 14 | 4 | 78.0% | 77.8% | 1.0E-05 | 0.0151 | 50 | 18 |
| MSH6 | | 0.26 | 37 | 12 | 15 | 4 | 75.5% | 79.0% | 5.7E-06 | | 49 | 19 |
| GADD45A | MME | 0.24 | 39 | 11 | 15 | 4 | 78.0% | 79.0% | 0.0010 | 0.0027 | 50 | 19 |
| GADD45A | MLH1 | 0.21 | 42 | 8 | 15 | 4 | 84.0% | 79.0% | 0.0053 | 0.0077 | 50 | 19 |
| ALDH1A1 | TNF | 0.20 | 40 | 10 | 15 | 4 | 80.0% | 79.0% | 0.0103 | 0.0002 | 50 | 19 |
| CA4 | NFKB1 | 0.19 | 39 | 11 | 15 | 4 | 78.0% | 79.0% | 0.0004 | 0.0043 | 50 | 19 |
| BAX | ITGA3 | 0.15 | 39 | 11 | 15 | 4 | 78.0% | 79.0% | 0.0040 | 0.0013 | 50 | 19 |

Table 1B

|  | Colon | Normals | Sum |  |
|---|---|---|---|---|
| Group Size | 27.5% | 72.5% | 100% |  |
| N = | 19 | 50 | 69 |  |
|  |  |  |  |  |
| Gene | Mean | Mean | Z-statistic | p-val |
| AXIN2 | 19.9 | 18.8 | 5.03 | 4.9E-07 |
| MSH2 | 18.5 | 17.7 | 4.96 | 7.2E-07 |
| MSH6 | 19.7 | 19.0 | 4.54 | 5.7E-06 |
| APC | 18.2 | 17.5 | 3.71 | 0.0002 |
| GADD45A | 18.8 | 19.5 | -3.20 | 0.0014 |
| TNF | 18.1 | 18.5 | -3.16 | 0.0016 |
| ZNF350 | 19.6 | 19.1 | 3.13 | 0.0018 |
| MLH1 | 18.0 | 17.5 | 3.10 | 0.0019 |
| MME | 15.4 | 14.8 | 2.91 | 0.0036 |
| GSK3B | 16.2 | 15.8 | 2.81 | 0.0050 |
| CA4 | 18.1 | 18.8 | -2.72 | 0.0065 |
| VIL1 | 19.9 | 20.6 | -2.69 | 0.0072 |
| TGFBR1 | 18.6 | 18.3 | 2.57 | 0.0103 |
| CA2 | 16.3 | 16.7 | -2.39 | 0.0167 |
| S100P | 16.3 | 17.2 | -2.35 | 0.0189 |
| BCL2 | 16.4 | 16.1 | 2.06 | 0.0397 |
| ITGA3 | 22.2 | 21.9 | 2.03 | 0.0427 |
| NFKB1 | 16.9 | 16.7 | 1.72 | 0.0859 |
| ALDH1A1 | 18.6 | 18.3 | 1.69 | 0.0914 |
| S100A4 | 12.9 | 13.1 | -1.68 | 0.0932 |
| IL8 | 22.2 | 21.7 | 1.64 | 0.1010 |
| BAX | 15.3 | 15.5 | -1.43 | 0.1529 |
| CCND3 | 14.1 | 14.3 | -1.38 | 0.1673 |
| CD44 | 13.7 | 13.9 | -1.34 | 0.1791 |
| ACSS2 | 19.3 | 19.1 | 1.29 | 0.1959 |
| AFAP | 18.3 | 18.1 | 1.25 | 0.2118 |
| PSEN2 | 19.4 | 19.6 | -1.22 | 0.2230 |
| VEGF | 23.0 | 23.3 | -1.18 | 0.2395 |
| CFD | 13.8 | 14.1 | -1.11 | 0.2684 |
| RPS3A | 15.9 | 16.1 | -1.10 | 0.2697 |
| TP53 | 16.0 | 15.9 | 1.03 | 0.3039 |
| ERBB2 | 22.4 | 22.2 | 1.02 | 0.3078 |

(continued)

| Gene | Mean | Mean | Z-statistic | p-val |
|------|------|------|-------------|-------|
| ZYX | 12.1 | 12.3 | -1.00 | 0.3173 |
| NME1 | 19.2 | 19.3 | -0.93 | 0.3537 |
| IGFBP4 | 21.3 | 21.4 | -0.83 | 0.4078 |
| CXCL1 | 19.1 | 19.3 | -0.81 | 0.4202 |
| BRAF | 17.2 | 17.1 | 0.80 | 0.4233 |
| MYC | 18.2 | 18.1 | 0.80 | 0.4247 |
| TCF4 | 19.6 | 19.5 | 0.78 | 0.4363 |
| RGC32 | 18.0 | 17.9 | 0.57 | 0.5685 |
| CD63 | 15.0 | 15.0 | -0.42 | 0.6754 |
| NUAK1 | 23.4 | 23.5 | -0.42 | 0.6774 |
| PTGS2 | 17.1 | 17.1 | -0.42 | 0.6780 |
| MUTYH | 19.4 | 19.4 | -0.39 | 0.6961 |
| MGMT | 19.4 | 19.5 | -0.36 | 0.7156 |
| IGF2 | 21.4 | 21.5 | -0.33 | 0.7407 |
| MKI67 | 22.2 | 22.2 | -0.15 | 0.8792 |
| ACSL5 | 17.8 | 17.8 | 0.15 | 0.8832 |
| CFLAR | 14.8 | 14.8 | 0.13 | 0.9000 |

Table 1C

| Patient ID | Group | MSH6 | PSEN2 | logit | odds | Predicted probability of colon cancer |
|------------|-------|------|-------|-------|------|----------------------------------------|
| CC-017 | Colon | 21.71 | 19.51 | 16.26 | 1.2E+07 | 1.0000 |
| CC-019 | Colon | 19.86 | 18.65 | 8.35 | 4.2E+03 | 0.9998 |
| CC-020 | Colon | 20.14 | 19.14 | 7.47 | 1.8E+03 | 0.9994 |
| CC-007 | Colon | 20.91 | 20.20 | 6.60 | 7.3E+02 | 0.9986 |
| CC-003 | Colon | 19.35 | 18.41 | 6.25 | 5.2E+02 | 0.9981 |
| CC-011 | Colon | 19.52 | 19.19 | 2.75 | 1.6E+01 | 0.9400 |
| CC-005 | Colon | 20.21 | 20.04 | 2.61 | 1.4E+01 | 0.9314 |
| CC-014 | Colon | 19.83 | 19.65 | 2.22 | 9.2E+00 | 0.9020 |
| CC-012 | Colon | 19.70 | 19.58 | 1.74 | 5.7E+00 | 0.8506 |
| CC-013 | Colon | 19.76 | 19.72 | 1.33 | 3.8E+00 | 0.7916 |
| CC-002 | Colon | 19.05 | 18.89 | 1.30 | 3.7E+00 | 0.7851 |
| CC-006 | Colon | 19.65 | 19.62 | 1.12 | 3.1E+00 | 0.7542 |
| CC-009 | Colon | 19.07 | 18.98 | 0.85 | 2.3E+00 | 0.6998 |
| CC-010 | Colon | 20.30 | 20.47 | 0.65 | 1.9E+00 | 0.6569 |
| HN-036-CC | Normal | 18.90 | 18.83 | 0.60 | 1.8E+00 | 0.6465 |

(continued)

| Patient ID | Group | MSH6 | PSEN2 | logit | odds | Predicted probability of colon cancer |
|---|---|---|---|---|---|---|
| HN-014-CC | Normal | 19.26 | 19.30 | 0.29 | 1.3E+00 | 0.5710 |
| HN-049-CC | Normal | 19.58 | 19.70 | 0.16 | 1.2E+00 | 0.5404 |
| CC-008 | Colon | 19.82 | 19.99 | 0.13 | 1.1E+00 | 0.5335 |
| HN-046-CC | Normal | 18.86 | 18.88 | -0.05 | 9.5E-01 | 0.4877 |
| HN-030-CC | Normal | 19.82 | 20.05 | -0.23 | 7.9E-01 | 0.4417 |
| HN-004-CC | Normal | 18.76 | 18.90 | -0.86 | 4.2E-01 | 0.2964 |
| CC-018 | Colon | 18.85 | 19.01 | -0.90 | 4.1E-01 | 0.2895 |
| HN-001-CC | Normal | 19.88 | 20.24 | -0.93 | 3.9E-01 | 0.2829 |
| HN-029-CC | Normal | 19.81 | 20.17 | -0.96 | 3.8E-01 | 0.2760 |
| HN-008-CC | Normal | 18.62 | 18.81 | -1.31 | 2.7E-01 | 0.2127 |
| HN-035-CC | Normal | 19.00 | 19.27 | -1.35 | 2.6E-01 | 0.2056 |
| HN-047-CC | Normal | 18.89 | 19.14 | -1.36 | 2.6E-01 | 0.2041 |
| HN-009-CC | Normal | 18.87 | 19.16 | -1.60 | 2.0E-01 | 0.1679 |
| HN-033-CC | Normal | 20.00 | 20.53 | -1.80 | 1.7E-01 | 0.1416 |
| HN-026-CC | Normal | 19.27 | 19.67 | -1.84 | 1.6E-01 | 0.1369 |
| CC-015 | Colon | 19.22 | 19.61 | -1.86 | 1.6E-01 | 0.1344 |
| HN-034-CC | Normal | 19.37 | 19.81 | -1.96 | 1.4E-01 | 0.1236 |
| HN-013-CC | Normal | 18.97 | 19.35 | -2.00 | 1.4E-01 | 0.1191 |
| CC-004 | Colon | 19.24 | 19.67 | -2.03 | 1.3E-01 | 0.1162 |
| HN-044-CC | Normal | 18.53 | 18.86 | -2.27 | 1.0E-01 | 0.0935 |
| HN-041-CC | Normal | 19.00 | 19.47 | -2.54 | 7.9E-02 | 0.0728 |
| HN-024-CC | Normal | 19.48 | 20.05 | -2.56 | 7.7E-02 | 0.0716 |
| HN-010-CC | Normal | 19.00 | 19.48 | -2.63 | 7.2E-02 | 0.0671 |
| HN-040-CC | Normal | 19.40 | 19.97 | -2.67 | 6.9E-02 | 0.0647 |
| HN-048-CC | Normal | 18.68 | 19.14 | -2.83 | 5.9E-02 | 0.0555 |
| CC-001 | Colon | 18.37 | 18.78 | -2.93 | 5.4E-02 | 0.0508 |
| HN-032-CC | Normal | 19.20 | 19.79 | -2.98 | 5.1E-02 | 0.0485 |
| HN-025-CC | Normal | 18.95 | 19.53 | -3.24 | 3.9E-02 | 0.0376 |
| HN-050-CC | Normal | 19.05 | 19.65 | -3.31 | 3.7E-02 | 0.0353 |

Table 1C

| HN-015-CC | Normal | 18.93 | 19.54 | -3.49 | 3.1E-02 | 0.0296 |
|---|---|---|---|---|---|---|
| HN-011-CC | Normal | 19.04 | 19.75 | -3.88 | 2.1E-02 | 0.0201 |
| HN-016-CC | Normal | 19.37 | 20.17 | -4.10 | 1.7E-02 | 0.0162 |
| HN-039-CC | Normal | 18.42 | 19.06 | -4.18 | 1.5E-02 | 0.0151 |
| HN-038-CC | Normal | 18.61 | 19.31 | -4.34 | 1.3E-02 | 0.0129 |

(continued)

| | | | | | | |
|---|---|---|---|---|---|---|
| HN-031-CC | Normal | 18.84 | 19.63 | -4.64 | 9.6E-03 | 0.0095 |
| HN-022-CC | Normal | 19.98 | 21.01 | -4.72 | 8.9E-03 | 0.0088 |
| HN-003-CC | Normal | 18.85 | 19.70 | -4.93 | 7.2E-03 | 0.0072 |
| HN-019-CC | Normal | 18.77 | 19.62 | -5.05 - | 6.4E-03 | 0.0064 |
| HN-023-CC | Normal | 18.52 | 19.33 | -5.08 | 6.2E-03 | 0.0062 |
| HN-043-CC | Normal | 18.59 | 19.42 | -5.12 | 6.0E-03 | 0.0060 |
| HN-04S-CC | Normal | 18.77 | 19.64 | -5.16 | 5.7E-03 | 0.0057 |
| HN-027-CC | Normal | 18.73 | 19.62 | -5.33 | 4.9E-03 | 0.0048 |
| HN-021-CC | Normal | 18.49 | 19.34 | -5.38 | 4.6E-03 | 0.0046 |
| HN-018-CC | Normal | 18.46 | 19.34 | -5.57 | 3.8E-03 | 0.0038 |
| HN-028-CC | Normal | 19.05 | 20.05 | -5.65 | 3.5E-03 | 0.0035 |
| HN-012-CC | Normal | 18.64 | 19.57 | -5.66 | 3.5E-03 | 0.0035 |
| HN-006-CC | Normal | 18.52 | 19.45 | -5.86 | 2.9E-03 | 0.0029 |
| HN-042-CC | Normal | 18.35 | 19.26 | -5.88 | 2.8E-03 | 0.0028 |
| HN-005-CC | Normal | 18.36 | 19.38 | -6.52 | 1.5E-03 | 0.0015 |
| HN-020-CC | Normal | 18.26 | 19.50 | -7.96 | 3.5E-04 | 0.0003 |
| HN-007-CC | Normal | 18.08 | 19.38 | -8.44 | 2.2E-04 | 0.0002 |
| HN-017-CC | Normal | 18.93 | 20.51 | -9.22 | 9.9E-05 | 0.0001 |

Table 2a

| 2-gene models and | 1-gene models | Entropy R-sq | #normals Correct | #normal FALSE | #ci Correct | #ci FALSE | Normal N= 32 Correct Classification | Colon 18 Correct Classification | p-val 1 | p-val 2 | total used (excludes missing) #normal | # disease |
|---|---|---|---|---|---|---|---|---|---|---|---|---|
| HMOX1 | TXNRD1 | 0.67 | 30 | 2 | 17 | 1 | 93.8% | 94.4% | 2.3E-09 | 2.1E-08 | 32 | 18 |
| C1QA | LTA | 0.61 | 28 | 4 | 16 | 2 | 87.5% | 88.9% | 8.3E-08 | 0.0017 | 32 | 18 |
| DPP4 | IL32 | 0.60 | 29 | 3 | 16 | 2 | 90.6% | 88.9% | 5.7E-09 | 6.3E-08 | 32 | 18 |
| C1QA | TXNRD1 | 0.59 | 29 | 3 | 16 | 2 | 90.6% | 88.9% | 3.9E-08 | 0.0030 | 32 | 18 |
| CCR5 | DPP4 | 0.58 | 28 | 4 | 16 | 2 | 87.5% | 88.9% | 1.4E-07 | 6.1E-08 | 32 | 18 |
| C1QA | PTG52 | 0.57 | 29 | 3 | 16 | 2 | 90.6% | 88.9% | 2.5E-09 | 0.0060 | 32 | 18 |
| APAF1 | C1QA | 0.57 | 29 | 3 | 16 | 2 | 30.6% | 88.9% | 0.0069 | S.5E-08 | 32 | 18 |
| CCR5 | LTA | 0.56 | 30 | 2 | 16 | 2 | 93.8% | 88.9% | 3.8E-07 | 1.0E-07 | 32 | 18 |
| C1QA | PTPRC | 0.55 | 27 | 5 | 16 | 2 | 84.4% | 88.9% | 6.1E-09 | 0.0118 | 32 | 18 |
| C1QA | TNFRSF13 | 0.55 | 27 | 5 | 15 | 3 | 84.4% | 83.3% | 9.6E-09 | 0.0118 | 32 | 18 |
| C1QA | IL8 | 0.55 | 29 | 3 | 16 | 2 | 90.6% | 88.9% | 7.3E.07 | 0.0122 | | 18 |
| C1QA | TLR4 | 0.55 | 26 | 6 | 16 | 2 | 81.3% | 88.9% | 7.8E-09 | 0.0126 | 32 | 18 |
| C1QA | CASP3 | 0.54 | 30 | 2 | 16 | 2 | 93.8% | 88.9% | 2.7E-07 | 0.0160 | 32 | 18 |
| C1QA | HSPA1A | 0.54 | 30 | 2 | 16 | 2 | 93.8% | 88.9% | 2.8E-09 | 0.0173 | 32 | 18 |
| TGFB1 | TXNRD1 | 0.54 | 29 | 3 | 15 | 3 | 90.6% | 83.3% | 2.4E-07 | 1.6E-06 | 32 | 18 |
| APAF1 | PLAUR | 0.54 | 28 | 4 | 16 | 2 | 87.5% | 88.9% | 2.7E-07 | 1.6E-07 | 32 | 18 |
| C1QA | MMP12 | 0.53 | 29 | 3 | 16 | 2 | 90.6% | 88.9% | 3.6E-09 | 0.0234 | 32 | 18 |
| C1QA | IL5 | 0.53 | 26 | 6 | 15 | 3 | 81.3% | 83.3% | 5.9E-09 | 0.0250 | 32 | 18 |
| C1QA | IL15 | 0.52 | 27 | 5 | 15 | 3 | 84.4% | 83.3% | 2.0E-08 | 0.0406 | 32 | 18 |
| CCL5 | LTA | 0.51 | 28 | 4 | 16 | 2 | 87.5% | 88.9% | 1.8E-06 | 8.2E-06 | 32 | 18 |
| CCR5 | TNFSF5 | 0.51 | 27 | 5 | 15 | 3 | 84.4% | 83.3% | 9.0E-07 | 5.0E-07 | 32 | 18 |

(continued)

| 2-gene models and | | Entropy | #normals | #normal | #ci | #ci | Normal<br>Correct | Colon<br>Correct | | | total used (excludes missing) | |
|---|---|---|---|---|---|---|---|---|---|---|---|---|
| | | | | | | N= | 32 | 18 | | | | |
| 1-gene models | | R-sq | Correct | FALSE | Correct | FALSE | Classification | Classification | p-val 1 | p-val 2 | #normal | # disease |
| TNF | TNFSF5 | 0.51 | 28 | 4 | 16 | 2 | 87.5% | 88.9% | 9.1E-07 | 6.4E-06 | 32 | 18 |
| CD4 | TGFB1 | 0.51 | 29 | 3 | 15 | 3 | 90.6% | 83.3% | 4.1E-06 | 1.2E-08 | 32 | 18 |
| LTA | TNF | 0.50 | 28 | 4 | 16 | 2 | 87.5% | 88.9% | 9.6E-06 | 2.8E-06 | 32 | 18 |
| NFKB1 | TGFB1 | 0.50 | 31 | 1 | 15 | 3 | 96.9% | 83.3% | 6.2E-O6 | 2.2E-08 | 32 | 18 |
| HMOX1 | PTPRC | 0.49 | 29 | 3 | 16 | 2 | 90.6% | 88.9% | 4.6E-08 | 1.0E-05 | 32 | 18 |
| LTA | TGFB1 | 0.49 | 27 | 5 | 15 | 3 | 84.4% | 83.3% | 7.6E-06 | 4.2E-06 | 32 | 18 |
| APAF1 | TLR2 | 0.48 | 28 | 4 | 16 | 2 | 87.5% | 88.3% | 1.5E-07 | 1.2E-06 | 32 | 18 |
| MAPK14 | TXNRD1 | 0.47 | 25 | 7 | 15 | 3 | 78.1% | 83.3% | 2.3E-06 | 3.7E-08 | 32 | 18 |
| PLAUR | TXNRD1 | 0.47 | 28 | 4 | 15 | 3 | 87.5% | 83.3% | 2.5E-06 | 2.8E-06 | | 18 |
| TIMP1 | TXNRD1 | 0.47 | 26 | 6 | 15 | 3 | 81.3% | 83.3% | 2.6E-06 | 1.8E-07 | 32 | 18 |
| APAF1 | TGFB1 | 0.46 | 28 | 4 | 15 | 3 | 87.5% | 83.3% | 2.2E-05 | 2.1E-06 | 32 | 18 |
| HMOX1 | NFKB1 | 0.46 | 26 | 6 | 15 | 3 | 81.3% | 83.3% | 8.1E-08 | 3.4E-05 | 32 | 18 |
| C1QA | | 0.46 | 28 | 4 | 15 | 3 | 87.5% | 83.3% | 4.9E-08 | | 32 | 18 |
| HMOX1 | LTA | 0.45 | 26 | 6 | 15 | 3 | 81.3% | 83.3% | 1.5E-05 | 3.7E-05 | 32 | 18 |
| IL32 | TNFSF5 | 0.45 | 26 | 6 | 15 | 3 | 81.3% | 83.3% | 6.9E-06 | 7.9E-07 | 32 | 18 |
| IL32 | TOSO | 0.45 | 27 | 4 | 16 | 2 | 87.1% | 88.9% | 9.2E-07 | 1.2E-06 | 31 | 18 |
| ICAM1 | TXNRD1 | 0.45 | 27 | 5 | 15 | 3 | 84.4% | 83.3% | 4.3E-06 | 1.1E-06 | 32 | 18 |
| APAF1 | HMOX1 | 0.45 | 27 | 5 | 16 | 2 | 84.4% | 88.9% | 4.5E-05 | 3.0E-06 | 32 | 18 |
| APAF1 | CASP1 | 0.44 | 26 | 6 | 15 | 3 | 81.3% | 83.3% | 5.7E-07 | 3.5E-06 | 32 | 18 |
| CCL5 | TNFSF5 | 0.44 | 27 | 5 | 15 | 3 | 84.4% | 83.3% | 9.8E-06 | 9.4E-05 | 32 | 18 |
| DPP4 | TNF | 0.44 | 27 | 5 | 15 | 3 | 84.4% | 83.3% | 7.1E-05 | 1.3E-05 | 32 | 18 |

(continued)

| 2-gene models and | | Entropy | #normals | #normal | #ci | #ci | Normal | Colon | | | total used (excludes missing) | |
|---|---|---|---|---|---|---|---|---|---|---|---|---|
| | | | | | | N= | 32 | 18 | | | | |
| | | | | | | | Correct | Correct | | | | |
| 1-gene models | | R-sq | Correct | FALSE | Correct | FALSE | Classification | Classification | p-val 1 | p-val 2 | #normal | # disease |
| IL18BP | TOSO | 0.44 | 25 | 5 | 15 | 3 | 83.3% | 83.3% | 2.6E-06 | 6.2E-07 | 30 | 18 |
| CCL5 | TOSO | 0.43 | 25 | 6 | 15 | 3 | 80.7% | 83.3% | 1.7E-06 | 0.0002 | 31 | 18 |
| CCR5 | TOSO | 0.43 | 24 | 7 | 14 | 4 | 77.4% | 77.8% | 2.1E-06 | 2.2E-05 | 31 | 18 |
| CCL5 | DPP4 | 0.42 | 27 | 5 | 15 | 3 | 84.4% | 83.3% | 2.7E-05 | 0.0002 | 32 | 18 |
| TGFB1 | TNFSF5 | 0.42 | 26 | 6 | 14 | 4 | 81.3% | 77.8% | 2.5E-05 | 9.8E-05 | 32 | 18 |
| IL32 | LTA | 0.42 | 27 | 5 | 15 | 3 | 84.4% | 83.3% | 5.5E-05 | 2.9E-06 | 32 | 18 |
| ADAM17 | HMOX1 | 0.41 | 26 | 6 | 15 | 3 | 81.3% | 83.3% | 0.0002 | 2.1E-06 | 32 | 18 |
| CCR5 | TXNRD1 | 0.41 | 26 | 6 | 15 | 3 | 81.3% | 83.3% | 2.0E-05 | 2.0E-05 | 32 | 18 |
| DPP4 | TGFB1 | 0.41 | 27 | 5 | 14 | 4 | 84.4% | 77.8% | 0.0001 | 4.8E-05 | 32 | 18 |
| PLAUR | PTGS2 | 0.40 | 26 | 6 | 15 | 3 | 81.3% | 83.3% | 7.1E-07 | 2.4E-05 | 32 | 18 |
| ADAM17 | CASP1 | 0.40 | 26 | 6 | 15 | 3 | 81.3% | 83.3% | 2.4E-06 | 2.7E-06 | 32 | 18 |
| CD4 | HMOX1 | 0.40 | 26 | 6 | 15 | 3 | 81.3% | 83.3% | 0.0002 | 4.4E-07 | 32 | 18 |
| CASP1 | TXNRD1 | 0.40 | 26 | 6 | 15 | 3 | 81.3% | 83.3% | 2.4E-05 | 2.6E-06 | 32 | 18 |
| ALOX5 | TXNRD1 | 0.40 | 25 | 7 | 14 | 4 | 78.1% | 77.8% | 2.6E-05 | 5.3E-07 | 32 | 18 |
| MHC2TA | TNFSF5 | 0.40 | 29 | 3 | 15 | 3 | 90.6% | 83.3% | 5.1E-05 | 1.2E-05 | 32 | 18 |
| IL18BP | LTA | 0.39 | 27 | 4 | 15 | 3 | 87.1% | 83.3% | 0.0001 | 1.8E-06 | 31 | 18 |
| TNF | TXNRD1 | 0.39 | 27 | 5 | 15 | 3 | 84.4% | 83.3% | 3.1E-05 | 0.0004 | 32 | 18 |
| MYC | TNF | 0.39 | 27 | 5 | 15 | 3 | 84.4% | 83.3% | 0.0004 | 1.1E-06 | 32 | 18 |
| CCL5 | MYC | 0.39 | 25 | 7 | 15 | 3 | 78.1% | 83.3% | 1.1E-06 | 0.0006 | 32 | 18 |
| SERPINA1 | TXNRD1 | 0.39 | 26 | 6 | 15 | 3 | 81.3% | 83.3% | 3.7E-05 | 7.7E-07 | 32 | 18 |
| MHC2TA | PLA2G7 | 0.39 | 28 | 4 | 15 | 3 | 87.5% | 83.3% | 2.5E-05 | 1.7E-05 | 32 | 18 |

(continued)

| 2-gene models and | | Entropy | #normals | | #ci | | Normal N= 32 | Colon 18 | | | total used (excludes missing) | |
|---|---|---|---|---|---|---|---|---|---|---|---|---|
| 1-gene models | | R-sq | Correct | FALSE | Correct | FALSE | Correct Classification | Correct Classification | p-val 1 | p-val 2 | #normal | # disease |
| HMOX1 | TNFSF5 | 0.38 | 29 | 3 | 15 | 3 | 90.6% | 83.3% | 8.5E-05 | 0.0005 | 32 | 18 |
| DPP4 | HMOX1 | 0.38 | 28 | 4 | 16 | 2 | 87.5% | 88.9% | 0.0005 | 0.0001 | 32 | 18 |
| APAF1 | MINDA | 0.38 | 25 | 7 | 15 | 3 | 78.1% | 83.3% | 2.6E-06 | 3.2E-05 | 32 | 18 |
| NFKB1 | PLAUR | 0.38 | 27 | 5 | 15 | 3 | 84.4% | 83.3% | 5.5E-05 | 1.1E-06 | 32 | 18 |
| EGR1 | IL8 | 0.38 | 28 | 4 | 14 | 4 | 87.5% | 77.8% | 0.0003 | 0.0051 | 32 | 18 |
| DPP4 | IL18BP | 0.38 | 25 | 6 | 15 | 3 | 80.7% | 83.3% | 3.1E-06 | 0.0001 | 31 | 18 |
| HMOX1 | PLA2G7 | 0.37 | 26 | 6 | 15 | 3 | 81.3% | 83.3% | 4.0E-05 | 0.0006 | 32 | 18 |
| DPP4 | MHC2TA | 0.37 | 25 | 7 | 15 | 3 | 78.1% | 83.3% | 2.8E-05 | 0.0002 | 32 | 18 |
| EGR1 | LTA | 0.37 | 24 | 8 | 14 | 4 | 75.0% | 77.8% | 0.0003 | 0.0070 | 32 | 18 |
| MNDA | TXNRD1 | 0.37 | 26 | 6 | 15 | 3 | 81.3% | 83.3% | 7.0E-05 | 3.7E-06 | 32 | 18 |
| EGR1 | MHC2TA | 0.37 | 26 | 6 | 15 | 3 | 81.3% | 83.3% | 3.1E-05 | 0.0073 | 32 | 18 |
| PTPRC | TNF | 0.37 | 27 | 5 | 15 | 3 | 84.4% | 83.3% | 3.1E-06 | 3.1E-06 | 32 | 18 |
| LTA | PLAUR | 0.37 | 26 | 6 | 15 | 3 | 81.3% | 83.3% | 8.0E-05 | 0.0003 | 32 | 18 |
| EGR1 | PLAUR | 0.37 | 25 | 7 | 14 | 4 | 78.1% | 77.8% | 8.9E-05- | 0.0084 | 32 | 18 |
| TNF | TOSO | 0.36 | 25 | 6 | 14 | 4 | 80.7% | 77.8% | 1.9E-05 | 0.0012 | 31 | 18 |
| EGR1 | HMOX1 | 0.36 | 28 | 4 | 14 | 4 | 87.5% | 77.896 | 0.0010 | 0.0099 | 32 | 18 |
| HMOX1 | HSPA1A | 0.36 | 26 | 6 | 15 | 3 | 81.3% | 83.3% | 1.4E-06 | 0.0010 | 32 | 18 |
| IL1RN | TXNRD1 | 0.36 | 28 | 4 | 16 | 2 | 87.5% | 88.9% | 0.0001 | 3.5E-06 | 32 | 18 |
| CCR5 | CTLA4 | 0.36 | 26 | 6 | 14 | 4 | 81.396 | 77.8% | 5.4E-06 | 0.0001 | 32 | 18 |
| CCL5 | TXNRD1 | 0.35 | 25 | 7 | 5 | 3 | 78.1% | 83.3% | 0.0001 | 0.0022 | 32 | 18 |
| TLR2 | TXNRD1 | 0.35 | 25 | 7 | 15 | 3 | 78.1% | 83.3% | 0.0001 | 8.9E-06 | 32 | 18 |

| 2-gene models and | | Entropy | #normals | #normal | #ci | #ci | Normal N= 32 Correct Classification | Colon N= 18 Correct Classification | | | total used (excludes missing) | |
|---|---|---|---|---|---|---|---|---|---|---|---|---|
| 1-gene models | | R-sq | Correct | FALSE | Correct | FALSE | Classification | Classification | p-val 1 | p-val 2 | #normal | # disease |
| PLAUR | TLR4 | 0.35 | 25 | 7 | 14 | 4 | 78.1% | 77.8% | 6.2E-06 | 0.0001 | 32 | 18 |
| IRF1 | LTA | 0.35 | 24 | 8 | 14 | 4 | 75.0% | 77.8% | 0.0005 | 2.7E-05 | 32 | 18 |
| EGR1 | TLR2 | 0.35 | 27 | 5 | 14 | 4 | 84.4% | 77.8% | 1.0E-05 | 0.0144 | 32 | 18 |
| EGR1 | TXNRD1 | 0.35 | 26 | 6 | 14 | 4 | 81.3% | 77.8% | 0.0001 | 0.0148 | 32 | 18 |
| CASP3 | PLAUR | 0.35 | 24 | 8 | 14 | 4 | 75.0% | 77.8% | 0.0002 | 0.0002 | 32 | 18 |
| PTPRC | TGFB1 | 0.35 | 26 | 6 | 14 | 4 | 81.3% | 77.8% | 0.0010 | 6.0E-06 | 32 | 18 |
| TGFB1 | TOSO | 0.35 | 24 | 7 | 15 | 3 | 77.4% | 83.3% | 3.2E-05 | 0.0023 | 31 | 18 |
| SS13 | TXNRD1 | 0.35 | 25 | 7 | 15 | 3 | 78.1% | 83.3% | 0.0002 | 2.0E-05 | 32 | |
| CASP3 | HMOX1 | 0.35 | 29 | 3 | 15 | 3 | 90.6% | 83.3% | 0.0017 | 0.0002 | 32 | 18 |
| TNFRSF1A | TXNRD1 | 0.34 | 27 | 5 | 15 | 3 | 84.4% | 83.3% | 0.0002 | 2.8E-06 | 32 | 18 |
| CASP1 | CASP3 | 0.34 | 25 | 7 | 14 | 4 | 78.1% | 77.8% | 0.0003 | 1.9E-05 | 32 | 18 |
| MMP9 | TXNRD1 | 0.34 | 26 | 6 | 15 | 3 | 81.3% | 83.3% | 0.0002 | 4.5E-06 | 32 | 18 |
| IFI16 | IL8 | 0.34 | 27 | 5 | 15 | 3 | 84.4% | 83.3% | 0.0012 | 0.0002 | 32 | 18 |
| EGR1 | TNFSF5 | 0.33 | 24 | 8 | 14 | 4 | 75.0% | 77.8% | 0.0004 | 0.0274 | 32 | 18 |
| ADAM17 | PLAUR | 0.33 | 26 | 6 | 14 | 4 | 81.3% | 77.8% | 0.0003 | 2.8E-05 | 32 | 18 |
| CXCR3 | TNFSF5 | 0.33 | 25 | 7 | 14 | 4 | 78.1% | 77.8% | 0.0005 | 5.0E-06 | 32 | 18 |
| CXCR3 | DPP4 | 0.33 | 26 | 6 | 14 | 4 | 81.3% | 77.8% | 0.0006 | 5.1E-06 | 32 | 18 |
| TGFB1 | TNFRSF13 | 0.33 | 24 | 8 | 14 | 4 | 75.0% | 77.8% | 1.7E-05 | 0.0019 | 32 | 18 |
| EGR1 | IL10 | 0.33 | 26 | 6 | 15 | 3 | 81.3% | 83.3% | 0.0001 | 0.0312 | 32 | 18 |
| ICAM1 | LTA | 0.33 | 26 | 6 | 15 | 3 | 81.3% | 83.3% | 0.0011 | 7.0E-05 | 32 | 18 |
| IFI16 | LTA | 0.33 | 25 | 7 | 14 | 4 | 78.1% | 77.8% | 0.0012 | 0.0002 | 32 | 18 |

EP 2 402 464 A1

| 2-gene models and | | Entropy | #normals | #normal | #ci | #ci | Normal<br>N= 32<br>Correct | Colon<br>18<br>Correct | | | total used (excludes missing) | |
|---|---|---|---|---|---|---|---|---|---|---|---|---|
| 1-gene models | | R-sq | Correct | FALSE | Correct | FALSE | Classification | Classification | p-val 1 | p-val 2 | #normal | # disease |
| IL1R1 | PLAUR | 0.32 | 25 | 7 | 14 | 4 | 78.1% | 77.8% | 0.0004 | 1.8E-05 | 32 | 18 |
| IL8 | TGF81 | 0.32 | 27 | 5 | 15 | 3 | 84.4% | 83.3% | 0.0024 | 0.0018 | 32 | 18 |
| CCR5 | EGR1 | 0.32 | 25 | 7 | 14 | 4 | 78.1% | 77.8% | 0.0394 | 0.0003 | 32 | 18 |
| EGR1 | PTPRC | 0.32 | 24 | 8 | 14 | 4 | 75.0% | 77.8% | 1.4E-05 | 0.0409 | 32 | 18 |
| LTA | MHC2TA | 0.32 | 26 | 6 | 15 | 3 | 81.3% | 83.3% | 0.0002 | 0.0014 | 32 | 18 |
| HMOX1 | MYC | 0.32 | 26 | 6 | 15 | 3 | 81.3% | 83.3% | 1.1E-05 | 0.0038 | 32 | 18 |
| EGR1 | TNF | 0.32 | 25 | 7 | 14 | 4 | 78.1% | 77.8% | 0.0052 | 0.0431 | 32 | 18 |
| CCR5 | MIF | 0.32 | 25 | 7 | 14 | 4 | 78.1% | 77.8% | 1.4E-05 | 0.0004 | 32 | 18 |
| CASP3 | TGFB1 | 0.32 | 25 | 7 | 15 | 3 | 78.1% | 83.3% | 0.0028 | 0.0006 | 32 | 18 |
| CASP1 | EGR1 | 0.32 | 29 | 3 | 14 | 4 | 90.6% | 77.8% | 0.0455 | 3.9E-05 | 32 | 18 |
| CTLA4 | TGFB1 | 0.32 | 26 | 6 | 14 | 4 | 81.3% | 77.8% | 0.0029 | 1.9E-05 | 32 | 18 |
| ADAM17 | TGFB1 | 0.32 | 26 | 6 | 15 | 3 | 81.3% | 83.3% | 0.0030 | 4.6E-05 | 32 | 18 |
| IRF1 | TXNRD1 | 0.32 | 27 | 5 | 14 | 4 | 84.4% | 77.8% | 0.0004 | 7.9E-05 | 32 | 18 |
| HMOX1 | TNFRSF13 | 0.32 | 25 | 7 | 14 | 4 | 78.1% | 77.8% | 2.7E-05 | 0.0043 | 32 | 18 |
| PLA2G7 | PLAUR | 0.32 | 24 | 8 | 14 | 4 | 75.0% | 77.8% | 0.0005 | 0.0003 | 32 | 18 |
| TGFB1 | TLR4 | 0.32 | 26 | 6 | 14 | 4 | 81.3% | 77.8% | 2.2E-05 | 0.0033 | 32 | 18 |
| HMOX1 | IL1R1 | 0.32 | 28 | 4 | 15 | 3 | 87.5% | 83.3% | 2.5E-05 | 0.0043 | 32 | 18 |
| CASP3 | CCR5 | 0.32 | 26 | 6 | 14 | 4 | 81.3% | 77.8% | 0.0005 | 0.0007 | 32 | 18 |
| HMOX1 | IL18 | 0.32 | 27 | 5 | 14 | 4 | 84.4% | 77.8% | 4.7E-05 | 0.0049 | 32 | 18 |
| CASP3 | TLR2 | 0.31 | 25 | 7 | 14 | 4 | 78.1% | 77.8% | 3.4E-05 | 0.0007 | 32 | 18 |
| CCL5 | PTPRC | 0.31 | 27 | 5 | 15 | 3 | 84.4% | 83.3% | 1.9E-05 | 0.0092 | 32 | 18 |

EP 2 402 464 A1

| 2-gene models and | | Entropy | #normals | #normal | #ci | #ci | Normal<br>N= 32<br>Correct<br>Classification | Colon<br>18<br>Correct<br>Classification | | | total used (excludes missing) | |
|---|---|---|---|---|---|---|---|---|---|---|---|---|
| 1-gene models | | R-sq | Correct | FALSE | Correct | FALSE | Classification | Classification | p-val 1 | p-val 2 | #normal | # disease |
| TNF | TNFRSF13 | 0.31 | 25 | 7 | 14 | 4 | 78.1% | 77.8% | 3.1E-05 | 0.0069 | 32 | 18 |
| APAF1 | TNF | 0.31 | 24 | 8 | 14 | 4 | 75.0% | 77.8% | 0.0072 | 0.0003 | 32 | 18' |
| NMOX1 | TLR4 | 0.31 | 26 | 6 | 15 | 3 | 81.3% | 83.3% | 2.5E-05 | 0.0054 | 32 | 18 |
| HMOX1 | TOSO | 0.31 | 27 | 4 | 16 | 2 | 87.1% | 88.9% | 0.0001 | 0.0056 | 31 | 18 |
| DPP4 | IFI16 | 0.31 | 25 | 7 | 15 | 3 | 78.1% | 83.3% | 0.0004 | 0.0013 | 32 | 18 |
| CXCR3 | TOSO | 0.31 | 25 | 6 | 15 | 3 | 80.7% | 83.3% | 0.0001 | 1.4E-05 | 31 | 18 |
| APAF1 | ICAM1 | 0.31 | 25 | 7 | 14 | 4 | 78.1% | 77.8% | 0.0001 | 0.0004 | 32 | 18 |
| APAF1 | CCR5 | 0.31 | 26 | 6 | 14 | 4 | 81.3% | 77.8% | 0.0006 | 0.0004 | 32 | 18 |
| NFKB1 | TNF | 0.31 | 24 | 8 | 14 | 4 | 75.0% | 77.8% | 0.0093 | 1.4E-05 | 32 | 18 |
| IL8 | LTA | 0.31 | 26 | 6 | 14 | 4 | 81.3% | 77.8% | 0.0026 | 0.0036 | 32 | 18 |
| CLASP3 | IL10 | 0.30 | 26 | 6 | 14 | 4 | 81.3% | 77.8% | 0.0003 | 0.0010 | 32 | 18 |
| PLA2G7 | TNF | 0.30 | 25 | 7 | 14 | 4 | 78.1% | 77.8% | 0.0104 | 0.0004 | 32 | 18 |
| IL1B | TXNRD1 | 0.30 | 28 | 4 | 16 | 2 | 87.5% | 88.9% | 0.0008 | 2.1E-05 | 32 | 18 |
| HMOX1 | IL8 | 0.30 | 25 | 7 | 15 | 3 | 78.1% | 83.3% | 0.0043 | 0.0083 | 32 | 18 |
| CCR5 | PLA2G7 | 0.30 | 24 | 8 | 14 | 4 | 75.0% | 77.8% | 0.0005 | 0.0008 | 32 | 18 |
| CD8A | LTA | 0.30 | 24 | 8 | 14 | 4 | 75.0% | 77.8% | 0.0032 | 2.0E-05 | 32 | 18 |
| CCL5 | CD4 | 0.30 | 27 | 5 | 14 | 4 | 84.4% | 77.8% | 1.4E-05 | 0.0165 | 32 | 18 |
| MYC | TGFB1 | 0.30 | 25 | 7 | 14 | 4 | 78.1% | 77.8% | 0.0063 | 2.6E-05 | 32 | 18 |
| CASP3 | TNF | 0.30 | 24 | 8 | 14 | 4 | 75.0% | 77.8% | 0.0127 | 0.0013 | 32 | 18 |
| CTLA4 | IL32 | 0.30 | 26 | 6 | 14 | 4 | 81.3% | 77.8% | 0.0002 | 4.3E-05 | 32 | 18 |
| IFI16 | TXNRD1 | 0.30 | 24 | 8 | 14 | 4 | 75.0% | 77.8% | 0.0009 | 0.0007 | 32 | 18 |

EP 2 402 464 A1

| | | | | | | Normal | Colon | | | | |
|---|---|---|---|---|---|---|---|---|---|---|---|
| | | | | | N= | 32 | 18 | | | | |
| 2-gene models and | | Entropy | #normals | #normal | #ci | #ci | Correct | Correct | | | total used (excludes missing) |
| 1-gene models | | R-sq | Correct | FALSE | Correct | FALSE | Classification | Classification | p-val 1 | p-val 2 | #normal | # disease |
| CD8A | DPP4 | 0.30 | 24 | 8 | 14 | 4 | 75.0% | 77.8% | 0.0022 | 2.2E-05 | 32 | 18 |
| IL10 | TXNRD1 | 0.29 | 26 | 6 | 14 | 4 | 81.3% | 77.8% | 0.0010 | 0.0005 | 32 | 18 |
| HMOX1 | MIF | 0.29 | 26 | 6 | 15 | 3 | 81.396 | 83.3% | 3.6E-05 | 0.0108 | 32 | 18 |
| IL8 | PLAUR | 0.29 | 28 | 4 | 16 | 2 | 87.596 | 88.9% | 0.0011 | 0.0058 | 32 | 18 |
| PLAUR | TNFSF5 | 0.29 | 26 | 6 | 14 | 4 | 81.3% | 77.8% | 0.0019 | 0.0011 | 32 | 18 |
| CASP1 | TLR4 | 0.29 | 25 | 7 | 14 | 4 | 78.1% | 77.8% | 5.5E-05 | 0.0001 | 32 | 18 |
| PLA2G7 | TGFB1 | 0.29 | 24 | 8 | 14 | 4 | 75.0% | 77.8% | 0.0085 | 0.0007 | 32 | 18 |
| IFI16 | TNF5F5 | 0.29 | 27 | 5 | 14 | 4 | 84.4% | 77.8% | 0.0021 | 0.0009 | 32 | 18 |
| CCL5 | PLAUR | 0.29 | 25 | 7 | 14 | 4 | 78.1% | 77.8% | 0.0014 | 0.0250 | 32 | 18 |
| CCR5 | PTPRC | 0.29 | 28 | 4 | 14 | 4 | 87.5% | 77.8% | 5.3E-05 | 0.0013 | 32 | 18 |
| CASP3 | LTA | 0.28 | 25 | 7 | 14 | 4 | 78.1% | 77.8% | 0.0056 | 0.0021 | | 18 |
| CASP3 | CCL5 | 0.28 | 26 | 6 | 14 | 4 | 81.3% | 77.8% | 0.0285 | 0.0022 | 32 | 18 |
| HMOX1 | PTGS2 | 0.28 | 26 | 6 | 15 | 3 | 81.3% | 83.3% | 4.4E-05 | 0.0162 | 32 | 18 |
| DPP4 | IRF1 | 0.28 | 25 | 7 | 14 | 4 | 78.1% | 77.8% | 0.0003 | 0.0037 | 32 | 18 |
| CCL5 | TNFRSF13 | 0.28 | 25 | 7 | 14 | 4 | 78.1% | 77.8% | 9.5E-05 | 0.0307 | 32 | 18 |
| DPP4 | PLAUR | 0.28 | 25 | 7 | 14 | 4 | 78.1% | 77.8% | 0.0017 | 0.0039 | 32 | 18 |
| CD19 | MHC2TA | 0.28 | 26 | 6 | 15 | 3 | 81.3% | 83.3% | 0.0007 | 5.9E-05 | 32 | 18 |
| IL8 | IRF1 | 0.28 | 26 | 6 | 14 | 4 | 81.3% | 77.8% | 0.0003 | 0.0091 | 32 | 18 |
| CCL5 | MIF | 0.28 | 25 | 7 | 14 | 4 | 78.1% | 77.8% | 6.2E-05 | 0.0363 | | 18 |
| CASP1 | IL15 | 0.28 | 25 | 7 | 15 | 3 | 78.1% | 83.3% | 7.9E-05 | 0.0002 | 32 | 18 |
| CASP1 | IL18 | 0.28 | 24 | 8 | 14 | 4 | 75.0% | 77.8% | 0.0002 | 0.0002 | 32 | 18 |

EP 2 402 464 A1

68

| | | | | | | | Normal | Colon | | | | |
|---|---|---|---|---|---|---|---|---|---|---|---|---|
| | | | | | | N= | 32 | 18 | | | | |
| 2-gene models and | | Entropy | #normals | #normal | #ci | #ci | Correct | Correct | | | total used (excludes missing) | |
| 1-gene models | | R-sq | Correct | FALSE | Correct | FALSE | Classification | Classification | p-val 1 | p-val 2 | #normal | # disease |
| CCL5 | IL8 | 0.27 | 26 | 6 | 14 | 4 | 81.3% | 77.8% | 0.0110 | 0.0401 | 32 | 18 |
| CCL5 | NFKB1 | 0.27 | 24 | 8 | 14 | 4 | 75.0% | 77.8% | 4.1E-05 | 0.0411 | 32 | 18 |
| CCR5 | TNFRSF13 | 0.27 | 25 | 7 | 14 | 4 | 78.1% | 77.8% | 0.0001 | 0.0020 | 32 | 18 |
| CCL5 | HMOX1 | 0.27 | 25 | 7 | 14 | 4 | 78.1% | 77.8% | 0.0230 | 0.0428 | 32 | 18 |
| APAF1 | IL10 | 0.27 | 26 | 6 | 15 | 3 | 81.3% | 83.3% | 0.0010 | 0.0013 | 32 | 18 |
| HSPA1A | PLAUR | 0.27 | 27 | 5 | 15 | 3 | 84.4% | 83.3% | 0.0023 | 2.6E-05 | 32 | 18 |
| IL8 | TNF | 0.27 | 24 | 8 | 14 | 4 | 75.0% | 77.8% | 0.0317 | 0.0118 | 32 | 18 |
| CCL5 | SS13 | 0.27 | 26 | 6 | 15 | 3 | 81.3% | 83.3% | 0.0003 | 0.0437 | 32 | 18 |
| IL8 | MHC2TA | 0.27 | 26 | 6 | 15 | 3 | 81.3% | 83.3% | 0.0009 | 0.0120 | 32 | 18 |
| APAF1 | TIMP1 | 0.27 | 24 | 8 | 14 | 4 | 75.0% | 77.8% | 0.0001 | 0.0014 | 32 | 18 |
| IL1R1 | TGFB1 | 0.27 | 24 | 8 | 14 | 4 | 75.0% | 77.8% | 0.0167 | 0.0001 | 32 | 18 |
| HMOX1 | IL15 | 0.27 | 28 | 4 | 15 | 3 | 87.5% | 83.3% | 9.6E-05 | 0.0251 | 32 | 18 |
| IL10 | PLA2G7 | 0.27 | 27 | 5 | 15 | 3 | 84.4% | 83.3% | 0.0014 | 0.0011 | 32 | 18 |
| CCL5 | IFI16 | 0.27 | 27 | 5 | 15 | 3 | 84.4% | 83.3% | 0.0018 | 0.0495 | 32 | 18 |
| CCL5 | CTLA4 | 0.27 | 27 | 5 | 15 | 3 | 84.4% | 83.3% | 0.0001 | 0.0498 | | 18 |
| CCR5 | CD19 | 0.27 | 25 | 7 | 14 | 4 | 78.1% | 77.8% | 8.7E-05 | 0.0024 | 32 | 18 |
| IL8 | PLA2G7 | 0.27 | 24 | 8 | 14 | 4 | 75.0% | 77.8% | 0.0015 | 0.0137 | 32 | 18 |
| IL23A | TGFB1 | 0.27 | 24 | 8 | 14 | 4 | 75.0% | 77.8% | 0.0186 | 7.2E-05 | 32 | 18 |
| PTPRC | TIMP1 | 0.27 | 24 | 8 | 14 | 4 | 75.0% | 77.8% | 0.0002 | 9.7E-05 | 32 | 18 |
| ADAM17 | TLR2 | 0.27 | 25 | 7 | 14 | 4 | 78.1% | 77.8% | 0.0002 | 0.0003 | 32 | 18 |
| ICAM1 | NFKB1 | 0.27 | 26 | 6 | 15 | 3 | 81.3% | 83.3% | 5.5E-05 | 0.0006 | 32 | 18 |

| 2-gene models and | | Entropy | #normals | #normal | #ci | #ci | Normal<br>Correct | Colon<br>Correct | | | total used (excludes missing) | |
|---|---|---|---|---|---|---|---|---|---|---|---|---|
| | | | | | | N= | 32 | 18 | | | | |
| 1-gene models | | R-sq | Correct | FALSE | Correct | FALSE | Classification | Classification | p-val 1 | p-val 2 | #normal | # disease |
| CXCL1 | HMOX1 | 0.26 | 24 | 8 | 14 | 4 | 75.0% | 77.8% | 0.0318 | 4.5E-05 | 32 | 18 |
| GZMB | LTA | 0.26 | 26 | 6 | 14 | 4 | 81.3% | 77.8% | 0.0114 | 9.3E-05 | 32 | 18 |
| CCR5 | IL8 | 0.26 | 27 | 5 | 14 | 4 | 84.4% | 77.8% | 0.0169 | 0.0029 | 32 | 18 |
| TLR2 | TLR4 | 0.26 | 25 | 7 | 14 | 4 | 78.1% | 77.8% | 0.0001 | 0.0002 | 32 | 18 |
| DPP4 | ICAM1 | 0.26 | 25 | 7 | 14 | 4 | 78.1% | 77.8% | 0.0008 | 0.0082 | 32 | 18 |
| HMOX1 | SERPINA1 | 0.26 | 25 | 7 | 14 | 4 | 78.1% | 77.8% | 6.5E-05 | 0.0411 | 32 | 18 |
| CASP1 | IL8 | 0.26 | 25 | 7 | 14 | 4 | 78.1% | 77.8% | 0.0214 | 0.0004 | 32 | 18 |
| MHC2TA | TXNRD1 | 0.26 | 27 | 5 | 15 | 3 | 84.4% | 83.3% | 0.0037 | 0.0015 | 32 | 18 |
| HLADRA | LTA | 0.26 | 26 | 6 | 15 | 3 | 81.3% | 83.3% | 0.0154 | 0.0003 | 32 | 18 |
| IL32 | MIF | 0.26 | 27 | 5 | 14 | 4 | 84.4% | 77.8% | 0.0001 | 0.0007 | 32 | 18 |
| HMOX1 | TNFRSF1A | 0.25 | 26 | 6 | 15 | 3 | 81.3% | 83.3% | 5.9E-05 | 0.0485 | 32 | 18 |
| HLADRA | IL8 | 0.25 | 24 | 8 | 14 | 4 | 75.0% | 77.8% | 0.0244 | 0.0003 | 32 | 18 |
| IL8 | TXNRD1 | 0.25 | 25 | 7 | 14 | 4 | 78.1% | 77.8% | 0.0042 | 0.0251 | 32 | 18 |
| IL32 | TXNRD1 | 0.25 | 25 | 7 | 15 | 3 | 78.1% | 83.3% | 0.0043 | 0.0008 | 32 | 18 |
| CD4 | PLAUR | 0.25 | 24 | 8 | 14 | 4 | 75.0% | 77.8% | 0.0050 | 7.4E-05 | 32 | 18 |
| CD19 | TGFB1 | 0.25 | 24 | 8 | 14 | 4 | 75.0% | 77.8% | 0.0374 | 0.0002 | 32 | 18 |
| DPP4 | IL10 | 0.25 | 26 | 6 | 14 | 4 | 81.3% | 77.8% | 0.0025 | 0.0134 | 32 | 18 |
| ADAM17 | IRF1 | 0.25 | 24 | 8 | 15 | 3 | 75.0% | 83.3% | 0.0010 | 0.0006 | 32 | 18 |
| IL8 | MNDA | 0.25 | 26 | 6 | 15 | 3 | 81.3% | 83.3% | 0.0003 | 0.0332 | 32 | 18 |
| ADAM17 | IL10 | 0.24 | 24 | 8 | 14 | 4 | 75.0% | 77.8% | 0.0026 | 0.0006 | 32 | 18 |
| IL32 | IL8 | 0.24 | 28 | 4 | 15 | 3 | 87.5% | 83.3% | 0.0337 | 0.0011 | 32 | 18 |

(continued)

| 2-gene models and | | Entropy | #normals | #normal | #ci | #ci | Normal | Colon | | | total used (excludes missing) | |
|---|---|---|---|---|---|---|---|---|---|---|---|---|
| | | | | | | N= | 32 | 18 | | | | |
| | | | | | | | Correct | Correct | | | | |
| 1-gene models | | R-sq | Correct | FALSE | Correct | FALSE | Classification | Classification | p-val 1 | p-val 2 | #normal | # disease |
| IL8 | TLR2 | 0.24 | 27 | 5 | 15 | 3 | 84.4% | 83.3% | 0.0004 | 0.0388 | 32 | 18 |
| ICAM1 | TLR4 | 0.24 | 26 | 6 | 14 | 4 | 81.3% | 77.8% | 0.0003 | 0.0015 | 32 | 18 |
| CD8A | TOSO | 0.24 | 24 | 7 | 14 | 4 | 77.4% | 77.8% | 0.0013 | 0.0002 | 31 | 18 |
| ICAM1 | IL8 | 0.24 | 27 | 5 | 15 | 3 | 84.4% | 83.3% | 0.0430 | 0.0016 | 32 | 18 |
| CCL3 | IL8 | 0.24 | 24 | 7 | 14 | 4 | 77.4% | 77.8% | 0.0356 | 0.0006 | 31 | 18 |
| CASP1 | LTA | 0.24 | 24 | 8 | 14 | 4 | 75.0% | 77.8% | 0.0320 | 0.0007 | 32 | 18 |
| IL10 | IL1R1 | 0.23 | 24 | 8 | 14 | 4 | 75.0% | 77.8% | 0.0004 | 0.0038 | 32 | 18 |
| IFNG | IL8 | 0.23 | 25 | 7 | 14 | 4 | 78.1% | 77.8% | 0.0500 | 0.0002 | 32 | 18 |
| PLAUR | TNFRSF1A | 0.23 | 24 | 8 | 14 | 4 | 75.0% | 77.8% | 0.0001 | 0.0093 | 32 | 18 |
| DPP4 | TLR2 | 0.23 | 24 | 8 | 14 | 4 | 75.0% | 77.8% | 0.0006 | 0.0220 | 32 | 18 |
| LTA | MNDA | 0.23 | 25 | 7 | 14 | 14 | 78.1% | 77.8% | 0.0004 | 0.0405 | 32 | 18 |
| HLADRA | TNFSFS | 0.23 | 25 | 7 | 14 | 4 | 78.1% | 77.8% | 0.0177 | 0.0007 | 32 | 18 |
| TLR2 | TNFSF5 | 0.23 | 25 | 7 | 14 | 4 | 78.1% | 77.8% | 0.0182 | 0.0007 | 32 | 18 |
| CTLA4 | MHC2TA | 0.23 | 25 | 7 | 14 | 4 | 78.1% | 77.8% | 0.0041 | 0.0004 | 32 | 18 |
| TIMP1 | TNFSFS | 0.23 | 25 | 7 | 14 | 4 | 78.1% | 77.8% | 0.0208 | 0.0007 | 32 | 18 |
| IL18BP | TXNRD1 | 0.22 | 24 | 7 | 14 | 4 | 77.4% | 77.8% | 0.0088 | 0.0006 | 31 | 18 |
| DPP4 | GZMB | 0.22 | 24 | 8 | 14 | 4 | 75.0% | 77.8% | 0.0004 | 0.0348 | 32 | 18 |
| HSPA1A | TXNRD1 | 0.22 | 24 | 8 | 14 | 4 | 75.0% | 77.8% | 0.0136 | 0.0002 | 32 | 18 |
| CASP3 | TNFSF6 | 0.22 | 24 | 8 | 14 | 4 | 75.0% | 77.8% | 0.0003 | 0.0223 | 32 | 18 |
| IL10 | TLR4 | 0.22 | 27 | 5 | 15 | 3 | 84.4% | 83.3% | 0.0007 | 0.0071 | 32 | 18 |
| IL10 | PTPRC | 0.21 | 25 | 7 | 14 | 4 | 78.1% | 77.8% | 0.0006 | 0.0083 | 32 | 18 |

(continued)

| 2-gene models and | | Entropy | #normals | #normal | #ci | #ci | Normal | Colon | | | total used (excludes missing) | |
|---|---|---|---|---|---|---|---|---|---|---|---|---|
| | | | | | | N= | 32 | 18 | | | | |
| | | | | | | | Correct | Correct | | | | |
| 1-gene models | | R-sq | Correct | FALSE | Correct | FALSE | Classification | Classification | p-val 1 | p-val 2 | #normal | # disease |
| PTGS2 | TLR2 | 0.20 | 25 | 7 | 14 | 4 | 78.1% | 77.8% | 0.0018 | 0.0008 | 32 | 18 |
| ICAM1 | PTG52 | 0.20 | 24 | 8 | 14 | 4 | 75.0% | 77.8% | 0.0008 | 0.0065 | 32 | 18 |
| CCR5 | IFI16 | 0.20 | 25 | 7 | 14 | 4 | 78.1% | 77.8% | 0.0233 | 0.0304 | 32 | 18 |
| MIF | PLAUR | 0.19 | 25 | 7 | 14 | 4 | 78.1% | 77.8% | 0.0396 | 0.0011 | 32 | 18 |
| ADAM17 | MHC2TA | 0.19 | 24 | 8 | 14 | 4 | 75.0% | 77.8% | 0.0154 | 0.0038 | 32 | 18 |
| CD8A | TXNRD1 | 0.19 | 24 | 8 | 14 | 4 | 75.0% | 77.8% | 0.0419 | 0.0009 | 32 | 18 |
| IL1R1 | IRF1 | 0.19 | 24 | 8 | 14 | 4 | 75.0% | 77.8% | 0.0078 | 0.0021 | 32 | 18 |
| APAF1 | CD86 | 0.19 | 26 | 6 | 15 | 3 | 81.3% | 83.3% | 0.0005 | 0.0294 | 32 | 18 |
| ICAM1 | MYC | 0.17 | 24 | 8 | 14 | 4 | 75.0% | 77.8% | 0.0024 | 0.0211 | 32 | 18 |
| IL32 | TNFRSF13 | 0.17 | 24 | 8 | 14 | 4 | 75.0% | 77.8% | 0.0057 | 0.0186 | 32 | 18 |
| MMP9 | TLR4 | 0.16 | 25 | 7 | 14 | 4 | 78.1% | 77.8% | 0.0055 | 0.0025 | 32 | 18 |
| HSPA1A | IRF1 | 0.16 | 25 | 7 | 14 | 4 | 78.1% | 77.8% | 0.0243 | 0.0014 | 32 | 18 |
| TIMP1 | TOSO | 0.16 | 24 | 7 | 14 | 4 | 77.4% | 77.8% | 0.0264 | 0.0128 | 31 | 18 |
| IL18 | SSI3 | 0.15 | 24 | 8 | 14 | 4 | 75.0% | 77.8% | 0.0177 | 0.0135 | 32 | 18 |
| ALOX5 | TLR4 | 0.15 | 24 | 8 | 14 | 4 | 75.0% | 77.8% | 0.0075 | 0.0028 | 32 | 18 |
| HLADRA | IL15 | 0.12 | 24 | 8 | 14 | 4 | 75.0% | 77.8% | 0.0211 | 0.0424 | 32 | 18 |

Table 2B

|  | Colon | Normals | Sum |
|---|---|---|---|
| Group Size | 36.0% | 64.0% | 100% |
| N = | 18 | 32 | 50 |
|  |  |  |  |
| Gene | Mean | Mean | p-val |
| C1QA | 19.1 | 20.9 | 4.9E-08 |
| EGR1 | 18.7 | 19.7 | 3.9E-05 |
| CCL5 | 11.6 | 12.2 | 0.0002 |
| TNF | 18.2 | 18.7 | 0.0003 |
| HMO3X1 | 16.0 | 16.6 | 0.0004 |
| TGFB1 | 12.3 | 12.8 | 0.0005 |
| IL8 | 22.3 | 21.3 | 0.0007 |
| LTA | 20.4 | 19.9 | 0.0010 |
| DPP4 | 19.0 | 18.4 | 0.0016 |
| TNF5F5 | 18.1 | 17.5 | 0.0021 |
| CASP3 | 20.2 | 19.8 | 0.0025 |
| PLAUR | 14.6 | 15.0 | 0.0036 |
| CCR5 | 17.2 | 17.7 | 0.0039 |
| TXNRD1 | 17.3 | 16.8 | 0.0039 |
| IFI16 | 15.2 | 16.0 | 0.0050 |
| APAF1 | 17.2 | 16.7 | 0.0062 |
| PLA2G7 | 19.7 | 19.0 | 0.0064 |
| IL10 | 22.9 | 23.7 | 0.0084 |
| MHC2TA | 15.8 | 16.1 | 0.0095 |
| ICAM1 | 16.8 | 17.2 | 0.0175 |
| IL32 | 13.3 | 13.7 | 0.0216 |
| IRF1 | 12.5 | 12.8 | 0.0219 |
| TOSO | 15.9 | 15.5 | 0.0241 |
| SS13 | 17.2 | 17.7 | 0.0343 |
| ADAM17 | 18.7 | 18.4 | 0.0396 |
| CASP1 | 15.6 | 15.9 | 0.0454 |
| IL18 | 21.8 | 21.4 | 0.0457 |
| HLADRA | 12.0 | 12.2 | 0.0551 |
| CCL3 | 19.8 | 20.2 | 0.0649 |
| TLR2 | 15.8 | 16.1 | 0.0657 |
| TIMP1 | 14.1 | 14.4 | 0.0726 |
| TNFR5F138 | 20.1 | 19.7 | 0.0752 |

(continued)

| Gene | Mean | Mean | p-val |
|---|---|---|---|
| IL1R1 | 20.6 | 20.2 | 0.0918 |
| MNDA | 12.4 | 12.7 | 0.1000 |
| TLR4 | 15.1 | 14.8 | 0.1036 |
| CTLA4 | 19.4 | 19.1 | 0.1046 |
| IL18BP | 16.7 | 17.0 | 0.1125 |
| IL15 | 21.4 | 21.0 | 0.1153 |
| PTPRC | 11.8 | 11.6 | 0.1310 |
| CD19 | 19.2 | 18.9 | 0.1402 |
| MIF | 15.5 | 15.3 | 0.1497 |
| GZMB | 15.9 | 16.4 | 0.1570 |
| IL1RN | 16.1 | 16.3 | 0.1705 |
| IL23A | 21.5 | 21.3 | 0.1820 |
| MYC | 18.2 | 18.0 | 0.1840 |
| PTGS2 | 17.1 | 16.8 | 0.1845 |
| IL1B | 15.6 | 15.9 | 0.2112 |
| TNFSF6 | 19.8 | 20.0 | 0.2370 |
| CD8A | 15.3 | 15.6 | 0.2424 |
| IFNG | 22.8 | 23.1 | 0.2554 |
| MMP9 | 14.1 | 14.5 | 0.2737 |
| NFKB1 | 16.8 | 16.7 | 0.2991 |
| SERPINA1 | 12.5 | 12.7 | 0.3440 |
| IL5 | 21.7 | 21.5 | 0.3448 |
| CXCR3 | 17.2 | 17.4 | 0.3481 |
| ALOX5 | 16.0 | 16.1 | 0.3599 |
| CD4 | 15.3 | 15.2 | 0.4102 |
| CXCL1 | 19.2 | 19.0 | 0.4253 |
| CCR3 | 16.6 | 16.5 | 0.4941 |
| TNFRSF1A | 14.8 | 14.9 | 0.5094 |
| SERPINE1 | 20.5 | 20.6 | 0.5422 |
| CD86 | 17.9 | 17.9 | 0.5875 |
| MAPK14 | 14.8 | 14.9 | 0.5938 |
| ELA2 | 20.7 | 20.9 | 0.6264 |
| VEGF | 23.2 | 23.3 | 0.7141 |
| HSPA1A | 14.4 | 14.3 | 0.7476 |
| MMP12 | 23.3 | 23.1 | 0.7872 |
| HMGB1 | 16.9 | 16.9 | 0.9920 |

Table 2C

| Patient ID | Group | HMOX1 | TXNRD1 | logit | odds | Predicted probability of Colon Inf |
|---|---|---|---|---|---|---|
| CC-019 | Colon | 16.02 | 18.00 | 8.34 | 4194.09 | 0.9998 |
| CC-020 | Colon | 15.13 | 17.16 | 7.92 | 2748.70 | 0.9996 |
| CC-003 | Colon | 16.03 | 17.77 | 6.62 | 747.28 | 0.9987 |
| CC-014 | Colon | 15.84 | 17.50 | 5.82 | 336.20 | 0.9970 |
| CC-004 | Colon | 16.20 | 17.59 | 4.26 | 71.14 | 0.9861 |
| CC-018 | Colon | 15.49 | 16.95 | 4.15 | 63.21 | 0.9844 |
| CC-002 | Colon | 15.68 | 17.04 | 3.58 | 35.72 | 0.9728 |
| CC-005 | Colon | 16.59 | 17.79 | 3.16 | 23.58 | 0.9593 |
| CC-011 | Colon | 15.12 | 16.48 | 3.06 | 21.39 | 0.9553 |
| CC-007 | Colon | 16.46 | 17.60 | 2.63 | 13.87 | 0.9327 |
| CC-006 | Colon | 16.22 | 17.38 | 2.54 | 12.71 | 0.9271 |
| CC-012 | Colon | 16.05 | 17.16 | 2.05 | 7.74 | 0.8856 |
| CC-008 | Colon | 16.07 | 17.17 | 2.03 | 7.65 | 0.8844 |
| CC-009 | Colon | 16.47 | 17.45 | 1.45 | 4.28 | 0.8107 |
| HN-003 | Normals | 15.71 | 16.69 | 0.88 | 2.42 | 0.7073 |
| CC-001 | Colon | 15.06 | 16.11 | 0.82 | 2.26 | 0.6933 |
| CC-013 | Colon | 16.93 | 17.70 | 0.37 | 1.44 | 0.5905 |
| HN-001 | Normals | 16.73 | 17.49 | 0.14 | 1.15 | 0.5353 |
| CC-015 | Colon | 16.57 | 17.33 | 0.01 | 1.01 | 0.5031 |
| HN-020 | Normals | 16.11 | 16.82 | -0.72 | 0.49 | 0.3267 |
| HN-016 | Normals | 16.94 | 17.51 | -1.10 | 0.33 | 0.2494 |
| HN-010 | Normals | 16.62 | 17.21 | -1.15 | 0.32 | 0.2397 |
| HN-011 | Normals | 16.57 | 17.10 | -1.65 | 0.19 | 0.1617 |
| HN-004 | Normals | 15.07 | 15.77 | -1.65 | 0.19 | 0.1615 |
| HN-029 | Normals | 16.92 | 17.35 | -2.14 | 0.12 | 0.1052 |
| HN-022 | Normals | 17.98 | 18.19 | -2.84 | 0.06 | 0.0550 |
| HN-023 | Normals | 16.44 | 16.80 | -3.03 | 0.05 | 0.0462 |
| HN-032 | Normals | 16.47 | 16.80 | -3.19 | 0.04 | 0.0394 |
| HN-028 | Normals | 16.47 | 16.78 | -3.34 | 0.04 | 0.0342 |
| HN-027 | Normals | 16.76 | 17.02 | -3.46 | 0.03 | 0.0305 |
| HN-021 | Normals | 16.39 | 16.68 | -3.53 | 0.03 | 0.0286 |
| HN-026 | Normals | 16.41 | 16.69 | -3.62 | 0.03 | 0.0260 |
| HN-019 | Normals | 16.49 | 16.75 | -3.65 | 0.03 | 0.0253 |
| HN-018 | Normals | 16.25 | 16.52 | -3.82 | 0.02 | 0.0215 |
| HN-017 | Normals | 16.95 | 17.12 | -3.98 | 0.02 | 0.0183 |

(continued)

| Patient ID | Group | HMOX1 | TXNRD1 | logit | odds | Predicted probability of Colon Inf |
|---|---|---|---|---|---|---|
| HN-031 | Normals | 16.79 | 16.95 | -4.14 | 0.02 | 0.0157 |
| HN-014 | Normals | 16.26 | 16.48 | -4.17 | 0.02 | 0.0152 |
| HN-009 | Normals | 16.59 | 16.75 | -4.38 | 0.01 | 0.0124 |
| HN-012 | Normals | 16.63 | 16.77 | -4.39 | 0.01 | 0.0123 |
| CC-010 | Colon | 16.46 | 16.61 | -4.47 | 0.01 | 0.0114 |
| HN-015 | Normals | 16.87 | 16.96 | -4.61 | 0.01 | 0.0099 |
| HN-007 | Normals | 16.29 | 16.44 | -4.67 | 0.01 | 0.0093 |
| HN-024 | Normals | 17.19 | 17.23 | -4.69 | 0.01 | 0.0091 |
| HN-002 | Normals | 17.18 | 17.21 | -4.80 | 0.01 | 0.0082 |
| HN-030 | Normals | 17.42 | 17.29 | -5.72 | 0.00 | 0.0033 |
| HN-006 | Normals | 16.94 | 16.82 | -6.07 | 0.00 | 0.0023 |
| HN-008 | Normals | 15.87 | 15.79 | -6.61 | 0.00 | 0.0013 |
| HN-005 | Normals | 16.50 | 16.24 | -7.45 | 0.00 | 0.0006 |
| HN-013 | Normals | 16.66 | 16.32 | -7.89 | 0.00 | 0.0004 |
| HN-025 | Normals | 17.06 | 16.53 | -8.91 | 0.00 | 0.0001 |

Table 3A

| 2-gene and 1-gene models | | Entropy R-sq | #normal correct | #normal FALSE | #cc Correct | #cc FALSE | Normal N = 50 Correct Classification | Colon N = 23 Correct Classification | p-val 1 | p-val 2 | total used (excludes missing) # normals | # disease |
|---|---|---|---|---|---|---|---|---|---|---|---|---|
| ATM | CDKN2A | 0.64 | 44 | 6 | 21 | 2 | 88.0% | 91.3% | 4.2E-07 | 2.8E-08 | 50 | 23 |
| CDK4 | CDKN2A | 0.62 | 47 | 3 | 21 | 2 | 94.0% | 91.3% | 1.1E-06 | 2.2E-13 | 50 | 23 |
| CDKN2A | ITGB1 | 0.62 | 47 | 3 | 21 | 2 | 94.0% | 91.3% | 7.0E-12 | 1.2E-06 | 50 | 23 |
| CDKN2A | TNFRSF10A | 0.62 | 46 | 4 | 20 | 3 | 92.0% | 87.0% | 1.9E-11 | 1.3E-06 | 50 | 23 |
| RHOC | SMAD4 | 0.58 | 44 | 6 | 20 | 3 | 88.0% | 87.0% | 1.3E-09 | 1.6E-07 | 50 | 23 |
| ATM | GZMA | 0.58 | 43 | 7 | 20 | 3 | 86.0% | 87.0% | 8.3E-11 | 5.9E-07 | 50 | 23 |
| CDK4 | RHOC | 0.56 | 43 | 7 | 20 | 3 | 86.0% | 87.0% | 4.3E-07 | 3.7E-12 | 50 | 23 |
| ATM | RHOC | 0.56 | 43 | 7 | 20 | 3 | 86.0% | 87.0% | 5.1E-07 | 1.5E-06 | 50 | 23 |
| CDKN2A | ITGAE | 0.56 | 45 | 5 | 21 | 2 | 90.0% | 91.3% | 1.5E-09 | 2.5E-05 | 50 | 23 |
| CDKN2A | MSH2 | 0.56 | 42 | 8 | 20 | 3 | 84.0% | 87.0% | 5.4E-07 | 2.6E-05 | 50 | 23 |
| EGR1 | NAME4 | 0.54 | 44 | 6 | 20 | 3 | 88.0% | 87.0% | 2.6E-11 | 1.7E-07 | 50 | 23 |
| RHOC | VHL | 0.54 | 47 | 3 | 21 | 2 | 94.0% | 91.3% | 1.1E-11 | 1.4E-06 | 50 | 23 |
| CDKN2A | ITGA3 | 0.54 | 42 | 7 | 19 | 4 | 85.7% | 82.6% | 7.8E-12 | 8.1E-05 | 49 | 23 |
| ITGAE | RHOC | 0.54 | 43 | 7 | 20 | 3 | 86.0% | 87.0% | 1.5E-06 | 4.IE-09 | 50 | 23 |
| BCL2 | COKN2A | 0.53 | 46 | 4 | 20 | 3 | 92.0% | 87.0% | 9.6E-05 | 1.8E-11 | 50 | 23 |
| CDKN2A | SMAD4 | 0.52 | 44 | 6 | 20 | 3 | 88.0% | 87.0% | 2.4E-08 | 0.0002 | 50 | 23 |
| SMAD4 | TNF | 0.52 | 42 | 8 | 20 | 3 | 84.0% | 87.0% | 1.8E-07 | 2.6E-08 | 50 | 23 |
| CDKN2A | PITCH1 | 0.51 | 43 | 7 | 20 | 3 | 86.0% | 87.0% | 1.3E-11 | 0.0002 | 50 | 23 |
| ATM | TNF | 0.51 | 44 | 6 | 20 | 3 | 88.0% | 87.0% | 2.4E-07 | 1.3E-05 | 50 | 23 |
| COKN2A | COL18A1 | 0.51 | 45 | 5 | 20 | 3 | 90.0% | 87.0% | 2.3E-11 | 0.0002 | 50 | 23 |
| BCL2 | RHOC | 0.50 | 40 | 10 | 20 | 3 | 80.0% | 87.0% | 6.5E-06 | 5.6E-11 | 50 | 23 |

(continued)

| 2-gene and models | 1-gene models | Entropy R-sq | #normal correct | #normal FALSE | #cc Correct | #cc FALSE | Normal 50 Correct Classification | Colon 23 Correct Classification | p-val 1 | p-val 2 | total used (excludes missing) # normals | # disease |
|---|---|---|---|---|---|---|---|---|---|---|---|---|
| ATM | NRAS | 0.50 | 45 | 5 | 19 | 4 | 90.0% | 82.6% | 1.5E-10 | 2.2E-05 | 50 | 23 |
| CDKN2A | ERBB2 | 0.50 | 41 | 9 | 19 | 4 | 82.0% | 82.6% | 4.7E-11 | 0.0004 | 50 | 23 |
| NRAS | SMAD4 | 0.50 | 43 | 7 | 20 | 3 | 86.0% | 87.0% | 6.9E-08 | 1.8E-10 | 50 | 23 |
| CDKN2A | HRAS | 0.49 | 41 | 9 | 19 | 4 | 82.0% | 82.6% | 4.0E-11 | 0.0007 | 50 | 23 |
| RHOC | TNFRSF10A | 0.48 | 40 | 10 | 18 | 5 | 80.0% | 78.3% | 1.0E-08 | 1.7E-05 | 50 | 23 |
| MSH2 | RHOC | 0.48 | 43 | 7 | 20 | 3 | 86.0% | 87.0% | 2.1E-05 | 2.0E-05 | 50 | 23 |
| CDKN2A | SKIL | 0.48 | 43 | 7 | 20 | 3 | 86.0% | 87.0% | 5.9E-07 | 0.0011 | 50 | 23 |
| ATM | PCNA | 0.48 | 44 | 6 | 20 | 3 | 88.0% | 87.0% | 4.2E-11 | 7.0E-05 | 50 | 23 |
| NFKB1 | RHOC | 0.47 | 42 | 8 | 20 | 3 | 84.0% | 87.0% | 3.7E-05 | 1.5E-10 | 50 | 23 |
| RHOC | TP53 | 0.47 | 42 | 8 | 20 | 3 | 84.0% | 87.0% | 7.7E-11 | 4.0E-05 | 50 | 23 |
| CDKN2A | SKI | 0.47 | 40 | 10 | 19 | 4 | 80.0% | 82.6% | 4.2E-10 | 0.0021 | 50 | 23 |
| CDKN2A | EGR1 | 0.46 | 39 | 11 | 19 | 4 | 78.0% | 82.6% | 6.5E-06 | 0.0024 | 50 | 23 |
| CDKN2A | IFITM1 | 0.46 | 42 | 8 | 20 | 3 | 84.0% | 87.0% | 4.1E-08 | 0.0028 | 50 | 23 |
| CDKN2A | VHL | 0.46 | 41 | 9 | 20 | 3 | 82.0% | 87.0% | 4.3E-10 | 0.0029 | 50 | 23 |
| CDKN2A | IL8 | 0.46 | 39 | 11 | 19 | 4 | 78.0% | 82.6% | 1.3E-07 | 0.0029 | 50 | 23 |
| CDKN2A | NAME4 | 0.46 | 44 | 6 | 19 | 4 | 88.0% | 82.6% | 1.2E-09 | 0.0032 | 50 | 23 |
| CDKN2A | NFKB1 | 0.46 | 42 | 8 | 19 | 4 | 84.0% | 82.6% | 2.6E-10 | 0.0034 | 50 | 23 |
| SMAD4 | TEMP1 | 0.45 | 39 | 11 | 18 | 5 | 78.0% | 78.3% | 4.9E-09 | 5.3E-07 | 50 | 23 |
| CDK2 | CDKN2A | 0.45 | 42 | 8 | 19 | 4 | 84.0% | 82.6% | 0.0041 | 1.4E-10 | 50 | 23 |
| ITGB1 | RHOC | 0.45 | 41 | 9 | 19 | 4 | 82.0% | 82.6% | 7.9E-05 | 1.8E-08 | 50 | 23 |
| CASP8 | CDKN2A | 0.45 | 40 | 10 | 19 | 4 | 80.0% | 82.6% | 0.0050 | 1.5E-09 | 50 | 23 |

| | models | | | | | | Normal | Colon | | | | |
|---|---|---|---|---|---|---|---|---|---|---|---|---|
| | | | | | | N = | 50 | 23 | | | | |
| 2-gene and | | Entropy | #normal | #normal | #cc | #cc | Correct | Correct | | | total used (excludes missing) | |
| 1-gene models | | R-sq | correct | FALSE | Correct | FALSE | Classification | Classification | p-val 1 | p-val 2 | # normals | # disease |
| CDKN2A | TP53 | 0.45 | 40 | 10 | 19 | 4 | 80.0% | 82.6% | 1.8E-10 | 0.0051 | 50 | 23 |
| PTCH1 | RHOC | 0.45 | 42 | 8 | 19 | 4 | 84.0% | 82.6% | 0.0001 | 3.3E-10 | 50 | 23 |
| ERBB2 | RHOC | 0.44 | 39 | 11 | 19 | 4 | 78.0% | 82.6% | 0.0001 | 6.8E-10 | 50 | 23 |
| NME4 | RHOC | 0.44 | 42 | 8 | 19 | 4 | 84.0% | 82.6% | 0.0001 | 2.4E-09 | 50 | 23 |
| ITGA3 | RHOC | 0.44 | 41 | 8 | 19 | 4 | 83.7% | 82.6% | 0.0001 | 6.7E-10 | 50 | 23 |
| ITGAE | TNF | 0.44 | 40 | 10 | 18 | 5 | 80.0% | 78.3% | 7.7E-06 | 3.7E-07 | 49 | 23 |
| CDKN2A | MYC | 0.44 | 38 | 12 | 19 | 4 | 76.0% | 82.6% | 6.6E-10 | 0.0086 | 50 | 23 |
| CDKN2A | PCNA | 0.44 | 42 | 8 | 20 | 3 | 84.0% | 87.0% | 3.1E-10 | 0.0097 | 50 | 23 |
| APAF1 | CDKN2A | 0.43 | 41 | 9 | 19 | 4 | 82.0% | 82.6% | 0.0101 | 7.5E-08 | 50 | 23 |
| MASH2 | NME4 | 0.43 | 41 | 9 | 19 | 4 | 82.0% | 82.6% | 4.0E-09 | 0.0002 | 50 | 23 |
| GZMA | MSH2 | 0.43 | 43 | 7 | 19 | 4 | 86.0% | 82.6% | 0.0002 | 1.0E-07 | 50 | 23 |
| RHOC | SRC | 0.43 | 42 | 8 | 20 | 3 | 84.0% | 87.0% | 8.9E-10 | 0.0003 | 50 | 23 |
| AKT1 | RHOC | 0.42 | 41 | 9 | 18 | 5 | 82.0% | 78.3% | 0.0003 | 6.3E-10 | 50 | 23 |
| CDKN2A | FOS | 0.42 | 39 | 10 | 18 | 5 | 79.6% | 78.3% | 2.1E-08 | 0.0205 | 50 | 23 |
| CDKN2A | NME1 | 0.42 | 44 | 6 | 19 | 4 | 88.0% | 82.6% | 6.5E-10 | 0.0225 | 49 | 23 |
| ATM | WANT1 | 0.42 | 43 | 7 | 19 | 4 | 86.0% | 82.6% | 8.4E-09 | 0.0012 | 50 | 23 |
| RHOC | SKI | 0.42 | 39 | 11 | 18 | 5 | 78.0% | 78.3% | 3.9E-09 | 0.0004 | 50 | 23 |
| MYCL1 | RHOC | 0.42 | 41 | 9 | 19 | 4 | 82.0% | 82.6% | 0.0004 | 7.7E-10 | 50 | 23 |
| ITGB1 | TNF | 0.41 | 39 | 11 | 19 | 4 | 78.0% | 82.6% | 2.9E-05 | 1.3E-07 | 50 | 23 |
| ATM | TGFB1 | 0.41 | 42 | 8 | 20 | 3 | 84.0% | 87.0% | 6.0E-08 | 0.0020 | 50 | 23 |
| ABL2 | RHOC | 0.41 | 41 | 9 | 18 | 5 | 82.0% | 78.3% | 0.0007 | 1.4E-09 | 50 | 23 |

EP 2 402 464 A1

| | models | | | | | N = | Normal 50 | Colon 23 | | | total used (excludes missing) | |
|---|---|---|---|---|---|---|---|---|---|---|---|---|
| 2-gene and | | Entropy | #normal | #normal | #cc | #cc | Correct | Correct | | | | |
| 1-gene models | | R-sq | correct | FALSE | Correct | FALSE | Classification | Classification | p-val 1 | p-val 2 | # normals | # disease |
| HRAS | RHOC | 0.41 | 42 | 8 | 19 | 4 | 84.0% | 82.6% | 0.0007 | 1.6E-09 | 50 | 23 |
| MYC | RHOC | 0.41 | 42 | 8 | 19 | 4 | 84.0% | 82.6% | 0.0007 | 2.7E-09 | 50 | 23 |
| AKT1 | CDKN2A | 0.41 | 40 | 10 | 19 | 4 | 80.0% | 82.6% | 0.0441 | 1.4E-09 | 50 | 23 |
| CDKN2A | E2F1 | 0.41 | 42 | 8 | 19 | 4 | 84.0% | 82.6% | 3.6E-06 | 0.0453 | 50 | 23 |
| CDKN2A | IL18 | 0.40 | 42 | 8 | 19 | 4 | 84.0% | 82.6% | 1.9E-08 | 0.0491 | 50 | 23 |
| RHOC | SKIL | 0.40 | 45 | 5 | 20 | 3 | 90.0% | 87.0% | 2.1E-05 | 0.0008 | 50 | 23 |
| ABL1 | CDKN2A | 0.40 | 41 | 9 | 18 | 5 | 82.0% | 78.3% | 0.0500 | 1.8E-09 | 50 | 23 |
| MASH2 | PCNA | 0.40 | 39 | 11 | 18 | 5 | 78.0% | 78.3% | 1.4E-09 | 0.0008 | 50 | 23 |
| EGR1 | RHOC | 0.40 | 38 | 12 | 18 | 5 | 76.0% | 78.3% | 0.0009 | 0.0001 | 50 | 23 |
| ATM | TIMP1 | 0.40 | 42 | 8 | 18 | 5 | 84.0% | 78.3% | 6.0E-08 | 0.0030 | 50 | 23 |
| TNF | TNFRSF10A | 0.40 | 39 | 11 | 19 | 4 | 78.0% | 82.6% | 5.3E-07 | 5.0E-05 | 50 | 23 |
| EGR1 | ITGAE | 0.40 | 42 | 8 | 19 | 4 | 84.0% | 82.6% | 2.6E-06 | 0.0001 | 50 | 23 |
| GZMA | SMAD4 | 0.40 | 45 | 5 | 18 | 5 | 90.0% | 78.3% | 7.9E-06 | 4.6E-07 | 50 | 23 |
| MSH2 | TNF | 0.40 | 42 | 8 | 20 | 3 | 84.0% | 87.0% | 6.1E-05 | 0.0012 | 50 | 23 |
| ATM | BAX | 0.40 | 38 | 12 | 19 | 4 | 76.0% | 82.6% | 2.6E-09 | 0.0039 | 50 | 23 |
| TNF | VHL | 0.39 | 42 | 8 | 18 | 5 | 84.0% | 78.3% | 9.3E-09 | 6.8E-05 | 50 | 23 |
| ATM | IFNG | 0.39 | 40 | 10 | 19 | 4 | 80.0% | 82.6% | 2.7E-09 | 0.0044 | 50 | 23 |
| ATM | BAD | 0.39 | 42 | 8 | 19 | 4 | 84.0% | 82.6% | 3.8E-09 | 0.0048 | 50 | 23 |
| NOTCH2 | RHOC | 0.39 | 43 | 7 | 18 | 5 | 86.0% | 78.3% | 0.0015 | 2.7E-09 | 50 | 23 |
| SKIL | TNFRSF6 | 0.39 | 42 | 8 | 19 | 4 | 84.0% | 82.6% | 2.6E-09 | 4.3E-05 | 50 | 23 |
| EGR1 | GZMA | 0.39 | 42 | 8 | 20 | 3 | 84.0% | 87.0% | 7.9E-07 | 0.0003 | 50 | 23 |

(continued)

| 2-gene and | | Entropy | #normal | #normal | #cc | #cc | Normal N = 50 Correct Classification | Colon 23 Correct Classification | p-val 1 | p-val 2 | total used (excludes missing) | |
|---|---|---|---|---|---|---|---|---|---|---|---|---|
| 1-gene models | | R-sq | correct | FALSE | Correct | FALSE | | | | | # normals | # disease |
| GZMA | SKIL | 0.39 | 39 | 11 | 19 | 4 | 78.0% | 82.6% | 5.1E-05 | 8.0E-07 | 50 | 23 |
| SKI | TGFB1 | 0.38 | 39 | 11 | 19 | 4 | 78.0% | 82.6% | 2.0E-07 | 2.0E-08 | 50 | 23 |
| NFKB1 | TNF | 0.38 | 40 | 10 | 18 | 5 | 80.0% | 78.3% | 0.0001 | 8.1E-09 | 50 | 23 |
| RHOC | SEMA4D | 0.38 | 40 | 10 | 18 | 5 | 80.0% | 78.3% | 4.9E-09 | 0.0027 | 50 | 23 |
| RHOC | TNFRSF10B | 0.38 | 39 | 11 | 18 | 5 | 78.0% | 78.3% | 9.3E-09 | 0.0027 | 50 | 23 |
| MSH2 | TGFB1 | 0.38 | 43 | 7 | 19 | 4 | 86.0% | 82.6% | 2.4E-07 | 0.0027 | 50 | 23 |
| ATM | EGR1 | 0.38 | 41 | 9 | 19 | 4 | 82.0% | 82.6% | 0.0004 | 0.0095 | 50 | 23 |
| ATM | TP53 | 0.38 | 39 | 11 | 18 | 5 | 78.0% | 78.3% | 5.0E-09 | 0.0098 | 50 | 23 |
| ITGAE | TGFB1 | D.38 | 38 | 12 | 18 | 5 | 76.0% | 78.3% | 2.7E-07 | 7.1E-06 | 50 | 23 |
| CASP8 | RHOC | 0.38 | 40 | 10 | 18 | 5 | 80.0% | 78.3% | 0.0033 | 4.5E-08 | 50 | 23 |
| ATM | ITGA1 | 0.37 | 38 | 12 | 18 | 5 | 76.0% | 78.3% | 8.2E-09 | 0.0127 | 50 | 23 |
| ATM | NME4 | 0.37 | 40 | 10 | 19 | 4 | 80.0% | 82.6% | 7.2E-08 | 0.0145 | 50 | 23 |
| ATM | TNFRSF6 | 0.37 | 40 | 10 | 18 | 5 | 80.0% | 78.3% | 6.6E-09 | 0.0145 | 50 | 23 |
| RHOA | RHOC | 0.37 | 40 | 10 | 18 | 5 | 80.0% | 78.3% | 0.0050 | 8.2E-09 | 50 | 23 |
| CDK4 | TNF | 0.37 | 38 | 12 | 18 | 5 | 76.0% | 78.3% | 0.0002 | 3.3E-08 | 50 | 23 |
| BCL2 | TNF | 0.37 | 38 | 12 | 18 | 5 | 76.0% | 78.3% | 0.0003 | 3.6E-08 | 50 | 23 |
| APAF1 | RHOC | 0.37 | 41 | 9 | 19 | 4 | 82.0% | 82.6% | 0.0056 | 2.0E-06 | 50 | 23 |
| ATM | PLAUR | 0.37 | 40 | 9 | 19 | 4 | 81.6% | 82.6% | 5.2E-08 | 0.0145 | 49 | 23 |
| ATM | IFITM1 | 0.36 | 39 | 11 | 18 | 5 | 78.0% | 78.3% | 3.8E-06 | 0.0193 | 50 | 23 |
| CDK5 | SMAD4 | 0.36 | 45 | 5 | 18 | 5 | 30.0% | 78.3% | 4.0E-05 | 1.9E-08 | 50 | 23 |
| FOS | RHOC | 0.36 | 38 | 11 | 19 | 4 | 77.6% | 82.6% | 0.0156 | 3.4E-07 | 49 | 23 |

(continued)

| | models | | | | | | Normal | Colon | | | | |
|---|---|---|---|---|---|---|---|---|---|---|---|---|
| | | | | | | N = | 50 | 23 | | | | |
| 2-gene and | | Entropy | #normal | #normal | #cc | #cc | Correct | Correct | | | total used (excludes missing) | |
| 1-gene models | | R-sq | correct | FALSE | Correct | FALSE | Classification | Classification | p-val 1 | p-val 2 | # normals | # disease |
| SKIL | TNF | 0.36 | 41 | 9 | 19 | 4 | 82.0% | 82.6% | 0.0003 | 0.0002 | 50 | 23 |
| RHOA | SMAD4 | 0.36 | 41 | 9 | 18 | 5 | 82.0% | 78.3% | 4.1E-05 | 1.0E-08 | 50 | 23 |
| ATM | TNFRSF1A | 0.36 | 44 | 6 | 18 | 5 | 88.0% | 78.3% | 4.4E-08 | 0.0208 | 50 | 23 |
| ABL1 | RHOC | 0.36 | 42 | 8 | 19 | 4 | 84.0% | 82.6% | 0.0065 | 1.3E-08 | 50 | 23 |
| ABL1 | ATM | 0.36 | 42 | 8 | 18 | 5 | 84.0% | 78.3% | 0.0215 | 1.3E-08 | 50 | 23 |
| ATM | IGFBP3 | 0.36 | 40 | 10 | 18 | 5 | 80.0% | 78.3% | 1.6E-08 | 0.0218 | 50 | 23 |
| CDKN2A | | 0.36 | 40 | 10 | 18 | 5 | 80.0% | 78.3% | 9.5E-09 | | 50 | 23 |
| NAME4 | TNF | 0.36 | 40 | 10 | 18 | 5 | 80.0% | 78.3% | 0.0003 | 1.1E-07 | 50 | 23 |
| COL18A1 | RHOC | 0.36 | 39 | 11 | 19 | 4 | 78.0% | 82.6% | 0.0073 | 2.6E-08 | 50 | 23 |
| SMAD4 | TNFRSF1A | 0.36 | 40 | 10 | 18 | 5 | 80.0% | 78.3% | 5.3E-08 | 5.0E-05 | 50 | 23 |
| ATM | ITGAE | 0.36 | 38 | 12 | 18 | 5 | 76.0% | 78.3% | 1.7E-05 | 0.0261 | 50 | 23 |
| NRAS | SKIL | 0.36 | 44 | 6 | 19 | 4 | 88.0% | 82.6% | 0.0002 | 1.3E-07 | 50 | 23 |
| BRCA1 | RHOC | 0.36 | 39 | 11 | 18 | 5 | 78.0% | 78.3% | 0.0094 | 8.7E-08 | 50 | 23 |
| GZMA | ITGB1 | 0.35 | 40 | 10 | 18 | 5 | 80.0% | 78.3% | 2.0E-06 | 3.7E-06 | 50 | 23 |
| ATM | FOS | 0.35 | 38 | 11 | 18 | 5 | 77.6% | 78.3% | 5.8E-07 | 0.0340 | 49 | 23 |
| EGR1 | SMAD4 | 0.35 | 41 | 9 | 18 | 5 | 82.0% | 78.3% | 7.1E-05 | 0.0014 | 50 | 23 |
| MSH2 | NRAS | 0.35 | 39 | 11 | 19 | 4 | 78.0% | 82.6% | 1.9E-07 | 0.0122 | 50 | 23 |
| IFITM1 | SKIL | 0.35 | 41 | 9 | 18 | 5 | 82.0% | 78.3% | 0.0003 | 7.7E-06 | 50 | 23 |
| BAX | MSH2 | 0.35 | 38 | 12 | 19 | 4 | 76.0% | 82.6% | 0.0125 | 2.3E-08 | 50 | 23 |
| ATM | RHOA | 0.35 | 38 | 12 | 18 | 5 | 76.0% | 78.3% | 2-0E-09 | 0.0449 | 50 | 23 |
| ATM | PTCH1 | 0.35 | 40 | 10 | 18 | 5 | 80.0% | 78.3% | 3.0E-08 | 0.0450 | 50 | 23 |

| 2-gene and | | Entropy | #normal | #normal | #cc | #cc | Normal | Colon | | | total used (excludes missing) | |
|---|---|---|---|---|---|---|---|---|---|---|---|---|
| models | | | | | | | N = | 50 | 23 | | | |
| | | | | | | | Correct | Correct | | | | |
| 1-gene models | | R-sq | correct | FALSE | Correct | FALSE | Classification | Classification | p-val 1 | p-val 2 | # normals | # disease |
| MSH2 | TIMP1 | 0.35 | 41 | 9 | 19 | 4 | 82.0% | 82.6% | 7.4E-07 | 0.0134 | 50 | 23 |
| ATM | RB1 | 0.35 | 39 | 11 | 18 | 5 | 78.0% | 78.3% | 2.-1E-07 | 0.0468 | 50 | 23 |
| ATM | IL8 | 0.35 | 39 | 11 | 18 | 5 | 78.0% | 78.3% | 2.7E-05 | 0.0476 | 50 | 23 |
| SKIL | TIMP1 | 0.35 | 42 | 8 | 19 | 4 | 84.0% | 82.6% | 7.9E-07 | 0.0003 | 50 | 23 |
| CDK5 | RHOC | 0.35 | 41 | 9 | 19 | 4 | 82.0% | 82.6% | 0.0152 | 4.3E-08 | 50 | 23 |
| CFLAR | RHOC | 0.34 | 40 | 10 | 18 | 5 | 80.0% | 78.3% | 0.0167 | 2.8E-07 | 50 | 23 |
| ITGAE | TIMP1 | 0.34 | 39 | 11 | 18 | 5 | 78.0% | 78.3% | 8.8E-07 | 3AE-05 | 50 | 23 |
| BAX | RHOC | 0.34 | 42 | 8 | 18 | 5 | 84.0% | 78.3% | 0.0168 | 2.9E-08 | 50 | 23 |
| TNF | TP53 | 0.34 | 40 | 10 | 8 | 5 | 80.0% | 78.3% | 2.5E-08 | 0.0008 | 50 | 23 |
| ITGAE | MSH2 | 0.34 | 44 | 6 | 18 | 5 | 88.0% | 78.3% | 0.0175 | 37E-05 | 50 | 23 |
| MSH2 | NME1 | 0.34 | 39 | 11 | 18 | 5 | 78.0% | 78.3% | 24E-08 | 0.0177 | 50 | 23 |
| MSH2 | WNT1 | 0.34 | 42 | 8 | 19 | 4 | 84.0% | 82.6% | 3.1E-07 | 0.0178 | 50 | 23 |
| SMAD4 | WNT1 | 0.34 | 41 | 9 | 19 | 4 | 82.0% | 82.6% | 3.3E-07 | 0.0001 | 50 | 23 |
| MSH2 | S100A4 | 0.34 | 41 | 9 | 19 | 4 | 82.0% | 82.6% | 3.4E-08 | 0.0191 | 50 | 23 |
| RB1 | RHOC | 0.34 | 41 | 9 | 19 | 4 | 82.0% | 82.6% | 0.0208 | 3.0E-07 | 50 | 23 |
| ITGB1 | NRAS | 0.34 | 42 | 8 | 18 | 5 | 84.0% | 78.3% | 3.2E-07 | 4.0E-06 | 50 | 23 |
| IFITM1 | MSH2 | 0.34 | 40 | 10 | 18 | 5 | 80.0% | 78.3% | 0.0230 | 1.4E-05 | 50 | 23 |
| E2F1 | RHOC | 0.34 | 39 | 11 | 18 | 5 | 78.0% | 78.3% | 0.0247 | 9.9E-05 | 50 | 23 |
| CDK5 | MSH2 | 0.34 | 44 | 6 | 19 | 4 | 88.0% | 82.6% | 0.0246 | 6.9E-08 | 50 | 23 |
| EGR1 | MSH2 | 0.34 | 39 | 11 | 19 | 4 | 78.0% | 82.6% | 0.0251 | 0.0031 | 50 | 23 |
| BAD | MSH2 | 0.34 | 40 | 10 | 18 | 5 | 80.0% | 78.3% | 0.0256 | 5.4E-08 | 50 | 23 |

EP 2 402 464 A1

| | models | | | | | | Normal | Colon | | | | |
|---|---|---|---|---|---|---|---|---|---|---|---|---|
| | | | | | | N = | 50 | 23 | | | | |
| 2-gene and | | Entropy | #normal | #normal | #cc | #cc | Correct | Correct | | | total used (excludes missing) | |
| 1-gene models | | R-sq | correct | FALSE | Correct | FALSE | Classification | Classification | p-val 1 | p-val 2 | # normals | # disease |
| APAF1 | IFITM1 | 0.33 | 39 | 11 | 18 | 5 | 78.0% | 78.3% | 1.6E-05 | 9.0E-06 | 50 | 23 |
| IL8 | RHOC | 0.33 | 40 | 10 | 18 | 5 | 80.0% | 78.3% | 0.0301 | 5.4E-05 | 50 | 23 |
| APAF1 | TNF | 0.33 | 38 | 12 | 18 | 5 | 76.0% | 78.3% | 0.0014 | 1.0E-05 | 50 | 23 |
| BRAF | RHOC | 0.33 | 40 | 10 | 18 | 5 | 80.0% | 78.3% | 0.0340 | 1.2E-07 | 50 | 23 |
| ABL2 | SMAD4 | 0.33 | 40 | 10 | 19 | 4 | 80.0% | 82.6% | 0.0002 | 5.9E-08 | 50 | 23 |
| MSH2 | PLAUR | 0.33 | 37 | 12 | 18 | 5 | 75.5% | 78.3% | 3.1E-07 | 0.0299 | 49 | 23 |
| GZMA | RHOC | 0.33 | 42 | 8 | 19 | 4 | 84.0% | 82.6% | 0.0434 | 1.4E-05 | 50 | 23 |
| FOS | MSH2 | 0.32 | 40 | 9 | 19 | 4 | 81.6% | 82.6% | 0.0436 | 2.1E-06 | 49 | 23 |
| IL8 | MSH2 | 0.32 | 39 | 11 | 18 | 5 | 78.0% | 78.3% | 0.0448 | 8.0E-05 | 50 | 23 |
| EGR1 | SKIL | 0.32 | 41 | 9 | 18 | 5 | 82.0% | 78.3% | 0.0011 | 0.0057 | 50 | 23 |
| NME4 | SKIL | 0.32 | 39 | 11 | 18 | 5 | 78.0% | 78.3% | 0.0012 | 7.0E-07 | 50 | 23 |
| E2F1 | ITGAE | 0.32 | 39 | 11 | 18 | 5 | 78.0% | 78.3% | 0.0001 | 0.0002 | 50 | 23 |
| E2F1 | GZMA | 0.32 | 39 | 11 | 18 | 5 | 78.0% | 78.3% | 2.2E-05 | 0.0003 | 50 | 23 |
| APAF1 | FOS | 0.31 | 38 | 11 | 18 | 5 | 77.6% | 78.3% | 3.5E-06 | 2.0E-05 | 49 | 23 |
| BRAF | TNF | 0.31 | 41 | 9 | 18 | 5 | 82.0% | 78.3% | 0.0035 | 2.7E-07 | 50 | 23 |
| GZMA | IL8 | 0.31 | 40 | 10 | 18 | 5 | 80.0% | 78.3% | 0.0002 | 2.8E-05 | 50 | 23 |
| SKIL | TGFB1 | 0.31 | 41 | 9 | 18 | 5 | 82.0% | 78.3% | 6.6E-06 | 0.0021 | 50 | 23 |
| FOS | SKIL | 0.31 | 40 | 9 | 18 | 5 | 81.6% | 78.3% | 0.0018 | 4.7E-06 | 49 | 23 |
| TGFB1 | TNFRSF10A | 0.30 | 40 | 10 | 18 | 5 | 80.0% | 78.3% | 5.0E-05 | 8.5E-06 | 50 | 23 |
| IL1B | SKIL | 0.30 | 42 | 8 | 18 | 5 | 84.0% | 78.3% | 0.0032 | 2.9E-07 | 50 | 23 |
| SEMA4D | TNF | 0.30 | 42 | 8 | 18 | 5 | 84.0% | 78.3% | 0.0073 | 2.3E-07 | 50 | 23 |

| models | | | | | | Normal | Colon | | | | |
|---|---|---|---|---|---|---|---|---|---|---|---|
| | models | | | | | N = | 50 | 23 | | | |
| 2-gene and | | Entropy | #normal | #normal | #cc | #cc | Correct | Correct | | | total used (excludes missing) |
| 1-gene models | | R-sq | correct | FALSE | Correct | FALSE | Classification | Classification | p-val 1 | p-val 2 | # normals | # disease |
| APAF1 | EGR1 | 0.30 | 40 | 10 | 18 | 5 | 80.0% | 78.3% | 0.0211 | 5.0E-05 | 50 | 23 |
| SKIL | TNFRSF1A | 0.30 | 42 | 8 | 18 | 5 | 84.0% | 78.3% | 9.4E-07 | 0.0038 | 50 | 23 |
| APAF1 | TGFB1 | 0.30 | 40 | 10 | 19 | 4 | 80.0% | 82.6% | 1.3E-05 | 5.4E-05 | 50 | 23 |
| EGR1 | SKI | 0.29 | 40 | 10 | 19 | 4 | 80.0% | 82.6% | 1.3E-06 | 0.0247 | 50 | 23 |
| PLAUR | SKIL | 0.29 | 38 | 11 | 18 | 5 | 77.6% | 78.3% | 0.0038 | 1.5E-06 | 49 | 23 |
| IL8 | TNF | 0.29 | 39 | 11 | 18 | 5 | 78.0% | 78.3% | 0.0105 | 0.0004 | 50 | 23 |
| CDK5 | SKIL | 0.29 | 38 | 12 | 18 | 5 | 76.0% | 78.3% | 0.0057 | 6.1E-07 | 50 | 23 |
| EGR1 | MYC | 0.29 | 38 | 12 | 18 | 5 | 76.0% | 78.3% | 7.6E-07 | 0.0363 | 50 | 23 |
| BAD | SMAD4 | 0.29 | 39 | 11 | 18 | 5 | 78.0% | 78.3% | 0.0017 | 5.4E-07 | 50 | 23 |
| COL18A1 | EGR1 | 0.29 | 40 | 10 | 18 | 5 | 80.0% | 78.3% | 0.0390 | 8.5E-07 | 50 | 23 |
| PCNA | SMAD4 | 0.29 | 42 | 8 | 19 | 4 | 84.0% | 82.6% | 0.0017 | 3.4E-07 | 50 | 23 |
| GZMA | IFITM1 | 0.29 | 41 | 9 | 18 | 5 | 82.0% | 78.3% | 0.0002 | 9.4E-05 | 50 | 23 |
| CFLAR | TNF | 0.29 | 39 | 11 | 18 | 5 | 78.0% | 78.3% | 0.0141 | 4.5E-06 | 50 | 23 |
| BCL2 | EGR1 | 0.28 | 41 | 9 | 18 | 5 | 82.0% | 78.3% | 0.0434 | 1.7E-06 | 50 | 23 |
| MMP9 | SKIL | 0.28 | 41 | 9 | 19 | 4 | 82.0% | 82.6% | 0.0084 | 1.9E-06 | 50 | 23 |
| RHOC | | 0.28 | 38 | 12 | 18 | 5 | 76.0% | 78.3% | 4.2E-07 | | 50 | 23 |
| E2F1 | TNF | 0.28 | 38 | 12 | 18 | 5 | 76.0% | 78.3% | 0.0178 | 0.0015 | 50 | 23 |
| MSH2 | | 0.28 | 41 | 9 | 19 | 4 | 82.0% | 82.6% | 4.4E07 | | 50 | 23 |
| BAX | TNFRSF10A | 0.28 | 38 | 12 | 18 | 5 | 76.0% | 78.3% | 0.0002 | 7.4E-07 | 50 | 23 |
| NRAS | VHL | 0.28 | 39 | 11 | 18 | 5 | 78.0% | 78.3% | 2.5E-06 | 6.4E-06 | 50 | 23 |
| NRAS | TNFRSF10A | 0.27 | 40 | 10 | 18 | 5 | 80.0% | 78.3% | 0.0002 | 6.6E-06 | 50 | 23 |

EP 2 402 464 A1

| | models | | | | | | Normal | Colon | | | | |
|---|---|---|---|---|---|---|---|---|---|---|---|---|
| | | | | | | N = | 50 | 23 | | | | |
| 2-gene and | | Entropy | #normal | #normal | #cc | #cc | Correct | Correct | | | total used (excludes missing) | |
| 1-gene models | | R-sq | correct | FALSE | Correct | FALSE | Classification | Classification | p-val 1 | p-val 2 | # normals | # disease |
| ITGA1 | SKIL | 0.27 | 39 | 11 | 18 | 5 | 78.0% | 78.3% | 0.0126 | 8.4E-07 | 50 | 23 |
| IFITM1 | ITGAE | 0.27 | 40 | 10 | 18 | 5 | 80.0% | 78.3% | 0.0011 | 0.0003 | 50 | 23 |
| PCNA | SKIL | 0.27 | 45 | 5 | 18 | 5 | 90.0% | 78.3% | 0.0176 | 8.1E-07 | 50 | 23 |
| ITGAE | PLAUR | 0.27 | 38 | 11 | 18 | 5 | 77.6% | 78.3% | 5.6E-06 | 0.0013 | 49 | 23 |
| ABL1 | SMAD4 | 0.26 | 40 | 10 | 18 | 5 | 80.0% | 78.3% | 0.0053 | 1.3E-06 | 50 | 23 |
| BAX | ITGAE | 0.26 | 39 | 11 | 18 | 5 | 78.0% | 78.3% | 0.0017 | 1.3E-06 | 50 | 23 |
| SERPINE1 | SKIL | 0.26 | 38 | 12 | 18 | 5 | 76.0% | 78.3% | 0.0269 | 2.8E-05 | 50 | 23 |
| NOTCH2 | SMAD4 | 0.26 | 39 | 11 | 18 | 5 | 78.0% | 78.3% | 0.0069 | 1.4E-06 | 50 | 23 |
| BAX | SMAD4 | 0.26 | 43 | 7 | 19 | 4 | 86.0% | 82.6% | 0.0072 | 1.7E-06 | 50 | 23 |
| BAD | ITGAE | 0.26 | 40 | 10 | 18 | 5 | 80.0% | 78.3% | 0.0024 | 2.2E-06 | 50 | 23 |
| ITGAE | WNT1 | 0.25 | 38 | 12 | 18 | 5 | 76.0% | 78.3% | 2.0E-05 | 0.0027 | 50 | 23 |
| CFLAR | TGFB1 | 0.25 | 38 | 12 | 18 | 5 | 76.0% | 78.3% | 0.0001 | 2.6E-05 | 50 | 23 |
| CDK2 | SMAD4 | 0.24 | 39 | 11 | 18 | 5 | 78.0% | 78.3% | 0.0139 | 2.4E-06 | 50 | 23 |
| S100A4 | SMAD4 | 0.24 | 40 | 10 | 18 | 5 | 80.0% | 78.3% | 0.0151 | 3.4E-06 | 50 | 23 |
| FOS | PTEN | 0.24 | 38 | 11 | 18 | 5 | 77.6% | 78.3% | 8.0E-05 | 0.0001 | 49 | 23 |
| ITGB1 | WNT1 | 0.24 | 38 | 12 | 18 | 5 | 76.0% | 78.3% | 3.6E-05 | 0.0004 | 50 | 23 |
| EGR1 | | 0.24 | 39 | 11 | 18 | 5 | 78.0% | 78.3% | 3.0E-06 | | 50 | 23 |
| FOS | IL8 | 0.24 | 38 | 11 | 18 | 5 | 77.6% | 78.3% | 0.0071 | 0.0001 | 49 | 23 |
| ITGAE | SMAD4 | 0.24 | 40 | 10 | 18 | 5 | 80.0% | 78.3% | 0.0224 | 0.0071 | 50 | 23 |
| CDK4 | TGF81 | 0.23 | 38 | 12 | 18 | 5 | 76.0% | 78.3% | 0.0003 | 2,1E-05 | 50 | 23 |
| BAD | TNFRSF10A | 0.23 | 38 | 12 | 18 | 5 | 76.0% | 78.3% | 0.0018 | 7.6E-06 | 50 | 23 |

EP 2 402 464 A1

| | models | | | | | | Normal | Colon | | | | |
|---|---|---|---|---|---|---|---|---|---|---|---|---|
| | | | | | | N = | 50 | 23 | | | | |
| 2-gene and | | Entropy | #normal | #normal | #cc | #cc | Correct | Correct | | | total used (excludes missing) | |
| 1-gene models | | R-sq | correct | FALSE | Correct | FALSE | Classification | Classification | p-val 1 | p-val 2 | # normals | # disease |
| CDKN1A | NME4 | 0.23 | 40 | 10 | 18 | 5 | 80.0% | 78.3% | 6.2E-05 | 0.0001 | 50 | 23 |
| IFITM1 | TNPRSF10A | 0.22 | 38 | 12 | 18 | 5 | 76.0% | 78.3% | 0.0025 | 0.0033 | 50 | 23 |
| ABL2 | TNFRSF10A | 0.22 | 40 | 10 | 18 | 5 | 80.0% | 78.3% | 0.0025 | 8.3E-06 | 50 | 23 |

EP 2 402 464 A1

Table 3B

|  | Colon | Normals | Sum |
|---|---|---|---|
| Group Size | 31.5% | 68.5% | 100% |
| N= | 23 | 50 | 73 |
|  |  |  |  |
| Gene | Mean | Mean | p-val |
| CDKN2A | 20.1 | 21.1 | 9.5E-09 |
| ATM | 17.3 | 16.5 | 1.4E-07 |
| RHOC | 15.9 | 16.6 | 4.2E-07 |
| MSH2 | 18.7 | 17.9 | 4.4E-07 |
| EGR1 | 18.9 | 19.8 | 3.0E-06 |
| TNF | 18.1 | 18.7 | 8.0E-06 |
| SKIL | 18.6 | 17.8 | 1.5E-05 |
| SMAD4 | 17.3 | 16.9 | 5.7E-05 |
| E2F1 | 19.5 | 20.2 | 8.4E-05 |
| ITGAE | 24.3 | 23.3 | 0.0002 |
| IL8 | 22.3 | 21.4 | 0.0002 |
| IFITM1 | 8.4 | 9.0 | 0.0006 |
| TNFRSF10A | 21.2 | 20.7 | 0.0008 |
| GZMA | 17.3 | 17.8 | 0.0010 |
| APAF1 | 17.5 | 17.0 | 0.0011 |
| ITGB1 | 14.9 | 14.5 | 0.0020 |
| TGFB1 | 12.4 | 12.7 | 0.0050 |
| TIMP1 | 14.1 | 14.5 | 0.0076 |
| PTEN | 14.2 | 13.8 | 0.0088 |
| FOS | 15.1 | 15.6 | 0.0091 |
| SERPINE1 | 20.6 | 21.1 | 0.0139 |
| SOCS1 | 16.4 | 16.8 | 0.0139 |
| CDKN1A | 15.9 | 16.3 | 0.0149 |
| ANGPT1 | 21.1 | 20.6 | 0.0172 |
| IL18 | 22.1 | 21.7 | 0.0226 |
| WMT1 | 21.2 | 21.6 | 0.0258 |
| CFLAR | 14.9 | 14.6 | 0.0262 |
| NRAS | 16.8 | 17.0 | 0.0309 |
| RB1 | 17.8 | 17.5 | 0.0310 |
| NME4 | 17.6 | 17.3 | 0.0313 |
| CASP8 | 15.2 | 15.0 | 0.0380 |
| BRCA1 | 21.6 | 21.3 | 0.0548 |
| SKI | 17.5 | 17.2 | 0.0638 |

(continued)

|  | Colon | Normals | Sum |
|---|---|---|---|
| Group Size | 31.5% | 68.5% | 100% |
| N= | 23 | 50 | 73 |
|  |  |  |  |
| Gene | Mean | Mean | p-val |
| PLAUR | 14.6 | 14.9 | 0.0695 |
| ICAM1 | 16.8 | 17.0 | 0.0697 |
| TNFRSF1A | 15.1 | 15.4 | 0.0809 |
| 8CL2 | 17.3 | 17.1 | 0.0859 |
| MMP9 | 14.1 | 14.6 | 0.0877 |
| CDK4 | 17.8 | 17.6 | 0.0890 |
| VHL | 17.4 | 17.2 | 0.0929 |
| CDC25A | 22.7 | 23.1 | 0.1161 |
| ERBB2 | 22.6 | 22.4 | 0.1360 |
| BRAF | 16.9 | 16.7 | 0.1511 |
| G1P3 | 15.1 | 15.4 | 0.1615 |
| COL18A1 | 23.8 | 23.3 | 0.1790 |
| CCNE1 | 22.8 | 23.1 | 0.1892 |
| MYC | 18.3 | 18.1 | 0.1898 |
| ITGA3 | 22.0 | 21.8 | 0.2006 |
| TNFRSF10B | 17.2 | 17.0 | 0.2062 |
| NPKB1 | 16.8 | 16.7 | 0.2158 |
| CDK5 | 18.5 | 18.6 | 0.2245 |
| RAF1 | 14.5 | 14.3 | 0.2450 |
| TH8S1 | 17.1 | 17.4 | 0.2556 |
| SRC | 18.1 | 18.3 | 0.2746 |
| IL1B | 15.6 | 15.8 | 0.2977 |
| PTCH1 | 20.1 | 19.9 | 0.3142 |
| IGFBP3 | 22.1 | 22.4 | 0.3151 |
| BAD | 18.1 | 18.2 | 0.3319 |
| HRAS | 20.2 | 20.0 | 0.3962 |
| ITGA1 | 21.0 | 21.1 | 0.4121 |
| FGFR2 | 22.5 | 22.8 | 0.4215 |
| ABL1 | 18.1 | 18.2 | 0.4378 |
| S100A4 | 13.0 | 13.2 | 0.4606 |
| ABL2 | 20.1 | 20.2 | 0.4676 |
| BAX | 15.6 | 15.7 | 0.4717 |
| IFNG | 23.1 | 23.3 | 0.5189 |
| SEMA4D | 14.3 | 14.2 | 0.5559 |

(continued)

|  | Colon | Normals | Sum |
|---|---|---|---|
| Group Size | 31.5% | 68.5% | 100% |
| N= | 23 | 50 | 73 |
|  |  |  |  |
| Gene | Mean | Mean | p-val |
| AKT1 | 15.1 | 15.0 | 0.5652 |
| PLAU | 23.9 | 24.0 | 0.6255 |
| RHOA | 11.6 | 11.6 | 0.6256 |
| NOTCH2 | 16.0 | 15.9 | 0.6295 |
| TP53 | 16.3 | 16.2 | 0.7109 |
| MYCL1 | 18.5 | 18.6 | 0.7168 |
| JUN | 20.9 | 20.9 | 0.8098 |
| CDK2 | 19.2 | 19.2 | 0.8892 |
| VEGF | 22.7 | 22.8 | 0.9203 |
| TNFRSF6 | 16.4 | 16.4 | 0.9420 |
| NME1 | 19.3 | 19.3 | 0.9578 |
| PCNA | 18.1 | 18.1 | 0.9609 |

Table 3C

| | | | | | | Predicted |
| | | | | | | probability |
| Patient ID | Group | ATM | CDKN2A | logit | odds | of colon cancer |
|---|---|---|---|---|---|---|
| CC-035 | Colon Cancer | 19.12 | 20.14 | 11.66 | 1.2E+05 | 1.0000 |
| CC-020 | Colon Cancer | 18.09 | 19.23 | 9.86 | 1.9E+04 | 0.9999 |
| CC-019 | Colon Cancer | 18.11 | 19.40 | 9.39 | 1.2E+04, | 0.9999 |
| CC-005 | Colon Cancer | 17.88 | 19.87 | 6.71 | 8.2E+02 | 0.9988 |
| CC-014 | Colon Cancer | 18.04 | 20.26 | 6.14 | 4.7E+02 | 0.9979 |
| CC-004 | Colon Cancer | 17.38 | 19.40 | 5.95 | 3.8E+02 | 0.9974 |
| CC-031 | Colon Cancer | 16.78 | 19.26 | 3.60 | 3.7E+01 | 0.9734 |
| CC-013 | Colon Cancer | 17.61 | 20.60 | 2.98 | 2.0E+01 | 0.9516 |
| CC-034 | Colon Cancer | 16.87 | 19.64 | 2.77 | 1.6E+01 | 0.9413 |
| CC-007 | Colon Cancer | 17.45 | 20.48 | 2.64 | 1.4E+01 | 0.9337 |
| CC-018 | Colon Cancer | 16.35 | 19.03 | 2.35 | 1.0E+01 | 0.9129 |
| CC-006 | Colon Cancer | 17.11 | 20.13 | 2.25 | 9.4E+00 | 0.9043 |
| CC-003 | Colon Cancer | 17.35 | 20.48 | 2.19 | 9.0E+00 | 0.8997 |
| CC-032 | Colon Cancer | 16.98 | 19.96 | 2.16 | 8.6E+00 | 0.8963 |
| CC-009 | Colon Cancer | 16.64 | 19.60 | 1.79 | 6.0E+00 | 0.8575 |
| CC-012 | Colon Cancer | 17.18 | 20.41 | 1.62 | 5.1E+00 | 0.8353 |
| HN-040 | Normal | 17.42 | 20.77 | 1.56 | 4.8E+00 | 0.8269 |

(continued)

| Patient ID | Group | ATM | CDKN2A | logit | odds | Predicted probability of colon cancer |
|---|---|---|---|---|---|---|
| HN-049 | Normal | 17.05 | 20.42 | 0.97 | 2.6E+00 | 0.7244 |
| CC-011 | Colon Cancer | 16.60 | 19.80 | 0.94 | 2.6E+00 | 0.7190 |
| HN-035 | Normal | 16.61 | 19.82 | 0.93 | 2.5E+00 | 0.7166 |
| CC-002 | Colon Cancer | 17.03 | 20.52 | 0.52 | 1.7E+00 | 0.6264 |
| CC-008 | Colon Cancer | 17.30 | 20.94 | 0.43 | 1.5E+00 | 0.6051 |
| CC-010 | Colon Cancer | 17.49 | 21.31 | 0.07 | 1.1E+00 | 0.5168 |
| HN-041 | Normal | 16.70 | 20.26 | -0.12 | 8.9E-01 | 0.4711 |
| HN-016 | Normal | 17.14 | 21.12 | -0.96 | 3.8E-01 | 0.2773 |
| HN-012 | Normal | 16.28 | 19.97 | -1.14 | 3.2E-01 | 0.2426 |
| CC-033 | Colon Cancer | 16.39 | 20.15 | -1.22 | 3.0E-01 | 0.2285 |
| HN-019 | Normal | 16.72 | 20.66 | -1.41 | 2.4E-01 | 0.1959 |
| HN-014 | Normal | 16.79 | 20.82 | -1.59 | 2.0E-01 | 0.1697 |
| CC-015 | Colon Cancer | 16.73 | 20.76 | -1.70 | 1.8E-01 | 0.1549 |
| HN-050 | Normal | 16.38 | 20.33 | -1.87 | 1.5E-01 | 0.1335 |
| HN-104 | Normal | 16.39 | 20.36 | -1.91 | 1.5E-01 | 0.1286 |
| HN-001 | Normal | 17.04 | 21.30 | -2.02 | 1.3E-01 | 0.1173 |
| HN-005 | Normal | 16.22 | 20.17 | -2.06 | 1.3E-01 | 0.1133 |
| HN-039 | Normal | 16.63 | 20.76 | -2.13 | 1.2E-01 | 0.1058 |
| HN-004 | Normal | 16.55 | 20.65 | -2.15 | 1.2E-01 | 0.1045 |
| HN-030 | Normal | 16.82 | 21.05 | -2.25 | 1.1E-01 | 0.0956 |
| CC-001 | Colon Cancer | 16.53 | 20.74 | -2.53 | 8.0E-02 | 0.0738 |
| HN-036 | Normal | 16.76 | 21.12 | -2.72 | 6.6E-02 | 0.0619 |
| HN-020 | Normal | 16.59 | 20.94 | -2.93 | 5.4E-02 | 0.0509 |
| HN-047 | Normal | 16.43 | 20.72 | -2.97 | 5.2E-02 | 0.0490 |
| HN-007 | Normal | 16.18 | 20.46 | -3.22 | 4.0E-02 | 0.0383 |
| HN-034 | Normal | 16.73 | 21.22 | -3.23 | 4.0E-02 | 0.0382 |
| HN-029 | Normal | 17.15 | 21.83 | -3.28 | 3.8E-02 | 0.0363 |
| HN-038 | Normal | 16.47 | 20.88 | -3.28 | 3.8E-02 | 0.0363 |
| HN-106 | Normal | 16.09 | 20.34 | -3.28 | 3.8E-02 | 0.0362 |
| HN-045 | Normal | 16.35 | 20.79 | -3.55 | 2.9E-02 | 0.0280 |
| HN-101 | Normal | 16.11 | 20.46 | -3.57 | 2.8E-02 | 0.0274 |
| HN-044 | Normal | 16.24 | 20.66 | -3.61 | 2.7E-02 | 0.0264 |
| HN-002 | Normal | 17.32 | 22.28 | -4.01 | 1.8E-02 | 0.0179 |
| HN-003 | Normal | 16.73 | 21.51 | -4.16 | 1.6E-02 | 0.0153 |
| HN-022 | Normal | 17.26 | 22.31 | -4.39 | 1.2E-02 | 0.0122 |
| HN-013 | Normal | 16.48 | 21.24 | -4.44 | 1.2E-02 | 0.0116 |

(continued)

| Patient ID | Group | ATM | CDKN2A | logit | odds | Predicted probability of colon cancer |
|---|---|---|---|---|---|---|
| HN-028 | Normal | 16.12 | 20.79 | -4.63 | 9.8E-03 | 0.0097 |
| HN-107 | Normal | 16.48 | 21.36 | -4.85 | 7.8E-03 | 0.0078 |
| HN-032 | Normal | 16.37 | 21.24 | -4.95 | 7.1E-03 | 0.0070 |
| HN-037 | Normal | 16.83 | 21.92 | -5.09 | 6.1E-03 | 0.0061 |
| HN-010 | Normal | 15.87 | 20.59 | -5.15 | 5.8E-03 | 0.0058 |
| HN-024 | Normal | 16.54 | 21.60 | -5.34 | 4.8E-03 | 0.0048 |
| HN-102 | Normal | 16.03 | 20.91 | -5.47 | 4.2E-03 | 0.0042 |
| HN-026 | Normal | 16.62 | 21.77 | -5.54 | 3.9E-03 | 0.0039 |
| HN-008 | Normal | 15.93 | 20.89 | -5.85 | 2.9E-03 | 0.0029 |
| HN-009 | Normal | 16.36 | 21.57 | -6.10 | 2.2E-03 | 0.0022 |
| HN-103 | Normal | 15.65 | 20.59 | -6.17 | 2.1E-03 | 0.0021 |
| HN-027 | Normal | 16.17 | 21.37 | -6.33 | 1.8E-03 | 0.0018 |
| HN-015 | Normal | 16.47 | 21.80 | -6.35 | 1.7E-03 | 0.0017 |
| HN-025 | Normal | 16.09 | 21.46 | -7.02 | 8.9E-04 | 0.0009 |
| HN-105 | Normal | 16.21 | 21.67 | -7.16 | 7.8E-04 | 0.0008 |
| HN-042 | Normal | 15.94 | 21.36 | -7.36 | 6.3E-04 | 0.0006 |
| HN-017 | Normal | 16.74 | 22.53 | -7.53 | 5.4E-04 | 0.0005 |
| HN-018 | Normal | 16.46 | 22.16 | -7.61 | 4.9E-04 | 0.0005 |
| HN-033 | Normal | 17.15 | 23.74 | -9.65 | 6.4E-05 | 0.0001 |
| HN-021 | Normal | 16.07 | 22.74 | -11.39 | 1.1E-05 | 0.0000 |

Table 4A

| 2-gene models | | Entropy R-sq | #normal correct | #normal FALSE | #cc Correct | #cc FALSE | Normal Correct Classification | Colon Correct Classification | p-val | p-val 2 | # normals | total used (excludes missing) disease |
|---|---|---|---|---|---|---|---|---|---|---|---|---|
| | | | | | | N= | Normal 50 | Colon 22 | | | | |
| NAB2 | TGFB1 | 0.45 | 41 | 9 | 18 | 4 | 82.0% | 81.8% | 6.4E-09 | 4.6E-07 | 50 | 22 |
| MAP2K1 | TGFB1 | 0.45 | 44 | 6 | 18 | 4 | 88.0% | 81.8% | 7.6E-09 | 1.5E-09 | 50 | 22 |
| TGFB1 | TOP8P1 | 0.42 | 38 | 12 | 18 | 4 | 76.0% | 81.8% | 2.1E-06 | 2.9E-08 | 50 | 22 |
| ICAM1 | TOPBP1 | 0.30 | 41 | 9 | 18 | 4 | 82.0% | 81.8% | 0.0007 | 1.1E-06 | 50 | 22 |
| CEBPB | TOPBP1 | 0.29 | 39 | 11 | 17 | 5 | 78.0% | 77.3% | 0.0011 | 9.6E-07 | 50 | 22 |
| EGR1 | NAB2 | 0.28 | 41 | 9 | 18 | 4 | 82.0% | 81.8% | 0.0016 | 0.0002 | 50 | 22 |
| NR4A2 | TGFB1 | 0.27 | 40 | 10 | 17 | 5 | 80.0% | 77.3% | 2.8E-05 | 7.3E-05 | 50 | 22 |
| NAB2 | PDGFA | 0.27 | 39 | 11 | 17 | 5 | 78.0% | 77.3% | 6.4E-06 | 0.0025 | 50 | 22 |
| CREBBP | TOPBP1 | 0.27 | 41 | 9 | 17 | 5 | 82.0% | 77.3% | 0.0026 | 1.3E-06 | 50 | 22 |
| FOS | NR4A2 | 0.26 | 38 | 11 | 17 | 5 | 77.6% | 77.3% | 0.0001 | 4.7E-05 | 49 | 22 |
| NAB1 | TGFB1 | 0.26 | 40 | 10 | 17 | 5 | 80.0% | 77.3% | 4.5E-05 | 0.0002 | 50 | 22 |
| EGR1 | NR4A2 | 0.26 | 39 | 11 | 17 | 5 | 78.0% | 77.3% | 0.0001 | 0.0004 | 50 | 22 |
| TOPBP1 | TNFRSF6 | 0.26 | 39 | 11 | 17 | 5 | 78.0% | 77.3% | 2.1E-06 | 0.0046 | 50 | 22 |
| NFKB1 | TOPBP1 | 0.23 | 38 | 12 | 17 | 5 | 76.0% | 77.3% | 0.0165 | 1.4E-05 | 50 | 22 |
| SRC | TOPBP1 | 0.23 | 39 | 11 | 17 | 5 | 78.0% | 77.3% | 0.0176 | 8.7E-06 | 50 | 22 |
| NAB2 | TOPBP1 | 0.23 | 39 | 11 | 17 | 5 | 78.0% | 77.3% | 0.0204 | 0.0205 | 50 | 22 |
| FOS | PTEN | 0.22 | 38 | 11 | 17 | 5 | 77.6% | 77.3% | 0.0001 | 0.0003 | 49 | 22 |
| NAB2 | PTEN | 0.22 | 39 | 11 | 17 | 5 | 78.0% | 77.3% | 0.0002 | 0.0237 | 50 | 22 |
| EGR2 | NAB1 | 0.20 | 42 | 8 | 17 | 5 | 84.0% | 77.3% | 0.0039 | 0.0011 | 50 | 22 |

EP 2 402 464 A1

Table 4B'

| | Colon | Normals | Sum |
|---|---|---|---|
| Group Size | 30.6% | 69.4% | 100% |
| N = | 22 | 50 | 72 |
| | | | |
| Gene | Mean | Mean | p-val |
| NAB2 | 20.42 | 19.91 | 0.0001 |
| TOPBP1 | 18.53 | 18.03 | 0.0001 |
| EGR1 | 19.19 | 19.85 | 0.0013 |
| NAB1 | 17.27 | 16.92 | 0.0025 |
| NR4A2 | 21.49 | 20.88 | 0.0041 |
| EGR2 | 23.57 | 24.11 | 0.0089 |
| TGFB1 | 12.43 | 12.73 | 0.0114 |
| FOS | 15.10 | 15.59 | 0.0122 |
| SERPINE1 | 20.62 | 21.10 | 0.0146 |
| PTEN | 14.16 | 13.81 | 0.0190 |
| PDGFA | 19.05 | 19.40 | 0.0628 |
| MAP2K1 | 16.01 | 15.81 | 0.0117 |
| ICAM1 | 16.80 | 17.05 | 0.1086 |
| NFKB1 | 16.85 | 16.68 | 0.2021 |
| CEBPB | 14.55 | 14.73 | 0.2435 |
| CCND2 | 16.82 | 16.47 | 0.2787 |
| RAF1 | 14.49 | 14.34 | 0.2979 |
| S100A6 | 14.22 | 14.01 | 0.3606 |
| THBS1 | 17.19 | 17.43 | 0.3724 |
| CDKN2D | 14.95 | 14.87 | 0.3830 |
| SMAD3 | 18.03 | 17.91 | 0.4187 |
| SRC | 18.16 | 18.27 | 0.4484 |
| TP53 | 16.30 | 16.23 | 0.5315 |
| CREBBP | 15.12 | 15.05 | 0.5858 |
| PLAU | 23.92 | 24.04 | 0.6141 |
| ALOX5 | 15.59 | 15.68 | 0.6414 |
| TNFRSF6 | 16.34 | 16.40 | 0.6472 |
| EP300 | 16.43 | 16.39 | 0.7457 |
| NFATC2 | 16.07 | 16.04 | 0.8309 |
| JUN | 20.86 | 20.90 | 0.8333 |
| EGR3 | 23.01 | 22.98 | 0.8957 |
| FGF2 | 24.57 | 24.59 | 0.9403 |
| MAPK1 | 14.71 | 14.71 | 0.9789 |

Table 5A

| 2-gene models and | 1-gene models | Entropy R-sq | #normal correct | #normal FALSE | #cc correct | #cc FALSE | Normal<br>N = 50<br>Correct Classification | colon<br>23<br>Correct Classification | p-val 1 | p-val 2 | total used (excludes missing)<br># normal | # disease |
|---|---|---|---|---|---|---|---|---|---|---|---|---|
| AXIN2 | TNF | 0.62 | 46 | 3 | 19 | 2 | 93.9% | 90.5% | 9.0E-10 | 2.4E-05 | 49 | 21 |
| AXIN2 | ITGAL | 0.62 | 40 | 7 | 19 | 2 | 85.1% | 90.5% | 8.2E-13 | 3.2E-05 | 47 | 21 |
| AXIN2 | MTA1 | 0.61 | 43 | 4 | 19 | 2 | 91.5% | 90.5% | 7.7E-13 | 4,2E-05 | 47 | 21 |
| AXIN2 | CCL5 | 0.60 | 43 | 4 | 19 | 2 | 91.5% | 90.5% | 1.7E-09 | 1.7E-09 | 47 | 21 |
| AXIN2 | HMOX1 | 0.59 | 42 | 5 | 18 | 3 | 89.4% | 85.7% | 5.4E-10 | 0.0001 | 47 | 21 |
| AXIN2 | HEXA10 | 0.58 | 44 | 5 | 18 | 3 | 89.8% | 85.7% | 4.5E-11 | 0.0002 | 49 | 21 |
| AXIN2 | DIABLO | 0.56 | 43 | 6 | 18 | 3 | 87.8% | 85.7% | 4.1E-12 | 0.0004 | 49 | 21 |
| AXIN2 | HMGA1 | 0.56 | 43 | 6 | 18 | 3 | 87.8% | 85.7% | 5.1E-12 | 0.0004 | 49 | 21 |
| TNF | TNFSF5 | 0.55 | 42 | 5 | 18 | 3 | 89.4% | 85.7% | 1.9E-08 | 2.3E-08 | 47 | 21 |
| AXIN2 | SRF | 0.55 | 39 | 8 | 18 | 3 | 83.0% | 85.7% | 1.3E-11 | 0.0006 | 47 | 21 |
| AXIN2 | IKBKE | 0.55 | 40 | 7 | 18 | 3 | 85.1% | 85.7% | 1.2E-10 | 0.0006 | 47 | 21 |
| AXIN2 | IRF1 | 0.54 | 39 | 8 | 17 | 4 | 83.0% | 81.0% | 1.8E-10 | 0.0008 | 47 | 21 |
| HMOX1 | MSH6 | 0.54 | 41 | 5 | 18 | 3 | 89.1% | 85.7% | 3.3E-06 | 4.1E-09 | 46 | 21 |
| AXIN2 | C1QA | 0.54 | 38 | 9 | 17 | 4 | 80.9% | 81.0% | 5.1E-07 | 0.0008 | 47 | 21 |
| CCR7 | TNF | 0.53 | 48 | 2 | 20 | 3 | 96.0% | 87.0% | 8.8E-08 | 0.0001 | 50 | 23 |
| M5H6 | TNF | 0.53 | 39 | 8 | 17 | 4 | 83.0% | 81.0% | 4.9E-08 | 7.0E-06 | 47 | 21 |
| AXIN2 | TGFB1 | 0.53 | 44 | 5 | 18 | 3 | 89.8% | 85.7% | 2.5E-10 | 0.0020 | 49 | 21 |
| AXIN2 | BAX | 0.53 | 46 | 3 | 18 | 3 | 93.9% | 85.7% | 2.0E-11 | 0.0021 | 49 | 21 |
| AXIN2 | NRAS | 0.52 | 41 | 8 | 18 | 3 | 83.7% | 85.7% | 1.0E-10 | 0.0026 | 49 | 21 |
| AXIN2 | EGR1 | 0.52 | 44 | 5 | 18 | 3 | 89.8% | 85.7% | 2.1E-07 | 0.0030 | 49 | 21 |

EP 2 402 464 A1

(continued)

| 2-gene models and | | Entropy | #normal | #normal | #cc | #cc | Normal | colon | | | total used (excludes missing) | |
|---|---|---|---|---|---|---|---|---|---|---|---|---|
| | | | | | | N = | 50 | 23 | | | | |
| 1-gene models | | R-sq | correct | FALSE | correct | FALSE | Correct Classification | Correct Classification | p-val 1 | p-val 2 | # normal | # disease |
| C1QA | MSH6 | 0.52 | 37 | 9 | 18 | 3 | 80.4% | 85.7% | 1.1E-05 | 1.6E-06 | 46 | 21 |
| AXIN2 | C1QB | 0.51 | 44 | 5 | 17 | 4 | 89.8% | 81.0% | 3.3E-06 | 0.0037 | 49 | 21 |
| CCL5 | TNFSF5 | 0.51 | 40 | 6 | 18 | 3 | 87.0% | 85.7% | 1.2E-07 | 6.6E-08 | 46 | 21 |
| CCLS | MSH6 | 0.51 | 37 | 10 | 18 | 3 | 78.7% | 85.7% | 1.9E-05 | 8.9E-08 | 47 | 21 |
| AXIN2 | ST14 | 0.51 | 41 | 8 | 17 | 4 | 83.7% | 81.0% | 8.3E-11 | 0.0057 | 49 | 21 |
| AXIN2 | USP7 | 0.50 | 40 | 7 | 18 | 3 | 85.1% | 85.7% | 7.7E-11 | 0.0049 | 47 | 21 |
| AXIN2 | LARGE | 0.50 | 41 | 8 | 18 | 3 | 83.7% | 85.7% | 1.5E-10 | 0.0065 | 49 | 21 |
| AXIN2 | IFI16 | 0.50 | 41 | 6 | 17 | 4 | 87.2% | 81.0% | 1.5E-09 | 0.0058 | 47 | 21 |
| AXIN2 | MYC | 0.50 | 41 | 8 | 18 | 3 | 83.7% | 85.7% | 2.3E-10 | 0.0068 | 49 | 21 |
| CCL5 | CCR7 | 0.50 | 38 | 9 | 18 | 3 | 80.9% | 85.7% | 0.0003 | 1.2E-07 | 47 | 21 |
| MSH6 | NRAS | 0.50 | 41 | 6 | 18 | 3 | 87.2% | 85.7% | 4.0E-10 | 3.1E-05 | 47 | 21 |
| AXIN2 | MTF1 | 0.49 | 38 | 9 | 18 | 3 | 80.9% | 85.7% | 3.2E-10 | 0.0092 | 47 | 21 |
| CCR7 | HMOX1 | 0.49 | 40 | 7 | 18 | 3 | 85.1% | 85.7% | 4.2E-08 | 0.0005 | 47 | 21 |
| AXIN2 | CTSD | 0.49 | 40 | 9 | 18 | 3 | 81.6% | 85.7% | 1.4E-10 | 0.0134 | 49 | 21 |
| AXIN2 | IL8 | 0.49 | 41 | 8 | 18 | 3 | 83.7% | 85.7% | 7.4E-08 | 0.0135 | 49 | 21 |
| IRF1 | MSH6 | 0.49 | 37 | 9 | 17 | 4 | 80.4% | 81.0% | 3.8E-05 | 2.2E-09 | 46 | 21 |
| CCR7 | HMGA1 | 0.49 | 42 | 8 | 20 | 3 | 84.0% | 87.0% | 1.1E-10 | 0.0014 | 50 | 21 |
| AXIN2 | G6PD | 0.48 | 41 | 8 | 18 | 3 | 83.7% | 85.7% | 3.9E-10 | 0.0154 | 49 | 21 |
| AXIN2 | DAD1 | 0.48 | 39 | 10 | 17 | 4 | 79.6% | 81.0% | 1.4E-10 | 0.0169 | 49 | 21 |
| AXIN2 | IGF2BP2 | 0.48 | 43 | 6 | 18 | 3 | 87.8% | 85.7% | 2.7E-10 | 0.0176 | 49 | 21 |
| AXIN2 | IGFBP3 | 0.48 | 41 | 8 | 18 | 3 | 83.7% | 85.7% | 3.2E-10 | 0.0193 | 49 | 21 |

| 2-gene models and | | Entropy | #normal | #normal | #cc | #cc | Normal<br>N =<br>50<br>Correct | colon<br>23<br>Correct | | | total used (excludes missing) | |
|---|---|---|---|---|---|---|---|---|---|---|---|---|
| 1-gene models | | R-sq | correct | FALSE | correct | FALSE | Classification | Classification | p-val 1 | p-val 2 | # normal | # disease |
| AXIN2 | CASP9 | 0.48 | 39 | 8 | 18 | 3 | 83.0% | 85.7% | 2.3E-10 | 0.0170 | 47 | 21 |
| AXIN2 | NBEA | 0.48 | 45 | 4 | 17 | 4 | 91.8% | 81.0% | 1.8E-05 | 0.0219 | 49 | 21 |
| MSH6 | TGFB1 | 0.48 | 40 | 7 | 18 | 3 | 85.1% | 85.7% | 2.8E-09 | 7.2E-05 | 47 | 21 |
| AXIN2 | FOS | 0.48 | 41 | 7 | 17 | 4 | 85.4% | 81.0% | 4.5E-09 | 0.0259 | 48 | 21 |
| AXIN2 | MYD88 | 0.48 | 41 | 8 | 18 | 3 | 83.7% | 85.7% | 4.3E-10 | 0.0240 | 49 | 21 |
| AXIN2 | CD97 | 0.48 | 36 | 10 | 18 | 3 | 78.3% | 85.7% | 3.2E-10 | 0.0189 | 46 | 21 |
| CCL5 | LTA | 0.47 | 39 | 8 | 17 | 4 | 83.0% | 81.0% | 8.3E-09 | 3.6E-07 | 47 | 21 |
| ITGAL | MSH6 | 0.47 | 38 | 9 | 17 | 4 | 80.9% | 81.0% | 7.9E-05 | 3.7E-10 | 47 | 21 |
| AXIN2 | TIMP1 | 0.47 | 41 | 8 | 18 | 3 | 83.7% | 85.7% | 3.2E-09 | 0.0254 | 49 | 21 |
| AXIN2 | XK | 0.47 | 39 | 10 | 18 | 3 | 79.6% | 85.7% | 2.9E-10 | 0.0261 | 49 | 21 |
| C1QB | MSH6 | 0.47 | 36 | 11 | 17 | 4 | 76.6% | 81.0% | 0.0001 | 1.9E-05 | 47 | 21 |
| IFI16 | MSH6 | 0.47 | 39 | 8 | 17 | 4 | 83.0% | 81.0% | 0.0001 | 6.0E-09 | 47 | 21 |
| AXIN2 | ZNF185 | 0.47 | 40 | 7 | 17 | 4 | 85.1% | 81.0% | 5.0E-10 | 0.0285 | 47 | 21 |
| AXIN2 | S100A4 | 0.47 | 41 | 8 | 18 | 3 | 83.7% | 85.7% | 2.7E-10 | 0.0363 | 49 | 21 |
| AXIN2 | PLXDC2 | 0.47 | 43 | 6 | 17 | 4 | 87.8% | 81.0% | 6.2E-10 | 0.0377 | 49 | 21 |
| CNKSR2 | TNF | 0.47 | 44 | 5 | 18 | 3 | 89.8% | 85.7% | 9.7E-07 | 5.0E-05 | 49 | 21 |
| AXIN2 | GNB1 | 0.47 | 42 | 7 | 17 | 4 | 85.7% | 81.0% | 3.0E-10 | 0.0385 | 49 | 21 |
| AXIN2 | UBE2C | 0.47 | 38 | 9 | 17 | 4 | 80.9% | 81.0% | 1.0E-08 | 0.0312 | 47 | 21 |
| AXIN2 | VIM | 0.47 | 40 | 7 | 17 | 4 | 85.1% | 81.0% | 4.0E-10 | 0.0323 | 47 | 21 |
| AXIN2 | LGALS8 | 0.46 | 40 | 7 | 17 | 4 | 85.1% | 81.0% | 4.8E-10 | 0.0334 | 47 | 21 |
| CCR7 | EGR1 | 0.46 | 39 | 11 | 20 | 3 | 78.0% | 87.0% | 6.3E-06 | 0.0040 | 50 | 21 |

| 2-gene models and | | Entropy | #normal | #normal | #cc | #cc | Normal | colon | | | total used (excludes missing) | |
| | | | | | | | N = | | | | | |
| | | | | | | | 50 | 23 | | | | |
| | | | | | | | Correct | Correct | | | | |
| 1-gene models | | R-sq | correct | FALSE | correct | FALSE | Classification | Classification | p-val 1 | p-val 2 | # normal | # disease |
|---|---|---|---|---|---|---|---|---|---|---|---|---|
| CCR7 | IL8 | 0.46 | 42 | 8 | 19 | 4 | 84.0% | 82.6% | 1.2E-07 | 0.0044 | 50 | 23 |
| C1QA | ZNF350 | 0.46 | 38 | 9 | 17 | 4 | 80.9% | 81.0% | 4.1E-05 | 2.0E-05 | 47 | 23 |
| C1QB | ZNF350 | 0.46 | 38 | 11 | 17 | 4 | 77.6% | 81.0% | 5.8E-05 | 4.1E-05 | 49 | 21 |
| AXIN2 | CCL3 | 0.46 | 40 | 7 | 18 | 3 | 85.1% | 85.7% | 1.2E-09 | 0.0493 | 47 | 21 |
| AXIN2 | NUDT4 | 0.46 | 38 | 9 | 17 | 4 | 80.9% | 81.0% | 3.0E-09 | 0.0496 | 47 | 21 |
| CCR7 | HOXA10 | 0.46 | 42 | 7 | 17 | 4 | 85.7% | 81.0% | 1.2E-08 | 0.0027 | 49 | 21 |
| C1QB | CCR7 | 0.45 | 40 | 9 | 17 | 4 | 81.6% | 81.0% | 0.0029 | 5.0E-05 | 49 | 21 |
| CCR7 | TGFB1 | 0.45 | 43 | 7 | 20 | 3 | 86.0% | 87.0% | 7.7E-09 | 0.0068 | 50 | 23 |
| CCR7 | MYC | 0.45 | 41 | 9 | 18 | 5 | 82.0% | 78.3% | 3.9E-10 | 0.0081 | 50 | 23 |
| DIABLO | MSH6 | 0.45 | 37 | 10 | 17 | 4 | 78.7% | 81.0% | 0.0002 | 8.8E-10 | 47 | 21 |
| MSH6 | SRF | 0.45 | 39 | 7 | 17 | 4 | 84.8% | 81.0% | 1.2E-09 | 0.0002 | 46 | 21 |
| CCR7 | IRF1 | 0.45 | 39 | 8 | 17 | 4 | 83.0% | 81.0% | 1.1E-08 | 0.0030 | 47 | 21 |
| HMOX1 | ZNF350 | 0.45 | 37 | 10 | 18 | 3 | 78.7% | 85.7% | 7.7E-05 | 2.7E-07 | 47 | 21 |
| MSH6 | MTF1 | 0.44 | 38 | 9 | 17 | 4 | 80.9% | 81.0% | 2.5E-09 | 0.0003 | 47 | 21 |
| BAX | MSH6 | 0.44 | 40 | 7 | 17 | 4 | 85.1% | 81.0% | 0.0004 | 1.2E-09 | 47 | 21 |
| CCR7 | TIMP1 | 0.44 | 42 | 8 | 19 | 4 | 84.0% | 82.6% | 1.0E-08 | 0.0135 | 50 | 23 |
| CCR7 | NRAS | 0.44 | 39 | 11 | 18 | 5 | 78.0% | 78.3% | 3.2E-09 | 0.0155 | 50 | 23 |
| CCR7 | ITGAL | 0.44 | 37 | 10 | 17 | 4 | 78.7% | 81.0% | 1.9E-09 | 0.0051 | 47 | 21 |
| GSK3B | S100A11 | 0.43 | 39 | 8 | 17 | 4 | 83.0% | 81.0% | 9.1E-09 | 1.7E-07 | 47 | 21 |
| GSK3B | TNF | 0.43 | 41 | 8 | 18 | 3 | 83.7% | 85.7% | 4.6E-06 | 1.6E-07 | 49 | 21 |
| CNKSR2 | HMOX1 | 0.43 | 39 | 8 | 18 | 3 | 83.0% | 85.7% | 5.4E-07 | 0.0002 | 47 | 21 |

EP 2 402 464 A1

(continued)

|  |  | Entropy | #normal | #normal | #cc | #cc | Normal | colon |  |  | total used (excludes missing) | |
|---|---|---|---|---|---|---|---|---|---|---|---|---|
|  |  |  |  |  |  | N = | 50 | 23 |  |  | | |
| 2-gene models and | | Entropy | #normal | #normal | #cc | #cc | Correct | Correct |  |  | total used (excludes missing) | |
| 1-gene models | | R-sq | correct | FALSE | correct | FALSE | Classification | Classification | p-val 1 | p-val 2 | # normal | # disease |
| HMOX1 | TNFSF5 | 0.43 | 37 | 10 | 18 | 3 | 78.7% | 85.7% | 4.1E-06 | 5.5E-07 | 47 | 21 |
| CCL5 | CNKSR2 | 0.43 | 41 | 6 | 18 | 3 | 87.2% | 85.7% | 0.0003 | 2.7E-06 | 47 | 21 |
| CCR7 | ZNF350 | 0.43 | 43 | 6 | 17 | 4 | 87.8% | 81.0% | 0.0002 | 0.0095 | 49 | 21 |
| APC | C1QB | 0.43 | 37 | 12 | 17 | 4 | 75.5% | 81.0% | 0.0002 | 3.8E-06 | 49 | 21 |
| NRAS | ZNF350 | 0.43 | 40 | 9 | 18 | 3 | 81.6% | 85.7% | 0.0003 | 7.1E-09 | 49 | 21 |
| MSH6 | MTA1 | 0.43 | 36 | 11 | 17 | 4 | 76.6% | 81.0% | 2.2E-09 | 0.0007 | 47 | 21 |
| CCR7 | SPARC | 0.43 | 38 | 9 | 17 | 4 | 80.9% | 81.0% | 6.8E-06 | 0.0085 | 47 | 21 |
| APC | HMOX1 | 0.42 | 40 | 7 | 17 | 4 | 85.1% | 81.0% | 7.0E-07 | 3.1E-06 | 47 | 21 |
| C1QA | MLH1 | 0.42 | 39 | 7 | 17 | 4 | 84.8% | 81.0% | 2.0E-07 | 2.0E-07 | 46 | 21 |
| HOXA10 | MSH6 | 0.42 | 40 | 7 | 18 | 3 | 85.1% | 85.7% | 0.0009 | 5.2E-08 | 47 | 21 |
| C1QA | TNFSF5 | 0.42 | 40 | 7 | 18 | 3 | 85.1% | 85.7% | 6.0E-06 | 0.0001 | 47 | 21 |
| CCR7 | SRF | 0.42 | 40 | 7 | 17 | 4 | 85.1% | 81.0% | 3.7E-09 | 0.0110 | 47 | 21 |
| APC | C1QA | 0.42 | 38 | 9 | 17 | 4 | 80.9% | 81.0% | 0.0001 | 3.8E-06 | 47 |  |
| CCR7 | MYD88 | 0.42 | 39 | 11 | 18 | 5 | 78.0% | 78.3% | 2.1E-09 | 0.0397 | 50 | 21 |
| CCR7 | G6PD | 0.42 | 39 | 11 | 18 | 5 | 78.0% | 78.3% | 5.4E-09 | 0.0397 | 50 | 23 |
| MSH6 | S100A4 | 0.42 | 41 | 6 | 18 | 3 | 87.2% | 85.7% | 3.1E-09 | 0.0010 | 47 | 23 21 |
| TNF | ZNF350 | 0.42 | 38 | 11 | 16 | 5 | 77.6% | 76.2% | 0.0004 | 8.4E-06 | 49 | 21 |
| CCR7 | SERPINE1 | 0.42 | 43 | 7 | 20 | 3 | 86.0% | 87.0% | 7.1E-09 | 0.0419 | 50 | 23 |
| IFI16 | ZNF350 | 0.42 | 40 | 7 | 18 | 3 | 85.1% | 85.7% | 0.0003 | 5.8E-08 | 47 | 23 |
| AXIN2 |  | 0.42 | 41 | 8 | 17 | 4 | 83.7% | 81.0% | 2.4E-09 |  | 49 | 21 |
| CASP9 | MSH6 | 0.42 | 39 | 8 | 17 | 4 | 83.0% | 81.0% | 0.0011 | 3.5E-09 | 47 | 21 |

EP 2 402 464 A1

(continued)

| 2-gene models and | 1-gene models | Entropy R-sq | #normal correct | #normal FALSE | #cc correct | #cc FALSE | Normal Correct Classification | colon Correct Classification | p-val 1 | p-val 2 | total used (excludes missing) # normal | # disease |
|---|---|---|---|---|---|---|---|---|---|---|---|---|
| | | | | | | | N = 50 | 23 | | | | |
| MSH6 | TIMP1 | 0.41 | 37 | 10 | 16 | 5 | 78.7% | 76.2% | 5.7E-08 | 0.0013 | 47 | 21 |
| APC | TNFRSF1A | 0.41 | 40 | 9 | 17 | 4 | 81.6% | 81.0% | 6.1E-09 | 7.0E-06 | 49 | 21 |
| GSK3B | PLXDC2 | 0.41 | 40 | 9 | 17 | 4 | 81.6% | 81.0% | 6.9E-09 | 3.8E-07 | 49 | 21 |
| C1QB | GSK3B | 0.41 | 38 | 11 | 17 | 4 | 77.6% | 81.0% | 3.8E-07 | 0.0003 | 49 | 21 |
| MLH1 | TNF | 0.41 | 37 | 10 | 17 | 4 | 78.7% | 81.0% | 8.7E-06 | 3.9E-07 | 47 | 21 |
| CCR7 | IFI16 | 0.41 | 38 | 9 | 17 | 4 | 80.9% | 81.0% | 8.0E-08 | 0.0181 | 47 | 21 |
| CCR7 | DIABLO | 0.41 | 37 | 12 | 16 | 5 | 75.5% | 76.2% | 3.9E-09 | 0.0265 | 49 | 21 |
| CCR7 | USP7 | 0.41 | 38 | 9 | 16 | 5 | 80.9% | 76.2% | 5.5E-09 | 0.0219 | 47 | 21 |
| IRF1 | ZNF350 | 0.41 | 39 | 8 | 18 | 3 | 83.0% | 85.7% | 0.0005 | 7.1E-08 | 47 | 21 |
| HMOX1 | MLH1 | 0.40 | 39 | 7 | 18 | 3 | 84.8% | 85.7% | 4.4E-07 | 1.6E-06 | 46 | 21 |
| MSH6 | MYD88 | 0.40 | 37 | 10 | 16 | 5 | 78.7% | 76.2% | 1.3E-08 | 0.0019 | 47 | 21 |
| APC | IRF1 | 0.40 | 39 | 8 | 17 | 4 | 83.0% | 81.0% | 7.6E-08 | 7.6E-06 | 47 | 21 |
| CCR7 | E2F1 | 0.40 | 41 | 6 | 17 | 4 | 87.2% | 81.0% | 3.5E-06 | 0.0248 | 47 | 21 |
| TNFRSF1A | TNF350 | 0.40 | 40 | 9 | 17 | 4 | 81.6% | 81.0% | 0.0008 | 9.6E-09 | 49 | 21 |
| G6PD | MSH6 | 0,40 | 38 | 9 | 17 | 4 | 80.9% | 81.0% | 0.0021 | 2.1E-08 | 47 | 21 |
| C1QA | TXNRD1 | 0.40 | 37 | 10 | 18 | 3 | 78.7% | 85.7% | 2.1E-07 | 0.0003 | 47 | 21 |
| MAPK14 | MSH6 | 0.40 | 36 | 11 | 16 | 5 | 76.6% | 76.2% | 0.0024 | 8.3E-09 | 47 | 21 |
| C1QA | GSK38 | 0.40 | 39 | 8 | 17 | 4 | 83.0% | 81.0% | 5.8E-07 | 0.0003 | 47 | 21 |
| TNF | XRCC1 | 0.40 | 43 | 6 | 17 | 4 | 87.8% | 81.0% | 2.2E-08 | 2.0E-05 | 49 | 21 |
| MSH6 | USP7 | 0.40 | 37 | 9 | 17 | 4 | 80.4% | 81.0% | 9.1E-09 | 0.0021 | 46 | 21 |
| NBEA | TNF | 0.40 | 40 | 9 | 17 | 4 | 81.6% | 81.0% | 2.2E-05 | 0.0007 | 49 | 21 |

| 2-gene models and | | Entropy | #normal | #normal | #cc | #cc | Normal<br>50<br>Correct | colon<br>23<br>Correct | | | total used (excludes missing) | |
|---|---|---|---|---|---|---|---|---|---|---|---|---|
| | N = | | | | | | | | | | | |
| 1-gene models | | R-sq | correct | FALSE | correct | FALSE | Classification | Classification | p-val 1 | p-val 2 | # normal | # disease |
| MSH2 | TNF | 0.40 | 42 | 8 | 20 | 3 | 84.0% | 87.0% | 6.1E-05 | 0.0012 | 50 | 21 |
| CCR7 | ING2 | 0.40 | 37 | 12 | 17 | 4 | 75.5% | 81.0% | 3.5E-06 | 0.0487 | 49 | 23 |
| C1QB | TXNRD1 | 0.39 | 38 | 9 | 17 | 4 | 80.9% | 81.0% | 2.7E-07 | 0.0007 | 47 | 21 |
| HMOX1 | MSH2 | 0.39 | 41 | 6 | 18 | 3 | 87.2% | 85.7% | 0.0002 | 2.6E-06 | 47 | 21 |
| MSH6 | UBE2C | 0.39 | 37 | 9 | 16 | 5 | 80.4% | 76.2% | 2.2E-07 | 0.0024 | 46 | 21 |
| APC | TNF | 0.39 | 39 | 10 | 17 | 4 | 79.6% | 81.0% | 2.5E-05 | 1.6E-05 | 49 | 21 |
| CCR7 | MTF1 | 0.39 | 37 | 10 | 16 | 5 | 78.7% | 76.2% | 2.3E-08 | 0.0404 | 47 | 21 |
| DAD1 | MSH6 | 0.39 | 39 | 8 | t 17 | 4 | 83.0% | 81.0% | 0.0033 | 1.1E-08 | 47 | 21 |
| GSK3B | HMOX1 | 0.39 | 38 | 9 | 17 | 4 | 80.9% | 81.0% | 2.9E-06 | 8.1E-07 | 47 | 21 |
| MYD88 | ZNF350 | 0.39 | 39 | 10 | 16 | 5 | 79.6% | 76.2% | 0.0013 | 1.9E-08 | 49 | 21 |
| LTA | TNF | 0.39 | 37 | 10 | 16 | 5 | 78.7% | 76.2% | 2.2E-05 | 3.4E-07 | 47 | 21 |
| C1QA | MSH2 | 0.39 | 37 | 10 | 17 | 4 | 78.7% | 81.0% | 0.0003 | 0.0005 | 47 | 21 |
| MSH6 | PLXDC2 | 0.39 | 36 | 11 | 17 | 4 | 76.6% | 81.0% | 2.5E-08 | 0.0038 | 47 | 21 |
| CTSD | MSH6 | 0.39 | 37 | 10 | 17 | 4 | 78.7% | 81.0% | 0.0039 | 1.4E-08 | 47 | 21 |
| APC | S100A11 | 0.39 | 37 | 10 | 16 | 5 | 78.7% | 76.2% | 6.9E-08 | 2.0E-05 | 47 | 21 |
| CD59 | ZNF350 | 0.39 | 42 | 7 | 18 | 3 | 85.7% | 85.7% | 0.0015 | 3.1E-08 | 49 | 21 |
| C1QB | TNFSF5 | 0.39 | 40 | 7 | 17 | 4 | 85.1% | 81.0% | 2.8E-05 | 0.0010 | 47 | 21 |
| C1QA | CNKSR2 | 0.38 | 39 | 8 | 18 | 3 | 83.0% | 85.7% | 0.0016 | 0.0006 | 47 | 21 |
| C1QB | NBEA | 0.38 | 39 | 10 | 17 | 4 | 79.6% | 81.0% | 0.0013 | 0.0012 | 49 | 21 |
| C1QB | MLH1 | 0.38 | 37 | 10 | 17 | 4 | 78.7% | 81.0% | 1.2E-06 | 0.0008 | 47 | 21 |
| MSH6 | RBM5 | 0.38 | 38 | 9 | 17 | 4 | 80.9% | 81.0% | 4.8E-08 | 0.0050 | 47 | 21 |

EP 2 402 464 A1

| 2-gene models and | | Entropy | #normal | #normal | #cc | #cc | Normal | colon | | | total used (excludes missing) | |
|---|---|---|---|---|---|---|---|---|---|---|---|---|
| | | | | | | N = | 50 | 23 | | | | |
| 1-gene models | | R-sq | correct | FALSE | correct | FALSE | Correct Classification | Correct Classification | p-val 1 | p-val 2 | # normal | # disease |
| MAPK14 | ZNF350 | 0.38 | 36 | 11 | 17 | 4 | 76.6% | 81.0% | 0.0015 | 1.7E-08 | 47 | 21 |
| TLR2 | ZNF350 | 0.38 | 41 | 6 | 17 | 4 | 87.2% | 81.0% | 0.0014 | 2.1E-08 | 47 | 21 |
| MSH6 | TLR2 | 0.38 | 37 | 9 | 16 | 5 | 80.4% | 76.2% | 2.5E-08 | 0.0045 | 46 | 21 |
| FOS | MSH6 | 0.38 | 35 | 11 | 16 | 5 | 76.1% | 76.2% | 0.0049 | 4.5E-07 | 46 | 21 |
| MSH6 | TNFRSF1A | 0.38 | 37 | 10 | 16 | 5 | 78.7% | 76.2% | 3.5E-08 | 0.0058 | 47 | 21 |
| MSH2 | TGFB1 | 0.38 | 43 | 7 | 19 | 4 | 86.0% | 82.6% | 2.4E-07 | 0.0027 | 50 | 23 |
| APC | IFI16 | 0.38 | 37 | 10 | 16 | 5 | 78.7% | 76.2% | 3.0E-07 | 2.8E-05 | 47 | 21 |
| MSH6 | S100A11 | 0.38 | 38 | 9 | 17 | 4 | 80.9% | 81.0% | 9.9E-08 | 0.0061 | 47 | 21 |
| C1QB | CNKSR2 | 0.38 | 37 | 12 | 18 | 3 | 75.5% | 85.7% | 0.0028 | 0.0016 | 49 | 21 |
| CCL5 | XRCC1 | 0.38 | 38 | 9 | 17 | 4 | 80.9% | 81.0% | 7.1E-08 | 2.6E-05 | 47 | 21 |
| APC | MAPK14 | 0.38 | 39 | 8 | 16 | 5 | 83.0% | 76.2% | 2.2E-08 | 3.1E-05 | 47 | 21 |
| APC | PLXDC2 | 0.38 | 38 | 11 | 16 | 5 | 77.6% | 76.2% | 3.2E-08 | 3.4E-05 | 49 | 21 |
| CA4 | MSH6 | 0.38 | 36 | 10 | 16 | 5 | 78.3% | 76.2% | 0.0054 | 3.4E-07 | 49 | 21 |
| CNKSR2 | ZNF350 | 0.38 | 38 | 11 | 17 | 4 | 77.6% | 81.0% | 0.0025 | 0.0032 | 46 | 21 |
| CNKSR2 | HMGA1 | 0.38 | 42 | 7 | 18 | 3 | 85.7% | 85.7% | 1.9E-08 | 0.0032 | 49 | 21 |
| C1QB | ING2 | 0.37 | 37 | 12 | 16 | 5 | 75.5% | 76.2% | 9.3E-06 | 0.0019 | 49 | 21 |
| HMOX1 | IKBKE | 0.37 | 39 | 8 | 17 | 4 | 83.0% | 81.0% | 2.6E-07 | 6.5E-06 | 49 | 21 |
| CA4 | ZNF350 | 0.37 | 36 | 11 | 17 | 4 | 76.6% | 81.0% | 0.0021 | 3.2E-07 | 47 | 21 |
| HMOX1 | TXNRD1 | 0.37 | 37 | 10 | 17 | 4 | 78.7% | 81.0% | 7.2E-07 | 6.6E-06 | 47 | 21 |
| CCR7 | | 0.37 | 39 | 11 | 18 | 5 | 78.0% | 78.3% | 5.9E-09 | I | 50 | 23 |
| CCL5 | MLH1 | 0.37 | 39 | 8 | 17 | 4 | 83.0% | 81.0% | 2.1F-06 | 3.3E-05 | 47 | 23 |

| 2-gene models and | | Entropy | #normal | #normal | #cc | #cc | Normal | colon | | | total used (excludes missing) | |
| | | | | | | | N = 50 | N = 23 | | | | |
| 1-gene models | | R-sq | correct | FALSE | correct | FALSE | Correct Classification | Correct Classification | p-val 1 | p-val 2 | # normal | # disease |
|---|---|---|---|---|---|---|---|---|---|---|---|---|
| G6PD | GSK3B | 0.37 | 39 | 10 | 16 | 5 | 79.6% | 76.2% | 2.4E-06 | 5.9E-08 | 47 | 21 |
| MSH6 | NBEA | 0.37 | 36 | 11 | 17 | 4 | 76.6% | 81.0% | 0.0020 | 0.0091 | 49 | 21 |
| C1QB | MSH2 | 0.37 | 38 | 11 | 17 | 4 | 77.6% | 81.0% | 0.0009 | 0.0023 | 47 | 21 |
| MSH6 | SPARC | 0.37 | 35 | 11 | 16 | 5 | 76.1% | 76.2% | 6.8E-05 | 0.0075 | 49 | 21 |
| TGFB1 | ZNF350 | 0.37 | 40 | 9 | 17 | 4 | 81.6% | 81.0% | 0.0034 | 27E-07 | 46 | 21 |
| C1QA | NBEA | 0.37 | 37 | 10 | 16 | 5 | 78.7% | 76.2% | 0.0024 | 0.0012 | 47 | 21 |
| CNKSR2 | IL8 | 0.37 | 40 | 9 | 17 | 4 | 81.6% | 81.0% | 1.7E-05 | 0.0053 | 49 | 21 |
| CNKSR2 | NRAS | 0.36 | 40 | 9 | 18 | 3 | 81.6% | 85.7% | 1.1E-07 | 0.0055 | 49 | 21 |
| APC | TGFB1 | 0.36 | 42 | 7 | 17 | 4 | 85.7% | 81.0% | 3.4E-07 | 6.1E-05 | 49 | 21 |
| MSH6 | ST14 | 0.36 | 38 | 9 | 17 | 4 | 80.9% | 81.0% | 5.5E-08 | 0.0131 | 47 | 21 |
| GSK3B | TIMP1 | 0.36 | 39 | 10 | 17 | 4 | 79.6% | 81.0% | 4.6E-07 | 3.5E-06 | 49 | 21 |
| EGR1 | TNFSF5 | 0.36 | 38 | 9 | 16 | 5 | 80.9% | 76.2% | 8.0E-05 | 0.0002 | 49 | 21 |
| CD97 | MSH6 | 0.36 | 37 | 9 | 16 | 5 | 80.4% | 76.2% | 0.0108 | 4.0E-08 | 47 | 21 |
| MTF1 | ZNF350 | 0.36 | 36 | 11 | 16 | 5 | 76.6% | 76.2% | 0.0040 | 8.8E-08 | 46 | 21 |
| FOS | ZNF350 | 0.36 | 39 | 9 | 17 | 4 | 81.3% | 81.0% | 0.0040 | 6.7E-07 | 48 | 21 |
| ADAM17 | C1QA | 0.36 | 38 | 8 | 17 | 4 | 82.6% | 81.0% | 0.0014 | 1.6E-06 | 46 | 21 |
| TNF | TXNRD1 | 0.36 | 36 | 11 | 17 | 4 | 76.6% | 81.0% | 1.2E-06 | 1.0E-04 | 47 | 21 |
| MSH6 | VIM | 0.36 | 36 | 10 | 16 | 5 | 78.3% | 76.2% | 4.2E-08 | 0.0112 | 46 | 21 |
| CNK5R2 | SPARC | 0.36 | 41 | 6 | 17 | 4 | 87.2% | 81.0% | 0.0001 | 0.0048 | 47 | 21 |
| E2F1 | MSH6 | 0.36 | 36 | 10 | 16 | 5 | 78.3% | 76.2% | 0.0116 | 19E-05 | 47 | 21 |
| APC | MYD88 | 0.36 | 40 | 9 | 17 | 4 | 81.6% | 81.0% | 6.9E-08 | 7.2E-05 | 449 | 21 |

(continued)

| 2-gene models and | | Entropy | #normal | #normal | #cc | #cc | Normal | colon | | | total used (excludes missing) | |
|---|---|---|---|---|---|---|---|---|---|---|---|---|
| | | | | | | N = | 50 | 23 | | | | |
| | | | | | | | Correct | Correct | | | | |
| 1-gene models | | R-sq | correct | FALSE | correct | FALSE | Classification | Classification | p-val 1 | p-val 2 | # normal | # disease |
| HMOX1 | XRCC1 | 0.36 | 37 | 10 | 16 | 5 | 78.79G | 76.296 | 1.4E-07 | 1.2E-05 | 46 | 21 |
| PLXDC2 | ZNF350 | 0.36 | 39 | 1D | 16 | 5 | 79.6% | 76.2% | 0.0054 | 6.9E-08 | 47 | 21 |
| NBEA | SPARC | 0.36 | 39 | 8 | 18 | 3 | 83.0% | 85.7% | 0.0001 | 0.0038 | 49 | 21 |
| CNKSR2 | EGR1 | 0.36 | 43 | 6 | 18 | 3 | 87.8% | 85.7% | 0.0003 | 0.0075 | 47 | 21 |
| HMGA1 | MSH6 | 0.36 | 37 | 10 | 17 | 4 | 78.7% | 81.0% | 0.0180 | 5.5E-08 | 49 | 21 |
| CNKSR2 | NBEA | 0.35 | 38 | 11 | 16 | 5 | 77.6% | 76.2% | 0.0054 | 0.0091 | 47 | 21 |
| EGR1 | ZNF350 | 0.35 | 39 | 10 | 17 | 4 | 79.6% | 81.0% | 0.0074 | 0.0004 | 49 | 21 |
| APC | G6PD | 0.35 | 39 | 10 | 16 | 5 | 79.6% | 76.2% | 1.3E-07 | 0.0001004 | 49 | 21 |
| CNKSR2 | IRF1 | 0.35 | 39 | 8 | 17 | 4 | 83.0% | 81.0% | 6.9E-07 | 0.0069 | 49 | 21 |
| MSH6 | XK | 0.35 | 37 | 10 | 17 | 4 | 78.7% | 81.0% | 8.5E-08 | 0.0228 | 47 | 21 |
| C1Q8 | LTA | 0.35 | 39 | 8 | 17 | 4 | 83.0% | 81.0% | 1.9E-06 | 0.0040 | 47 | 21 |
| MSH6 | SERPINE1 | 0.35 | 36 | 11 | 16 | 5 | 76.6% | 76.2% | 3.9E-07 | 0.0239 | 47 | 21 |
| MSH2 | NRAS | 0.35 | 39 | 11 | 19 | 4 | 78.0% | 82.6% | 1.9E-07 | 0.0122 | 50 | 23 |
| APC | CA4 | 0.35 | 37 | 10 | 16 | 5 | 78.7% | 76.2% | 8.8E-07 | 8.3E-05 | 47 | 21 |
| BAX | MSH2 | 0.35 | 38 | 12 | 19 | 4 | 76.0% | 82.6% | 0.0125 | 2.3E-08 | 50 | 23 |
| HOXA10 | ZNF350 | 0.35 | 39 | 10 | 16 | 5 | 79.6% | 76.2% | 0.0090 | 1.3E-06 | 49 | 21 |
| EGR1 | NBEA | 0.35 | 41 | 8 | 16 | 5 | 83.7% | 76.2% | 0.0067 | 0.0004 | 49 | 21 |
| BEAM | MASH6 | 0.35 | 38 | 8 | 17 | 4 | 82.6% | 81.0% | 0.0205 | 9.9E-08 | 46 | 21 |
| CAV1 | MSH6 | 0.35 | 37 | 10 | 17 | 4 | 78.7% | 81.0% | 0.0266 | 2.4E-06 | 47 | 21 |
| SIAH2 | XK | 0.35 | 37 | 10 | 17 | 4 | 78.7% | 81.0% | 1.1E-07 | 2.7E-05 | 47 | 21 |
| APC | TLR2 | 0.35 | 40 | 7 | 18 | 3 | 85.1% | 85.7% | 9.7E-08 | 9.8E-05 | 47 | 21 |

| | | | | | | | Normal | colon | | | | |
|---|---|---|---|---|---|---|---|---|---|---|---|---|
| | | | | | | N = | 50 | 23 | | | | |
| 2-gene models and | | Entropy | #normal | #normal | #cc | #cc | Correct | Correct | | | total used (excludes missing) | |
| 1-gene models | | R-sq | correct | FALSE | correct | FALSE | Classification | Classification | p-val 1 | p-val 2 | # normal | # disease |
| CCL5 | TNF350 | 0.35 | 37 | 10 | 16 | 5 | 78.7% | 76.2% | 0.0086 | 0.0001 | 47 | 21 |
| APC | FOS | 0.35 | 38 | 10 | 17 | 4 | 79.2% | 81.0% | 1.4E-06 | 0.0001 | 48 | 21 |
| MSH6 | PLAU | 0.34 | 36 | 11 | 16 | 5 | 76.6% | 76.2% | 7.7E-08 | 0.0313 | 47 | 21 |
| MSH6 | RP51077B9.4 | 0.34 | 36 | 11 | 16 | 5 | 76.6% | 76.2% | 1.8E-06 | 0.0318 | 47 | 21 |
| NBEA | ZNF350 | 0.34 | 39 | 10 | 16 | 5 | 79.6% | 76.2% | 0.0115 | 0.0085 | 49 | 21 |
| ADAM17 | HMOX1 | 0.34 | 36 | 10 | 16 | 5 | 78.3% | 76.2% | 2.3E-05 | 3.6E-06 | 46 | 21 |
| CNKSR2 | E2F1 | 0.34 | 37 | 10 | 17 | 4 | 78.7% | 81.0% | 4.8E-05 | 0.0107 | 47 | 21 |
| GSK3B | TGFB1 | 0.34 | 40 | 9 | 16 | 5 | 81.6% | 76.2% | 8.8E-07 | 8.5E-06 | 49 | 21 |
| CNKSR2 | HEXA10 | 0.34 | 43 | 6 | 17 | 4 | 87.8% | 81.0% | 1.7E-06 | 0.0160 | 49 | 21 |
| MSH2 | S100A4 | 0.34 | 41 | 9 | 19 | 4 | 82.0% | 82.6% | 3.4E-08 | 0.0191 | 50 | 23 |
| ETS2 | MSH6 | 0.34 | 36 | 11 | 16 | 5 | 76.6% | 76.2% | 0.0380 | 1.1M7 | 47 | 21 |
| MNDA | MASH6 | 0.34 | 38 | 9 | 17 | 4 | 80.9% | 81.0% | 0.0389 | 1.0E-07 | 47 | 21 |
| MSH6 | SERPINA1 | 0.34 | 37 | 10 | 16 | 5 | 78.7% | 76.2% | 9.1E-08 | 0.0389 | 47 | 21 |
| C1Q8 | CEACAM1 | 0.34 | 39 | 10 | 17 | 4 | 79.6% | 81.0% | 1.4E-07 | 0.0094 | 49 | 21 |
| CNKSR22 | TNF91 | 0.34 | 41 | 8 | 18 | 3 | 83.7% | 85.7% | 9.9E-07 | 0.0175 | 49 | 21 |
| CNKSR2 | MSH6 | 0.34 | 38 | 9 | 17 | 4 | 80.9% | 81.0% | 0,0405 | 0.0153 | 47 | 21 |
| APC | MIF1 | 0.34 | 36 | 11 | 16 | 5 | 76.6% | 76.2% | 2.4E-07 | 0.0002 | 47 | 21 |
| C1QA | IKBKE | 0.34 | 36 | 11 | 16 | 5 | 76.6% | 76.2% | 1.1E-06 | 0.0045 | 47 | 21 |
| G6PD | ZNF350 | 0.34 | 39 | 10 | 16 | 5 | 79.6% | 76.2% | 0.0147 | 2.4E-07 | 49 | 21 |
| HOXA10 | TNFSF5 | 0.34 | 38 | 9 | 17 | 4 | 80.996 | 81.0% | 0.0002 | 2.2E-06 | 47 | 21 |
| PTPRk | TNF | 0.34 | 39 | 11 | 19 | 4 | 78.0% | 82.6% | 0.0010 | 2.0E-C6 | 50 | 23 |

EP 2 402 464 A1

(continued)

| 2-gene models and | 1-gene models | Entropy R-sq | #normal correct | #normal FALSE | #cc correct | #cc FALSE (N =) | Normal 50 Correct Classification | colon 23 Correct Classification | p-val 1 | p-val 2 | total used (excludes missing) # normal | # disease |
|---|---|---|---|---|---|---|---|---|---|---|---|---|
| IQGAP1 | TNF | 0.34 | 39 | 11 | 18 | 5 | 78.0% | 78.3% | 0.0010 | 8.3E-08 | 50 | 23 |
| MSH2 | NBEA | 0.34 | 38 | 11 | 16 | 5 | 77.6% | 76.2% | 0.0113 | 0.0038 | 49 | 21 |
| IRF1 | MSH2 | 0.34 | 36 | 11 | 16 | 5 | 76.6% | 76.2% | 0.0032 | 1.3E-06 | 47 | 21 |
| CCL5 | IKBKE | 0.34 | 36 | 10 | 17 | 4 | 78.3% | 81.0% | 1.9E-06 | 0.0001 | 46 | 21 |
| CNKSR22 | IFI16 | 0.34 | 39 | 8 | 17 | 4 | 83.0% | 81.0% | 1.9E-06 | 0.0168 | 47 | 21 |
| CCL3 | MSH6 | 0.34 | 35 | 11 | 16 | 5 | 76.1% | 76.2% | 0.0349 | 2.1E-07 | 46 | 21 |
| IL8 | MSH6 | 0.34 | 39 | 8 | 17 | 4 | 83.0% | 81.0% | 0.0457 | 4.9E-05 | 47 | 21 |
| GSK3B | MAP14 | 0.34 | 37 | 10 | 17 | 4 | 78.7% | 81.8% | 1.3E-07 | 1.2E-05 | 47 | 21 |
| MMP9 | MSH6 | 0.34 | 37 | 10 | 17 | 4 | 78.7% | 81.0% | 0.0469 | 2.7E-07 | 47 | 21 |
| CNKSR22 | MSH2 | 0.34 | 38 | 11 | 17 | 4 | 77.6% | 81.0% | 0.0041 | 0.0211 | 49 | 21 |
| CA4 | MME | 0.34 | 38 | 9 | 16 | 5 | 80.9% | 76.2% | 1.2E-06 | 1.6E-06 | 47 | 21 |
| EGR1 | MSH2 | 0.34 | 39 | 11 | 19 | 4 | 78.0% | 82.6% | 0.0251 | 0.0031 | 50 | 23 |
| IKBKE | TNF | 0.33 | 41 | 6 | 16 | 5 | 87.2% | 76.2% | 0.0003 | 1.4E-06 | 47 | 21 |
| NBEA | SIAH2 | 0.33 | 37 | 10 | 16 | 5 | 78.7% | 76.2% | 4.7E-05 | 0.0116 | 47 | 21 |
| CNKSR2 | MYC | 0.33 | 44 | 5 | 17 | 4 | 89.8% | 81.0% | 4.0E-07 | 0.0251 | 49 | 21 |
| SRF | ZNF350 | 0.33 | 37 | 10 | 16 | 5 | 78.7% | 76.2% | 0.0138 | 1.7E-07 | 47 | 21 |
| SPARC | TNF5F5 | 0.33 | 38 | 9 | 17 | 4 | 80.9% | 81.0% | 0.0003 | 0.0005 | 47 | 21 |
| 6SK3B | TNFRSF1A | 0.33 | 39 | 10 | 17 | 4 | 79.6% | 81.0% | 2.3E-07 | 1.5E-05 | 49 | 21 |
| CCLS | NBEA | 0.33 | 36 | 11 | 17 | 4 | 76.6% | 81.0% | 0.0134 | 0.0002 | 47 | 21 |
| CAV1 | ZNF350 | 0.33 | 39 | 10 | 17 | 4 | 79.6% | 81.0% | 0.0228 | 4.6E-06 | 49 | 21 |
| CNKSR22 | MTA1 | 0.33 | 39 | 8 | 17 | 4 | 83.0% | 81,0% | 1.4E-07 | 0.0244 | 47 | 21 |

| 2-gene models and | | Entropy | #normal | #normal | #cc | #cc | Normal | colon | | | total used (excludes missing) | |
| | | | | | | | 50 | 23 | | | | |
| | | | | | | N = | Correct | Correct | | | | |
| 1-gene models | | R-sq | correct | FALSE | correct | FALSE | Classification | Classification | p-val 1 | p-val 2 | # normal | # disease |
|---|---|---|---|---|---|---|---|---|---|---|---|---|
| LGALS8 | ZNF350 | 0.33 | 37 | 10 | 16 | 5 | 78.7% | 76.2% | 0.0181 | 1.7E-07 | 47 | 21 |
| APC | ERAS | 0.33 | 42 | 7 | 16 | 5 | 85.7% | 76.2% | 5.1E-07 | 0.0003 | 49 | 21 |
| ADAM17 | TNF | 0.33 | 36 | 11 | 16 | 5 | 76.6% | 76.2% | 0.0003 | 7.3E-06 | 47 | 21 |
| GNB1 | TNF | 0.33 | 43 | 6 | 17 | 4 | 87.8% | 81.0% | 0.0005 | 1.3E-07 | 49 | 21 |
| MNC1A | ZNF350 | 0.33 | 36 | 11 | 16 | 5 | 76.6% | 76.2% | 0.0193 | 1.7E-07 | 47 | 21 |
| ETS2 | ZNF350 | 0.33 | 37 | 12 | 16 | 5 | 75.5% | 76.2% | 0.0255 | 1.5E-07 | 49 | 21 |
| CTSD | ZNF350 | 0.33 | 38 | 11 | 16 | 5 | 77.6% | 76.2% | 0.0255 | 1.6E-117 | 49 | 21 |
| APC | CNKSR22 | 0.33 | 38 | 11 | 17 | 4 | 77.6% | 81.0% | 0.0323 | 0.0003 | 49 | 21 |
| E352 | GSK39 | 0.33 | 38 | 11 | 16 | 5 | 77.6% | 76.2% | 1.7E-05 | 1.5E-07 | 49 | 21 |
| SPARC | ZNF350 | 0.33 | 38 | 9 | 16 | 5 | 80.9% | 76.296 | 0.0169 | 0.0005 | 47 | 21 |
| CNKSR2 | SERP1NG1 | 0.33 | 39 | 10 | 17 | 4 | 79.6% | 81.0% | 1.0M6 | 0.0331 | 49 | 21 |
| G6PD | MASH2 | 0.33 | 43 | 7 | 18 | 5 | 86.0% | 78.3% | 0.0405 | 4.1E-07 | 50 | 23 |
| C1Q8 | IL8 | 0.33 | 41 | 8 | 18 | 3 | 83.7% | 85.7% | 9.7E-05 | 0.0182 | 49 | 21 |
| C1QA | GALS8 | 0.33 | 38 | 8 | 17 | 4 | 82.6% | 81.0% | 2.1E-07 | 0.0072 | 46 | 21 |
| CNKSR2 | ITGAL | 0.33 | 38 | 9 | 17 | 4 | 80.9% | 81.0% | 2.4E-07 | 0.0299 | 47 | 21 |
| FOS | MSH2 | 0.32 | 40 | 9 | 19 | 4 | 81.6% | 82.6% | 0.0436 | 2.1E-06 | 49 | 21 |
| UBE2C | ZNF350 | 0.32 | 38 | 9 | 17 | 4 | 80.9% | 81.0% | 0.0192 | 4.7E-06 | 47 | 23 |
| IL8 | MSH2 | 0.32 | 39 | 11 | 18 | 5 | 78.0% | 78.3% | 0.0448 | 8.0E-05 | | 21 |
| HMOX1 | RBM5 | 0.32 | 39 | 7 | 17 | 4 | 84.8% | 81.0% | 6.0E-07 | 5.3E-05 | 50 | 23 |
| CNKSR2 | ING2 | 0.32 | 39 | 10 | 17 | 4 | 79.6% | 81.0% | 8.5E-05 | 0.0381 | 46 | 21 |
| APC | EGR1 | 0.32 | 41 | 8 | 17 | 4 | 83.7% | 81.0% | 0.0013 | 0.0004 | 49 | 21 |

EP 2 402 464 A1

(continued)

|  | | Entropy | #normal | #normal | #cc | #cc | Normal | colon | | | | |
|  | | | | | | | | | | | | |
|  | | | | | | N = | 50 | 23 | | | | |
| 2-gene models and | | Entropy | #normal | #normal | #cc | #cc | Correct | Correct | | | total used (excludes missing) | |
| 1-gene models | | R-sq | correct | FALSE | correct | FALSE | Classification | Classification | p-val 1 | p-val 2 | # normal | # disease |
| APC | SERPINA1 | 0.32 | 36 | 11 | 16 | 5 | 76.6% | 76.2% | 2.1E-01 | 0.0004 | 49 47 | 21 |
| E2F1 | ZNF350 | 0.32 | 36 | 11 | 16 | 5 | 76.6% | 76.2% | 0.0229 | 0.0001 | 47 | 21 |
| C1QB | PTEN | 0.32 | 38 | 11 | 16 | 5 | 77.6% | 76.2% | 8.6E-07 | 0.0234 | 21 | 21 |
| CNKSR2 | DIABLO | 0.32 | 38 | 11 | 17 | 4 | 77.6% | 81.0% | I:8E-07 | 0.0457 | 49 | 21 |
| ST14 | ZNF350 | 0.32 | 37 | 12 | 16 | 5 | 75.5% | 76.2% | 0.0359 | 2.8E-07 | 49 | 21 |
| IFI16 | TXNRD2 | 0.32 | 39 | 7 | 16 | 5 | 84.8% | 76.2% | 7.3E-06 | 4.9E-06 | 46 | 21 |
| CAV1 | CNKSR2 | 0.32 | 39 | 10 | 16 | 5 | 79.6% | 76.2% | 0.0480 | 7.3E-06 | 49 | 21 |
| CTNNA1 | ZNF350 | 0.32 | 37 | 12 | 16 | 5 | 75.5% | 76.2% | 0.0378 | 2.3E-07 | 49 | 21 |
| CCL5 | PTPRK | 0.32 | 39 | 8 | 17 | 4 | 83.0% | 81.0% | 6.8E-06 | 0.0003 | 47 | 21 |
| SERPING1 | ZNF350 | 0.32 | 39 | 10 | 16 | 5 | 79.6% | 76.2% | 0.0391 | 1.4E-06 | 49 | 21 |
| IL8 | NBEA | 0.32 | 41 | 8 | 16 | 5 | 83.7% | 76.2% | 0.0297 | 0.0001 | 49 | 21 |
| C1QB | MME | 0.32 | 39 | 8 | 16 | 5 | 83.0% | 76.2% | 2.7E-06 | 0.0232 | 47 | 21 |
| CCL5 | MYC | 0.32 | 36 | 11 | 16 | 5 | 76.6% | 76.2% | 8.6E-07 | 0.0004 | 47 | 21 |
| GSK3B | IRF1 | 0.32 | 36 | 11 | 16 | S | 76.6% | 76.2% | 3.3E-06 | 2.1E-05 | 47 | 21 |
| CNKSR2 | USP7 | D.32 | 40 | 7 | 18 | 3 | 85.1% | 85.7% | 2.6E-07 | 0.0380 | 49 | 21 |
| EGR1 | G5K3B | 0.32 | 39 | 10 | 16 | 5 | 79.6% | 76.2% | 2.7E-05 | 0.0019 | 49 | 21 |
| IL8 | TNF350 | 0.32 | 40 | 9 | 17 | 4 | 81.6% | 81.0% | 0.0444 | 0.0002 | 47 | 21 |
| BAX | ZNF350 | 0.32 | 38 | 11 | 16 | 5 | 77.6% | 76.2% | 0.0450 | 2.0E-07 | 49 | 21 |
| C1QB | XRCC1 | 0.32 | 39 | 10 | 16 | 5 | 79.6% | 76.2% | 8.5E-07 | 0.0308 | 49 | 21 |
| NBEA | SERPINE1 | 0.32 | 40 | 9 | 17 | 4 | 81.6% | 81.0% | 1.3E-06 | 0.0340 | 49 | 21 |
| RBMS TNF | | 0.32 | 36 | 11 | 16 | 5 | 76.6% | 76.2% | 0.0006 | 9.1E-07 | 49 | 21 |

(continued)

| 2-gene models and | | Entropy | #normal | #normal | #cc | #cc | Normal | colon | | | total used (excludes missing) | |
|---|---|---|---|---|---|---|---|---|---|---|---|---|
| | | | | | | | N = 50 | 23 | | | | |
| | | | | | | | Correct | Correct | | | | |
| 1-gene models | | R-sq | correct | FALSE | correct | FALSE | Classification | Classification | p-val 1 | p-val 2 | # normal | # disease |
| C1QA | RBMS | 0.32 | 36 | 10 | 16 | 5 | 78.3% | 76.2% | 8.9E-07 | 0.0119 | 47 | 21 |
| MSH2 | ZNF350 | 0.31 | 42 | 7 | 16 | 5 | 85.7% | 76.296 | 0.0485 | 0.0113 | 46 | 21 |
| TGFB1 | TNFSFS | 0.31 | 38 | 9 | 17 | 4 | 80.9% | 81.0% | 0.0007 | 4.2E-06 | 49 | 21 |
| C1QA | SIAH2 | 0.31, | 36 | 10 | 16 | 5 | 78.3% | 76.2% | 0.0001 | 0.0133 | 46 | 21 |
| C1QB | IQGAP1 | 0.31 | 38 | 11 | 16 | 5 | 77.6% | 76.2% | 5.3E-07 | 0.0368 | 47 | # |
| CNKSR2 | SRF | 0.31 | 36 | 11 | 17 | 4 | 76.6% | 81.0% | 4.0E-07 | 0.0487 | 49 47 | 21 |
| HMOX1 | ING2 | 0.31 | 36 | 11 | 16 | 5 | 76.6% | 76.2% | 0.0001 | 0.0001004 | | 21 |
| EGR1 | PTPRK | 0.31 | 40 | 10 | 19 | 4 | 80.0% | 82.6% | 7.3E-D6 | 0.0106 | 47 | 21 |
| C1QA | MME | 0.31 | 38 | 9 | 17 | 4 | 80.9% | 81.0% | 3.7E-06 | 0.0170 | 47 | 23 |
| C1C1A | ESR1 | 0.31 | 40 | 7 | 17 | 4 | 85.1% | 81.0% | 3.0E-06 | 0.0171 | 50 | 21 |
| C1Q8 | CCL5 | 0.31 | 38 | 9 | 17 | 4 | 80.9% | 81.0% | 0.0005 | 0.0257 | 47 | 21 |
| C1QB TNF | | 0.31 | 39 | 10 | 17 | 4 | 79.8% | 81.0% | 0.0011 | 0.0393 | 47 | 21 |
| HOXA10 | NBEA | 0.31 | 38 | 11 | 16 | 5 | 77.6% | 76.2% | 0.0429 | 7.0E-06 | 49 | 21 |
| C1QB | SPARC | 0.31 | 38 | 9 | 17 | 4 | 80.9% | 81.0% | 0.0011 | 0.0329 | 49 47 | 21 |
| ITGAL | TNFSFS | 0.31 | 39 | 7 | 16 | 5 | 84.8% | 76.2% | 0.0009 | 5.2E-07 | 46 | 21 |
| NRAS | TNFSF5 | 0.31 | 39 | 8 | 17 | 4 | 83.0% | 81,0% | 0.0008 | 1.4E-06 | 47 | 21 |
| E2F1 | NBEA | 0.31 | 39 | 8 | 16 | 5 | 83.0% | 76.296 | 0.0365 | 0.0002 | 47 | 21 |
| ADAM17 | MTF1 | 0.31 | 37 | 10 | 17 | 4 | 78.7% | 81.0% | B.SE-07 | 1.7E-05 | 47 | 21 |
| NBEA | TIMP1 | 0.31 | 37 | 12 | 16 | 5 | 75.5% | 76.2% | 4.9E-06 | 0.0467 | 46 | 21 |
| C1QA | CD97 | 0.31 | 38 | 8 | 17 | 4 | 82.6% | 81.0% | 3.9E-07 | 0.0161 | 49 | 21 |
| COB | 5P1 | 0.31 | 37 | 10 | 17 | 4 | 78.7% | 81.0% | 3.9E-07 | 0.0364 | 47 | 21 |

(continued)

| 2-gene models and | 1-gene models | Entropy R-sq | #normal correct | #normal FALSE | #cc correct | #cc FALSE (N =) | Normal 50 Correct Classification | colon 23 Correct Classification | p-val 1 | p-val 2 | total used (excludes missing) # normal | # disease |
|---|---|---|---|---|---|---|---|---|---|---|---|---|
| E2F1 | TNPSP5 | 0.31 | 36 | 11 | 17 | 4 | 76.6% | 81.0% | 0.0009 | 0.0002 | 47 | 21 |
| C1QA | SPARC | 0.31 | 39 | 8 | 17 | 4 | 83.0% | 81.0% | 0.0013 | 0.0194 | 47 | 21 |
| C1Q8 | RBMS | 0.31 | 37 | 10 | 16 | 5 | 78.7% | 76.2% | 1.3E-06 | 0.0309 | 47 | 21 |
| CCL5 | MTA1 | 0.31 | 37 | 10 | 17 | 4 | 78.7% | 81.0% | 4.0E-07 | 0.0006 | 47 | 21 |
| TNFSF5 | USP7 | 0.31 | 37 | 10 | 16 | 5 | 78.7% | 76.2% | 4.2E-07 | 0.0010 | 47 | 21 |
| C1QA | IL8 | 0.31 | 40 | 7 | 17 | 4 | 85.1% | 81.0% | 0.0002 | 0.0222 | 47 | 21 |
| CCL5 | MASH2 | 0.30 | 38 | 9 | 17 | 4 | 80.9% | 81.0% | 0.0139 | 0.0007 | 47 | 21 |
| SPARC | TXNRD1 | 0.30 | 40 | 7 | 17 | 4 | 85.1% | 81.0% | 1.4E-05 | 00015 | 47 | 21 |
| CTSD | GSK3B | 0.30 | 38 | 11 | 16 | 5 | 77.6% | 76.2% | 4.8E-05 | 4.5E-07 | 49 | 21 |
| CA4 | MSH2 | 0.30 | 38 | 9 | 16 | 5 | 80.9% | 76.2% | 0.0157 | 6.5E-06 | 47 | 21 |
| C1QB | PTPRC | 0.30 | 39 | 8 | 16 | 5 | 83.0% | 76.2% | 6.2E-07 | 0.0369 | 47 | 21 |
| MSH2 | 7tK | 0.30 | 38 | 11 | 16 | 5 | 77.6% | 76.2% | 5.4E-07 | 0.0197 | 47 | 21 |
| APC | TEGT | 0.30 | 39 | 10 | 16 | 5 | 79.6% | 76.2% | 3.6E-07 | 0.0010 | 49 | 21 |
| IRF1 | TXNRD1 | 0.30 | 39 | 8 | 17 | 4 | 83.0% | 81.0% | 1.5E-05 | 6.1E-06 | 49 | 21 |
| EGR1 | TXNRD1 | 0.30 | 37 | 10 | 17 | 4 | 78.7% | 81.0% | 1.6E-05 | 0.0036 | 47 | 21 |
| APC | CTSD | 0.30 | 39 | 10 | 17 | 4 | 79.6% | 81.096 | 5.3E-07 | 0.0011 | 49 | 21 |
| IGF2BP2 | SIAH2 | 0.30 | 40 | 7 | 18 | 3 | 85.1% | 85.7% | 0.0002 | 9.7E-07 | 47 | 21 |
| C1QA | MYC | 0.30 | 38 | 9 | 17 | 4 | 80.9% | 81.0% | 1.8E-06 | 0.0289 | 47 | 21 |
| HMOX1 | LTA | 0.30 | 37 | 9 | 17 | 4 | 80.4% | 81.0% | 1.5E-05 | 0.0002 | 46 | 21 |
| C1QA | TNF | 0.30 | 36 | 11 | 16 | 5 | 76.6% | 76.2% | 0.0017 | 0.0322 | 47 | 21 |
| IF116 | MSH2 | 0.30 | 36 | 11 | 17 | 4 | 76.6% | 81.0% | 0.0194 | 1.1E-05 | 47 | 21 |

(continued)

| 2-gene models and | | Entropy | #normal | #normal | #cc | #cc | Normal<br>50 | colon<br>23 | | | total used (excludes missing) | |
| --- | --- | --- | --- | --- | --- | --- | --- | --- | --- | --- | --- | --- |
| | | | | | | N = | Normal | colon | | | | |
| | | | | | | | 50 | 23 | | | | |
| 1-gene models | | R-sq | correct | FALSE | correct | FALSE | Correct<br>Classification | Correct<br>Classification | p-val 1 | p-val 2 | # normal | #<br>disease |
| ING2 | SPARC | 0.29 | 38 | 9 | 16 | 5 | 80.9% | 76.2% | 0.0024 | 0.0003 | 47 | 21 |
| C1QA | PTPRK | 0.29 | 39 | 8 | 17 | 4 | 83.0% | 91.0% | 2.1E-05 | 0.0412 | 47 | 21 |
| APC | ETS2 | 0.29 | 37 | 12 | 16 | 5 | 75.5% | 76.2% | 7.4E-07 | 0.0016 | 49 | 21 |
| GSK3B | SERPINA1 | 0.29 | 37 | 10 | 17 | 4 | 78.7% | 81.0% | 7.5E-07 | 8.6E-05 | 47 | 21 |
| C1QA | CCL5 | 0.29 | 35 | 11 | 17 | 4 | 76.1% | 81.0% | 0.0010 | 0.0364 | 46 | 21 |
| C1QA | GNB1 | 0.29 | 40 | 7 | 16 | 5 | 85.1% | 76.2% | 8.1E-07 | 0.0440 | 47 | 21 |
| NCOA1 | TNF | 0.29 | 38 | 12 | 18 | 5 | 76.0% | 78.3% | 0.0104 | 2.9E-07 | 50 | 23 |
| IL8 | TNF | 0.29 | 39 | 11 | 18 | 5 | 78.0% | 78.3% | 0.0105 | 0.0004 | 50 | 23 |
| G6PD | TXNRD1 | 0.29 | 42 | 5 | 16 | 5 | 89.4% | 76.2% | 2.6E-05 | 3.2E-06 | 47 | 21 |
| C1QA | IQGAP1 | 0.29 | 37 | 10 | 16 | 5 | 78.7% | 76.2% | 1.5E-06 | 0.0459 | 47 | 21 |
| GNB1 | HMOX1 | 0.29 | 38 | 9 | 16 | 5 | 80.9% | 76.2% | 0.0003 | 8.BE-07 | 47 | 21 |
| MSH6 | | 0.29 | 37 | 10 | 17 | 4 | 78.7% | 81.0% | 8.1E-07 | | 47 | 21 |
| MTA1 | TNFSF5 | 0.29 | 36 | 10 | 17 | 4 | 78.3% | 81.0% | 0.0024 | 9.7E-07 | 46 | 21 |
| EGR1 | MYC | 0.29 | 38 | 12 | 18 | 5 | 76.0% | 78.3% | 7.6E-07 | 0.0363 | 50 | 23 |
| GSK3B | NRAS | 0.29 | 38 | 11 | 17 | 4 | 77.6% | 81.0% | 3.4E-06 | 0.0001 | 49 | 21 |
| TIMP1 | TNFSF5 | 0.29 | 42 | 5 | 16 | 5 | 89.4% | 76.2% | 0.0025 | 2.1E-05 | 47 | 21 |
| MSH2 | SPARC | 0.28 | 38 | 9 | 17 | 4 | 80.9% | 81.0% | 0.0041 | 0.0440 | 47 | 21 |
| MSH2 | | 0.28 | 41 | 9 | 19 | 4 | 82.0% | 82.6% | 4.4E-07 | | 50 | 23 |
| IQGAP1 | TIMP1 | 0.28 | 38 | 12 | 18 | 5 | 76.0% | 78.3% | 1.9E-05 | 1.3M6 | 50 | 23 |
| APC | CTNNA1 | 0.28 | 37 | 12 | 16 | 5 | 75.5% | 76.2% | 1.3E-06 | 0.0029 | 49 | 21 |
| ADAM17 | S100A11 | 0.28 | 39 | 8 | 16 | 5 | 83.0% | 76.2% | 7.7E-06 | 6.6E-05 | 47 | 21 |

EP 2 402 464 A1

(continued)

| 2-gene models | 1-gene models | Entropy R-sq | #normal correct | #normal FALSE | #cc correct | #cc FALSE | Normal 50 Correct Classification | colon 23 Correct Classification | p-val 1 | p-val 2 | # normal | # disease |
|---|---|---|---|---|---|---|---|---|---|---|---|---|
| HMOX1 | MYC | 0.28 | 38 | 9 | 18 | 3 | 80.9% | 85.7% | 5.3E-06 | 0.0005 | 47 | 21 |
| LTA | SPARC | 0.28 | 36 | 10 | 16 | 5 | 78.3% | 76.2% | 0.0047 | 4.5E-05 | 46 | 21 |
| CNKSR22 | | 0.27 | 39 | 10 | 17 | 4 | 79.6% | 81.0% | 1.3E-06 | | 49 | 21 |
| ADAM17 | IRF1 | 0.27 | 37 | 9 | 17 | 4 | 80.4% | 81.0% | 2.3E-05 | 7.7E-05 | 46 | 21 |
| LARGE | TNF | 0.27 | 39 | 10 | 16 | 5 | 79.6% | 76.2% | 0.0064 | 3.7E-06 | 49 | 21 |
| SIAH2 | TNF | 0.27 | 36 | 11 | 16 | 5 | 76.6% | 76.2% | 0.0044 | 0.0007 | 47 | 21 |
| CCL5 | ING2 | 0.27 | 37 | 10 | 16 | 5 | 78.7% | 76.2% | 0.0012 | 0.0031 | 47 | 21 |
| EGR1 | MLH1 | 0.27 | 37 | 10 | 16 | 5 | 78.7% | 76.2% | 0.0002 | 0.0133 | 47 | 21 |
| CCL5 | GNB1 | 0.27 | 36 | 11 | 16 | 5 | 76.6% | 76.2% | 2.2E-06 | 0.0034 | 47 | 21 |
| HMOX1 | SIAH2 | 0.27 | 36 | 10 | 16 | 5 | 78.3% | 76.2% | 0.0007 | 0.0006 | | 21 |
| HMOX1 | LGALS8 | 0.27 | 38 | 8 | 16 | 5 | 82.6% | 76.2% | 2.6E-06 | 0.0006 | 46 | 21 |
| E2F1 | ING2 | 0.27 | 36 | 11 | 16 | 5 | 76.6% | 76.2% | 0.0010 | 0.0014 | 46 | 21 |
| SRF | TNFSF5 | 0.26 | 37 | 10 | 16 | 5 | 78.7% | 76.2% | 0.0066 | 3.1E-06 | 47 | 21 |
| EGR1 | SIAH2 | 0.26 | 37 | 10 | 16 | 5 | 78.7% | 76.2% | 0.0010 | 0.0184 | 47 | 21 |
| MLH1 | TGFB1 | 0.26 | 36 | 11 | 16 | 5 | 76.6% | 76.2% | 3.2E-05 | 0.0003 | 47 | 21 |
| DiABLO | TNFSF5 | 0.26 | 36 | 11 | 16 | 5 | 76.6% | 76.2% | 0.0078 | 3.0E-06 | 47 | 21 |
| HMOX1 | MME | 0.26 | 38 | 9 | 17 | 4 | 80.9% | 81.0% | 3.3E-05 | 0.0009 | 47 | 21 |
| ING2 | NRAS | 0.26 | 40 | 9 | 16 | 5 | 81.6% | 76.2% | 1.1E-05 | 0.0015 | 47 | 21 |
| C1Q8 | | 0.26 | 39 | 10 | 17 | 4 | 79.6% | 81.0% | 2.3E-06 | | 49 | 21 |
| CCL5 | SIAH2 | 0.26 | 36 | 11 | 16 | 5 | 76.6% | 76.2% | 0.0012 | 0.0051 | 47 | 21 |
| ING2 | S100A11 | 0.26 | 36 | 11 | 16 | 5 | 76.6% | 76.2% | 1.8E-05 | 0.0019 | 47 | 21 |

| 2-gene models and | | Entropy | #normal | #normal | #cc | #cc | Normal<br>50<br>N =<br>Correct | colon<br>23<br>Correct | | | total used (excludes missing) | |
|---|---|---|---|---|---|---|---|---|---|---|---|---|
| 1-gene models | | R-sq | correct | FALSE | correct | FALSE | Classification | Classification | p-val 1 | p-val 2 | # normal | # disease |
| CCL5 | LARGE | 0.26 | 37 | 10 | 16 | 5 | 78.7% | 76.2% | 9.8E-0B | 0.0060 | 47 | 21 |
| APC | MNDA | 0.26 | 36 | 11 | 16 | 5 | 76.6% | 76.2% | 3.8E-06 | 0.0070 | 47 | 21 |
| GSK3B | TLR2 | 0.26 | 37 | 10 | 17 | 4 | 78.7% | 81.0% | 4.7E-06 | 0.0003 | 47 | 21 |
| IL8 | ING2 | 0.26 | 37 | 12 | 16 | 5 | 75.5% | 76.2% | 0.0019 | 0.0024 | 49 | 21 |
| SPARC | XRCC1 | 0.26 | 37 | 10 | 16 | S | 78.7% | 76.2% | 1.3E-05 | 0.0140 | 47 | 21 |
| DIABLO | HMOX1 | 0.26 | 38 | 9 | 17 | 4 | 80.9% | 81.0% | 0.0012 | 3.7E-06 | 47 | 21 |
| CCLS | DIABLO | 0.26 | 37 | 10 | 16 | 5 | 78.7% | 76.2% | 3.8E-06 | 0.0062 | 47 | 21 |
| MLH1 | SPARC | 0.26 | 36 | 10 | 16 | 5 | 78.3% | 76.2% | 0.0115 | 0.0003 | 46 | 21 |
| ADAM7 | MAPK14 | 0.26 | 36 | 11 | 16 | 5 | 76.6% | 76.2% | 4.3E-06 | 0.0002 | 47 | 21 |
| APC | HSPA1A | 0.25 | 37 | 12 | 16 | 5 | 75.5% | 76.2% | 3.3E-06 | 0.0101 | 49 | 21 |
| PTPRK | SPARC | 0.25 | 37 | 10 | 17 | 4 | 78.7% | 81.0% | 0.0163 | 0.0001 | 47 | 21 |
| EGR1 | GNB1 | 0.25 | 38 | 11 | 16 | 5 | 77.6% | 76.2% | 3.7E-06 | 0.0398 | 47 | 21 |
| IQGAP1 | MYD88 | 0.25 | 39 | 11 | 18 | 5 | 78.0% | 78.3% | 5.5E-06 | 4.9E-06 | 49 | 21 |
| TNF | USP7 | 0.25 | 37 | 10 | 17 | 4 | 78.7% | 81.0% | 4.6E-06 | 0.0155 | 50 | 23 |
| G6PD | TNFSFS | 0.25 | 40 | 7 | 16 | 5 | 85.1% | 76.2% | 0.0133 | 1.9E-05 | 47 | 21 |
| CCL5 | EGR1 | 0.25 | 38 | 9 | 16 | 5 | 80.9% | 76.2% | 0.0362 | 0.0083 | 47 | 21 |
| PLEK2 | SIAH2 | 0.25 | 37 | 10 | 17 | 4 | 78.7% | 81.0% | 0.0020 | 7.0E-06 | 47 | 21 |
| SPARC | TNF | 0.25 | 38 | 9 | 16 | 5 | 80.9% | 76.2% | 0.0167 | 0.0196 | 47 | 21 |
| ADAM17 | TLR2 | 0.25 | 35 | 11 | 16 | 5 | 76.1% | 76.2% | 7.3E-06 | 0.0002 | 46 | 21 |
| DAD1 | TNF | 0.25 | 37 | 12 | 16 | 5 | 75.5% | 76.2% | 0.0211 | 4.3E-06 | 49 | 21 |
| EGR1 | SPARC | 0.25 | 39 | 8 | 16 | 5 | 83.0% | 76.2% | 0.0221 | 0.0462 | 47 | 21 |

EP 2 402 464 A1

(continued)

| 2-gene models and | | Entropy | #normal | #normal | #cc | #cc | Normal | colon | | | total used (excludes missing) | |
| --- | --- | --- | --- | --- | --- | --- | --- | --- | --- | --- | --- | --- |
| | | | | | | | N = | N = | | | | |
| | | | | | | | 50 | 23 | | | | |
| 1-gene models | | R-sq | correct | FALSE | correct | FALSE | Correct Classification | Correct Classification | p-val 1 | p-val 2 | # normal | # disease |
| APC | BAX | 0.25 | 37 | 12 | 16 | 5 | 75.5% | 76.2% | 4.4E-06 | 0.0138 | 49 | 21 |
| EGR1 | HMOX1 | 0.25 | 36 | 11 | 16 | 5 | 76.6% | 76.2% | 0.0018 | 0.0490 | 47 | 21 |
| APC | NCOA1 | 0.25 | 37 | 12 | 16 | 5 | 75.5% | 76.2% | 5.3E-06 | 0.0142 | 49 | 21 |
| ADAM17 | TIMP1 | 0.25 | 36 | 11 | 16 | 5 | 76.6% | 76.2% | 8.6E-05 | 0.0003 | 47 | 21 |
| HMOX1 | SPARC | 0.24 | 40 | 7 | 17 | 4 | 85.1% | 81.0% | 0.0246 | 0.0020 | 47 | 21 |
| CAV1 | TNF | 0.24 | 39 | 10 | 16 | 5 | 79.6% | 76.2% | 0.0251 | 0.0002 | 49 | 21 |
| E2F1 | TNF | 0.24 | 38 | 9 | 16 | 5 | 80.9% | 76.2% | 0.0218 | 0.0043 | 47 | 21 |
| ING2 | TNFRSF1A | 0.24 | 39 | 10 | 16 | 5 | 79.6% | 76.2% | 1.1E-05 | 0.0034 | 49 | 21 |
| APC | SERPINE1 | 0.24 | 38 | 21 | 16 | 5 | 77.6% | 76.2% | 3.4E-05 | 0.0163 | 49 | 21 |
| G1QA | | 0.24 | 37 | 10 | 16 | 5 | 78.7% | 76.2% | 6.1E-06 | | 47 | 21 |
| FOS | PTEN | 0.24 | 38 | 11 | 18 | 5 | 77.6% | 78.3% | 8.0E-05 | 0.0001 | 49 | 23 |
| SPARC | ZNF185 | 0.24 | 38 | 9 | 17 | 4 | 80.9% | 81.0% | 9.1E-06 | 0.0280 | 47 | 21 |
| HMOX1 | PTPRK | 0.24 | 38 | 9 | 16 | 5 | 80.9% | 76.2% | 0.0002 | 0.0023 | 47 | 21 |
| CCL5 | ITGAL | 0.24 | 36 | 11 | 16 | 5 | 76.6% | 76.2% | 9.3E-06 | 0.0124 | 47 | 21 |
| APC | CAV1 | 0.24 | 38 | 11 | 16 | 5 | 77.6% | 76.2% | 0.0002 | 0.0181 | 49 | 21 |
| SIAH2 | TNFSF5 | 0.24 | 36 | 10 | 16 | 5 | 78.3% | 76.2% | 0.0219 | 0.0027 | 46 | 21 |
| MLH1 | MTF1 | 0.24 | 37 | 10 | 16 | 5 | 78.7% | 76.2% | 1.8E-05 | 0.0007 | 47 | 21 |
| EGR1 | | 0.24 | 39 | 11 | 18 | 5 | 78.0% | 78.3% | 3.0E-06 | | 50 | 23 |
| FOS | IL8 | 0.24 | 38 | 11 | 18 | 5 | 77.6% | 78.3% | 0.0071 | 0.0001 | 49 | 23 |
| CD59 | ING2 | 0.24 | 37 | 12 | 16 | 5 | 75.5% | 76.2% | 0.0045 | 2.4E-05 | 49 | 21 |
| ADAM17 | G6PD | 0.24 | 37 | 10 | 16 | 5 | 78.7% | 76.2% | 2.7E-05 | 0.0004 | 47 | 21 |

(continued)

| 2-gene models and | 1-gene models | Entropy R-sq | #normal correct | #normal FALSE | #cc correct | #cc FALSE N = | Normal 50 Correct Classification | colon 23 Correct Classification | p-val 1 | p-val 2 | # normal total used (excludes missing) | # disease |
|---|---|---|---|---|---|---|---|---|---|---|---|---|
| GSK3B | IL8 | 0.24 | 37 | 12 | 16 | 5 | 75.5% | 76.2% | 0.0058 | 0.0010 | 49 | 21 |
| CD97 | HMOX1 | 0.24 | 35 | 11 | 16 | 5 | 76.1% | 76.2% | 0.0026 | 8.9E-06 | 46 | 21 |
| HMOX1 | VIM | 0.23 | 38 | 9 | 17 | 4 | 80.9% | 81.0% | 9.3E-06 | 0.0033 | 47 | 21 |
| ESR1 | HMOX1 | 0.23 | 38 | 9 | 16 | 5 | 80.9% | 76.2% | 0.0035 | 9.5E-05 | 47 | 21 |
| MYD88 | TNFSF5 | 0.23 | 37 | 10 | 16 | 5 | 78.7% | 76.2% | 0.0305 | 2.4E-05 | 47 | 21 |
| TLR2 | TXNRD1 | 0.23 | 36 | 11 | 16 | 5 | 76.6% | 76.2% | 0.0003 | 1.4E-05 | 47 | 21 |
| HOXA10 | LTA | 0.23 | 41 | 6 | 17 | 4 | 87.2% | 81.0% | 0.0004 | 0.0002 | 47 | 21 |
| IL8 | SPARC | 0.23 | 37 | 10 | 16 | 5 | 78.7% | 76.2% | 0.0487 | 0.0055 | 47 | 21 |
| SERPINE1 | TNFSF5 | 0.23 | 37 | 10 | 16 | 5 | 78.7% | 76.2% | 0.0338 | 7.8E-0.5 | 47 | 21 |
| MME | SPARC | 0.23 | 39 | 8 | 16 | 5 | 83.0% | 76.2% | 0.0494 | 0.0001 | 47 | 21 |
| HMOX1 | LARGE | 0.23 | 37 | 10 | 16 | 5 | 78.7% | 76.2% | 3.1E-05 | 0.0039 | 47 | |
| CCL5 | IL8 | 0.23 | 37 | 10 | 17 | 4 | 78.7% | 81.0% | 0.0066 | 0.0227 | 47 | 21 |
| APC | ITGAL | 0.23 | 36 | 11 | 16 | 5 | 76.6% | 76.2% | 1.6E-05 | 0.0273 | 47 | 21 |
| IKBKE | TGFB1 | 0.23 | 37 | 10 | 16 | 5 | 78.7% | 76.2% | 0.0002 | 0.0002 | 47 | 21 |
| HOXA10 | SIAH2 | 0.23 | 36 | 11 | 17 | 4 | 76.6% | 81.0% | 0.0055 | 0.0003 | 47 | 21 |
| CAV1 | ING2 | 0.23 | 38 | 11 | 16 | 5 | 77.6% | 76.2% | 0.0077 | 0.0005 | 49 | 21 |
| IRF1 | MME | 0.23 | 39 | 8 | 16 | 5 | 83.0% | 76.2% | 0.0002 | 0.0002 | 47 | 21 |
| MLH1 | PLXDC2 | 0.23 | 37 | 10 | 16 | 5 | 78.7% | 76.2% | 3.1E-05 | 0.0014 | | 21 |
| HMOX1 | NCOA1 | 0.23 | 36 | 11 | 16 | 5 | 76.6% | 76.2% | 1.5E-05 | 0.0049 | 47 | 21 |
| CTSD | TNFSF5 | 0.22 | 38 | 9 | 16 | 5 | 80.9% | 76.2% | 0.0467 | 2.0E-05 | 47 | 21 |
| ING2 | MAPK14 | 0.22 | 36 | 11 | 17 | 4 | 76.6% | 81.0% | 1.8E-05 | 0.0107 | 47 | 21 |

| 2-gene models and | | Entropy | #normal | #normal | #cc | #cc | Normal | colon | | | total used (excludes missing) | |
| | | | | | | | N = | | | | | |
| | | | | | | | 50 | 23 | | | | |
| | | | | | | | Correct | Correct | | | | |
| 1-gene models | | R-sq | correct | FALSE | correct | FALSE | Classification | Classification | p-val 1 | p-val 2 | # normal | # disease |
| APC | PTGS2 | 0.22 | 39 | 10 | 16 | 5 | 79.6% | 76.2% | 1.3E-05 | 0.0468 | 47 49 | 21 |
| LTA | TGFB1 | 0.22 | 36 | 11 | 16 | 5 | 76.6% | 76.2% | 0.0002 | 0.0006 | 47 | 21 |
| CCL5 | ESR1 | 0.22 | 36 | 11 | 16 | 5 | 76.6% | 76.2% | 0.0003 | 0.0364 | 47 | 21 |
| ADAM17 | RP51077B9.4 | 0.21 | 36 | 11 | 16 | 5 | 76.6% | 76.2% | 0.0006 | 0.0013 | 47 | 21 |
| CCL5 | HMOX1 | 0.21 | 37 | 9 | 16 | 5 | 80.4% | 76.2% | 0.0092 | 0.0484 | 46 | 21 |
| CA4 | PTEN | 0.21 | 37 | 10 | 16 | 5 | 78.7% | 76.2% | 0.0001 | 0.0005 | 47 | 21 |
| HOXA10 | IKBKE | 0.20 | 36 | 11 | 17 | 4 | 76.6% | 81.0% | 0.0004 | 0.0008 | 47 | 21 |
| G6PD | ING2 | 0.20 | 39 | 10 | 16 | 5 | 79.6% | 76.2% | 0.0251 | 0.0001 | 49 | 21 |
| ADAM17 | UBE2C | 0.20 | 36 | 10 | 17 | 4 | 78.3% | 81.0% | 0.0010 | 0.0019 | 49 | 21 |
| ING2 | SERPINE1 | 0.20 | 40 | 9 | 16 | 5 | 81.6% | 76.2% | 0.0002 | 0.0277 | 46 | 21 |
| BCAM | SIAH2 | 0.20 | 35 | 11 | 16 | 5 | 76.1% | 76.2% | 0.0175 | 6.2E-05 | 49 | 21 |
| IFI16 | XRCC1 | 0.20 | 37 | 10 | 16 | 5 | 78.7% | 76.2% | 0.0002 | 0.0008 | 47 | 21 |
| HMOX1 | PTPRC | 0.20 | 38 | 8 | 16 | 5 | 82.6% | 76.2% | 6.4E-05 | 0.0163 | 46 | 21 |
| S100A4 | SIAH2 | 0.20 | 36 | 11 | 16 | 5 | 76.6% | 76.2% | 0.0238 | 5.0E-05 | 46 | 21 |
| CTNNA1 | ING2 | 0.19 | 38 | 11 | 16 | 5 | 77.6% | 76.2% | 0.0365 | 6.0E-05 | 47 | 21 |
| GSK3B | HSPA1A | 0.19 | 37 | 12 | 16 | 5 | 75.5% | 76.2% | 4.7E-05 | 0.0078 | 49 | 21 |
| PTEN | S100A11 | 0.19 | 39 | 8 | 17 | 4 | 83.0% | 81.0% | 0.0004 | 0.0003 | 49 | 21 |
| IRF1 | SP1 | 0.19 | 40 | 7 | 17 | 4 | 85.1% | 81.0% | 8.7E-05 | 0.0011 | 47 | 21 |
| HMOX1 | S100A4 | 0.19 | 38 | 9 | 17 | 4 | 80.9% | 81.0% | 8.3E-05 | 0.0327 | 47 | 21 |
| HMOX1 | PTEN | 0.19 | 37 | 10 | 17 | 4 | 78.7% | 81.0% | 0.0004 | 0.0333 | 47 | 21 |
| GSK3B | ST14 | 0.18 | 39 | 10 | 17 | 4 | 79.6% | 81.0% | 0.0001 | 0.0123 | 47 | 21 |

| 2-gene models and | | Entropy | #normal | #normal | #cc | #cc | Normal | colon | | | total used (excludes missing) | |
|---|---|---|---|---|---|---|---|---|---|---|---|---|
| | | | | | | | N = | 50 | 23 | | | |
| | | | | | | | Correct | Correct | | | | |
| 1-gene models | | R-sq | correct | FALSE | correct | FALSE | Classification | Classification | p-val 1 | p-val 2 | # normal | # disease |
| HMOX1 | USP7 | 0.18 | 36 | 11 | 16 | 5 | 76.6% | 76.2% | 0.0001 | 0.0451 | 47 | 21 |
| CD97 | IRF1 | 0.16 | 36 | 10 | 16 | 5 | 78.3% | 76.2% | 0.0032 | 0.0002 | 46 | 21 |
| CASP9 | MLH1 | 0.16 | 36 | 11 | 16 | 5 | 76.6% | 76.2% | 0.0294 | 0.0002 | 47 | 21 |
| CCL3 | MLH1 | 0.15 | 35 | 11 | 16 | 5 | 76.1% | 76.2% | 0.0344 | 0.0007 | 46 | 21 |
| IQGAP1 | IRF1 | 0.15 | 37 | 10 | 16 | 5 | 78.7% | 76.2% | 0.0067 | 0.0008 | 47 | 21 |
| IRF1 | LGALS8 | 0.14 | 35 | 11 | 16 | 5 | 76.1% | 76.2% | 0.0006 | 0.0077 | 46 | 21 |
| GNB1 | IFI16 | 0.14 | 36 | 11 | 16 | 5 | 76.6% | 76.2% | 0.0126 | 0.0007 | 47 | 21 |
| LGALS8 | TGFB1 | 0.13 | 36 | 11 | 16 | 5 | 76.6% | 76.2% | 0.0138 | 0.0012 | 47 | 21 |
| ESR1 | HOXA10 | 0.13 | 38 | 11 | 16 | 5 | 77.6% | 76.2% | 0.0324 | 0.0098 | 49 | 21 |
| HOXA10 | NUDT4 | 0.12 | 40 | 7 | 16 | 5 | 85.1% | 76.2% | 0.0071 | 0.0345 | 47 | 21 |

|  | Colon | Normals | Sum |
|---|---|---|---|
| Group Size | 31.5% | 68.5% | 100% |
| N = | 23 | 50 | 73 |
|  |  |  |  |
| Gene | Mean | Mean | p-val |
| AXIN2 | 20.3 | 19.2 | 2.4E-09 |
| CCR7 | 15.8 | 14.8 | 5.9E-09 |
| MSH2 | 18.7 | 17.9 | 4.4E-07 |
| MSH6 | 20.0 | 19.3 | 8.1E-07 |
| CNKSR2 | 22.1 | 21.2 | 1.3E-06 |
| ZNF350 | 19.9 | 19.3 | 1.6E-06 |
| NBEA | 22.7 | 21.6 | 2.1E-06 |
| C1QB | 19.7 | 21.2 | 2.3E-06 |
| EGR1 | 18.9 | 19.8 | 3.0E-06 |
| C1QA | 19.3 | 20.7 | 6.1E-06 |
| TNF | 18.1 | 18.7 | 8.0E-06 |
| SPARC | 14.0 | 14.8 | 8.2E-05 |
| APC | 18.4 | 17.8 | 0.0001 |
| TNFSF5 | 18.3 | 17.7 | 0.0001 |
| CCL5 | 11.7 | 12.3 | 0.0002 |
| IL8 | 22.3 | 21.4 | 0.0002 |
| E2F1 | 19.5 | 20.2 | 0.0004 |
| ING2 | 19.9 | 19.6 | 0.0005 |
| SIAH2 | 13.1 | 14.0 | 0.0007 |
| HMOX1 | 15.7 | 16.3 | 0.0009 |
| GSK3B | 16.2 | 15.8 | 0.0021 |
| MLH1 | 18.1 | 17.8 | 0.0030 |
| PTPRK | 22.4 | 21.7 | 0.0042 |
| TGFB1 | 12.4 | 12.7 | 0.0050 |
| ADAM17 | 18.6 | 18.2 | 0.0060 |
| CAV1 | 22.9 | 23.7 | 0.0072 |
| TIMP1 | 14.4 | 14.7 | 0.0074 |
| PTEN | 14.2 | 13.8 | 0.0088 |
| FOS | 15.1 | 15.6 | 0.0091 |
| TXNRD1 | 17.2 | 16.9 | 0.0093 |
| LTA | 19.6 | 19.3 | 0.0095 |
| HOXA10 | 22.4 | 23.1 | 0.0115 |
| UBE2C | 20.4 | 20.8 | 0.0118 |
| RP5107789.4 | 16.3 | 16.6 | 0.0130 |

(continued)

|  | Colon | Normals | Sum |
|---|---|---|---|
| Group Size | 31.5% | 68.5% | 100% |
| N = | 23 | 50 | 73 |
|  |  |  |  |
| Gene | Mean | Mean | p-val |
| SERPING1 | 17.5 | 18.3 | 0.0144 |
| IFI16 | 14.3 | 14.6 | 0.0178 |
| CA4 | 18.5 | 19.1 | 0.0225 |
| IRF1 | 12.5 | 12.8 | 0.0252 |
| IKBKE | 17.0 | 16.7 | 0.0280 |
| MME | 15.5 | 15.1 | 0.0295 |
| NRAS | 16.8 | 17.0 | 0.0309 |
| SERPINE1 | 20.5 | 20.9 | 0.0339 |
| GADD45A | 19.0 | 19.3 | 0.0353 |
| ESR1 | 22.3 | 21.9 | 0.0383 |
| ESR2 | 24.5 | 23.9 | 0.0417 |
| G6PD | 15.4 | 15.7 | 0.0437 |
| S100A11 | 11.0 | 11.3 | 0.0628 |
| CDH1 | 20.1 | 20.4 | 0.0691 |
| NUDT4 | 15.7 | 16.1 | 0.0732 |
| TNFRSF1A | 15.1 | 15.4 | 0.0809 |
| ST14 | 17.6 | 17.9 | 0.0857 |
| MMP9 | 14.1 | 14.6 | 0.0877 |
| XRCC1 | 18.6 | 18.4 | 0.0960 |
| HMGA1 | 15.6 | 15.8 | 0.1154 |
| NEDD4L | 18.3 | 18.5 | 0.1201 |
| CD59 | 17.5 | 17.7 | 0.1205 |
| RBMS | 16.1 | 15.9 | 0.1214 |
| MYD88 | 14.3 | 14.5 | 0.1359 |
| IQGAP1 | 14.0 | 13.8 | 0.1550 |
| LARGE | 22.3 | 22.0 | 0.1674 |
| MTF1 | 17.6 | 17.9 | 0.1794 |
| MYC | 18.3 | 18.1 | 0.1898 |
| PLXDC2 | 16.6 | 16.7 | 0.1958 |
| CCL3 | 20.0 | 20.2 | 0.2456 |
| CEACAM1 | 18.3 | 18.5 | 0.2484 |
| IGF2BP2 | 15.7 | 15.9 | 0.2504 |
| IGFBP3 | 22.1 | 22.4 | 0.3151 |
| DLC1 | 23.3 | 23.5 | 0.3424 |

(continued)

|  | Colon | Normals | Sum |
|---|---|---|---|
| Group Size | 31.5% | 68.5% | 100% |
| N = | 23 | 50 | 73 |
|  |  |  |  |
| Gene | Mean | Mean | p-val |
| XK | 17.6 | 17.9 | 0.3635 |
| PLEK2 | 18.2 | 18.5 | 0.3701 |
| ANLN | 22.2 | 22.4 | 0.3744 |
| PTPRC | 12.4 | 12.3 | 0.4140 |
| ZNF185 | 16.9 | 17.0 | 0.4201 |
| ITGAL | 14.6 | 14.7 | 0.4241 |
| TLR2 | 16.0 | 16.1 | 0.4248 |
| BCAM | 20.4 | 20.7 | 0.4396 |
| CTSD | 13.0 | 13.2 | 0.4600 |
| S100A4 | 13.0 | 13.2 | 0.4606 |
| CASP3 | 20.5 | 20.3 | 0.4626 |
| SRF | 16.3 | 16.4 | 0.4695 |
| BAX | 15.6 | 15.7 | 0.4717 |
| ETS2 | 17.3 | 17.4 | 0.4889 |
| CXCL1 | 19.8 | 19.7 | 0.5361 |
| ACPP | 18.0 | 17.9 | 0.5367 |
| MAP14 | 15.2 | 15.3 | 0.5479 |
| LGALS8 | 17.5 | 17.4 | 0.5731 |
| MEIS1 | 21.7 | 21.8 | 0.5828 |
| MNDA | 12.7 | 12.8 | 0.6082 |
| PLAU | 23.9 | 24.0 | 0.6255 |
| SP1 | 15.8 | 15.7 | 0.6356 |
| GNB1 | 13.5 | 13.4 | 0.6407 |
| NCOA1 | 16.2 | 16.2 | 0.6518 |
| CTNNA1 | 16.9 | 17.0 | 0.6903 |
| DIABLO | 18.5 | 18.5 | 0.6940 |
| HSPA1A | 14.5 | 14.5 | 0.7229 |
| USP7 | 15.2 | 15.2 | 0.7383 |
| DAD1 | 15.3 | 15.3 | 0.7470 |
| POV1 | 18.2 | 18.2 | 0.7579 |
| PTGS2 | 17.2 | 17.2 | 0.7953 |
| CASP9 | 18.1 | 18.0 | 0.8087 |
| SERPINA1 | 12.7 | 12.7 | 0.8238 |
| TEGT | 12.4 | 12.4 | 0.8779 |

(continued)

|  | Colon | Normals | Sum |
|---|---|---|---|
| Group Size | 31.5% | 68.5% | 100% |
| N = | 23 | 50 | 73 |
|  |  |  |  |
| Gene | Mean | Mean | p-val |
| VEGF | 22.7 | 22.8 | 0.9203 |
| MTA1 | 19.4 | 19.5 | 0.9261 |
| ELA2 | 20.9 | 20.8 | 0.9542 |
| VIM | 11.4 | 11.4 | 0.9681 |
| CD97 | 12.9 | 12.9 | 0.9862 |

| Patient ID | Group | AXIN2 | TNF | logit | odds | Predicted probability of colon cancer |
|---|---|---|---|---|---|---|
| CC-010:XS:200072430 | Colon Cancer | 22.23 | 18.09 | 12.34 | 2.3E+05 | 1.0000 |
| CC-007:XS:200072427 | Colon Cancer | 21.66 | 18.20 | 9.29 | 10865.66 | 0.9999 |
| CC-004:XS:200072424 | Colon Cancer | 21.76 | 18.57 | 8.42 | 4538.86 | 0.9998 |
| CC-008:XS:200072428 | Colon Cancer | 20.98 | 17.94 | 7.18 | 1307.55 | 0.9992 |
| CC-002:XS:200072422 | Colon Cancer | 21.33 | 18.56 | 6.49 | 660.48 | 0.9985 |
| CC-011:XS:200072431 | Colon Cancer | 20.36 | 17.45 | 6.11 | 449.07 | 0.9978 |
| CC-003:XS:200072423 | Colon Cancer | 20.31 | 17.65 | 5.14 | 170.20 | 0.9942 |
| CC-034:XS:200072442 | Colon Cancer | 20.18 | 17.64 | 4.59 | 98.65 | 0.9900 |
| CC-031:XS:200072439 | Colon Cancer | 19.70 | 17.08 | 4.42 | 83.04 | 0.9881 |
| CC-014:XS:200072434 | Colon Cancer | 20.46 | 18.41 | 3.00 | 20.17 | 0.9528 |
| CC-006:XS:200072426 | Colon Cancer | 20.09 | 18.13 | 2.38 | 10.83 | 0.9155 |
| HN-041-XS:200073106 | Normal | 19.78 | 17.89 | 1.85 | 6.35 | 0.8639 |
| CC-018:XS:200072436 | Colon Cancer | 19.84 | 18.03 | 1.62 | 5.04 | 0.8344 |
| CC-019:XS:200072437 | Colon Cancer | 20.02 | 18.26 | 1.56 | 4.77 | 0.8268 |
| CC-013:XS:200072433 | Colon Cancer | 20.68 | 19.18 | 1.23 | 3.43 | 0.7742 |
| HN-001-XS:200072922 | Normal | 19.95 | 18.32 | 1.04 | 2.83 | 0.7388 |
| CC-032:XS:200072440 | Colon Cancer | 19.61 | 18.03 | 0.52 | 1.68 | 0.6264 |
| CC-005:XS:200072425 | Colon Cancer | 20.11 | 18.67 | 0.50 | 1.65 | 0.6231 |
| CC-033:XS:200072441 | Colon Cancer | 19.28 | 17.69 | 0.28 | 1.32 | 0.5686 |
| CC-009-XS:20CO72429 | Colon Cancer | 19.20 | 17.62 | 0.15 | 1.16 | 0.5370 |
| HN-050-XS:200073113 | Normal | 19.36 | 17.87 | 0.00 | 1.00 | 0.5010 |
| CC-012:XS:200072432 | Colon Cancer | 20.04 | 18.81 | -0.32 | 0.72 | 0.4197 |
| HN-004-XS:200072925 | Normal | 19.54 | 18.23 | -0.52 | 0.60 | 0.3738 |
| HN-029-XS:200073095 | Normal | 20.31 | 19.33 | -1.02 | 0.36 | 0.2647 |

(continued)

| Patient ID | Group | AXIN2 | TNF | logit | odds | Predicted probability of colon cancer |
|---|---|---|---|---|---|---|
| HN-026-XS:200073092 | Normal | 20.17 | 19.24 | -1.35 | 0.26 | 0.2063 |
| HN-012-XS:200072931 | Normal | 19.57 | 18.52 | -1.48 | 0.23 | 0.1855 |
| HN-010-XS:200072930 | Normal | 19.13 | 18.06 | -1.78 | 0.17 | 0.1446 |
| HN-015-XS:200072934 | Normal | 19.34 | 18.39 | -2.04 | 0.13 | 0.1153 |
| HN-007-XS:200072927 | Normal | 19.50 | 18.60 | -2.04 | 0.13 | 0.1149 |
| NN-049-XS:200073112 | Normal | 19.67 | 18.82 | -2.08 | 0.12 | 0.1111 |
| HN-035-XS:200073100 | Normal | 19.41 | 18.52 | -2.15 | 0.12 | 0.1046 |
| HN-040-XS:200073105 | Normal | 19.04 | 18.06 | -2.18 | 0.11 | 0.1014 |
| CC-015:XS:200072435 | Colon Cancer | 19.55 | 18.71 | -2.23 | 0.11 | 0.0968 |
| HN-106-XS:200073119 | Normal | 19.12 | 18.20 | -2.35 | 0.10 | 0.0873 |
| HN-034-XS:200073099 | Normal | 19.26 | 18.40 | -2.44 | 0.09 | 0.0801 |
| HN-008-XS:200072928 | Normal | 19.26 | 18.42 | -2.49 | 0.08 | 0.0766 |
| HN-002-XS:200072923 | Normal | 19.52 | 18.76 | -2.52 | 0.08 | 0.0746 |
| HN-038-XS:200073103 | Normal | 19.23 | 18.40 | -2.57 | 0.08 | 0.0708 |
| HN-025-XS:200073091 | Normal | 19.40 | 18.67 | -2.79 | 0.06 | 0.0578 |
| HN-102-XS:200073115 | Normal | 18.93 | 18.10 | -2.84 | 0.06 | 0.0554 |
| CC-001:XS:200072421 | Colon Cancer | 19.05 | 18.26 | -2.87 | 0.06 | 0.0536 |
| HN-044-XS:200073109 | Normal | 19.16 | 18.41 | -2.93 | 0.05 | 0.0507 |
| HN-042-XS:200073107 | Normal | 19.06 | 18.29 | -2.93 | 0.05 | 0.0506 |
| HN-039-XS:200073104 | Normal | 18.66 | 17.81 | -3.02 | 0.05 | 0.0466 |
| HN-022-XS:200072948 | Normal | 19.95 | 19.45 | -3.09 | 0.05 | 0.0434 |
| HN-020-XS:200072946 | Normal | 19.24 | 18.57 | -3.15 | 0.04 | 0.0410 |
| HN-104-XS:200073117 | Normal | 19.29 | 18.73 | -3.48 | 0.03 | 0.0300 |
| HN-019-XS:200072945 | Normal | 19.05 | 18.45 | -3.57 | 0.03 | 0.0274 |
| HN-027-XS:200073093 | Normal | 19.19 | 18.65 | -3.67 | 0.03 | 0.0249 |
| HN-045-XS:200073110 | Normal | 19.18 | 18.67 | -3.76 | 0.02 | 0.0227 |
| HN-014-XS:200072933 | Normal | 18.90 | 18.32 | -3.77 | 0.02 | 0.0224 |
| HN-016-XS:200072935 | Normal | 18.98 | 18.42 | -3.80 | 0.02 | 0.0219 |
| HN-030-XS:200073096 | Normal | 19.67 | 19.32 | -3.92 | 0.02 | 0.0194 |
| HN-017-XS:200072936 | Normal | 19.11 | 18.68 | -4.15 | 0.02 | 0.0156 |
| HN-032-XS:200073097 | Normal | 19.30 | 18.99 | -4.41 | 0.01 | 0.0120 |
| HN-105-XS:200073118 | Normal | 19.23 | 18.95 | -4.59 | 0.01 | 0.0101 |
| HN-047-XS:200073111 | Normal | 18.79 | 18.44 | -4.73 | 0.01 | 0.0087 |
| HN-033-XS:200073098 | Normal | 19.77 | 19.74 | -5.01 | 0.01 | 0.0066 |
| HN-036-XS:200073101 | Normal | 18.95 | 18.76 | -5.19 | 0.01 | 0.0055 |
| HN-018-XS:200072944 | Normal | 18.94 | 18.78 | -5.29 | 0.01 | 0.0050 |

(continued)

| Patient ID | Group | AXIN2 | TNF | logit | odds | Predicted probability of colon cancer |
|---|---|---|---|---|---|---|
| HN-005-XS:200072926 | Normal | 18.83 | 18.80 | -5.87 | 0.00 | 0.0028 |
| HN-037-XS:200073102 | Normal | 18.62 | 18.56 | -5.94 | 0.00 | 0.0026 |
| HN-101-XS:200073114 | Normal | 18.74 | 18.75 | -6.07 | 0.00 | 0.0023 |
| HN-009-XS:200072929 | Normal | 19.09 | 19.30 | -6.50 | 0.00 | 0.0015 |
| HN-003-XS:200072924 | Normal | 18.25 | 18.27 | -6.57 | 0.00 | 0.0014 |
| HN-103-XS:200073116 | Normal | 18.53 | 18.71 | -6.90 | 0.00 | 0.0010 |
| HN-024-XS:200073090 | Normal | 19.26 | 19.73 | -7.33 | 0.00 | 0.0007 |
| HN-028-XS:200073094 | Normal | 19.47 | 20.03 | -7.43 | 0.00 | 0.0006 |
| HN-107-XS:200073120 | Normal | 18.44 | 18.95 | -8.18 | 0.00 | 0.0003 |
| HN-021-XS:200072947 | Normal | 18.26 | 19.27 | -10.20 | 0.00 | 0.0000 |

Statements of the Invention

1. A method for evaluating the presence of colon cancer in a subject based on a sample from the subject, the sample providing a source of RNAs, comprising: a) determining a quantitative measure of the amount of at least one constituent of any constituent of any one table selected from the group consisting of Tables 1, 2, 3, 4, and 5 as a distinct RNA constituent in the subject sample, wherein such measure is obtained under measurement conditions that are substantially repeatable and the constituent is selected so that measurement of the constituent distinguishes between a normal subject and a colon cancer-diagnosed subject in a reference population with at least 75% accuracy; and b) comparing the quantitative measure of the constituent in the subject sample to a reference value.

2. A method for assessing or monitoring the response to therapy in a subject having colon cancer based on a sample from the subject, the sample providing a source of RNAs, comprising: a) determining a quantitative measure of the amount of at least one constituent of any constituent of Tables 1, 2, 3, 4, and 5 as a distinct RNA constituent, wherein such measure is obtained under measurement conditions that are substantially repeatable to produce subject data set; and b) comparing the subject data set to a baseline data set.

3. A method for monitoring the progression of colon cancer in a subject, based on a sample from the subject, the sample providing a source of RNAs, comprising: a) determining a quantitative measure of the amount of at least one constituent of any constituent of Tables 1, 2, 3, 4, and 5 as a distinct RNA constituent in a sample obtained at a first period of time, wherein such measure is obtained under measurement conditions that are substantially repeatable to produce a first subject data set; b) determining a quantitative measure of the amount of at least one constituent of any constituent of Tables 1, 2, 3, 4, and 5 as a distinct RNA constituent in a sample obtained at a second period of time, wherein such measure is obtained under measurement conditions that are substantially repeatable to produce a second subject data set; and c) comparing the first subject data set and the second subject data set.

4. A method for determining a colon cancer profile based on a sample from a subject known to have colon cancer, the sample providing a source of RNAs, the method comprising: a) using amplification for measuring the amount of RNA in a panel of constituents including at least 1 constituent from Tables 1, 2, 3, 4, and 5 and b) arriving at a measure of each constituent, wherein the profile data set comprises the measure of each constituent of the panel and wherein amplification is performed under measurement conditions that are substantially repeatable.

5. The method of any one of statements 1-4, wherein said constituent is selected from the group consisting of AXIN2, CIQA, CDKN2A, CCR7, CNKSR2, CIQB, EGR1, MSH2, MSH[beta]and RHOC. 6. The method of any one of statements 1-4, comprising measuring at least two constituents from a) Table 1, wherein the first constituent is selected from the group consisting of ACSL5, ALDHIA1, APC, AXIN2, BAX, CA4, CCND3, CD44, CD63, CFLAR, GADD45A, IGFBP4, ITGA3, MGMT, MSH2, and MSH6 and the second constituent is any other constituent selected from Table 1, wherein the constituent is selected so that measurement of the constituent distinguishes between a normal subject and a colon cancer-diagnosed subject in a reference population with at least 75% accuracy; b) Table 2, wherein the first constituent is selected from the group consisting of ADAM17, ALOX5, APAF1, CIQA, CASP1, CASP3, CCL3, CCL5, CCR5, CD19, CD4, CD8A, CTLA4, CXCL1, CXCR3, DPP4, EGR1, GZMB, HLADRA, HMOX1, HSPAIA, ICAM1, IFI16, IFNG, ILIO, IL18, IL18BP, ILIB, ILIR1, ILIRN, IL23A, IL32, IL8, IRF1, LTA, MAPK14, MHC2TA, MIF, MMP9, MNDA, MYC, NFKB1, PLA2G7, PLAUR, PTGS2, PTPRC, SERPINA1, SSI3, TGFB1, TIMP1, TLR2, YNF, and TNFRSFIA, and the second constituent is any other constituent selected from Table 2, wherein the constituent is selected so that measurement of the constituent distinguishes between a normal subject and a colon cancer-diagnosed subject in a reference population with at least 75% accuracy; c) Table 3 wherein the first constituent is selected from the group consisting of ABL1, ABL2, AKT1, APAF1, ATM, BAD, BAX, BCL2, BRAF, BRCA1, CASP8, CDK2, CDK4, CDK5, CDKNIA, CDKN2A, CFLAR, GOL18A1, E2F1, EGR1, ERBB2, FOS, GZMA, HRAS, IFITM1, ILIB, IL8, ITGA1, ITGA3, ITGAE, ITGB1, MMP9, MSH2, MYC, MYCL1, NFKB1, NME4, N0TCH2, NRAS, PCNA, PLAUR, PTCH1, RB1, RHOA, RHOC, S100A4, SEMA4D, SERPINE1, SK1, SKE., SMAD4, TGFB1, and TNF, and the second constituent is any other constituent selected from Table 3, wherein the constituent is selected so that measurement of the constituent distinguishes between a normal subject and a colon cancer-diagnosed subject in a reference population with at least 75% accuracy; d) Table 4 wherein the first constituent is selected from the group consisting of CEBPB, CREBBP, EGR1, EGR2, FOS, ICAM1, MAP2K1, NAB1, NFKB1, NR4A2, SRC, TGFB1, and TOPBP1 and the second constituent is from the group consisting of NAB1, NR4A2, PDGFA, PTEN, TGFB1, TNFRSF6, and TOPBP1, wherein the constituent is selected so that measurement of the constituent distinguishes between a normal subject and a colon cancer-diagnosed subject in a reference population with at least 75% accuracy; and e) Table 5 wherein the first constituent is selected from the group consisting of ADAM 17, APC, AXIN2, BAX, BCAM, CIQA, CIQB, CA4, CLASP9, CAV1, CCL3, CCL5, CCR7, CD59, CD97, CNKSR2, CTNNA1, CTSD, DAD1, DIABLO, E2F1, EGR1, ESR1, ETS2, FOS, G6PD, GNB1, GSK3B, HMGA1, HMOX1, HOXA1O, M16, IGF2BP2, KBKE, IL8, ING2, IQGAP1, IRF1, ITGAL, LARGE, LGALS8, LTA, MAPK14, MLH1, MME, MMP9, MNDA, MSH2, MSH6, MTA1, MTF1, MYD88, NBEA, NCOA1, NRAS, PLEK2, PLXDC2, PTEN, PTPRK, RBM5, S100A4, SERPINE1, SERPING1, SIAH2, SPARC, SRF, ST14, TGFB1, TIMP1, TLR2, TNF, TNFRSFIA, TNFSF5, and UBE2C and the second constituent is any other constituents selected from Table 5, wherein the constituent is selected so that measurement of the constituent distinguishes between a normal subject and a colon cancer-diagnosed subject in a reference population with at least 75% accuracy.

7. The method of any one of statements 1-6, wherein the combination of constituents are selected according to any of the models enumerated in Tables IA, 2 A, 3 A, 4 A, or 5 A.

8. The method of any one of statements 1, 5 and 6, wherein said reference value is an index value.

9. The method of statement 2, wherein said therapy is immunotherapy.

10. The method of statement 9, wherein said constituent is selected from Table 6.

11. The method of any one of statements 2, 9 or 10, wherein when the baseline data set is derived from a normal subject a similarity in the subject data set and the baseline date set indicates that said therapy is efficacious.

12. The method of any one of statements 2, 9 or 10, wherein when the baseline data set is derived from a subject known to have colon cancer a similarity in the subject data set and the baseline date set indicates that said therapy is not efficacious.

13. The method of any one of statements 1-12, wherein expression of said constituent in said subject is increased compared to expression of said constituent in a normal, reference sample.

14. The method of any one of statements 1-12, wherein expression of said constituent in said subject is decreased compared to expression of said constituent in a normal reference sample.

15. The method of any one of statements 1-12, wherein the sample is selected from the group consisting of blood, a blood fraction, a body fluid, a cells and a tissue.

16. The method of any one of statements 1-15, wherein the measurement conditions that are substantially repeatable are within a degree of repeatability of better than ten percent.

17. The method of any one of statements 1-16, wherein the measurement conditions that are substantially repeatable are within a degree of repeatability of better than five percent.

18. The method of any one of statements 1-17, wherein the measurement conditions that are substantially repeatable are within a degree of repeatability of better than three percent.

19. The method of any one of statements 1-18, wherein efficiencies of amplification for all constituents are substantially similar.

20. The method of any one of statements 1-19, wherein the efficiency of amplification for all constituents is within ten percent.

21. The method of any one of statements 1-20, wherein the efficiency of amplification for all constituents is within five percent.

22. The method of any one of statements 1-19, wherein the efficiency of amplification for all constituents is within three percent.

23. A kit for detecting colon cancer in a subject, comprising at least one reagent for the detection or quantification of any constituent measured according to any one of statements 1-22 and instructions for using the kit.

**Claims**

1. A method for determining a livelihood of colon cancer in a subject, comprising:

    a) determining a subject data set by determining in a subject blood sample a quantitative measure of the amount of RNA of a set of 2 genes; and
    b) determining the likelihood of colorectal cancer using an equation of a 2-gene logistic regression model, wherein the 2 genes are selected from the group consisting of:

    PSEN2 and MSH6;
    TXNRD1 and HMOX1;
    CDKN2A and ATM;
    TNF and AKIN2; and
    constituents of Tables 1, 2, 3, 4 and 5.

2. A method of evaluating the presence or absence of colon cancer, based on a sample from a subject the sample providing a source of RNAs, comprising:

    a) determining a quantitative measure of 2 colon cancer associated genes
    b) distinguishing between subjects suffering from colorectal cancer and normal subjects with at least 75% accuracy using a 2-gene logistic regression model, wherein the 2 genes are selected from the group consisting of:-

    i) PTEN2 and MSH6;
    ii) TXNRD1 and HMOX1;
    iii) CDKN2A and ATM; and
    iv) constituents of Tables 1, 2, 3, 4 and 5.

3. The method of claim 1 or 2, wherein the equation is MSH6=2.861677+0.840724*PSEN2, where MSH6 and PSEN2 are values of the data set.

4. The method of claim 1 or 2, wherein the equation is HMOX1= -2.9520 + 1.1294*TXNRD1, where HMOX1 and TXNRD1 are values of the data set.

5. The method of claim 1 or 2, wherein the equation is
ATM=1.992988+0.71347*CDKN2A. where ATM and CDKN2A are values of the data set.

6. The method of claim 1 or wherein the equation is AXIN2= 4.9912+0.79925*TNF, where AXIN2 and TNF are values of the data set.

Fig. 1

MSH6 = 2.861677 + 0.840724 * PSEN2

Fig. 2

Fig. 3-1

Z-STATISTIC

Fig. 3-2

**SOURCE MDx DISEASE PROBABILITY INDEX**
COLON CANCER 2 - GENE MODEL (MSH6, PSEN) ENTROPY R2 = 0.5938

Fig. 4

HMOX1 = -2.9520 + 1.1294 * TXNRD1

# Fig. 5

ATM = 1.992988 + 0.71347 * CDKN2A

Fig. 6

$$AXIN2 = 4.9912 + 0.79925 * TNF$$

Fig. 7

Europäisches
Patentamt

European
Patent Office

Office européen
des brevets

## EUROPEAN SEARCH REPORT

Application Number

EP 11 18 2820

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 00/62070 A2 (HOLTEN ANDERSEN MADS [DK]; STEPHENS ROSS W [AU]; NIELSEN HANS JOERGEN) 19 October 2000 (2000-10-19) * the whole document * ----- | 1-6 | INV. C12Q1/68 |
| Y | WO 2006/066917 A2 (ROCHE DIAGNOSTICS GMBH [DE]; HOFFMANN LA ROCHE [CH]; ANDRES HERBERT [D) 29 June 2006 (2006-06-29) * the whole document * ----- | 1-6 | |
| Y | WO 2006/066915 A1 (ROCHE DIAGNOSTICS GMBH [DE]; HOFFMANN LA ROCHE [CH]; KARL JOHANN [DE];) 29 June 2006 (2006-06-29) * the whole document * ----- | 1-6 | |
| Y | EP 1 512 758 A (VERIDEX LLC [US]) 9 March 2005 (2005-03-09) * the whole document * ----- | 1-6 | |
| Y | PETERLONGO PAOLO ET AL: "MSH6 germline mutations are rare in colorectal cancer families.", INTERNATIONAL JOURNAL OF CANCER, vol. 107, no. 4, 20 November 2003 (2003-11-20), pages 571-579, XP002501667, ISSN: 0020-7136 * the whole document * ----- -/-- | 1-6 | TECHNICAL FIELDS SEARCHED (IPC) C12Q |

The present search report has been drawn up for all claims

| Place of search | Date of completion of the search | Examiner |
|---|---|---|
| The Hague | 10 November 2011 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 402 464 A1

Europäisches Patentamt
European Patent Office
Office européen des brevets

# EUROPEAN SEARCH REPORT

Application Number

EP 11 18 2820

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PLASCHKE JENS ET AL: "Eight novel MSH6 germline mutations in patients with familial and nonfamilial colorectal cancer selected by loss of protein expression in tumor tissue.", HUMAN MUTATION MAR 2004, vol. 23, no. 3, March 2004 (2004-03), page 285, XP002501668, ISSN: 1098-1004 * the whole document * | 1-6 | |
| Y | JOSEPH ANSAMMA ET AL: "Paired colonic epithelial cell lines derived from tumor and normal sites of the same individual's intestine as tools to study the pathophysiology of colon cancer", GASTROENTEROLOGY, vol. 120, no. 5 Supplement 1, April 2001 (2001-04), pages A.300-A.301, XP008145134, & 102ND ANNUAL MEETING OF THE AMERICAN GASTROENTEROLOGICAL ASSOCIATION AND DIGESTIVE DISEASE WEEK; ATLANTA, GEORGIA, USA; MAY 20-23, 2001 ISSN: 0016-5085 * the whole document * | 1-6 | |
| Y | BAIER PETER K ET AL: "Cytokine expression in colon carcinoma", ANTICANCER RESEARCH, vol. 25, no. 3B, May 2005 (2005-05), pages 2135-2139, XP002663245, ISSN: 0250-7005 * the whole document * | 1-6 | TECHNICAL FIELDS SEARCHED (IPC) |

-/--

The present search report has been drawn up for all claims

| Place of search | Date of completion of the search | Examiner |
|---|---|---|
| The Hague | 10 November 2011 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

136

Europäisches
Patentamt

European
Patent Office

Office européen
des brevets

# EUROPEAN SEARCH REPORT

**Application Number**

EP 11 18 2820

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HUGHES THOMAS A ET AL: "Regulation of axin2 expression at the levels of transcription, translation and protein stability in lung and colon cancer", CANCER LETTERS, vol. 233, no. 2, February 2006 (2006-02), pages 338-347, XP002663246, ISSN: 0304-3835 * the whole document * | 1-6 | |
| Y | YAN DONG ET AL: "Elevated expression of axin2 and hnkd mRNA provides evidence that Wnt/beta-catenin signaling is activated in human colon tumors", PROCEEDINGS OF THE NATIONAL ACADEMY OF SCIENCES OF THE UNITED STATES OF AMERICA, vol. 98, no. 26, 18 December 2001 (2001-12-18), pages 14973-14978, XP002663247, ISSN: 0027-8424 * the whole document * | 1-6 | |
| Y | WO 03/025203 A2 (QUARK BIOTECH INC [US]; GILAD SHLOMIT [IL]; YAHALOM JOACHIM [US]) 27 March 2003 (2003-03-27) * claim 1 * | 1-6 | TECHNICAL FIELDS SEARCHED (IPC) |
| Y | FANG JING YUAN ET AL: "Epigenetic modification regulates both expression of tumor-associated genes and cell cycle progressing in human colon cancer cell lines: Colo-320 and SW1116", CELL RESEARCH, vol. 14, no. 3, June 2004 (2004-06), pages 217-226, XP002663248, ISSN: 1001-0602 * the whole document * | 1-6 | |

-/--

The present search report has been drawn up for all claims

| Place of search | Date of completion of the search | Examiner |
|---|---|---|
| The Hague | 10 November 2011 | Botz, Jürgen |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**Europäisches Patentamt**
**European Patent Office**
**Office européen des brevets**

## EUROPEAN SEARCH REPORT

Application Number

EP 11 18 2820

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/068659 A2 (POMORSKA AKADEMIA MEDYCZNA [PL]; LUBINSKI JAN [PL]; CYBULSKI CEZARY [P) 28 July 2005 (2005-07-28)<br>* claim 32 *<br>----- | 1-6 | |
| | | | **TECHNICAL FIELDS SEARCHED (IPC)** |

The present search report has been drawn up for all claims

| Place of search | Date of completion of the search | Examiner |
|---|---|---|
| The Hague | 10 November 2011 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 2 402 464 A1

**ANNEX TO THE EUROPEAN SEARCH REPORT
ON EUROPEAN PATENT APPLICATION NO.**    EP 11 18 2820

This annex lists the patent family members relating to the patent documents cited in  the above-mentioned European search report.
The members are as contained in the European Patent Office EDP file on
The European Patent Office is in no way liable for these particulars which are merely given for the purpose of information.

10-11-2011

| Patent document cited in search report | | Publication date | Patent family member(s) | | | Publication date |
|---|---|---|---|---|---|---|
| WO 0062070 | A2 | 19-10-2000 | AT | 298890 | T | 15-07-2005 |
| | | | AU | 771309 | B2 | 18-03-2004 |
| | | | CA | 2366682 | A1 | 19-10-2000 |
| | | | CA | 2652131 | A1 | 19-10-2000 |
| | | | DE | 60021068 | D1 | 04-08-2005 |
| | | | DE | 60021068 | T2 | 18-05-2006 |
| | | | EP | 1171771 | A2 | 16-01-2002 |
| | | | EP | 1619206 | A2 | 25-01-2006 |
| | | | ES | 2244417 | T3 | 16-12-2005 |
| | | | JP | 4638608 | B2 | 23-02-2011 |
| | | | JP | 2002541481 | A | 03-12-2002 |
| | | | JP | 2010266455 | A | 25-11-2010 |
| | | | NO | 20014869 | A | 04-12-2001 |
| | | | NZ | 515311 | A | 26-11-2002 |
| | | | US | 2008261246 | A1 | 23-10-2008 |
| | | | US | 2009035794 | A1 | 05-02-2009 |
| | | | US | 7108983 | B1 | 19-09-2006 |
| WO 2006066917 | A2 | 29-06-2006 | AT | 426168 | T | 15-04-2009 |
| | | | CA | 2586654 | A1 | 29-06-2006 |
| | | | EP | 1831696 | A2 | 12-09-2007 |
| | | | ES | 2323429 | T3 | 15-07-2009 |
| | | | JP | 4673895 | B2 | 20-04-2011 |
| | | | JP | 2008523366 | A | 03-07-2008 |
| | | | US | 2009155820 | A1 | 18-06-2009 |
| WO 2006066915 | A1 | 29-06-2006 | AT | 430936 | T | 15-05-2009 |
| | | | CA | 2585788 | A1 | 29-06-2006 |
| | | | CN | 101088011 | A | 12-12-2007 |
| | | | CN | 101088012 | A | 12-12-2007 |
| | | | EP | 1831695 | A1 | 12-09-2007 |
| | | | ES | 2325277 | T3 | 31-08-2009 |
| | | | JP | 4606469 | B2 | 05-01-2011 |
| | | | JP | 2008522172 | A | 26-06-2008 |
| | | | US | 2008020414 | A1 | 24-01-2008 |
| EP 1512758 | A | 09-03-2005 | AU | 2004205270 | A1 | 17-03-2005 |
| | | | BR | PI0403812 | A | 07-06-2005 |
| | | | CA | 2475769 | A1 | 28-02-2005 |
| | | | CN | 1637151 | A | 13-07-2005 |
| | | | JP | 2006101701 | A | 20-04-2006 |
| | | | KR | 20050021916 | A | 07-03-2005 |
| WO 03025203 | A2 | 27-03-2003 | AU | 2002327013 | A1 | 01-04-2003 |
| | | | US | 2003162195 | A1 | 28-08-2003 |

EPO FORM P0459

For more details about this annex : see Official Journal of the European Patent Office, No. 12/82

139

## ANNEX TO THE EUROPEAN SEARCH REPORT
## ON EUROPEAN PATENT APPLICATION NO.

EP 11 18 2820

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2011

| Patent document cited in search report | Publication date | Patent family member(s) | Publication date |
|---|---|---|---|
| WO 2005068659 A2 | 28-07-2005 | EP 1743035 A2 | 17-01-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 85896506 P
- WO 0125473 A
- WO 9824935 A
- US 5744305 A

### Non-patent literature cited in the description

- **O'Marcaigh AS ; Jacobson RM.** Estimating the Predictive Value of a Diagnostic Test, How to Prevent Misleading or Confusing Results. *Clin. Ped.,* 1993, vol. 32 (8), 485-491
- **Pepe et al.** Limitations of the Odds Ratio in Gauging the Performance of a Diagnostic, Prognostic, or Screening Marker. *Am. J. Epidemiol,* 2004, vol. 159 (9), 882-890
- Clinical Interpretation of Laboratory Procedures. **Shultz ; Teitz.** Fundamentals of Clinical Chemistry. W.B. Saunders Company, 1996, 192-199
- **Zweig et al.** ROC Curve Analysis: An Example Showing the Relationships Among Serum Lipid and Apolipoprotein Concentrations in Identifying Subjects with Coronory Artery Disease. *Clin. Chem.,* 1992, vol. 38 (8), 1425-1428
- **Cook.** Use and Misuse of the Receiver Operating Characteristic Curve in Risk Prediction. *Circulation,* 2007, vol. 115, 928-935
- **Balkwill ; Mantovani.** *Lancet,* 2001, vol. 357, 539-545
- **Coussens L.M. ; Z. Werb.** *Nature,* 2002, vol. 429, 860-867
- **Kusmartsev ; Gabrilovic.** *Cancer Immunol. Immunother.,* 2002, vol. 51, 293-298
- **Serafini et al.** *Cancer Immunol. Immunther.,* 2004, vol. 53, 64-72
- **Bunt et al.** *J. Immunol.,* 1996, vol. 176, 284-290
- **Hirayama et al.** *Blood,* 1998, vol. 92, 46-52
- RNA Isolation Strategies. RNA Methodologies, A laboratory guide for isolation and characterization. Academic Press, 1998, 55-104
- RNA Methodologies. A Laboratory Guide for Isolation and Characterization. Academic Press, 1998, 143-151
- RNA Isolation and Characterization Protocols. Methods in Molecular Biology. Human Press, 1998, vol. 86
- METHODS IN MOLECULAR BIOLOGY. 55-72
- PCR Applications: protocols for functional genomics. Academic Press, 1999
- Nucleic acid detection methods. Molecular Methods for Virus Detection. Academic Press, 1995, 1-24
- **Y.S. Lie ; C.J. Petropolus.** Advances in Quantitative PCR Technology: 5' Nuclease Assays. *Current Opinion in Biotechnology,* 1998, vol. 9, 43-48
- Rapid Thermal Cycling and PCR Kinetics. PCR applications: protocols for functional genomics. Academic Press, 1999, 211-229
- **Magidson, Jay.** Qualitative Variance, Entropy and Correlation Ratios for Nominal Dependent Variables. *Social Science Research,* vol. 10, 177-194
- **Vermunt ; Magidson.** LG-Syntax™ User's Guide: Manual for Latent GOLD® 4.5 Syntax Module, Belmont MA: Statistical Innovations. 2007
- Latent Class Cluster Analysis. **Vermunt J.K. ; J. Magidson.** Applied Latent Class Analysis. Cambridge University Press, 2002, 89-106
- **Magidson, J.** maximum Likelihood Assessment of Clinical Trials Based on an Ordered Categorical Response. *Drug Information Journal, Maple Glen, PA: Drug Information Association,* 1996, vol. 30 (1), 143-170